# EUROPEAN PATENT SPECIFICATION

(11) **EP 0 831 799 B1**
(45) Date of publication and mention of the grant of the patent: **02.05.2003**
(21) Application number: 96918477.9
(22) Date of filing: 07.06.1996
(51) Int. Cl.: A61K 31/135, A61K 31/155, A61K 31/165, A61K 31/40, A61K 31/38, A61K 31/44, A61K 31/445

(54) **COMPOUNDS ACTIVE AT A NOVEL SITE ON RECEPTOR-OPERATED CALCIUM CHANNELS USEFUL FOR TREATMENT OF NEUROLOGICAL DISORDERS**
VERBINDUNGEN, DIE AN EINER NEUEN STELLE AUF REZEPTOR-STIMULIERTEN KALZIUMKANÄLEN WIRKSAM SIND, VERWENDBAR ZUR BEHANDLUNG VON NEUROLOGISCHEN KRANKHEITEN
COMPOSES ACTIFS AU NIVEAU D'UN NOUVEAU SITE DE CANAUX CALCIQUES ACTIVES PAR RECEPTEUR, UTILES DANS LE TRAITEMENT DE TROUBLES NEUROLOGIQUES

(30) Priority: 07.06.1995 US 485038
(43) Date of publication of application: 01.04.1998
(73) Proprietor: NPS PHARMACEUTICALS, INC., Salt Lake City Utah 84108 (US)
(72) Inventor: MUELLER, Alan, L., Salt Lake City, UT 84105 (US); MOE, Scott, T., Salt Lake City, UT 84105 (US); BALANDRIN, Manuel, F., Sandy, UT 84093 (US); DELMAR, Eric, G., Salt Lake City, UT 84108 (US); VANWAGENEN, Bradford, C., Salt Lake City, UT 84124 (US); ARTMAN, Linda, D., Salt Lake City, UT 84105 (US); BARMORE, Robert, M., Salt Lake City, UT 84102 (US); SMITH, Daryl, L., Murray, UT 84123 (US)
(74) Representative: VOSSIUS & PARTNER
(86) International application number: PCT/US96/10201
(87) International publication number: WO 96/040097

(56) References cited:
- EP-A- 0 399 504
- WO-A-93/04373
- WO-A-95/21612
- WO-A-96/05818
- DD-A- 33 285
- US-A- 4 018 895
- US-A- 5 281 624
- EUR. J. MED. CHEM., vol. 19, no. 3, 1984, pages 235-242, XP000605241 P. MELLONI ET AL.: "POTENTIAL ANTIDEPRESSANT AGENTS"
- EUROPEAN JOURNAL OF PHARMACOLOGY, vol. 48, 1978, pages 231-235, XP002016624 C.J. HELKE ET AL.: "ANTIEXTENSOR EFFECTS OF 3,3-DIPHENYL-n-PROPYLAMINE IN THE MOUSE"
- LIFE SCIENCES, vol. 54, no. 16, 1994, pages 271-275, XP000604402 C.L. CRAMER ET AL.: "KAINIC ACID AND 4-AMINOPYRIDINE SEIZURE MODELS IN MICE: EVALUATION OF EFFICACY OF ANTIEPILEPTIC AGNETS AND CALCIUM ANTAGONISTS"
- EPILEPSIA, vol. 34, no. 2, 1993, pages 372-380, XP002016625 G.C. PALMER ET AL.: "ANTICONVULSANT PROPERTIES OF CALCIUM CHANNEL BLOCKERS IN MICE"
- STN FILE SUPPLIER: MEDLINE; AN=90065508, XP002016626 "CALCIUM ANTAGONISTS (FINOPTIN AND SENSIT) IN THE TREATMENT OF CEREBROVASCULAR DISORDERS" & KLINICHESKAIA MEDITSINA, vol. 67, no. 9, 1989, pages 51-54, BURTSEV ET AL.:
- "THE MERCK INDEX" 1989 , MERCK & CO. , RAHWAY, N.J., U.S.A. XP002016631 see page 218 see page 337 see page 623 see page 655 see page 1148 see page 1227 see page 1444 see page 1597
- THE JOURNAL OF PHARMACOLOGY AND EXPERIMENTAL THERAPEUTICS, vol. 269, no. 1, 1994, pages 95-102, XP002016627 I.A. PAUL ET AL.: "ADAPTATION OF THE N-METHYL-D-ASPARTATE RECEPTOR COMPLEX FOLLOWING CHRONIC ANTIDEPRESSANT TREATMENTS"
- ACTA PHYSIOLOGICA ACADEMIAE SCIENTIARUM HUNGARICAE , vol. 29, 1966, pages 283-297, XP002016628 G. LESZKOVSZKY ET AL.: "THE PHARMACOLOGY OF DIPHENYLALKYL DERIVATIVES"
- EUR. J. MED. CHEM., vol. 27, no. 4, 1992, pages 321-330, XP002016629 A BUSCHAUER ET AL.: "SYNTHESIS AND HISTAMINE H2 AGONISTIC ACTIVITY OF ARPROMIDINE ANALOGUES"
- CHEMICAL ABSTRACTS, vol. 83, no. 7, 18 August 1975 Columbus, Ohio, US; abstract no. 58634, H. MIKIO ET AL.: "SYNTHESIS OF ANALGESICS" XP002016632 & YAKUGAKU ZASSHI, vol. 95, no. 2, 1975, pages 131-137,
- EUROPEAN JOURNAL OF PHARMACOLOGY, vol. 215, no. 2-3, 1992, pages 191-198, XP002016630 J.O. MARCUSSON ET AL.: cited in the application

## Description

### Field of the Invention

This invention relates to compounds useful as neuroprotectants, anticonvulsants, anxiolytics, analgesics, muscle relaxants or adjuvants to general anesthetics. The invention relates as well to the use of such compounds for the preparation of a pharmaceutical composition for the treatment of neurological disorders and diseases, including, but not limited to, global and focal ischemic and hemorrhagic stroke, head trauma, spinal cord injury, hypoxia-induced nerve cell damage such as in cardiac arrest or neonatal distress, epilepsy, anxiety, and neurodegenerative diseases such as Alzheimer's Disease, Huntington's Disease, Parkinson's Disease, and amyotrophic lateral sclerosis (ALS).

### Background of the Invention

The following is a description of relevant art.

Glutamate is the major excitatory neurotransmitter in the mammalian brain. Glutamate binds or interacts with one or more glutamate receptors which can be differentiated pharmacologically into several subtypes. In the mammalian central nervous system (CNS) there are three main subtypes of ionotropic glutamate receptors, defined pharmacologically by the selective agonists *N*-methyl-D-aspartate (NMDA), kainate (KA), and α-amino-3-hydroxy-5-methylisoxazole-4-propionic acid (AMPA). The NMDA receptor has been implicated in a variety of neurological pathologies including stroke, head trauma, spinal cord injury, epilepsy, anxiety, and neurodegenerative diseases such as Alzheimer's Disease (Watkins and Collingridge, *The NMDA Receptor,* Oxford: IRL Press, 1989). A role for NMDA receptors in nociception and analgesia has been postulated as well (Dickenson, A cure for wind-up: NMDA receptor antagonists as potential analgesics. *Trends Pharmacol. Sci.* 11: 307, 1990). More recently, AMPA receptors have been widely studied for their possible contributions to such neurological pathologies (Fisher and Bogousslavsky, Evolving toward effective therapy for acute ischemic stroke. *J. Amer. Med. Assoc.* 270: 360, 1993; Yamaguchi *et al.,* Anticonvulsant activity of AMPA/kainate antagonists: Comparison of GYKI 52466 and NBQX in maximal electroshock and chemoconvulsant seizure models. *Epilepsy Res.* 15: 179, 1993).

When activated by glutamate, the endogenous neurotransmitter, the NMDA receptor permits the influx of extracellular calcium (Ca²⁺) and sodium (Na⁺) through an associated ion channel. The NMDA receptor allows considerably more influx of Ca²⁺ than do kainate or AMPA receptors (but see below), and is an example of a receptor-operated Ca²⁻ channel. Normally, the channel is opened only briefly, allowing a localized and transient increase in the, concentration of intracellular Ca²⁺ ([Ca²⁺]), which, in turn, alters the functional activity of the cell. However, prolonged increases in [Ca²⁺]₁, resulting from chronic stimulation of the NMDA receptor, are toxic to the cell and lead to cell death The chronic elevation in [Ca²⁺]₁, resulting from stimulation of NMDA receptors, is said to be a primary cause of neuronal degeneration following a stroke (Choi, Glutamate neurotoxicity and diseases of the nervous system. *Neuron* 1: 623, 1988). Overstimulation of NMDA receptors is also said to be involved in the pathogenesis of some forms of epilepsy (Dingledine *et al*., Excitatory amino acid receptors in epilepsy. *Trends Pharmacol. Sci.* 11: 334, 1990), anxiety (Wiley and Balster, Preclinical evaluation of *N*-methyl-D-aspartate antagonists for antianxiety effects: A review. In: *Multiple Sigma and PCP Receptor Ligands: Mechanisms for Neuromodulation and Neuroprotection?* NPP Books, Ann Arbor, Michigan, pp. 801-815, 1992), neurodegenerative diseases (Meldrum and Garthwaite, Excitatory amino acid neurotoxicity and neurodegenerative disease. *Trends Pharmacol. Sci.* 11: 379, 1990), and hyperalgesic states (Dickenson, A cure for wind-up: NMDA receptor antagonists as potential analgesics. *Trends Pharmacol. Sci.* 11: 307, 1990).

The activity of the NMDA receptor-ionophore complex is regulated by a variety of modulatory sites that can be targeted by selective antagonists. Competitive antagonists, such as the phosphonate AP5, act at the glutamate binding site, whereas noncompetitive antagonists, such as phencyclidine (PCP), MK-801 or magnesium (Mg ²⁺), act within the associated ion channel (ionophore). There is also a glycine binding site that can be blocked selectively with compounds such as 7-chlorokynurenic acid. There is evidence suggesting that glycine acts as a co-agonist, so that both glutamate and glycine are necessary to fully elicit NMDA receptor-mediated responses. Other potential sites for modulation of NMDA receptor function include a zinc (Zn ²⁺) binding site and a sigma ligand binding site. Additionally, endogenous polyamines such as spermine are believed to bind to a specific site and so *potentiate* NMDA receptor function (Ransom and Stec, Cooperative modulation of [³H]MK-801 binding to the NMDA receptor-ion channel complex by glutamate, glycine and polyamines. *J. Neurochem.* 51: 830, 1988). The potentiating effect of polyamines on NMDA receptor function may be mediated via a specific receptor site for polyamines; polyamines demonstrating agonist, antagonist, and inverse agonist activity have been described (Reynolds, Arcaine is a competitive antagonist of the polyamine site on the NMDA receptor. *Europ. J. Pharmacol.* 177: 215, 1990; Williams *et al.*, Characterization of polyamines having agonist, antagonist, and inverse agonist effects at the polyamine recognition site of the NMDA receptor. *Neuron* 5: 199, 1990). Radioligand binding studies have demonstrated additionally that higher concentrations of polyamines *inhibit* NMDA receptor function (Reynolds and Miller, Ifenprodil is a novel type of NMDA receptor antagonist: Interaction with polyamines. *Molec. Pharmacol.* 36: 758, 1989; Williams *et al*., Effects of polyamines on the binding of [³H]MK-801 to the NMDA receptor: Pharmacological evidence for the existence of a polyamine recognition site. *Molec. Pharmacol*. 36: 575, 1989; Sacaan and Johnson, Characterization of the stimulatory and inhibitory effects of polyamines on [³H]TCP binding to the NMDA receptor-ionophore complex. *Molec. Pharmacol*. 37: 572, 1990). This inhibitory effect of polyamines on NMDA receptors is probably a nonspecific effect (*i.e.*, not mediated via the polyamine receptor) because patch clamp electro-physiological studies have demonstrated that this inhibition is produced by compounds previously shown to act at the polyamine receptor as either agonists or antagonists (Donevan *et al.*, Arcaine Blocks *N*-Methyl-D-Aspartate Receptor Responses by an Open Channel Mechanism: Whole-Cell and Single-Channel Recording Studies in Cultured Hippocampal Neurons. *Molec. Pharmacol.* 41: 727, 1992; Rock and Macdonald, Spermine and Related Polyamines Produce a Voltage-Dependent Reduction of NMDA Receptor Single-Channel Conductance. *Molec. Pharmacol.* 42: 157, 1992).

Recent studies have demonstrated the molecular diversity of glutamate receptors (reviewed by Nakanishi, Molecular Diversity of Glutamate Receptors and Implications for Brain Function. *Science* 258: 597, 1992). At least five distinct NMDA receptor subunits (NMDAR1 and NMDAR2A through NMDAR2D), each encoded by a distinct gene, have been identified to date. Also, in NMDAR1, alternative splicing gives rise to at least six additional isoforms. It appears that NMDAR1 is a necessary subunit, and that combination of NMDAR1 with different members of NMDAR2 forms the fully functional NMDA receptor-ionophore complex. The NMDA receptor-ionophore complex, thus, can be defined as a hetero-oligomeric structure composed of at least NMDAR1 and NMDAR2 subunits; the existence of additional, as yet undiscovered, subunits is not excluded by this definition. NMDAR1 has been shown to possess binding sites for glutamate, glycine, Mg ²⁺, MK-801, and Zn ²⁺. The binding sites for sigma ligands and polyamines have not yet been localized on NMDA receptor subunits, although ifenprodil recently has been reported to be more potent at the NMDAR2B subunit than at the NMDAR2A subunit (Williams, Ifenprodil discriminates subtypes of the *N*-Methyl-D-aspartate receptor: selectivity and mechanisms at recombinant heteromeric receptors. Mol. Pharmacol. 44: B51, 1993).

Several distinct subtypes of AMPA and kainate receptors have been cloned as well (reviewed by Nakanishi, Molecular diversity of glutamate receptors and implications for brain function. *Science* 258: 597, 1992). Of particular relevance are the AMPA receptors designated GluR1, GluR2, GluR3, and GluR4 (also termed GluRA through GluRD), each of which exists in one of two forms, termed flip and flop, which arise by RNA alternative splicing. GluR1, GluR3 and GluR4, when expressed as homomeric or heteromeric receptors, are permeable to Ca ²⁺, and are therefore examples of receptor-operated Ca ²⁺ channels. Expression of GluR2 alone or in combination with the other subunits gives rise to a receptor which is largely impermeable to Ca ²⁺. As most native AMPA receptors studied *in situ* are not Ca ²⁺-permeable (discussed above), it is believed that such receptors *in situ* possess at least one GluR2 subunit.

Furthermore, it is hypothesized that the GluR2 subunit is functionally distinct by virtue.of the fact that it contains an arginine residue within the putative pore-forming transmembrane region II; GluR1, GluR3 and GluR4 all contain a glutamine residue in this critical region (termed the Q/R site, where Q and R are the single letter designations for glutamine and arginine, respectively). The activity of the AMPA receptor is regulated by a number of modulatory sites that can be targeted by selective antagonists (Honore *et al.*, Quinoxalinediones: potent competitive non-NMDA glutamate receptor antagonists. *Science* 241: 701, 1988; Donevan and Rogawski, GYKI 52466, a 2,3-benzodiazepine, is a highly selective, noncompetitive antagonist of AMPA/kainate receptor responses. *Neuron* 10: 51, 1993). Competitive antagonists such as NBQX act at the glutamate binding site, whereas compounds such as GYKI 52466 appear to act noncompetitively at an associated allosteric site.

Compounds that act as competitive or noncompetitive antagonists at the NMDA receptor are said to be effective in preventing neuronal cell death in various *in vitro* neurotoxicity assays (Meldrum and Garthwaite, Excitatory amino acid neurotoxicity and neurodegenerative disease. *Trends Pharmacol. Sci.* 11: 379, 1990) and in *in vivo* models of stroke (Scatton, Therapeutic potential of NMDA receptor antagonists in ischemic cerebrovascular disease in *Drug Strategies* in the *Prevention and Treatment of Stroke,* IBC Technical Services Ltd., 1990). Such compounds are also effective anticonvulsants (Meldrum, Excitatory amino acid neurotransmission in epilepsy and anticonvulsant therapy in *Excitatory Amino Acids.* Meldrum, Moroni, Simon, and Woods (Eds.), New York: Raven Press, p. 655, 1991), anxiolytics (Wiley and Balster, Preclinical evaluation of *N*-methyl-D-aspartate antagonists for antianxiety effects: A review. In: *Multiple Sigma and PCP Receptor Ligands: Mechanisms for Neuromodulation and Neuroprotection?* NPP Books, Ann Arbor, Michigan, pp. 801-815, 1992), and analgesics (Dickenson, A cure for wind-up: NMDA receptor antagonists as potential analgesics. *Trends Pharmacol. Sci.* 11: 307, 1990), and certain NMDA receptor antagonists may lessen dementia associated with Alzheimer's Disease (Hughes, Merz' novel approach to the treatment of dementia. *Script* No. 1666: 24, 1991).

Similarly, AMPA receptor antagonists have come under intense scrutiny as potential therapeutic agents for the treatment of such neurological disorders and diseases. AMPA receptor antagonists have been shown to possess neuroprotectant (Fisher and Bogousslavsky, Evolving coward effective therapy for acute ischemic stroke. *J. Amer. Med.* *Assoc*. 270: 360, 1993) and anticonvulsant (Yamaguchi *et al*., Anticonvulsant activity of AMPA/kainate antagonists: comparison of GYKI 52466 and NBQX in maximal electroshock and chemoconvulsant seizure models. *Epilepsy Res.* 15: 179, 1993) activity in animal models of ischemic stroke and epilepsy, respectively.

The nicotinic cholinergic receptor present in the mammalian CNS is another example of a receptor-operated Ca ²⁺ channel (Deneris *et al*., Pharmacological and functional diversity of neuronal nicotinic acetylcholine receptors. *Trends Pharmacol*. Sci. 12: 34, 1991). Several distinct receptor subunits have been cloned, and these subunits can be expressed, in *Xenopus* oocytes for example, to form functional receptors with their associated cation channels. It is hypothesized that such receptor-ionophore complexes are heteropentameric structures. The possible role of nicotinic receptor-operated Ca²⁺ channels in the pathology of neurological disorders and diseases such as ischemic stroke, epilepsy and neurodegenerative diseases has been largely unexplored.

It has been demonstrated previously that certain spider and wasp venoms contain arylalkylamine toxins (also called polyamine toxins, arylamine toxins, acylpolyamine toxins or polyamine amide toxins) with activity against glutamate receptors in the mammalian CNS (for reviews see Jackson and Usherwood, Spider toxins as tools for dissecting elements of excitatory amino acid transmission. *Trends Neurosci*. 11: 278, 1988; Jackson and Parks, Spider Toxins: Recent Applications In Neurobiology. *Annu. Rev. Neurosci*. 12: 405, 1989; Saccomano et al., Polyamine spider toxins: Unique pharmacological tools. *Annu. Rep. Med. Chem.* 24: 287, 1989; Usherwood and Blagbrough, Spider Toxins Affecting Glutamate Receptors: Polyamines in Therapeutic Neurochemistry. *Pharmacol. Therap.* 52: 245, 1991; Kawai, Neuroactive Toxins of Spider Venoms. *J. Toxicol. Toxin Rev.* 10: 131, 1991). Arylalkylamine toxins were initially reported to be selective antagonists of the AMPA/kainate subtypes of glutamate receptors in 'the mammalian CNS (Kawai *et al.,* Effect of a spider toxin on glutaminergic synapses in the mammalian brain. *Biomed. Res.* 3: 353, 1982; Saito *et al.,* Spider Toxin (JSTX) blocks glutamate synapse in hippocampal pyramidal neurons. Brain Res. 346: 397, 1985; Saito *et al.*, Effects of a spider toxin (JSTX) on hippocampal CA1 neurons *in vitro. Brain Res.* 481: 16, 1989; Akaike *et al.*, Spider toxin blocks excitatory amino acid responses in isolated hippocampal pyramidal neurons. *Neurosci. Lett.* 79: 326, 1987; Ashe *et al.*, Argiotoxin-636 blocks excitatory synaptic transmission in rat hippocampal CAl pyramidal neurons. *Brain Res.* 480: 234, 1989; Jones *et al.*, Philanthotoxin blocks quisqualate-induced, AMPA-induced and kainate-induced, but not NMDA-induced excitation of rat brainstem neurones *in vivo. Br. J. Pharmacol.* 101: 968, 1990). Subsequent studies have demonstrated that while certain arylalkylamine toxins are both nonpotent and nonselective at various glutamate receptors, other arylalkylamines are both very potent and selective at antagonizing responses mediated by NMDA receptor activation in the mammalian CNS (Mueller *et al*., Effects of polyamine spider toxins on NMDA receptor-mediated transmission in rat hippocampus *in vitro. Soc. Neurosci. Abst.* 15: 945, 1989; Mueller *et al.*, Arylamine spider toxins antagonize NMDA receptor-mediated synaptic transmission in rat hippocampal slices. *Synapse* 9: 244, 1991; Parks *et al*., Polyamine spider toxins block NMDA receptor-mediated increases in cytosolic calcium in cerebellar granule neurons. *Soc. Neurosci. Abst.* 15: 1169, 1989; Parks *et al*., Arylamine toxins from funnel-web spider (*Agelenopsis aperta*) venom antagonize *N*-methyl-D-aspartate receptor function in mammalian brain. *J. Biol. Chem*. 266: 21523, 1991; Priestley *et al*., Antagonism of responses to excitatory amino acids on rat cortical neurones by the spider toxin, argiotoxin-636. *Br. J. Pharmacol.* 97: 1315, 1989; Draguhn *et al*., Argiotoxin-636 inhibits NMDA-activated ion channels expressed in *Xenopus* oocytes. *Neurosci. Lett.* 132: 187, 1991; Kiskin *et al*., A highly potent and selective *N*-methyl-D-aspartate receptor antagonist from the venom of the *Agelenopsis* aperta spider. *Neuroscience* 51: 11, 1992; Brackley *et al.*, Selective antagonism of native and cloned kainate and NMDA receptors by polyamine-containing toxins. *J. Pharmacol. Exp. Therap.* 266: 1573, 1993; Williams, Effects of *Agelenopsis aperta* toxins on the N-methyl-D-aspartate receptor: Polyamine-like and high-affinity antagonist actions. *J. Pharmacol. Exp. Therap*. 266: 231, 1993). Inhibition of nicotinic cholinergic receptors by the arylalkylamine toxin philanthotoxin has also been reported (Rozental *et al*., Allosteric inhibition of nicotinic acetylcholine receptors of vertebrates and insects by philanthotoxin. *J. Pharmacol. Exp. Therap.* 249: 123, 1989).

Parks *et al.* (Arylamine toxins from funnel-web spider (*Agelenopsis aperta*) venom antagonize *N*-methyl-D-aspartate receptor function in mammalian brain. *J. Biol. Chem.* 266: 21523, 1991), describe arylalkylamine spider toxins ( α-agatoxins) which antagonize NMDA receptor function in mammalian brain. The authors discuss the mechanism of action of arylalkylamine toxins, and indicate that an NMDA receptor-operated ion channel is the probable site of action of the α-agatoxins, and most probably other spider venom arylalkylamines. They state:
"The discovery that endogenous polyamines in the vertebrate brain modulate the function of NMDA receptors suggests that the arylamine toxins may produce their antagonism via a polyamine-binding site on glutamate receptors. Brackley *et al*. studied the effects of spermine and philanthotoxin 433 on the responses evoked by application of excitatory amino acids in *Xenopus* oocytes injected with mRNA from rat or chick brain. These authors reported that, at concentrations below those that antagonize glutamate receptor function, both spermine and philanthotoxin potentiate the effects of excitatory amino acids and some other neurotransmitters. On the basis of these and other data, Brackley *et al.* concluded that the arylamine toxins may, by binding nonspecifically to the membranes of excitable cells, reduce membrane fluidity and alter receptor function. The validity of this intriguing idea for NMDA receptor function is not well supported by two recent binding studies. Reynolds reported that argiotoxin 636 inhibits the binding of [³H]MK-801 to rat brain membranes in a manner that is insensitive to glutamate, glycine, or spermidine. This author concluded that argiotoxin 636 exerts a novel inhibitory effect on the NMDA receptor complex by binding to one of the Mg ²⁻ sites located within the NMDA-gated ion channel. Binding data reported by Williams *et al.* also support the conclusion that argiotoxin 636 does not act primarily at the polyamine modulatory site on the NMDA receptor, but rather acts directly to produce an activity-dependent block of the ion channel. It is already known that compounds such as phencyclidine and ketamine can block the ion channels associated with both arthropod muscle glutamate receptors and mammalian NMDA receptors. Thus, it seems possible that vertebrate and invertebrate glutamate receptors share additional binding sites for allosteric modulators of receptor function, perhaps related to divalent cation-binding sites. Clearly, considerable additional work will be needed to determine if the arylamines define such a novel regulatory site."

Usherwood and Blagbrough (Spider Toxins Affecting Glutamate Receptors: Polyamines in Therapeutic Neurochemistry. *Pharmacol. Therap.* 52: 245, 1991) describe a proposed intracellular binding site for arylalkylamine toxins (polyamine amide toxins) located within the membrane potential field referred to as the QUIS-R channel selectivity filter. The authors postulate that the binding site for polyamine amide toxins may occur close to the internal entrance of the channel gated by the QUIS-R of locust muscle. The authors also note that one such toxin, argiotoxin-636, selectively antagonizes the NMDA receptor in cultured rat cortical neurons.

Gullak *et al.* (CNS binding sites of the novel NMDA antagonist Arg-636. *Soc. Neurosci. Abst.* 15: 1168, 1989), describe argiotoxin-636 (Arg-636) as a polyamine (arylalkylamine) toxin component of a spider venom. This toxin *is* said to block NMDA-induced elevation of cGMP in a noncompetitive fashion. The authors state that:
"[¹²⁵I]Arg-636 bound to rat forebrain. membranes with K_{d} and Bₘₐₓ values of 11.25 µM and 28.95 pmol/mg protein (80% specific). The ability of other known polyamines and recently discovered polyamines from *Agelenopsis aperta* to inhibit binding paralleled neuroactivity as functional NMDA antagonists. No other compounds tested were able to block specific binding."

The authors then stated that polyamines (arylalkylamines) may antagonize responses to NMDA by interacting with membrane ion channels.

Seymour and Mena (*In vivo* NMDA antagonist activity of the polyamine spider venom component, argiotoxin-636. *Soc. Neurosci. Abst.* 15: 1168, 1989) describe studies that are said to show that argiotoxin-636 does not significantly affect locomotor activity at doses that are effective against audiogenic seizures in DBA/2 mice, and that it significantly antagonizes NMDA-induced seizures with a minimal effective dose of 32 mg/kg given subcutaneously (s.c.).

Herold and Yaksh (Anesthesia and muscle relaxation with intrathecal injections of AR636 and AG469, two acylpolyamine spider toxins, in rats. *Anesthesiology* 77: 507, 1992) describe studies that are said to show that the arylalkylamine argiotoxin-636 (AR636), but not agatoxin-489 (AG489). produces muscle relaxation and anesthesia following intrathecal administration in rats.

Williams (Effects of *Agelenopsis aperta* toxins on the *N*-methyl-D-aspartate receptor: Polyamine-like and high-affinity antagonist actions, *J. Pharmacol. Exp. Therap.* 266: 231, 1993) reports that the α-agatoxins (arylalkylamines) Agel-489 and Agel-505 enhance the binding of [³H]MK-801 to NMDA receptors on membranes prepared from rat brain by an action at the stimulatory polyamine receptor; polyamine receptor agonists occluded the stimulatory effects of Agel-489 and Agel-505 and polyamine receptor antagonists inhibited the stimulatory effect of Agel-505. Higher concentrations of Agel-489 and Agel-505, and argiotoxin-636 at all concentrations tested, had inhibitory effects on the binding of [³H]MK-801. In *Xenopus* oocytes voltage-clamped at -70 mV, Agel-505 inhibited responses to NMDA with an IC ₅₀ of 13 nM; this effect of Agel-505 occurred at concentrations approximately 10,000-fold lower than those that affected [³H]MK-801 binding. Responses to kainate were inhibited only 11% by 30 nM Agel-505. The antagonism of NMDA-induced currents by Agel-505 was strongly voltage-dependent, consistent with an open-channel blocking effect of the toxin. Williams states:
"Although α-agatoxins can interact at the positive allosteric polyamine site on the NNDA receptor, stimulatory effects produced by this interaction may be masked in functional assays due to a separate action of the toxins as high-affinity, noncompetitive antagonists of the receptor."

Brackley et al. (Selective antagonism of native and cloned kainate and NMDA receptors by polyamine-containing toxins, *J. Pharmacol. Exp. Therap.* 266: 1573, 1993) report that the polyamine-containing toxins (arylalkylamines) philanthotoxin-343 (PhTX-343) and argiotoxin-636 (Arg-636) produce reversible, noncompetitive, partly voltage-dependent antagonism of kainate- and NMDA-induced currents in *Xenopus* oocytes injected with rat brain RNA. Arg-636 was demonstrated to be selective for NMDA-induced responses (IC ₅₀ = 0.04 µM) compared to kainate-induced responses (IC ₅₀ = 0.07 µM), while PhTX-343 was selective for kainate-induced responses (IC ₅₀ = 0.12 µM) compared to NMDA-induced responses (IC ₅₀ = 2.5 µM). Arg-636 more potently antagonized responses co NMDA in *Xenopus* oocytes expressing cloned NMDAR1 subunits (IC ₅₀ = 0.09 µM) than responses to kainate in oocytes expressing either cloned GluR1 (IC ₅₀ = 3.4 µM) or GluR1+GluR2 subunits (IC ₅₀ ≈ 300 µM). PhTX-343, on the other hand, was equipotent at antagonizing NMDAR1 (IC ₅₀ = 2.19 µM) and GluR1 (IC ₅₀ = 2.8 µM), but much less potent against GluR1+GluR2 subunits (IC ₅₀ = 270 µM).

Raditsch *et al*. (Subunit-specific block of cloned NMDA receptors by argiotoxin-636. *FEBS Lett.* 324: 63, 1993) report that Arg-636 more potently antagonizes responses in *Xenopus* oocytes expressing NMDAR1+NMDAR2A subunits (IC ₅₀ = 9 nM) or NMDAR1+NMDAR2B subunits (IC ₅₀ = 2.5 nM) than NMDAR1+NMDAR2C subunits (IC ₅₀ = 460 nM), even though all of the receptor subunits contain an asparagine residue in the putative pore-forming transmembrane region II (the Q/R site, as discussed above). The authors state that the large difference in Arg-636 sensitivity between NMDAR1+NMDAR2A and NMDAR1+NMDAR2C channels "must be conferred by other structural determinants."

Herlitz *et al.* (Argiotoxin detects molecular differences in AMPA receptor channels. *Neuron* 10: 1131, 1993) report that Arg-636 antagonizes subtypes of AMPA receptors in a voltage- and use-dependent manner consistent with open-channel blockade. Arg-636 potently antagonizes Ca ²⁺-permeable AMPA receptors comprised of GluRAi (K ₁ = 0.35 µM), GluRCi (K ₁ = 0.23 µM), or GluRDi subunits (K ₁ = 0.43 µM), while being essentially ineffective against Ca ²⁺-impermeable GluRBi subunits at concentrations up to 10 µM. Other data reported by these investigators strongly suggest that the Q/R site in the purative pore-forming transmembrane region II is of primary importance in determining Arg-636 potency and Ca ²⁺ permeability.

Blaschke *et al.* (A single amino acid determines the subunit-specific spider toxin block of α-amino-3-hydroxy-5-methylisoxazole-4-propionate/kainate receptor channels. *Proc. Natl. Acad. Sci. USA* 90: 6528, 1993) report that the arylalkylamine JSTX-3 potently antagonizes responses to kainate in *Xenopus* oocytes expressing GluR1 (IC ₅₀ = 0.04 µM) or GluR3 (IC ₅₀ = 0.03 µM) subunits, but that expressed receptors in which a GluR2 subunit is present are essentially unaffected by the toxin. site-directed mutagenesis studies strongly implicate the Q/R site as the primary site influencing toxin potency.

Nakanishi *et al.* (Bioorganic studies of transmitter receptors with philanthotoxin analogs. *Pure Appl. Chem*., in press) have synthesized a number of highly potent photoaffinity labeled philanthotoxin (PhTX) analogs. Such analogs have been studied on expressed nicotinic cholinergic receptors as a model system for receptor-operated calcium channels. These investigators suggest that these PhTX analogs block the ion channel with the hydrophobic headpiece of the toxin binding to a site near the cytoplasmic surface while the polyamine tail extends into the ion channel from the cytoplasmic side.

In US-A 4,018,895 aryloxy phenyl propylamines are disclosed which are useful as antidepressants, wherein the phenyl ring is not substituted.

US-A 5,281,624 pertains to N-alkyl-3-phenyl-3(2-substituted phenoxy) propylamines useful to treat more pinephinine imbalance associated neurological disorders

EP-A 0 399 504 discloses aryloxyphenyl propylamines which may be used for the treatment of anoxia, migrane, ischemia, traumatic injury and neurodegenerative disease.

### Summary of the Invention

Applicant has determined that simplified arylalkylamines (see below) are potent, noncompetitive antagonists of the NMDA receptor-ionophore complex. For example, such compounds bind to the site labeled by [³H]MK-801 at concentrations ranging approximately 1 to 400-fold higher - than those which antagonize NMDA receptor-mediated function. Such simplified arylalkylamines possess one or more of the following additional biological properties: significant neuroprotectant activity, significant anticonvulsant activity, significant analgesic activity, no PCP-like stereotypic behavior in rodents (hyperexcitabilicy and head weaving) at effective neuroprotectant, anticonvulsant and analgesic doses, no generalization to PCP in a PCP discrimination assay at effective neuroprotectant, anticonvulsant and analgetic doses, no neuronal vacuolization at effective neuroprotectant, anticonvulsant and analgesic doses, significantly less potent activity against voltage-sensitive calcium channels, and minimal hypotensive activity at effective neuroprotectant, anticonvulsant and analgesic doses. Such compounds may, however, inhibit the induction of LTP in rat hippocampal slices and may produce motor impairment at neuroprotectant, anticonvulsant and analgesic doses.

By "neurological disorder or disease" is meant a disorder or disease of the nervous system including, but not limited to, global and focal ischemic and hemorrhagic stroke, head trauma, spinal cord injury, spinal cord ischemia, ischemia- or hypoxia-induced nerve cell damage, hypoxia-induced nerve cell damage as in cardiac arrest or neonatal distress, epilepsy, anxiety, neuropsychiatric or cognitive deficits due to ischemia or hypoxia such as those that frequently occur as a consequence of cardiac surgery under cardiopulmonary bypass, and neurodegenerative disease. Also meant by "neurological disorder or disease" are those disease states and conditions in which a neuroprotectant, anticonvulsant, anxiolytic, analgesic, muscle relaxant and/or adjunct in general anesthesia may be indicated, useful, recommended or prescribed.

By "neurodegeneracive disease" is meant diseases including, but not limited to, Alzheimer's Disease, Huntington's Disease, Patkinson's Disease, and amyotrophic lateral sclerosis (ALS).

By "neuroprotectant" is meant a compound capable of preventing the neuronal damage or death associated with a neurological disorder or disease.

By "anticonvulsant" is meant a compound capable of reducing convulsions produced by conditions such as simple partial seizures, complex partial seizures, status epilepticus, and trauma-induced seizures such as occur following head injury, including head surgery.

By "anxiolytic" is means a compound capable of relieving the feelings of apprehension, uncertainty and fear that are characteristic of anxiety.

By "analgesic" is meant a compound capable of relieving pain by altering perception of nociceptive stimuli without producing anesthesia or loss of consciousness.

By "muscle relaxant" is meant a compound that reduces muscular tension.

By "adjunct in general anesthesia" is meant a compound useful in conjunction with anesthetic agents in producing the loss of ability to perceive pain associated with the loss of consciousness.

By "potent" or "active" is meant that the compound has activity at receptor-operated calcium channels, including NMDA receptors, Ca ²⁺-permeable AMPA receptors, and nicotinic cholinergic receptors, with an IC ₅₀ value less than 10 µM, more preferably less than 100 nM, and even more preferably less than 1 nM.

By "selective" is meant that the compound is potent at receptor-operated calcium channels as defined above, but is less potent by greater than 10-fold, more preferably 50-fold, and even more preferably 100-fold, at other neurotransmitter receptors, neurotransmitter receptor-operated ion channels, or voltage-dependent ion channels.

By "biochemical and electrophysiological assays of receptor-operated calcium channel function" is meant assays designed to detect by biochemical or electrophysiological means the functional activity of receptor-operated calcium channels. Examples of such assays include, but are not limited to, the fura-2 fluorimetric assay for cytosolic calcium in cultured rat cerebellar granule cells (see Example 1 and Example 2), patch clamp electrophysiolocial assays (see Example 3 and Example 27), rat hippocampal slice synaptic transmission assays, radioligand binding assays Example 21, Example 22, and Example 23), and *in vitro* neuroprotectant assays (see Example 3).

By "efficacy" is meant that a statistically significant level of the desired activity is detectable with a chosen compound; by "significant" is meant a statistical significance at the p < 0.05 level.

By "neuroprotectant activity" is meant efficacy in treatment of neurological disorders or diseases including, but not limited to, global and focal ischemic and hemorrhagic stroke, .head trauma, spinal cord injury, spinal cord ischemia, ischemia- or hypoxia-induced nerve cell damage, hypoxia-induced nerve cell damage as in cardiac arrest or neonatal distress, neuropsychiatric or cognitive deficits due to ischemia or hypoxia such as those that frequently occur as a consequence of cardiac surgery under cardiopulmonary bypass, and neurodegenerative diseases such as Alzheimer's Disease, Huntington's Disease, Parkinson's Disease, and amyocropbic lateral sclerosis (ALS) (see Examples 4 and 5, below) .

By "anticonvulsant activity" is meant efficacy in reducing convulsions produced by conditions such as simple partial seizures, complex partial seizures, status epilepticus, and trauma-induced seizures such as occur following head injury, including head surgery (see Examples 6 and 7, below).

By "anxiolytic activity" is meant that a compound reduces the feelings of apprehension, uncertainty and fear that are characteristic of anxiety.

By "analgesic activity" is meant that a compound produces the absence of pain in response to a stimulus that would normally be painful. Such activity would be useful in clinical conditions of acute and chronic pain including, but not limited- to the following: preemptive preoperative analgesia; peripheral neuropathies such as occur with diabetes mellitus and multiple sclerosis; phantom limb pain; causalgia; neuralgias such as occur with herpes zoster; central pain such as that seen with spina cord lesions; hyperalgesia; and allodynia.

By "causalgia" is meant a painful disorder associated with injury of peripheral nerves.

By "neuralgia" is meant pain in the distribution of a nerve or nerves.

By "central pain" is meant pain associated with a lesion of the central nervous system.

By "byperalgesia" is meant an increased response to a stimulus that is normally painful.

By "allodynia" is meant pain due to a stimulus that does not normally provoke pain (see Examples 8 through 11, below).

By "induction of long-term potentiation in rat hippocampal slices" is meant the ability of tetanic electrical stimulation of afferent Schaffer collateral fibers to elicit long-term increases in the strength of synaptic transmission at the Schaffer collateral-CA1 pyramidal cell pathway in rat hippocampal slices maintained *in vitro* (see Example 16).

By "therapeutic dose" is meant an amount of a compound that relieves to some extent one or more symptoms of the disease or condition of the patient. Additionally, by "therapeutic dose" is meant an amount that rerurns to normal, either partially or completely, physiological or biochemical parameters associated with or causative of the disease or condition. Generally, id is an amount between about 1 nmole and 1 µmole of the compound, dependent on its EC ₅₀ (IC ₅₀ in the case of an antagonist) and on the age, size, and disease associated with the patient.

By "impair cognition" is meant the ability to impair the acquisition of memory or the performance of a learned task (see Example 17). Also by "impair congnition" is meant the ability to interfere with normal rational thought processes and reasoning.

By "disrupt motor function" is meant the ability to significantly alter locomotor activity (see Example 12) or elicit significant ataxia, loss of the righting reflex, sedation or muscle relaxation (see Example 13).

By "locomotor activity" is meant the ability to perform normal ambulatory movements.

By "loss of the righting reflex" is meant the ability of an animal, typically a rodent, to right itself after being placed in a supine position.

By "neuronal vacuolization" is meant the production of vacuoles in neurons of the cingulate cortex or retrosplenial cortex (see Example 15).

By "cardiovascular activity " is meant the ability to elicit significant changes in parameters including, but not limited to, mean arterial blood pressure and heart rate (see Examples 18 and 19).

By "hyperexcitability" is meant an enhanced susceptibility to an excitatory stimulus. Hyperexcitability is often manifested as a significant increase in locomotor activity in rodents administered a drug (see Example 12).

By "sedation" is meant a calmative effect, or the allaying of activity and excitement. Sedation is often manifested as a significant decrease in locomotor activity in rodents administered a drug (see Example 12).

By "PCP-like abuse potential" is meant the potential of a drug to be wrongfully used, as in the recreational use of PCP (i.e., "angel dust") by man. It is believed that PCP-like abuse potential can be predicted by the ability of a drug to generalize to PCP in rodents trained to discriminate PCP from saline (see Example 14.)

By "generalization to PCP" is meant that a compound is perceived as being PCP in rodents trained to discriminate PCP from saline (see Example 14).

By "PCP-like psychotomimetic activity" is meant the ability of a drug to elicit in man a behavioral syndrome resembling acute psychosis, including visual hallucinations, paranoia, agitation, and confusion. It is believed that PCP-like psychotomimetic activity can be predicted in rodents by the ability of a drug to produce PCP-like stereotypic behaviors including ataxia, head weaving, hyperexcitability, and generalization to PCP in rodents trained to discriminate PCP from saline (see Example 12, Example 13, and Example 14).

By "ataxia" is meant a deficit in muscular coordination.

By "head weaving" is meant the stereotypic behavior elicited in rodents by PCP in which the head is repeatedly moved slowly and broadly from side to side.

By "pharmaceutical composition" is meant a therapeutically effective amount of a compound of the present invention in a pharmaceutically acceptable carrier, *i.e.*, a formulation to which the compound can be added to dissolve or otherwise facilitate administration of the compound. Examples of pharmaceutically acceptable carriers include water, saline, and physiologically buffered saline. Such a pharmaceutical composition is provided in a suitable dose. Such compositions are generally those which are approved for use in treatment of a specified disorder by the FDA or its equivalent in non-U.S. countries.

Treatment involves the steps of first identifying a patient that suffers from a neurological disease or disorder by standard clinical methodology and then treating such a patient with a composition of the present invention.

One aspect of the present invention features the use of a compound having the formula: 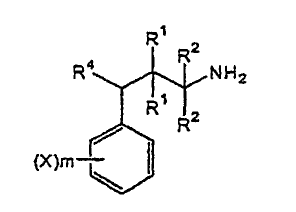 wherein:
X is independently selected from the group consisting of -Br, -Cl, -F, -I,-CF₃, alkyl, -OH, -OCF₃, -O-alkyl, and -O-acyl;
R₁ is independently selected from the group consisting of -H, C₁-C₄ alkyl, and -O-acyl;
R₂ is independently selected from the group consisting of -H, alkyl, and hydroxyalkyl, or both R₂s together are imino;
R₄ is phenoxy which is optionally substituted with -F, -Cl, -Br, -I, -CF_{3,} alkyl, -OH,- OCF₃ -O-alkyl, or -O-acyl; and
m is independently an integer from 0 to 5; and pharmaceutically acceptable salts and complexes thereof provided that said compound is not:
3-(p-isopropoxyphenoxy)-3-phenylpropylamine
3-(2'-methyl-4',5'-dichlorophenoxy)-3-phenylpropylamine
3-(p-t-butylphenoxy)-3-phenylpropylamine
3-(2',4'-dichlorophenoxy)-3-phenyl-2-methyl propylamine
3-(o-ethylphenoxy)-3-phenylpropylamine
3-(o-methoxyphenoxy)-3-phenylpropylamine
3-phenoxy-3-phenylpropylamine
for the preparation of a pharmaceutical composition for the treatment of a neurological disease or disorder.

Another aspect features the use of a compound having the formula 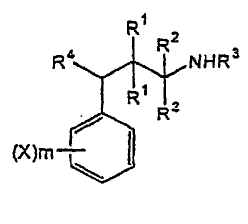 wherein:
X is independently selected from the group consisting of -F, -Cl, -Br, -I, -CF₃ alkyl, -OH, -OCF₃, -O-alkyl, and -O-acyl;
R₁ is independently selected from the group consisting of -H, C₁-C₄ alkyl, and -O-acyl;
R₂ is independently selected from the group consisting of -H, C₁-C₄ alkyl, and hydroxyalkyl, or both R₂s together are imino;
R₃ is selected from the group consisting of methyl and ethyl;
R₄ is phenoxy which is optionally substituted with -F, -Cl, -Br, -I, -CF₃, alkyl, -OH, -OCF₃, -O-alkyl, or -O-acyl;
and m is independently an integer from 0 to 5; and pharmaceutically acceptable salts and complexes thereof provided that said compound is not
N-methyl 3-(o-chloro-p-tolyloxy)-3-phenyl-1-methylpropylamine
N-methyl 3-(p-tolyloxy)-3-phenylpropylamine
N-methyl 3-(o-chloro-p-isopropylphenoxy)-3-phenyl-2-methylpropylamine
N-methyl 3-(p-iodophenoxy)-3-phenyl-propylamine
N-methyl 3-(3-n propylphenoxy)-3-phenyl-propylamine
N-methyl 3-(p-trifluoromethylphenoxy)-3-phenylpropylamine
N-methyl 3-(m-chlorophenoxy)-3-phenylpropylamine
N-methyl 3-(p-fluorophenoxy)-3-phenylpropylamine
N-methyl 3-(p-methoxyphenoxy)-3-phenylpropylamine
N-methyl 3-(o-methoxyphenoxy)-3-phenylpropylamine
N-methyl 3-(o-fluorophenoxy)-3-phenylpropylamine
N-methyl 3-(o-tolyloxy)-3-phenylpropylamine
N-methyl 3-(p-chlorophenoxy)-3-phenylpropylamine
N-methyl 3-(m-fluorophenoxy)-3-phenylpropylamine
N-methyl 3-phenoxy-3-phenyl-2-methylpropylamine
N-methyl 3-phenoxy-3-phenyl-1-methylpropylamine
N-methyl 3-phenoxy-3-phenylpropylamine
N-methyl 3-(o-trifluoromethylphenoxy)-3-phenylpropylamine
N-methyl 3-(m-methoxyphenoxy)-3-phenylpropylamine
N-ethyl 3-(o-iodophenoxy)-3-phenylpropylamine
N-ethyl-3-(o-chlorophenoxy)-3-phenylpropylamine
N-methyl-3-(o-bromophenoxy)-3-phenylpropylamine
N-methyl-3-(o-bromophenoxy)-3-phenylpropylamine
for the preparation of a pharmaceutical composition for the treatment of a neurological disease or disorder.

A third aspect features the use of a compound having the formula: 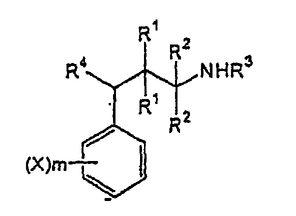 wherein:
(X)m is selected from the group consisting of meta-fluoro; meta-chloro, ortho-O-C₁-C₄ alkyl, ortho-methyl, ortho-fluoro, ortho-chloro, meta-O-C₁-C₄ alkyl, meta-methyl, ortho-OH, and meta-OH;
R₁ is H;
R₂ is H;
R₃ is selected from the group consisting of methyl and ethyl;
R₄ is phenoxy which is optionally substituted with -F, -Cl, -Br, -I, -CF₃, alkyl, -OH, -OCF₃, -O-alkyl, or -O-acyl; and pharmaceutically acceptable salts and complexes thereof, for the preparation of a pharmaceutical composition for the treatment of a neurological disease or disorder.

The compounds of the present invention are active at an NMDA receptor.

By "patient" is meant any animal that has a cell with an NMDA receptor. Preferably, the animal is a mammal. Most preferably, the animal is a human.

By "alkyl" is meant a branched or unbranched hydrocarbon chain containing between 1 and 6, preferably between 1 and 4, carbon atoms, such as, e.g., methyl, ethyl, *n*-propyl, *iso*-propyl, *n*-butyl, *sec*-butyl, *iso*-butyl, *tert*-butyl, 2-methylpentyl, cyclopropylmethyl, allyl, and cyclobutylmethyl.

By "lower alkyl" is meant a branched or unbranched hydrocarbon chain containing between 1 and 4 carbon atoms, of which examples are listed herein.

By "hydroxyalkyl" is meant an alkyl group as defined above, substituted with a hydroxyl group.

By "alkylphenyl" is meant an alkyl group as defined above, substituted with a phenyl group.

By "acyl" is meant -C(O)R, where R is H or alkyl as defined above, such as, e.g., formyl, acetyl, propionyl, or butyryl;
or R is -O-alkyl such as in alkyl carbonates or R is N-alkyl such as in alkyl carbamates.

By "cycloalkyl" is meant a branched or unbranched cyclic hydrocarbon chain containing between 3 and 12 carbon atoms.

Preferred aspects are those embodiments in which (X) m is independently selected from the group consisting of meta-fluoro, meta-chloro, ortho-O-lower alkyl, ortho-methyl, ortho-fluoro, ortho-chloro, meta-O-lower alkyl, meta-methyl, ortho-OH, and meta-OH.

Especially preferred aspects are those embodiments in which (X)m is meta-fluoro; NR³ is selected from the group consisting of NH and N-methyl; and each R¹ and R² is -H;

Other preferred embodiments of the present invention relate to the use of a compound which is selected from the group consisting of 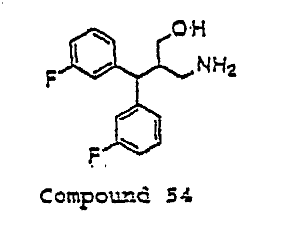 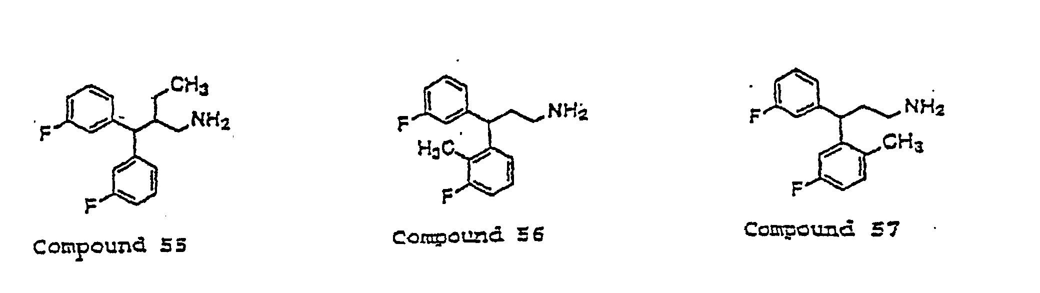 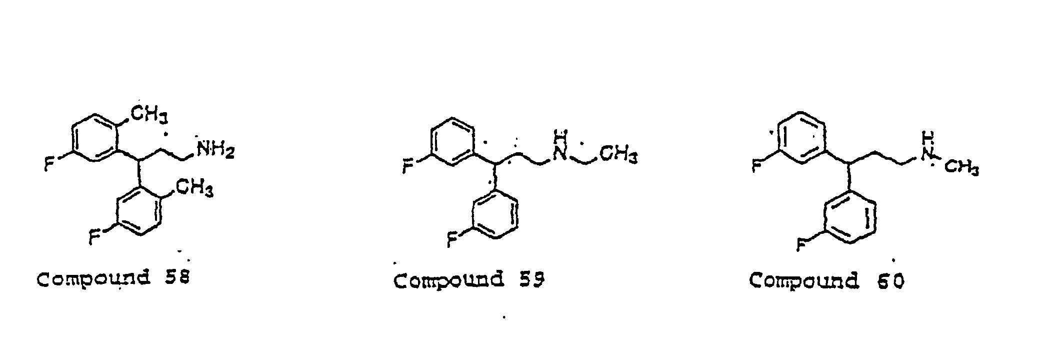 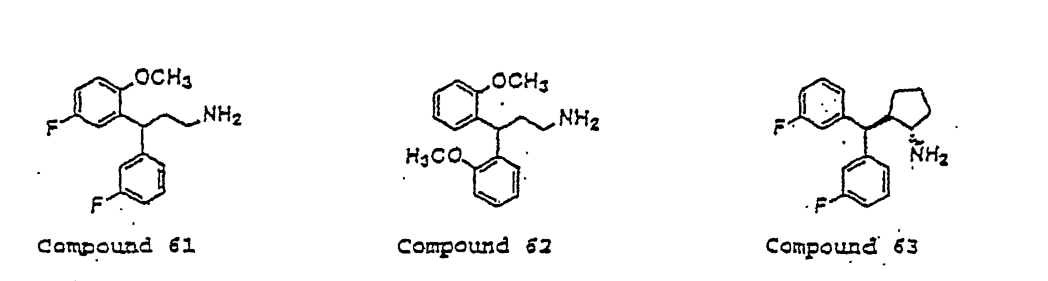 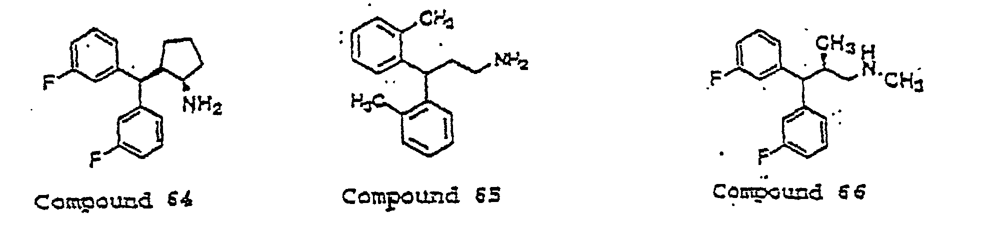 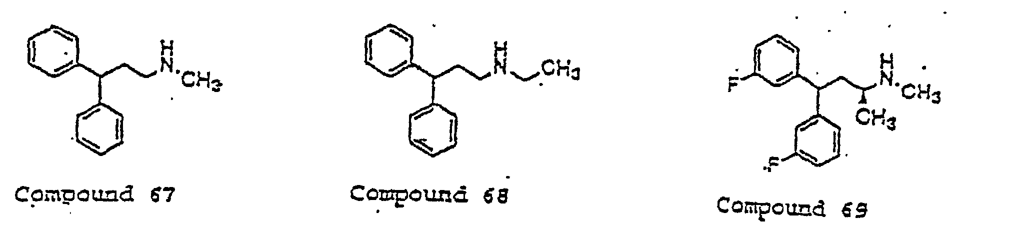 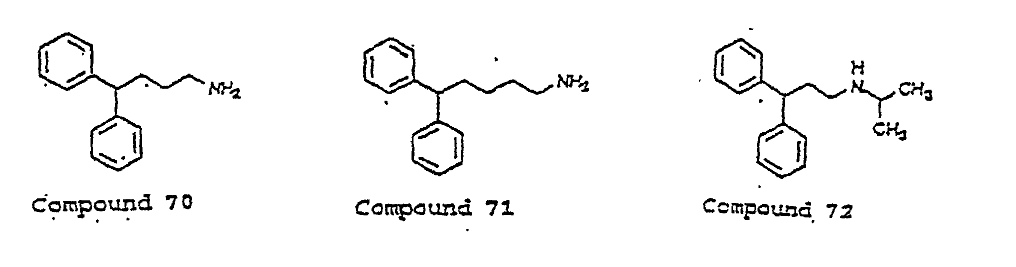 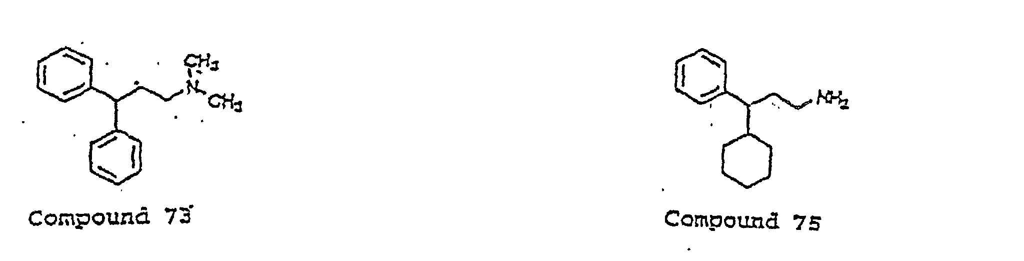 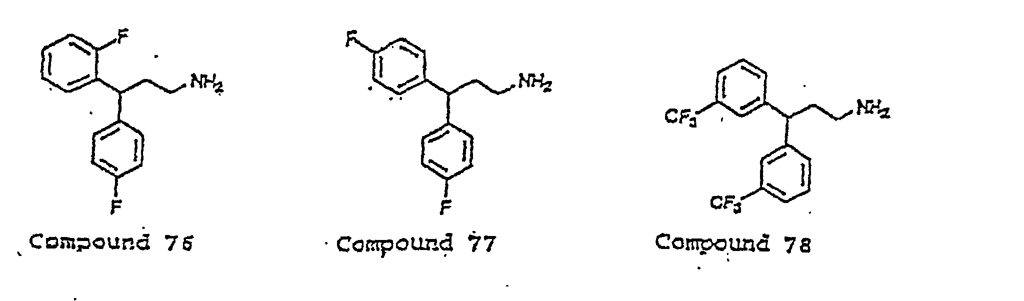 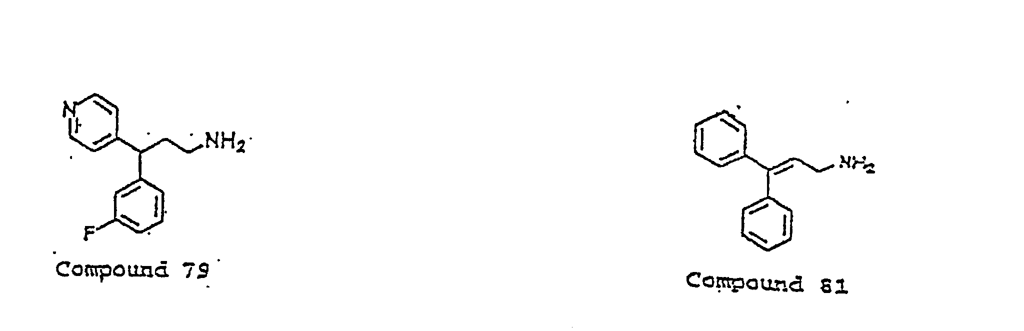 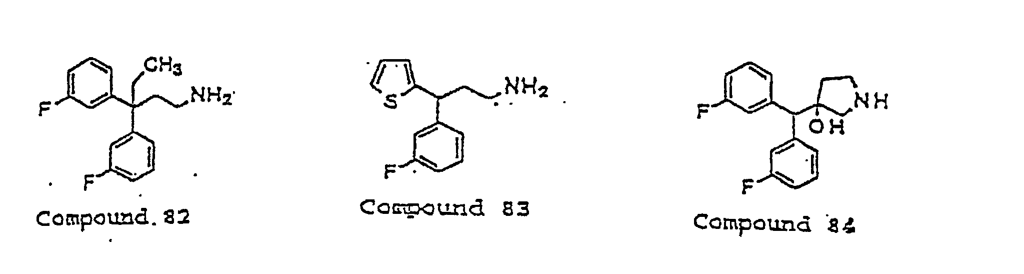 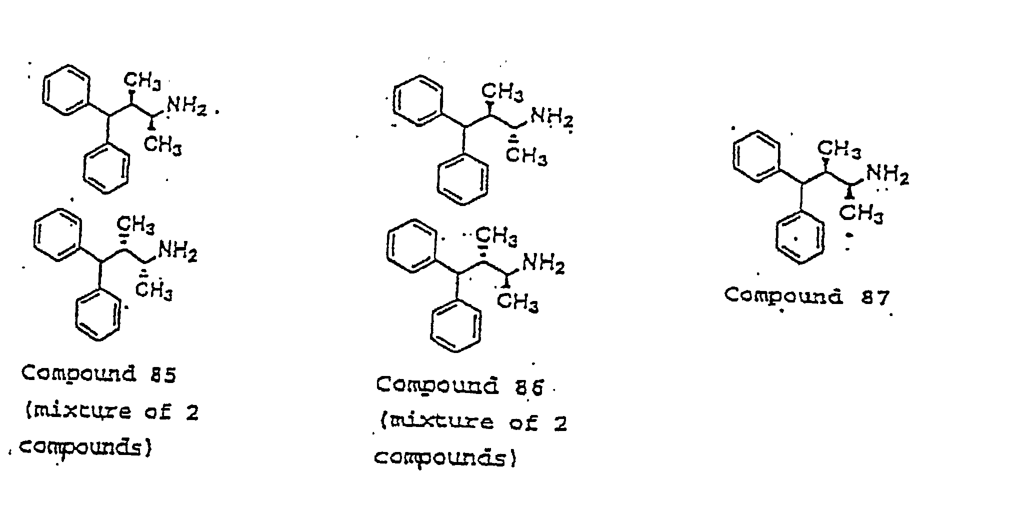 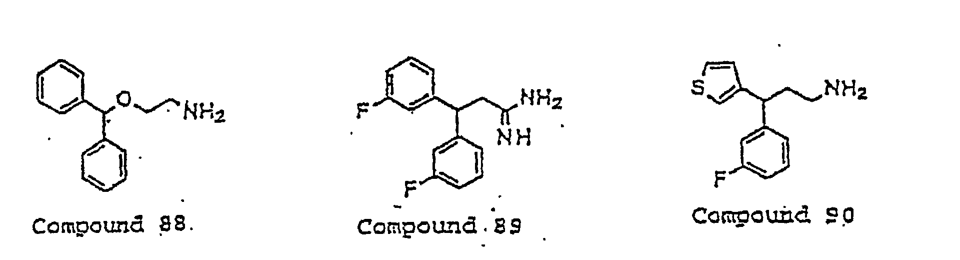 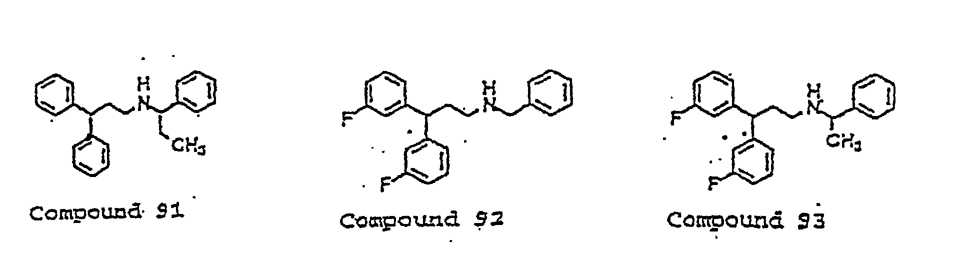 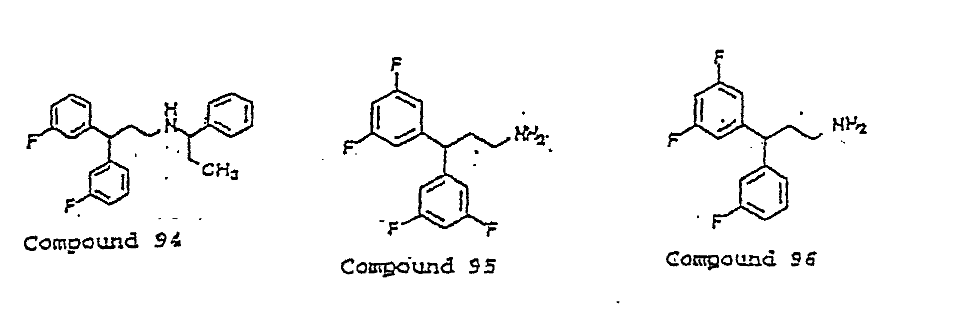 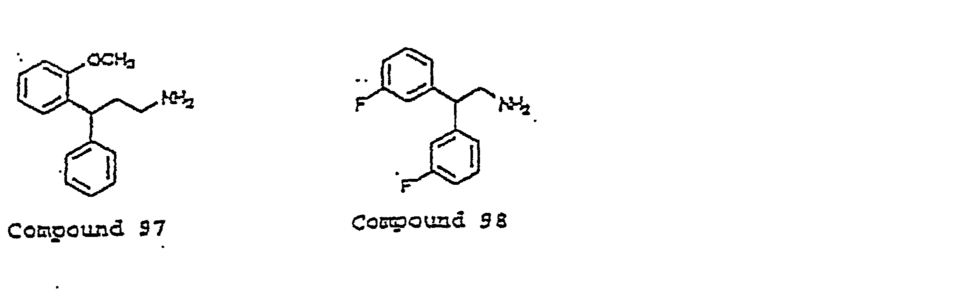 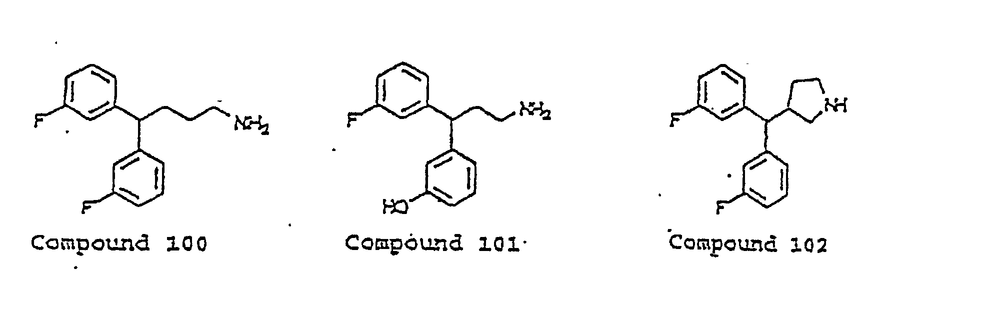 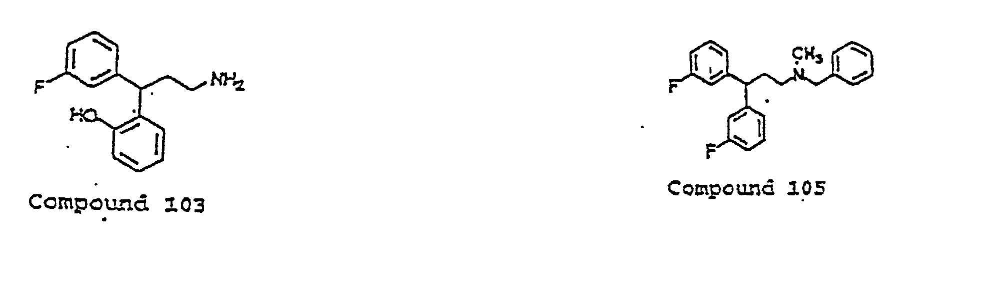 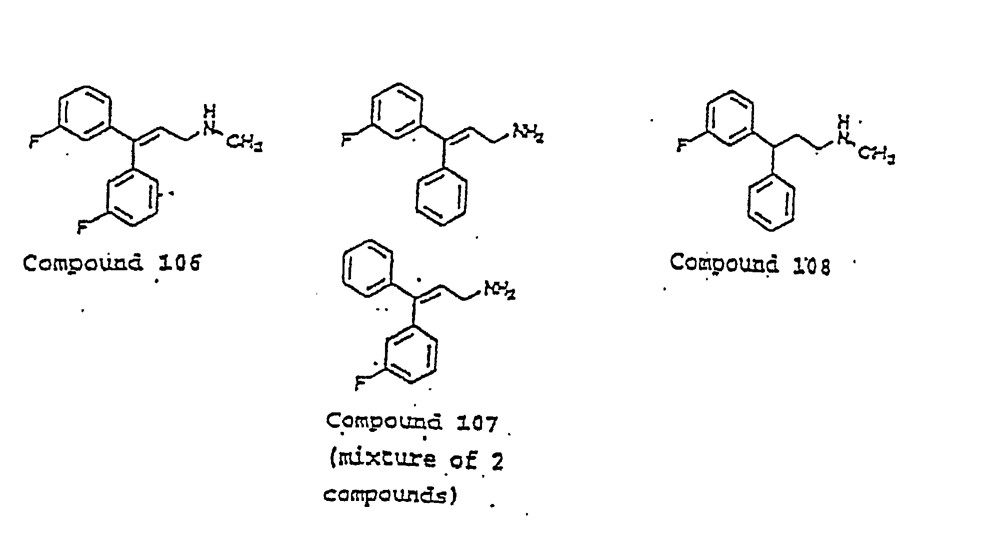 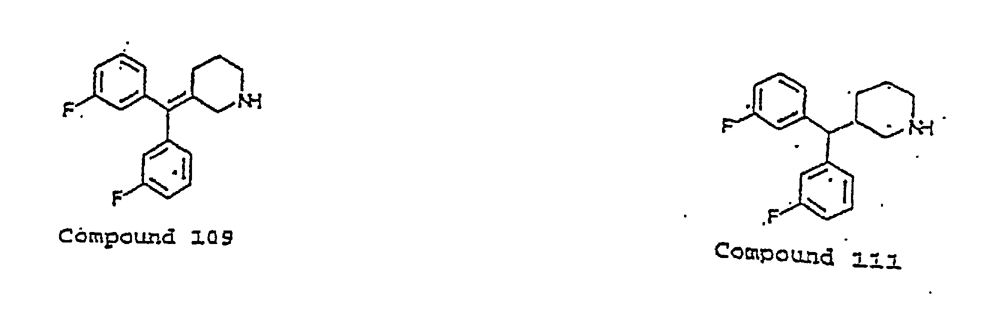 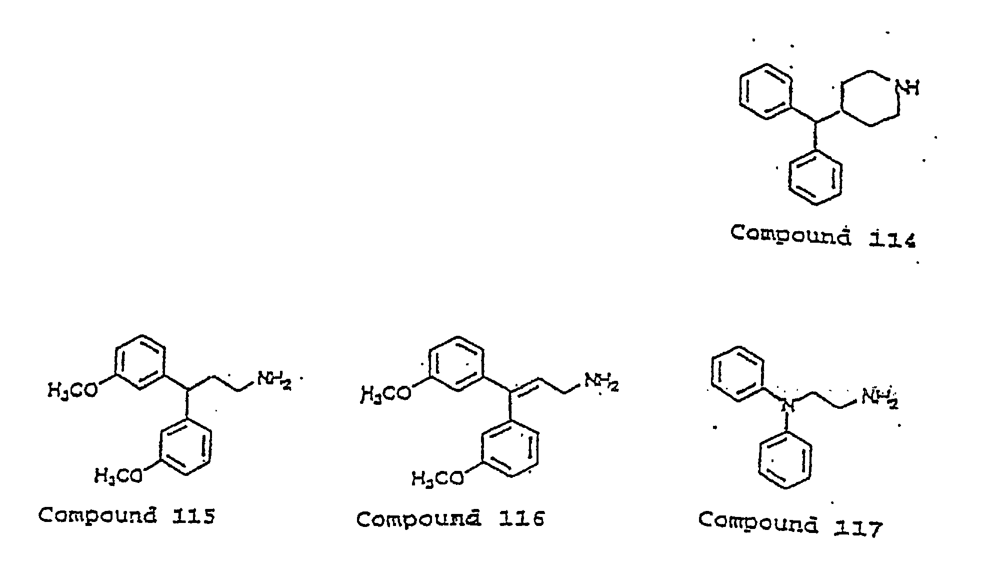 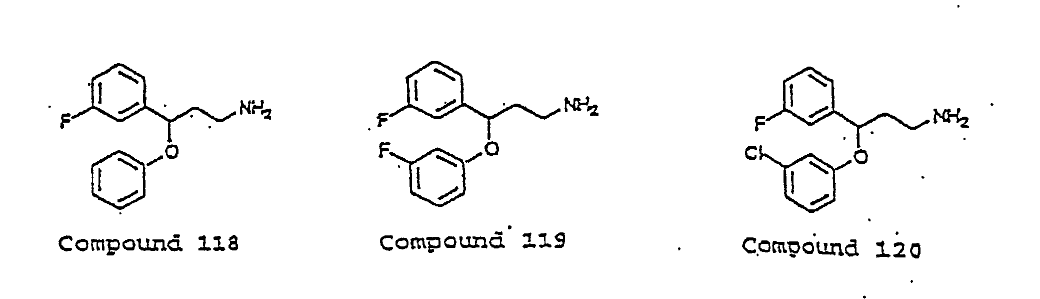 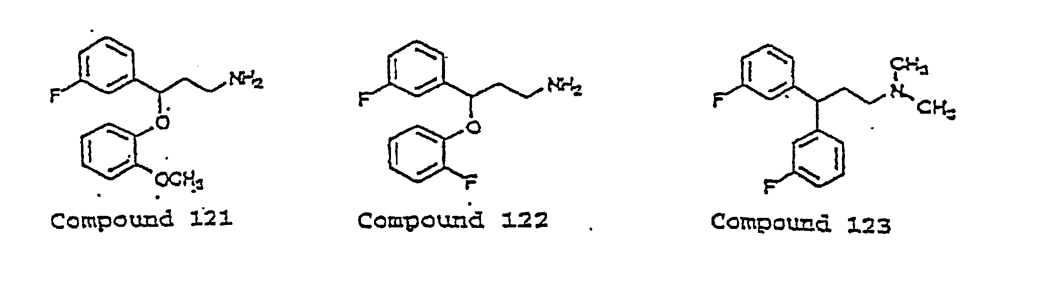 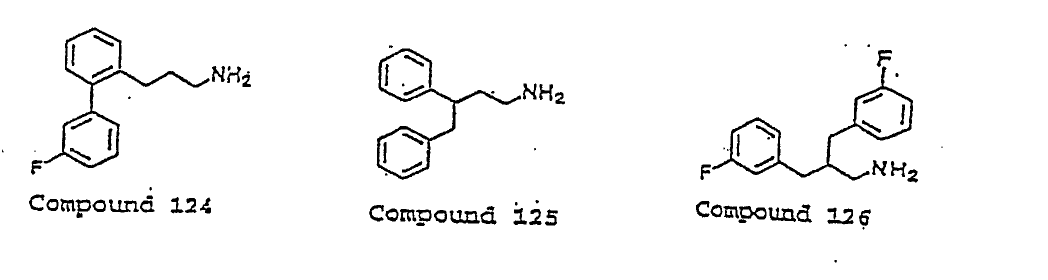 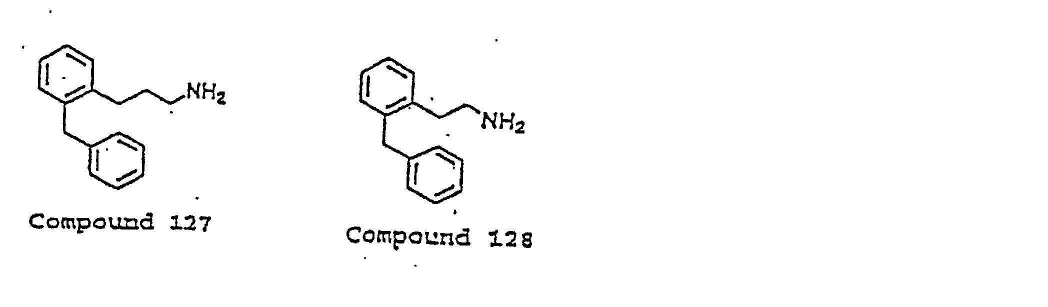 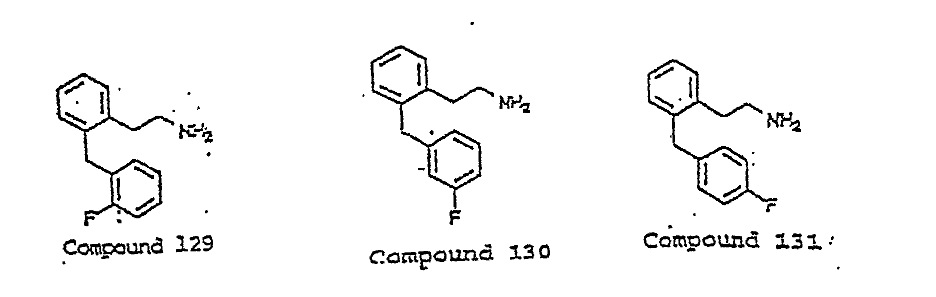 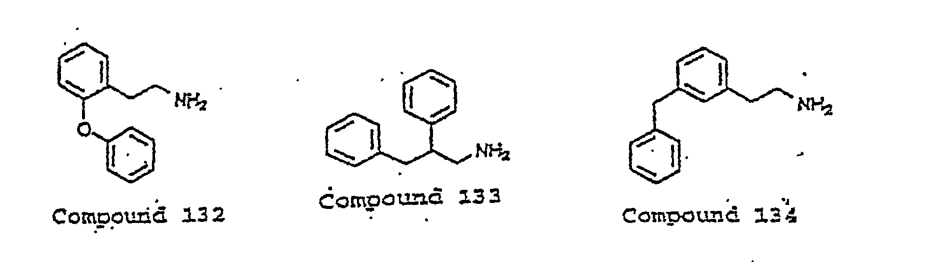 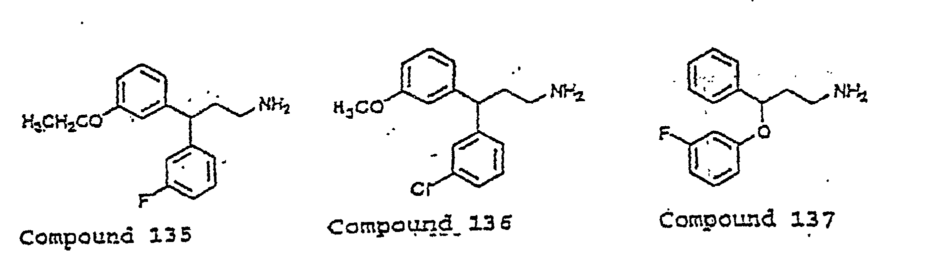 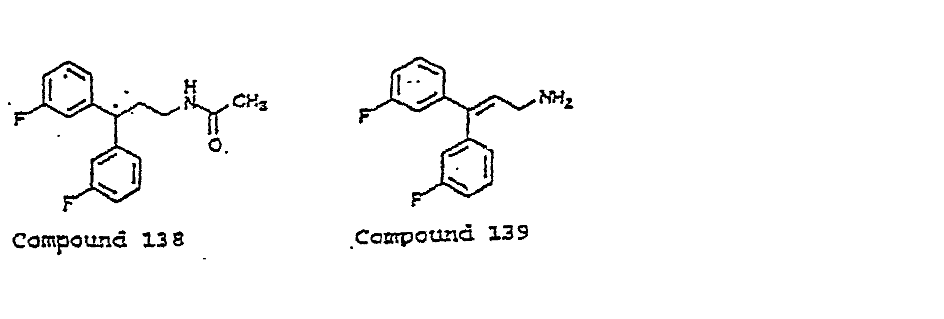 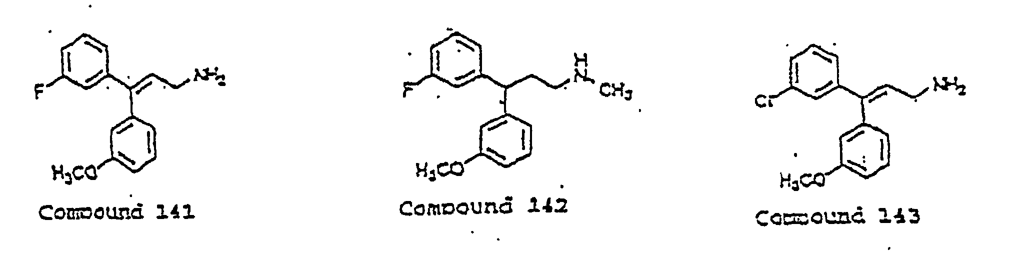 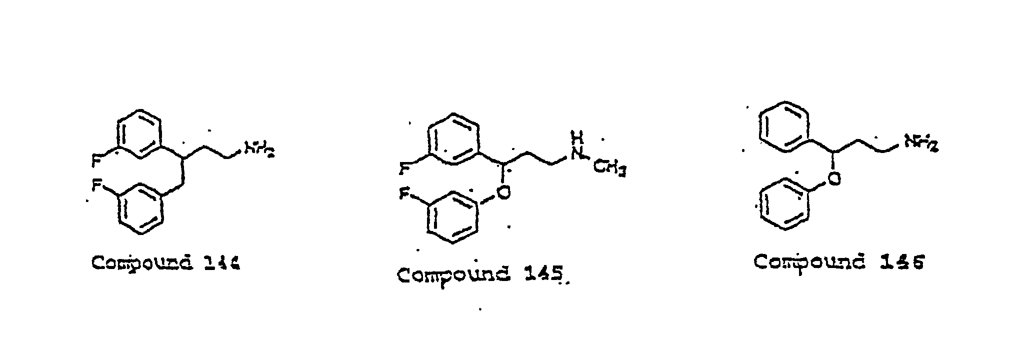 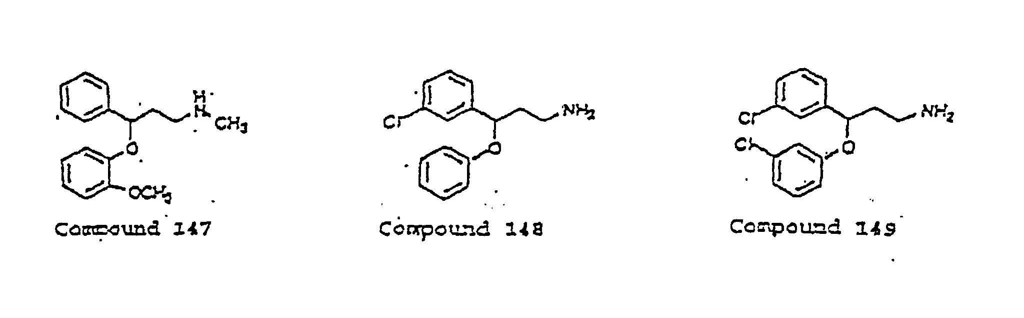 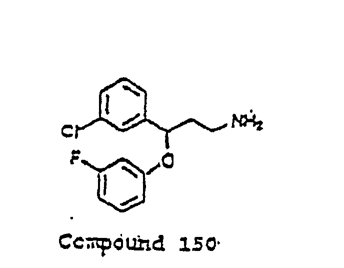 , and pharmaceutically acceptable salts and complexes thereof for the preparation of a pharmaceutical composition for the treatment of a neurological disease or disorder.

Preferably, the compound to be administered has an IC₅₀ ≤ 10 µM at an NMDA receptor, more preferably ≤ 2.5 µM, and most preferably ≤ 0.5 µM at an NMDA receptor.

In further preferred embodiments, the invention uses compounds with an I.C. ₅₀ ≤ 10µm at an MMDA receptor selected from the group consisting of Compound 54, 55, 56, 57, 58, 59, 60, 61, 62, 63, 64, 65, 66, 67, 68, 69, 70, 71, 72, 73, 75, 76, 77, 78, 79, 81, 82, 83, 84, 85, 86, 87, 88, 89, 90, 91, 92, 93, 94, 95, ,96, 97, 98, 100, 101, 102, 103, 105, 106, 107, 108, 109, 111, 114, 115, 116, 117, 118, 119, 120, 121, 122, 123, 124, 125, 126, 127, 128, 129, 130, 131, 132, 133, 134, 135, 136, 137, 138 (potential prodrug), 139, 141, 142, 143, 144, 145, 146, 147, 148, 149, and 150, and pharmaceutically acceptable salts and complexes thereof.

In more preferred embodiments, the invention uses compounds with an IC₅₀ ≤ 2.5 µM at an NMDA receptor selected from the group consisting of Compound 54, 55, 56, 57, 58, 59, 60, 61, 62, 63, 64, 65, 66, 69, 70, 75, 76, 81, 82, 83, 85, 86, 87, 88, 89, 90, 91, 92, 93, 94, 95, 96, 97, 100, 101, 102, 103, 105, 106, 108, 109, 111, 115, 118, 119, 120, 121, 122, 125, 126, 127, 128, 129, 130, 131, 132, 133, 135, 136, 137, 138 (potential prodrug), 139, 142, 144, 145, 146, 147, 148, 149, and 150, and pharmaceutically acceptable salts and complexes thereof. In other embodiments, the compound is selected from the group consisting of Compound 54, 55, 56, 57, 58, 59, 60, 61, 62, 63, 64, 65, 66, 69, 76, 82, 83, 88, 89, 90-, 92, 93, 94, 95, 96, 101, 102, 103, 105, 109, 111, 115, 118, 119, 120, 121, 122, 125, 126, 127, 129, 130, 131, 135, 136, 137, 138, 139, 142, 144, 145, 148, 149, and 150, and pharmaceutically acceptable salts and complexes thereof.

In particularly preferred embodiments, the invention uses a compound with an IC₅₀ ≤ 0.5 µM at an NMDA receptor selected from the group consisting of Compound 54, 55, 56, 57, 58, 59, 60, 61, 62, 63, 64, 65, 66, 65, 82, 83, 89, 90, 91, 93, 94, 95, 96, 97, 103, 111, 118, 119, 120, 122, 126, 135, 136, 137, 138 (potential prodrug), 142, 144, 145, 147, 148, 149, and 150, and pharmaceutically acceptable salts and complexes thereof.

In more preferred embodiments, the invention uses a compound selected from the group consisting of Compound 60, 66, 69, 103, 111, 118, 119, 120, 122, 136, 137, 138 (potential prodrug), 142, 144, 145, 148, 149, and 150, and pharmaceutically acceptable salts and complexes thereof.

In particularly preferred embodiments, the invention uses a compound selected from the group consisting of Compound 60, 119, and 144, and pharmaceutically acceptable salts and complexes thereof.

In other particularly preferred embodiments, the invention uses a compound selected from the group consisting of Compound 60, 119, and 144, and pharmaceutically acceptable salts and complexes thereof.

The present invention further provides simplified arylalkylamines as defined in the attached claims.

Preferably, the compound has an IC₅₀ ≤ 10 µM at an NMDA receptor. More preferably, the compound has an IC₅₀ ≤ 5 µM, more preferably ≤ 2.5 µM, and most preferably ≤ 0.5 µM at an NMDA receptor.

In preferred embodiments, the compound has an IC₅₀ ≤ 10 µM at an NMDA receptor and is selected from the group consisting of Compounds 54, 55, 56, 57, 58, 59, 60, 61, 62, 63, 64, 65, 66, 69, 76, 78, 79, 82, 83, 84, 88, 89, 90, 92, 93, 94, 95, 96, 98, 101, 102, 103, 105, 107, 108, 109, 111, 115, 116, 118, 119, 120, 121, 122, 124, 125, 126, 127, 129, 130, 131, 134, 135, 136, 137, 138 (potential prodrug), 139, 141, 142, 143, 144, 145, 148, 149, and 150. In other embodiments, the compound is selected from the group consisting of 54, 55, 56, 57, 58, 59, 60, 61, 62, 63, 64, 65, 66, 69, 76, 82, 83, 88, 89, 90, 92, 93, 94, 95, 96, 101, 102, 103, 105, 109, 111, 115, 118, 119, 120, 121, 122, 125, 126, 127, 129, 130, 131, 135, 136, 137, 138, 139, 142, 144, 145, 148, 149, and 150.

In more preferred embodiments, the compound has an IC₅₀ ≤ 2.5 µM at an NMDA receptor and is selected from the group consisting of Compound 54, 55, 56, 57, 58, 59, 60, 61, 62, 63, 64, 65, 66, 69, 76, 82, 83, 88, 89, 90, 92, 93, 94, 95, 96, 101, 102, 103, 105, 108, 109, 111, 115, 118, 119, 120, 121, 122, 125, 126, 127, 129, 130, 131, 135, 136, 137, 138 (potential prodrug), 139, 142, 144, 145, 148, 149, and 150.

In particularly preferred embodiments, the compound has an IC₅₀ ≤ 0.5 µM at an NMDA receptor and is selected from the group consisting of Compound 54, 55, 56, 57, 58, 59, 60, 61, 62, 63, 64, 65, 66, 69, 82, 83, 89, 90, 93, 94, 95, 96, 103, 111, 118, 119, 120, 122, 126, 135, 136, 137, 138 (potential prodrug), 142, 144, 145, 148, 149, and 150.

In preferred embodiments, the compound is selected from the group consisting of Compound 60, 66, 69, 103, 111, 118, 119, 120, 122, 136, 137, 138, 142, 144, 145, 148, 149, and 150.

In particularly preferred embodiments, the compound is selected from the group consisting of Compound 60, 119, and 144.

In more particularly preferred embodiments, the compound is selected from the group consisting of Compound 60, 119, and 144.

Also provided in an aspect of the invention are pharmaceutical compositions useful for treating a patient having a neurological disease or disorder as defined in the attached claims. The pharmaceutical compositions are provided in a pharmaceutically acceptable carrier and appropriate dose. The pharmaceutical compositions may be in the form of pharmaceutically acceptable salts and complexes, as is known to those skilled in the art'.

Preferably, the compound has an IC₅₀ ≤ 10 µM at an NMDA receptor. More preferably the compound has an IC₅₀ ≤ 5 µM, more preferably ≤ 2.5 µM, and most preferably ≤ 0.5 µM at an NMDA receptor.

In further preferred embodiments, the pharmaceutical composition comprises a compound with an IC₅₀ ≤ 10µM at an NMDA receptor and is selected from the group consisting of Compound 53, 54, 55, 56, 57, 58, 59, 60, 61, 62, 63, 64, 65, 66, 67, 68, 69, 70, 71, 72, 73, 75, 76, 77, 78, 79, 81, 82, 83, 84, 85, 86, 87, 88, 89, 90, 91, 92, 93, 94, 95, ,96, 97, 98, 100, 101, 102, 103, 105, 106, 107, 108, 109, 111, 114, 115, 116, 117, 118, 119, 120, 121, 122, 123, 124, 125, 126, 127, 128, 129, 130, 131, 132, 133, 134, 135, 136, 137, 138 (potential prodrug), 139, 141, 142, 143, 144, 145, 146, 147, 148, 149, and 150. Preferably, the compound is selected from the'group consisting of 54, 55, 56, 57, 58, 59, 60, 61, 62, 63, 64, 65, 66, 69, 76, 78, 79, 82, 83, 84, 88, 89, 90, 92, 93, 94, 95, 96, 98, 101, 102, 103, 105, 107, 108, 109, 111, 115, 116, 118, 119, 120, 121, 122, 124, 125, 126, 137, 129, 130, 131, 134, 135, 136, 137, 138 (potential prodrug), 139, 141, 142, 143, 144, 145, 148, 149, and 150.

In other embodiments, the-compound is selected from the group consisting of 54, 55, 56, 57, 58, 59, 60, 61, 62, 63, 64, 65, 66, 69, 76, 82, 83, 88, 89, 90, 92, 93, 94, 95, 96, 101, 102, 103, 105, 109, 111, 115, 118, 119, 120, 121, 122, 125, 126, 127, 129, 130, 131, 135, 136, 137, 138, 139, 142, 144, 145, 148, 149, and 150.

In more preferred embodiments, the pharmaceutical composition comprises a compound with an IC₅₀ ≤ 2.5 µM at an NMDA receptor and is selected from the group consisting of Compound 54, 55, 56, 57, 58, 59, 60, 61, 62, 63, 64, 65, 66, 69, 70, 75, 76, 81, 82, 83, 85, 86, 87, 88, 89, 90, 91, 92, 93, 94, 95, 96, 97, 100, 101, 102, 103, 105, 106, 108, 109, 111, 115, 118, 119, 120, 121, 122, 125, 126, 127, 128, 129, 130, 131, 132, 133, 135, 136, 137, 138 (potential prodrug), 139, 142, 144, 145, 146, 148, 149, and 150. Preferably, the compound is selected from the group consisting of 54, 55, 56, 57, 58, 59, 60, 61, 62, 63, 64, 65, 66, 69, 76, 82, 83, 88, 89, 90, 92, 93, 94, 95, 96, 101, 102, 103, 105, 108, 109, 111, 115, 118, 119, 120, 121, 122, 125, 126, 127, 129, 130, 131, 135, 136, 137, 138 (potential prodrug), 139, 142, 144, 145, 148, 149, and 150.

In particularly preferred embodiments, the pharmaceutical composition comprises a compound with an IC₅₀ ≤ 0.5 µM at an MMDA receptor and is selected from the group consisting of Compound 54, 55, 56, 57, 58, 59, 60, 61, 62, 63, 64, 65, 66, 69, 82, 83, 89, 90, 93, 94, 95, 96, 97, 103, 111, 118, 119, 120, 122, 126, 135, 136, 137, 138 (potential prodrug), 142, 144, 145, 148, 149, and 150. Preferably, the compound is selected from the group consisting of 54, 55, 56, 57, 58, 59, 60, 61, 62, 63, 64, 65, 66, 69, 82, 83, 89, 90, 93, 94, 95, 96, 103, 111, 118, 119, 120, 122, 126, 135, 136, 137, 138 (potential prodrug), 142, 144, 145, 148, 149, and 150.

In more preferred embodiments, the pharmaceutical composition comprises a compound selected from the group consisting of Compound 60, 66, 69, 103, 111, 118, 119, 120, 122, 136, 137, 138, 142, 144, 145, 148, 149, and 150. Preferably, the compound is selected from the group consisting of Compound 60, 66, 69, 103, 111, 118, 119, 120, 122, 136, 137, 138, 142, 144, 145, 148, 149, and 150.

In most particularly preferred embodiments, the pharmaceutical composition comprises a compound selected from the group consisting of Compound 60, 119, and 144.

Preferably, the compound is selected from the group consisting of 60, 119, and 144.

Structural modifications can be made to compounds such as 60 which do not add materially to the structure-activity relationships (SAR) illustrated here. For example, successful bioisosteric replacement or substitution of optionally substituted phenyl'groups, such as those occurring in compound 60, can be accomplished with other lipophilic or semi-polar aromatic (e.g., naphthyl, naphthoxy, benzyl, phenoxy, phenylthio), aliphatic (alkyl, e.g., isopropyl), cycloaliphatic (cycloalkyl, e.g., cyclohexyl), heterocyclic [e.g., pyridyl, furanyl, thiofuranyl (thiophenyl)], or other functional groups or systems, as is well known in the art, will afford clinically useful compounds (structural homologs, analogs, and/or congeners) with similar biopharmaceutical properties and activity at the NMDA receptor (e.g., *cf.* compounds 75, 79, 83, 89, 119-122, 125, 126, 128, 130, 132, 137, 144, and 145). For example, such replacements or substitutions have been used to great effect in the development of SAR among other groups of highly clinically and commercially successful synthetic pharmaceutical agents such as the classical H₁ -antihistamines, anticholinergics (antimuscarinics; e.g., anti-Parkinsonians), antidepressants (including tricyclic compounds), and opioid analgesics [See, Foye *et al*. (Eds.), *Principles of Medicinal Chemistry,* 4th ed., Lea and Febiger/Williams and Wilkins, Philadelphia, PA, 1995, pp. 233, 265, 281-282, 340-341, 418-427, and 430; Prous, J.R., *The Year's Drug News, Therapeutic Targets - 1995 Edition,* Prous Science Publishers, Barcelona, Spain, 1995, pp. 13, 55-56, 58-59, 74, 89, 144-145, 152, 296-297, and 317]. Similarly, bioisosteric replacement or substitution of the methylene or methine groups in the propyl backbone of compounds such as 60 with, e.g., oxygen, sulfur, or nitrogen, will afford clinically useful NMDA receptor-active compounds . with similarly useful biopharmaceutical properties, such as 88 (a modified "classical H₁-antihistamine-type" structure), which can be further optimized for activity at the NMDA receptor by preparing, e.g., the corresponding compound(s) containing, e.g., (bis) (3-fluorophenyl) group (s), as taught by the present invention. The propyl backbone of compounds such as **60** may also be modified successfully by the incorporation of ring systems (as in compounds **102** and **111**) and/or unsaturation (e.g., a double bond, as in compounds **81, 106, 109,** and **139**) to afford further clinically useful NMDA receptor-active compounds of the present invention (cf. compounds cited above).

The method for making a therapeutic agent may comprise the steps of screening for said agent by determining whether said agent is active on a receptor-operated calcium channel, and synthesizing said therapeutic agent in an amount sufficient to provide said agent in a therapeutically effective amount to a patient. Said screening may be performed by methods known to those of ordinary skill in the art, and may, for example be performed by the methods set out herein. Those skilled in the art are also familiar with methods used to synthesize therapeutic agents in amounts sufficient to be provided in a therapeutically effective amount.

In a preferred aspect, said receptor-operated calcium channel is an NMDA receptor. In a more preferred aspect, said method further comprises the step of adding a pharmaceucically acceptable carrier to said agent.

Other features and advantages of the invention will be apparent from the following description of the preferred embodiments thereof, and from the claims.

### Description of the Preferred Embodiments

### Assays for Potent and Selective Antagonists of Receptor-Operated Calcium Channels in the Mammalian CNS

Desired properties of a drug include: high affinity and selectivity for receptor-operated Ca²⁻ channels, such as those present in NMDA, AMPA and nicotinic cholinergic receptor-ionophore complexes (compared to responses mediated via other neurotransmitter receptors, neurotransmitter receptor-operated ion channels, or voltage-dependent ion channels) and a noncompetitive antagonism of said receptor-operated Ca²⁺ channels.

The MMDA receptor-ionophore complex is utilized as an example of a receptor-operated Ca²⁺ channel. Activation of the NMDA receptor opens a cation-selective channel that allows the influx of extracellular Ca²⁺ and Na⁺, resulting in increases in [Ca²⁺]ᵢ and depolarization of the cell membrane Measurements of [Ca²⁺]ᵢ were used as primary assays for detecting the activity of arylalkylamine compounds on NMDA receptors. Purified arylalkylamines, synthetic aryl-alkylamines, and synthetic analogs of arylalkylamines were examined for activity in in vitro assays capable of measuring glutamate receptor activity. simplified synthetic analogs generally consist of suitably substituted aromatic chromophoric groups attached to an alkyl(poly)amine moiety (see Compounds **54** through **69** below).

A primary assay that provides a functional index of glutamate receptor activity and that allows high-throughput screening was developed. Primary cultures of rat cerebellar granule cells loaded with the fluorimetric indicator fura-2 were used to measure changes in [Ca²⁺]ᵢ elicited by NMDA and its coagonist glycine. This assay provides an extremely sensitive and precise index of NMDA receptor activity. Increases in [Ca²⁺]ᵢ evoked by NMDA are dependent on the presence of glycine, and are blocked by extracellular Mg²⁺ or antagonists acting at the glutamate, glycine, or MK-801 binding sites. Increases in [Ca²⁺]ᵢ elicited by NMDA/glycine are readily distinguished from those resulting from depolarization by their refractoriness to inhibition by blockers of voltage-sensitive Ca²⁺ channels. The fidelity with which measurements of [Ca²⁺]₁ corroborate results obtained by electrophysiological and ligand-binding studies suggests that such measurements mirror closely activation of the NMDA receptor-ionophore complex.

### Example 1: Potent noncompetitive inhibition of NMDA receptor function

Preferential inhibitory effects of arylalkylamines on NMDA receptor-mediated increases in [Ca²⁺]ᵢ in cultured rat cerebellar granule cells were measured. Increases in [Ca²⁺]ᵢ were elicited by the addition of NMDA/glycine (50 µM/l µM) in the presence or absence of different concentrations of each test compound. The IC₅₀ values were derived for each test compound using from 2 to 8 separate experiments per test compound, and the standard error level was less than 10% of the mean value for each compound.

All of the arylalkylamines tested blocked increases in [Ca²⁺]ᵢ in cerebellar granule cells elicited by NMDA/glycine. Many of the arylalkylamines tested were more potent than competitive antagonists such as AP5 (IC₅₀ = 15 µM). The inhibitory effects of the arylalkylamines were not overcome by increasing the concentrations of NMDA or glycine. That is, no change was observed in the EC₅₀ for either NMDA or glycine. The arylalkylamines are thus noncompetitive antagonists at the NMDA receptor-ionophore complex, and act neither at the glutamate nor the glycine binding sites.

### Example 2: Activity against Kainate and AMPA receptor function

Measurements of [Ca²⁺]ᵢ in cerebellar granule cells can also be used to monitor activation of the native kainate or AMPA receptors present in this tissue. Although the increases in [Ca²⁺]ᵢ evoked by these agonists are of a lesser magnitude than those evoked by NMDA/glycine, such responses are robust and can be used to precisely assess the specificity of action of arylalkylamines on pharmacologically defined glutamate receptor subtypes. Comparative measurements of [Ca²⁺]ᵢ revealed a clear distinction in the receptor selectivity of the arylalkylamines. Some, like JSTX-3 (Joro Spider toxin from the spider *Nephila clavata*), were more potent antagonists of responses elicited by kainate (100 µM) or AMPA (30 µM).

### Neuroprotectant activity

Desired properties of a neuroprotectant drug include the following. (1) The drug can be administered by oral or injectable routes (*i.e.*, it is not significantly broken down in the stomach, intestine or vascular system and thus reaches the tissues to be treated in a therapeutically effective amount). Such drugs are easily tested in rodents to determine their bioavailability. (2) The drug exhibits neuroprotectant activity (*i.e.*, efficacy) when given after an ischemic insult (stroke, asphyxia) or traumatic injury (head trauma, spinal cord injury). (3) The drug is devoid of or has minimal side effects such as impairment of cognition, disruption of motor performance, sedation or hyperexcitability, neuronal vacuolization, cardiovascular activity, PCP-like abuse potential, or PCP-like psychotomimetic activity.

Although glutamate is the physiological synaptic transmitter, chronic exposure to glutamate leads to neuronal cell death. Much of the neurodegeneration caused by glutamate appears to be mediated by MKDA receptors and results directly from chronically elevated levels of cytosolic Ca²⁺. There is now extensive experimental support for the view that NMDA and AMPA receptors play a major role in mediating the neuronal degeneration following a stroke and other ischemic/hypoxic events (Choi, Glutamate neurotoxicity and diseases of the nervous system. *Neuron* **1**: 623, 1988). Most of this evidence is based on the ability of competitive or noncompetitive antagonists of the NMDA or AMPA receptor to effectively block neuronal cell death in both in vitro and *in vivo* models of stroke.

### Example 3: Cortical neuron protection

To assess the *in vitro* neuroprotectant effect of arylalkylamines, mouse cortical neurons grown in culture were exposed for 5 minutes to NMDA, and cell death after 24 hours was monitored by measuring the release of lactate dehydrogenase (LDH), a cytoplasmic enzyme that is released from dying cells (Choi *et al.,* Glutamate neurotoxicity in cortical cell culture. *J. Neurosci.* **7**: 357, 1987).

More rigorous testing to determine potential therapeutic efficacy involved *in vivo* stroke models. In these models, the blood supply is temporarily blocked by clamping the main arteries to the brain.

### Example 4: Bilateral carotid artery occlusion

The first assay was the bilateral common carotid artery occlusion model of forebrain ischemia performed in the gerbil (Karpiak *et al*., Animal models for the study of drugs in ischemic stroke. *Ann. Rev. Pharmacol. Toxicol.* **29**: 403, 1989; Ginsberg and Busto, Rodent models of cerebral ischemia. *Stroke* **20**: 1627, 1989). Blood flow to the brain was interrupted for 7 minutes by clamping the carotid arteries. The test compounds were administered as a single dose given intraperitoneally (i.p.) 30 minutes after reinstating blood flow. During the course of these experiments, the core body temperature of the animals was maintained at 37°C to prevent any hypothermia reaction. It has been shown that many NMDA receptor antagonists cause hypothermia and this effect can account for much of the protective effect of these compounds. The brains were examined for neuronal cell death 4 days later by silver staining sections of the brain and quantifying death by morphometric analysis.

### Example 5: Middle cerebral artery occlusion

The middle cerebral artery model of stroke performed in the rat (Karpiak *et al*., Animal models for the study of drugs in ischemic stroke. *Ann. Rev. Pharmacol. Toxicol.* **29**: 403, 1989; Ginsberg and Busto, Rodent models of cerebral ischemia. *Stroke* **20**: 1627, 1989) is different from the gerbil model because it results in a more restricted brain infarct, and thereby approximates a different kind of stroke (focal thrombotic stroke). In the first study using this stroke model, one cerebral artery was permanently occluded by surgical ligation. The test compounds were administered 30 minutes after the occlusion by a single intraperitoneal (i.p.) injection. During the course of these experiments, the core body temperature of the animals was maintained at 37°C to prevent any hypothermic reaction. Brains were assessed histologically for neuronal cell loss 24 hours later. Infarct volumes were calculated using the area of histological pallor from 10 slides and integrating the distance between each successive section.

In the second study of focal cerebral ischemia in the rat, the middle cerebral artery was permanently occluded by passing a small piece of suture thread through the carotid artery to the region of the middle cerebral artery. Core body temperature was maintained at 37°C.

In a third model of focal cerebral ischemia in the rat, an ischemic infarct was produced by a photothrombotic method using the dye Rose Bengal.

In a fourth model of focal cerebral ischemia in the rat, the middle cerebral artery was temporarily occluded by passing a small piece of suture thread through the carotid artery to the region of the middle cerebral artery. The suture thread was withdrawn after an ischemic period of 2 hr. Core body temperature was maintained at 37°C.

### Anticonvulsant activity

Desired properties of an anticonvulsant drug include: the drug can be administered by oral or injectable routes, the drug exhibits effective anticonvulsant activity against several seizure types, including, but not limited to, simple partial seizures, complex partial seizures; status epilepticus, and trauma-induced seizures such as occur following head injury, including head surgery; and the drug is devoid of or has minimal side effects such as impairment of cognition, disruption of motor performance, sedation or hyperexcitability, neuronal vacuolization, cardiovascular activity, PCP-like abuse potential, or PCP-like psychotomimetic activity.

Glutamate is the major excitatory transmitter in the brain, and thus may play at major role in seizure activity, and contribute to the pathogenesis of epilepsy. Much of the evidence favoring a major role for glutamate receptors in epilepsy derives from pharmacological studies demonstrating that glutamate receptor agonists elicit seizures, and that NMDA and AMPA receptor antagonists are effective anticonvulsants when administered *in vivo.* There are numerous *in vivo* models involving different kinds of seizures and behavioral effects that are relevant for clinically distinct forms of epilepsy. It is thus prudent to test for effects in several models, because it may be an oversimplification to suppose that the same mechanism underlies all forms of seizure activity.

### Example 6: Convulsant blocking activity

In initial studies, the ability of arylalkylamines to block seizures induced by kainate, picrotoxin or bicuculline were examined. Each of these convulsants acts through a different mechanism and seizures elicited by kainate are qualitatively different from those elicited by picrotoxin or bicuculline. In these experiments, a fraction of *Agelenopsis aperta* venom containing several arylalkylamine toxins was administered intravenously (iv) 5 min before picrotoxin or bicuculline, and 5 min after kainate administration. The arylalkylamines diminished the seizures induced by all three of these agents. The effects of picrotoxin or bicuculline were so severe that all 19 control animals died within 25 minutes. In contrast, there were no deaths in the 9 animals pretreated with the arylalkylamines. In fact, only about half the animals treated with the arylalkylamines showed any convulsions at all and those symptoms abated within an hour. These results demonstrate clear anticonvulsant effects of arylalkylamines and prompted further studies using purified' arylalkylamines and their analogs.

### Example 7: Seizure stimuli

Three different seizure-inducing test paradigms were used initially in this second group of studies and arylalkylamines proved to be effective anticonvulsants in two such paradigms. The first two models used DBA/2 mice which are prone to audiogenic seizures. Seizures were elicited by sound (bell tone at 109 dBs) or the intraperitoneal (ip) administration of NMDA (56 mg/kg). The test substances were administered 15-30 min before the convulsant stimulus. The number of clonic seizures was recorded for 1 min following the audiogenic stimulus or for 15 min following the administration of NMDA.

### Analgesic activity

Desired properties of an analgesic drug include: the drug can be administered by oral or injectable routes, the drug exhibits analgesic activity, the drug is devoid of or has minimal side effects such as impairment of cognition, disruption of motor performance, sedation or hyperexcitability, neuronal vacuolization, cardiovascular activity, PCP-like abuse potential, or PCP-like psychotomimetic activity.

Glutamate and NMDA receptor-mediated responses may play a role in certain kinds of pain perception (Dickenson, A cure for wind up: NMDA receptor antagonists as potential analgesics. *Trends Pharmacol. Sci.* **11**: 302, 1990).

### Example 8: Writhing response test

In the first series of experiments, the animals were administered an unpleasant stimulus (2-phenyl-1,4-benzoquinone, PBQ) which elicits a writhing response (abdominal stretching). Typically, the number of writhes occurring in a 5 min observation period is recorded. Classic analgesic drugs, such as morphine, are effective at decreasing the number of PBQ-elicited writhes (100% block of the writhing response at 4 mg/kg i.p.). Nonsteroidal antiinflammatory agents are likewise effective in this model.

### Example 9: Hot plate test

In this model of analgesic activity, mice were administered test substances s.c. 30 min before being placed on a hot plate (50°C). The time taken to lick the feet or jump off the plate is an index of analgesic activity, and effective analgesics increase the latency to licking or jumping. Morphine (5.6 mg/kg) increased the latency to jump by 765%.

### Example 10: Tail flick test

In this standard assay, the thermal nociceptive stimulus was 52°C warm water with the latency to tail flick or withdrawal taken as the endpoint.

### Example 11. Formalin test

Male Sprague-Dawley rats were habituated to an observation chamber for at least 1 hr before receiving an injection of dilute formalin (5%) in a volume of 50 µl into the left rear paw. Behavioral responses were monitored immediately after s.c. injection of formalin into the dorsal surface of the paw by counting the number of flinches exhibited by the animal. Behaviors were monitored for at least 50 min after formalin injection and'were recorded as early phase responses (0 - 10 min post-formalin) and late phase responses (20 - 50 min post-formalin). Compounds were injected intrathecally (i.th.) 10 min prior to formalin (pre-treatment) or 10 min after formalin (post-treatment) in a volume of 5 µl.

Intraplantal administration of formalin produced a typical biphasic response of flinching behavior, commonly described as the early and late phase responses.

Taken together, the results obtained with the hot plate, tail flick and formalin assays demonstrate that arylalkylamines have significant analgesic activity in several rodent models of acute pain. The formalin assay additionally demonstrates that arylalkylamines are effective in an animal model of chronic pain. Importantly, the arylalkylamines possess significant analgesic activity when administered after the formalin stimulus. This profile of activity -clearly distinguishes the arylalkylamines from standard NMDA receptcr antagonists such as MK-801.

### Side effects of arylalkylamines

Given the important role NMDA receptors play in diverse brain functions, it is not surprising to find that antagonists of this receptor are typically associated with certain unwelcome side effects. In fact, it is this property that provides the major obstacle to developing therapies that target NMDA receptors. The principal side effects, which characterize both competitive and noncompetitive antagonists, are a PCP-like psychotomimetic activity, impairment of motor performance, sedation or hyperexcitability, impairment of cognitive abilities, neuronal vacuolization, or cardiovascular effects (Willetts *et al.*, The behavioral pharmacology of NMDA receptor antagonists. *Trends Pharmacol. Sci.* **11**: 423, 1990; Oiney *et al*., Pathological changes induced in cerebrocortical neurons by phencyclidine and related drugs. *Science* **244**: 1360, 1989). The psychotomimetic effect associated with inhibition of NMDA receptor-mediated responses is epitomized in the response to phencyclidine (PCP) or "angel dust" which acts at the MK-801 binding site. Impairment of cognitive ability is associated with the important role that NMDA receptors normally play in learning and memory.

Relatively less is known concerning the side effect profile of AMPA receptor antagonists. However, it is becoming clear that such compounds also elicit motor impairment, ataxia and profound' sedation.

The activity of arylalkylamines was examined in animal models that index motor impairment, sedation and psychotomimetic activity as well as both *in vitro* and *in vivo* models of learning and memory.

### (a) PCP-like Psychotomimetic Activity

In rodents, both competitive and noncompetitive antagonists of the NMDA receptor produce a PCP-like stereotypic behavior characterized by hyperactivity, head-weaving, and ataxia (Willetts *et al.,* The behavioral pharmacology of MMDA receptor antagonists. *Trends Pharmacol. Sci.* **11**: 423, 1990; Snell and Johnson, In: *Excitatory* Amino *Acids in Health and Disease,* John Wiley & Sons, p. 261, 1988). We investigated whether the arylalkylamines would elicit such behaviors. In addition, we investigated whether the arylalkylamines would substitute for PCP in rats trained to discriminate PCP from saline (Willetts *et al.*, The behavioral pharmacology of MMDA receptor antagonists. *Trends Pharmacol. Sci*. **11**: 423, 1990), and whether the arylalkylamines would elicit a PCP-like neuronal vacuolization (Olney *et al.*, Pathological changes induced in cerebrocortical, neurons by phencyclidine and related drugs. *Science* **244**: 1360, 1989).

### Example 12: Locomotor activity

The first assay simply monitors locomoter activity during the first hour following peripheral (s.c. or i.p.) administration of test substance.

### Example 13: Motor impairment

In the first assay, generalized motor impairment, was examined in the inverted grid assay. In this assay, animals are placed on a wire-holed grid suspended from a rotating metal bar which can be inverted. The animals are then scored for their ability to climb to the top or hang on to the grid: Animals with severe motor impairment fall off the grid. This assay provides an index of "behavioral disruption" that may result from ataxia, loss of the righting reflex, sedation, or muscle relaxation.

The second assay of acute motor impairment was the rotorod assay. In this assay, Frings and CF1 mice were injected with test compound and placed on a knurled rod which rotated at a speed of 6 rpm. The ability of the mice to maintain equilibrium for long periods of time was determined; those mice that were unable to maintain equilibrium on the rotorod for 1 min in each of 3 trials were considered impaired.

### Example 14. PCP discrimination

In this assay, rats who have been trained to lever press for food reinforcement must select which of two levers in their cages is correct. The only stimulus they have for selecting the correct lever is their ability to detect whether they received a PCP or vehicle injection. After about two months of training, rats become very good at discriminating PCP from vehicle injections and can then be tested with other drugs to determine if they are discriminated as PCP. When tested in this procedure, other drugs which are known to produce a PCP-like intoxication substitute for PCP. These drugs include various PCP analogs such as ketamine and the noncompetitive NMDA receptor antagonist, MK-801.

### Example 15

The administration of compounds such as PCP and MK-801 to rats produces a neurotoxic effect termed neuronal vacuolization. Following a single dose of such compounds, vacuoles are found in particular central neurons, especially those in the cingulate cortex and retrosplenial cortex.

Taken together, the results on locomotor activity, motor impairment, PCP discrimination and neuronal vacuolization strongly suggest that arylalkylamines will be devoid of PCP-like side effects in man.

### (b) Cognitive impairment

One of the major reasons for postulating a role of NMDA receptors in memory and learning derives from cellular studies on long-term potentiation (LTP) in the rat hippocampus. LTP is a long-lasting increase in the magnitude of synaptic responses produced by brief yet incense synaptic stimulation. Since the discovery of this phenomenon, it has become the preeminent cellular model of learning in the vertebrate brain (Teyler and Discenna, Long-term potentiation. *Annu. Rev. Neurosci.* **10**: 131, 1987). Transmission at synapses formed by Schaffer collaterals onto CA1 pyramidal cells is mediated by NMDA and AMPA receptors. Following a brief tetanizing stimulus, the magnitude of the population spike (a measure of synaptic transmission) is greatly increased and remains so for hours. It has been shown that all known competitive and noncompetitive antagonists of NMDA recepcors block LTP in the rat hippocampus, whereas antagonists of non-NMDA receptors are without effect (Collingridge and Davis, In: *The NMDA Receptor*, IRL Press. p. 123, 1989). This supports a role of NMDA receptors in memory and learning.

### Example 16: LTP assay

The effects of selected arylalkylamines and literature standards were examined for effects on LTP in slices of rat hippocampus. As anticipated, all the conventional competitive (AP5 and AP7) and noncompetitive (MK-801 and ifenprodil) NMDA receptor antagonists inhibited the induction of LTP in the hippocampus. Slices of rat hippocampus were superfused for 30-60 min with a test compound before delivering a tetanizing stimulus consisting of 3 trains, separated by 500 msec, of 100 Hz for 1 sec each. The response amplitude was monitored for an additional 15 minutes post-tetanus. The tetanizing stimulus caused a mean 95% increase in the amplitude of the synaptic response. The induction of LTP was significantly blocked (p < 0.05) by competitive (AP5, AP7) or noncompetitive (MK-801, ifenprodil) NMDA receptor antagonists. Quite surprisingly, none of the arylalkylamines tested blocked the induction of LTP (p > 0.05), even when used at high concentrations (100-300 µM) that caused some inhibition of the control response.

These results highlight yet another unique and important feature of arylalkylamines. Arylalkylamines are the first, arid at present the only, class of compounds shown to be selective and potent antagonists of the NMDA receptor that do not block the induction of LTP. This likely reflects the novel mechanism and site of action of arylalkylamines and suggests that drugs which target the novel site on the NMDA receptor will similarly lack effects on LTP. As LTP is the primary cellular model for learning and memory in the mammalian CNS, it additionally suggests that such drugs will lack deleterious effects on cognitive performance.

### Example 17: Learning tests

In this test, rats were trained to alternate turning in a T maze for a food reward. MK-801 was included for comparison. Test compounds were administered i.p. 15 min before testing. Control animals made the correct choice about 80% of the time. Increasing doses of MK-801 progressively decreased the number of correct choices and this decrement in behavior was accompanied by hyperactivity.

Although MK-801 decreased learning performance in parallel with increases in locomotor activity, other studies using different paradigms in rodents and primates have shown a clear dissociation between the effects on learning and locomotion. Thus, both competitive and noncompetitive NMDA receptor antagonists impair learning at doses which do not cause any overt change in motor behavior. This demonstrates that conventional NMDA receptor antagonists impair learning independently of other side effects. The result's of the T-maze assay demonstrate that arylalkylamines, do not impair learning even at doses that cause some decrease in locomotor activity.

In a second series of experiments, the effect on learning in the Morris water maze task was evaluated. In this test, a hidden platform was placed in a fixed location in a circular steel tank, and submerged 2 cm below the surface of the water. Each rat was given 3 trials per day with a 10 min intertrial interval for 5 days. A trial was initiated by placing the rat in the water, nose facing the wall of the tank, at one of three predetermined starting locations. The order of the start location was varied daily. Learning was measured as a decrease in time required to swim to the platform. If an animal failed to locate the platform within 60 sec after the start of the trial, the rat was hand-guided to it. The animals remained on the platform for 10 sec before being removed from the tank. Ten min after the last training trial on day 5, the animals received a probe test. The platform was removed for this 1 trial task and the animals were allowed to swim for 60 sec to assess the spatial bias for the platform location. Two measures were recorded from this task: latency to first crossing the area where the platform had been, and total number of crossings.

The results of these learning tasks are encouraging. They suggest that arylalkylamines lack the learning and memory deficits that typify other NMDA receptor antagonists. In fact, there is a suggestion that the arylalkylamines may even be nootropic (memory enhancers).

### (c) Cardiovascular effects

*In vivo* studies with certain arylalkylamines revealed a hypotensive effect of these compounds, especially at high doses. On the basis of these results, a systematic study of the effects of arylalkylamines on cardiovascular function was performed.

### Example 18: Ca²⁺ channel inhibition

We have discovered that some of the arylalkylamines are quite potent inhibitors of voltage-sensitive Ca²⁺ channels, specifically those sensitive to inhibition by dihydropyridines (L-type channels). Such effects on vascular smooth muscle would be expected to dilate blood vessels and cause a drop in blood pressure, thus producing hypotension.

The ability of arylalkylamines to inhibit dihydropyridine-sensitive Ca²⁺ channels was examined in cerebellar granule cells and a rat aortic smooth muscle cell line, A₇r5 cells. Overall, we have observed a wide range of potencies against voltage-sensitive Ca²⁺ channels that does not correlate with potency against NMDA receptors. This strongly suggests that further structure-activity work based on chemical modification of the arylalkylamine molecule will lead to the development of compounds that are very potent NMDA antagonists with low potency against voltage-sensitive Ca²⁺channels.

Arylalkylamines are not, however, indiscriminate blockers of voltage-sensitive Ca²⁺ channels. They do not, for example, affect voltage-sensitive Ca²⁺ channels in cerebellar Purkinje cells (P-type channels), or those channels thought to be involved in neurotransmitter release (N-channels). The arylalkylamines that do block voltage-sensitive Ca²⁺ channels appear to target specifically L-type Ca²⁺ channels. Moreover, as mentioned above, there is a high degree of structural specificity in this effect. For example, one arylalkylamine is 57 times more potent than another arylalkylamine in blocking Ca²⁺ influx through L-type channels, where the only structural difference between the compounds is the presence or absence of a hydroxyl group.

### Example 19: In vivo cardiovascular studies

On the basis of these studies, it is anticipated that chemical efforts will minimize the cardiovascular side effects by (1) enhancing the uptake of arylalkylamine into the brain such that lower doses are required for neuroprocection, and (2) increasing the selectivity (potency ratio) of arylalkylamines for receptor-operated Ca²⁺ channels over voltage-sensitive Ca²⁺ channels.

### Example 20: Biological activity of Compound 54 and analogs

Compounds **54** - **139** had high potencies against NMDA-induced increases in [Ca²⁺]₁ in rat cerebellar granule cells grown in culture (Table 1). Compounds **54** - **147** inhibited [³H]MK-801 binding in membranes prepared from rat hippocampal and cortical tissue (Table 1).

Compound **57** possessed the following additional biological activities: significant anticonvulsant activity against sound-induced seizures in a genetically susceptible mouse model of reflex epilepsy (Frings mice) following i.p. administration (ED₅₀ = 1 mg/kg and TD₅₀ (motor impairment) between 6 and 8 mg/kg).

Compound **58** possessed the following additional biological activities: significant anticonvulsant activity against sound-induced seizures in a genetically susceptible mouse model of reflex epilepsy (Frings mice) following i.p. administration (ED₅₀ = 0.9 mg/kg and TD₅₀ (motor impairment) = 14.5 mg/kg); no significant neuroprotectant activity in a rat model of focal ischemic stroke following i.p. administration of 2 mg/kg 30 min prior to vessel occlusion and 2 mg/kg 3 hr post-occlusion; and no significant cardiovascular activity in anesthetized rats at doses up to 2 mg/kg i.v.

Compound **59** possessed the following additional biological activities: significant anticonvulsant activity against sound-induced seizures in a genetically susceptible mouse model of reflex epilepsy (Frings mice) following i.p. administration (ED₅₀ = 2.7 mg/kg and TD₅₀ (motor impairment) = 7.8 mg/kg); a reduction in seizure threshold as indexed by the i.v. Metrazol test in mice at the dose of. 11.7 mg/kg i.p.; no significant neuroprotectant activity in a rat model of focal ischemic stroke following i.p. administration of 2 mg/kg 30 min prior to vessel occlusion and 2 mg/kg 3 hr pose-occlusion; and no significant cardiovascular activity in anesthetized rats at doses up to 10 mg kg i.v.

Compound **60** possessed the following additional biological activities: significant anticonvulsant activity against sound-induced seizures in a genetically susceptible mouse model of reflex epilepsy (Frings mice) following i.p. administration (ED₅₀ = 4.4 mg/kg and TD₅₀ (motor impairment) = 9.2 mg/kg); significant anticonvulsant activity against sound-induced seizures in a genetically susceptible mouse model of reflex epilepsy (Frings mice) following oral administration (ED₅₀ = 10 mg/kg and TD₅₀ (motor impairment) = 25.6 mg/kg); significant anticonvulsant activity against maximal electroshock-induced seizures in mice following i.p. administration (ED₅₀ = 8.17 mg/kg and TD₅₀ (rotorod) = 17.30 mg/kg); no effect on seizure threshold as indexed by the i.v. Metrazol test in mice at the doses of 1 and 4 mg/kg i.p.; a reduction in seizure threshold as indexed by the i.v. Metrazol test in mice at the doses of 8 and 17 mg/kg i.p.; significant neuroprotectant activity in a rat model of temporary focal ischemic stroke following i.p. administration of 2 mg/kg 30 min prior to vessel occlusion and 2 mg/kg 3 hr post-occlusion; significant neuroprotectant activity in a rat model of temporary focal ischemic stroke following i.p. or i.v. administration of 1 mg/kg 2 hr and again 8 hr post-occlusion; significant neuroprotectant activity in a rat model of temporary focal ischemic stroke following i.v. administration of 1 mg/kg 2 hr post-occlusion; no significant neuroprotectant activity in a rat photothrombotic model of focal ischemia following the administration of 10 mg/kg i.p. at 15 min, 3 hr, and again 6 hr post-occlusion; no neuronal vacuolization when administered at doses of 20 mg/kg i.p. or 10 mg/kg i.v.; no significant cardiovascular activity in conscious beagle dogs at the dose of 0.3 mg/kg i.v. (60 sec bolus injection); transient increases in mean arterial pressure in conscious beagle dogs at the doses of 1 and 3 mg/kg i.v., with larger magnitude and longer duration effects seen at the dose of 10 mg/kg i.v. (60 sec bolus injection); transient increases in heart rate in conscious beagle dogs at the doses of 3 and 10 mg/kg i.v. (60 sec bolus injection); no significant changes in the ECG in conscious beagle dogs at doses ranging from 0.3 to 10 mg/kg i.v. (60 sec bolus injection); no significant behavioral effects in conscious beagle dogs at the doses of 0.3 and 1 mg/kg i.v. (60 sec bolus injection); a slight increase in respiratory rate in conscious beagle dogs at the dose of 3 mg/kg i.v. (60 sec bolus injection); dilated pupils, whole body tremors, salivation, and urination in conscious beagle dogs at the dose of 10 mg/kg i.v. (60 sec bolus injection); no significant behavioral effects in conscious male Wistar rats at doses up to 4 mg/kg i.v.; excitation, stereotypies, increased reactivity to touch, increased muscle tone, and tremor in conscious male Wistar rats at the dose of 8 mg/kg i.v.; Straub tail, convulsions, and death in conscious male Wistar rats at the dose of 16 mg/kg i.v.

Taken together, the results obtained with these simplified synthetic arylalkylamines suggest that such simplified molecules do not interact specifically with the arylalkylamine binding site on receptor-operated Ca²⁺ channels as do Compounds 1, 2 and 3. Specifically, Compounds **54** - **147** bind to the site labeled by [³H]MK-801 at concentrations ranging approximately 1 to 400-fold higher than those which antagonize the function of the MMDA receptor-ionophore complex. The fact that Compounds **54** - **147** at therapeutic doses do not generally produce PCP-like stereotypic behavior, substitute for PCP in drug discrimination assays, or elicit neuronal vacuolization suggests, however, that such compounds might be useful either as lead compounds or drug candidates for neurological disorders and diseases. It has been reported that compounds which bind with low affinity (relative to MK-801) to the site labeled by [³H]MK-801 might possess therapeutic utility and possess a more favorable side effect profile than that possessed by a high affinity antagonise such as MK-801 itself (Rogawski, Therapeutic potential of excitatory amino acid antagonists: channel blockers and 2,3-benzodiazepines. *Trends Pharmacol. Sci.* **14**: 325, 1993). The low affinity of certain compounds within the group of Compounds **54** - **147** (relative to MK-801) for the site labeled by [³H]MK-801 may place these compounds into this general class of low affinity noncompetitive antagonists.

### Identification of a novel modulatory site on receptor-operated calcium channels

Having identified arylalkylamines which have therapeutically useful properties as defined above, compounds can now be identified which act at the critical arylalkylamine binding site on receptor-operated Ca²⁺ channels, such as those present within NMDA, AMPA and nicotinic cholinergic receptor-ionophore complexes.

Examples of suitable tests now follow:

### Example 21: Radioligand binding in rat cortex or cerebellum.

The following assay can be utilized as a high throughput assay to screen product libraries (e.g., natural product libraries and compound files at major pharmaceutical companies) to identify new classes of compounds with activity at this unique arylalkylamine site. These new classes of compounds are then utilized as chemical lead structures for a drug development program targeting the arylalkylamine binding site on receptor-operated Ca²⁺ channels. The compounds identified by this assay offer a novel therapeutic approach to treatment of neurological disorders or diseases. Examples of such compounds include those provided in the generic chemical formulae above. Routine experiments can be performed to identify those compounds having the desired activities.

Rat brain membranes are prepared according to the method of Williams *et al.* (Effects of polyamines on the binding of [³H]MK-801 to the NMDA receptor: Pharmacological evidence for the existence of a polyamine recognition site. *Molec. Pharmacol.* **36**: 575, 1989) with the following alterations: Male Sprague-Dawley rats (Harlan Laboratories) weighing 100-200 g are sacrificed by decapitation. The cortex or cerebellum from 20 rats are cleaned and dissected. The resulting brain tissue is homogenized at 4°C with a polytron homogenizer at the lowest setting in 300 ml 0.32 M sucrose containing 5 mM K-EDTA (pH 7.0). The homogenate is centrifuged for 10 min at 1,000 x *g* and the supernatant removed and centrifuged at 30,000 x *g* for 30 minutes. The resulting pellet is resuspended in 250 ml 5 mM K-EDTA (pH 7.0) stirred on ice for 15 min, and then centrifuged at 30,000 x g for 30 minutes. The pellet is resuspended in 300 ml 5 mM K-EDTA (pH 7.0) and incubated at 32°C for 30 min. The suspension is then centrifuged at 100,000 x *g* for 30 min. Membranes are washed by resuspension in 500 ml 5 mM K-EDTA (pH 7.0), incubated at 32°C for 30 min, and centrifuged at 100,000 x *g* for 30 minutes. The wash procedure, including the 30 min incubation, is repeated. The final pellet is resuspended in 60 ml 5 mM K-EDTA (pH 7.0) and stored in aliquots at -80°C. The extensive washing procedure utilized in this assay was designed in an effort to minimize the concentrations of glutamate and glycine (co-agonists at the NMDA receptor-ionophore complex) present in the membrane preparation.

To perform a binding assay with [³H]arylalkylamine, aliquots of SPMs (Synaptic Plasma Membranes) are thawed, resuspended in 30 mls of 30 mM EPPS/1mM K-EDTA, pH 7.0, and centrifuged at 100,000 x *g* for 30 minutes. SPMs are resuspended in buffer A (30 mM EPPS/l mM K-EDTA, pH 7.0). The [³H]arylalkylamine is added to this reaction mixture. Binding assays are carried out in polypropylene test tubes. The final incubation volume is 500 µl. Nonspecific binding is determined in the presence of 100 µM nonradioactive arylalkylamine. Duplicate samples are incubated at 0°C for 1 hour. Assays are terminated by the addition of 3 ml of ice-cold buffer A, followed by filtration over glass-fiber filters (Schleicher & Schuell No. 30) that are presoaked in 0.33% polyethyleneimine (PEI). The filters are washed with another 3 x 3 ml of buffer A, and radioactivity is determined by scintillation counting at an efficiency of 35-40% for ³H.

In order to validate the above assay, the following experiments are also performed:
(a) The amount of nonspecific binding of the [³H]arylalkylamine to the filters is determined by passing 500 µl of buffer A containing various concentrations of [³H]arylalkylamine through the presoaked glass-fiber filters. The filters are washed with another 4 x 3 ml of buffer A, and radioactivity bound to the filters is determined by scintillation counting at an efficiency of 35-40% for ³H. In filters that are not pretreated with 0.33% PEI, it was found that 87% of the ³H-ligand was bound to the filter. Presoaking with 0.33% PEI reduces the nonspecific binding to 0.5 - 1.0% of the total ligand added.
(b) A saturation curve is constructed by resuspending SPMs in buffer A. The assay buffer (500 µl) contains 60 µg of protein. Concentrations of [³H]arylalkylamine are used, ranging from 1.0 nM to 400 µM in half-log units. A saturation curve is constructed from the data, and an apparent K_{D} value and Bₘₐₓ value determined by Scatchard analysis (Scatchard, The attractions of proteins for small molecules and ions. Ann. *N.Y. Acad. Sci.* **51**: 660, 1949). The cooperativity of binding of the [³H]arylalkylamine is determined by the construction of a Hill plot (Hill, A new mathematical treatment of changes of ionic concentrations in muscle and nerve under the action of electric currents, with a theory to their mode of excitation. J. *Physiol.* **40**: 190, 1910).
(c) The dependence of binding on protein (receptor) concentration is determined by resuspending SPMs in buffer A. The assay buffer (500 µl) contains a concentration of [³H]arylalkylamine equal to its K_{D} value and increasing concentrations of protein. The specific binding of [³H]arylalkylamine should be linearly related to the amount of protein (receptor) present.
(d) The time course of ligand-receptor binding is determined by resuspending SPMs in buffer A. The assay buffer (500 µl) contains a concentration of [³H]arylalkylamine equal to its K_{D} value and 100 µg of protein. Duplicate samples are incubated at 0°C for varying lengths of time; the time at which equilibrium is reached is determined, and this time point is routinely used in all subsequent assays.
(e) The pharmacology of the binding site can be analyzed by competition experiments. In such experiments, the concentration of [³H]arylalkylamine and the amount of protein are kept constant, while the concentration of test (competing) drug is varied. This assay allows for the determination of an IC₅₀ and an apparent K_{D} for the competing drug (Cheng and Prusoff, Relationship between the inhibition constant (K₁) and the concentration of inhibitor which causes 50 percent inhibition (IC₅₀) of an enzymatic reaction. *J. Biochem. Pharmacol.* **22**: 3099, 1973). The cooperativity of binding of the competing drug is determined by Hill plot analysis.
   Specific binding of the [³H]arylalkylamine represents binding to a novel site on receptor-operated Ca²⁻channels such as those present within NMDA-, AMPA- and nicotinic cholinergic receptor-ionophore complexes. As such, other arylalkylamines should compete with the binding of [³H]arylalkylamine in a competitive fashion, and their potencies in this assay should correlate with their inhibitory potencies in a functional assay of receptor-operated Ca²⁺ channel antagonism (*e.g.*, inhibition of NMDA receptor-induced increases in [Ca²⁺]₁ in cultures of rat cerebellar granule cells). Conversely, compounds which have activity at the other known sites on receptor-operated Ca²⁺ channels (*e.g*., MK-801, Mg²⁺, polyamines) should not displace [³H]arylalkylamine binding in a competitive manner. Rather, complex allosteric modulation of [³H]arylalkylamine binding, indicative of noncompetitive interactions, might be expected to occur. In preliminary experiments, MK-801 did not displace [³H]arylalykylamine binding at concentrations up to 100 µM.
(f) Studies to estimate the dissociation kinetics are performed by measuring the binding of [³H]arylalkylamine after it is allowed to come to equilibrium (see (d) above), and a large excess of nonradioactive competing drug is added to the reaction mixture. Binding of the [³H]arylalkylamine is then assayed at various time intervals. With this assay, the association and dissociation rates of binding of the [³H]arylalkylamine are determined (Titeler, Multiple *Dopamine Receptors: Receptor Binding* Studies *in Dopamine Pharmacology*. Marcel Dekker, Inc., New York, 1983). Additional experiments involve varying the reaction temperature (0°C to 37°C) in order to understand the temperature dependence of this parameter.

### Example 22: Radioligand binding in cerebellar granule cells

Primary cultures of cerebeiiar granule neurons are obtained from 8-day-old rats and plated onto Squares of Aclar plastic coated with poly-L-lysine. The plastic squares are placed in 24-well culture plates, and approximately 7.5 X 10⁵ granule cells are added to each well. Cultures are maintained in Eagles' medium (HyClone Laboratories) containing 25 mM KCl, 10% fetal calf serum (HyClone Laboratories), 2 mM glucamine, 100 µg/ml gentamicin, 50 U/ml penicillin, and 50 µg/ml streptomycin at 37°C in a humid atmosphere of 5% CO₂ in air for 24 hr before the addition of cytosine arabinoside (10 µM, final). No changes of culture medium are made until the cells are used for receptor binding studies 6-8 days after plating.

To perform a binding assay with [³H]arylalkylamine, the reaction mixture consists of 200 µl of buffer A (20 mM K-HEPES, 1 mM K-EDTA, pH 7.0) in each well of the 24-well plate. The [³H]arylalkylamine is added to this reaction mixture. Nonspecific binding is determined in the presence of 100 µM nonradioactive arylalkylamine. Triplicate samples are incubated at 0°C for 1 hour. Assays are terminated by manually scraping the cells off the Aclar squares and placing them into polypropylene test tubes. The membranes prepared from whole cells in this manner are suspended in 10 ml of ice-cold buffer A, and filtered over glass-fiber filters (Schleicher & Schuell No. 30) that are presoaked in 0.33% PEI. The filters are washed with another 3 x 3 ml of buffer A, and radioactivity on the filters is determined by scintillation counting at an efficiency of 35-40% for ³H. The assay may be terminated by centrifugation rather than filtration in order to minimize nonspecific binding.

Specific experiments to characterize and validate the assay are performed essentially as above, except that cells are used in place of membranes for the initial binding. The binding assay allows for the determination of an IC₅₀, value and an apparent K_{D} for the competing drug as described by Scatchard analysis (The attractions of proteins for small molecules and ions. *Ann. N.Y. Acad. Sci.* **51**: 660, 1949). Cooperativity of binding of the competing drug is determined by Hill plot analysis (A new mathematical treatment of changes of ionic concentrations in muscle and nerve under the action of electric currents, with a theory .to their mode of excitation. *J. Physiol.* **40:** 190, 1910). The specific binding of the [³H]arylalkylamine represents binding, to a novel site on receptor-operated calcium channels.

### Example 23: Patch-clamp electrophysiology assay

The following assay is performed for selected compounds identified in the above-mentioned radioligand binding assays as interacting in a highly potent and competitive fashion at the novel arylalkylamine binding site on receptor-operated Ca²⁺ channels, such as those present in NMDA-, AMPA- or nicotinic cholinergic receptor-ionophore complexes. This patch-clamp assay provides additional relevant data about the site and mechanism of action of said previously selected compounds. Specifically, the following pharmacological and physiological properties of the compounds interacting at the arylalkylamine binding site are determined, utilizing the NMDA receptor-ionophore complex as an example of receptor-operated Ca²⁺ channels: potency and efficacy at blocking MMDA receptor-mediated ionic currents, the noncompetitive nature of block with respect to glutamate and glycine, use-dependence of action, voltage-dependence of action, both with respect to onset and reversal of blocking, the kinetics of blocking and unblocking (reversal), and open-channel mechanism of blocking. Such data confirm that the compounds interacting at the arylalkylamine binding site retain the unique biological profile of the arylalkylamines, and do not have their primary activity at the known sites on the NMDA receptor-ionophore complex (glutamate binding site, glycine binding site, MK-801 binding site, Mg²⁺ binding site, Zn²⁺ binding site, sigma binding site, polyamine binding site).

Patch-clamp recordings of mammalian neurons (hippocampal, cortical, cerebellar granule cells) are carried out utilizing standard procedures (Donevan *et al.,* Arcaine blocks N-methyl-D-aspartate receptor responses by an open channel mechanism: whole-cell and single-channel recording studies in cultured hippocampal neurons. *Molec. Pharmacol.* **41**: 727, 1992; Rock and Macdonald, Spermine and related polyamines produce a voltage-dependent reduction of NMDA receptor single-channel conductance. *Molec. Pharmacol.* **42**: 157, 1992).

Alternatively, patch-clamp experiments can be performed on *Xenopus* oocytes or on a stably transfected mammalian cell line (*e.g*., HEK 293 cells) expressing specific subunits of receptor-operated Ca²⁺ channels. In this manner, for example, potency and efficacy at various glutamate receptor subtypes (*e.g.*, NMDAR1, NMDAR2A through NMDAR2D, GluR1 through GluR4) can be determined. Further information regarding the site of action of the arylalkylamines on these glutamate receptor subtypes can be obtained by using site-directed mutagenesis.

### Example 24: Synthesis of simplified arylalkylamines

Synthesis of Compound **20** (Reference Compound) was accomplished as follows.

A solution of sodium hydride (1.21 g, 50 mmol) in dimethoxyethane was treated with diethyl cyanomethylphosphonate (8.86 g, 50 mmol) and the reaction stirred 4 hr at room temperature. To this was added 3,3'-difluorobenzophenone (10 g, 46 mmol) in DME. The reaction was stirred 24 hr at room temperature, quenched with H₂O, and partitioned between diethyl ether and water. The ether fraction was dried over Na₂SO₄ and concentrated. GC/MS of this material showed 90% of the product A and 10% starting benzophenone.

A solution of this material in ethanol with a catalytic amount of Pd(OH)₂ was hydrogenated at 55 psi hydrogen for 4 hr at room temperature. The reaction was filtered and the catalyst washed with ethanol (3x). The filtrate and ethanol washes were combined and concentrated. GC/MS of this material showed 90% of the product B and 10% of the starting benzophenone.

A solution of this material in THF was treated with. 70 ml 1 M B₂H₆ (70 mmol) in THF and refluxed 1 hr. After cooling the reaction was treated with 6 N HCl (50 ml) and refluxed an additional hour. After cooling the reaction was basified to pH 14 with 10 N NaOH and equilibrated with ether. The ether layer was removed and washed with 10% HCl (3x). The acidic washes were combined, basified to pH 14 with 10 N NaOH and extracted with dichloromethane (3x). The organic washes were combined, dried over Na₂SO₄, and concentrated to yield an oil. GC/MS of this material showed 100% Compound **20**. GC/EI-MS (Rₜ =7.11 min) m/z (relative intensity) **247** (M⁺, 31), **230** (100), **215** (30), **201** (52), **183** (63), **134** (23), **121** (16), **101** (21), **95** (15), **77** (15). This material in diethyl ether was filtered and treated with 35 ml 1 M HCl in ether. The precipitate was collected, dried, and recrystallized from water-ethanol to afford 1.045 g of Compound **20**, as the hydrochloride salt. ¹H-NMR (CDCl₃) d 8.28 (3H, br s), 7.28-7.17 (2 H, m), 7.02-6.86 (6 H, m), 4.11 (1H). t, J=8 Hz), 2.89 (2H, br t, J=8 Hz), 2.48 (2H, br t, J=7 Hz); ¹³C-NMR (CDCl₃) d 164.6, 161.3, 144.8, 144.7, 130.4, 130.3, 123.3, 123.2, 114.7, 114.5, 114.1, 113.8, 47.4, 38.4, 32.7.

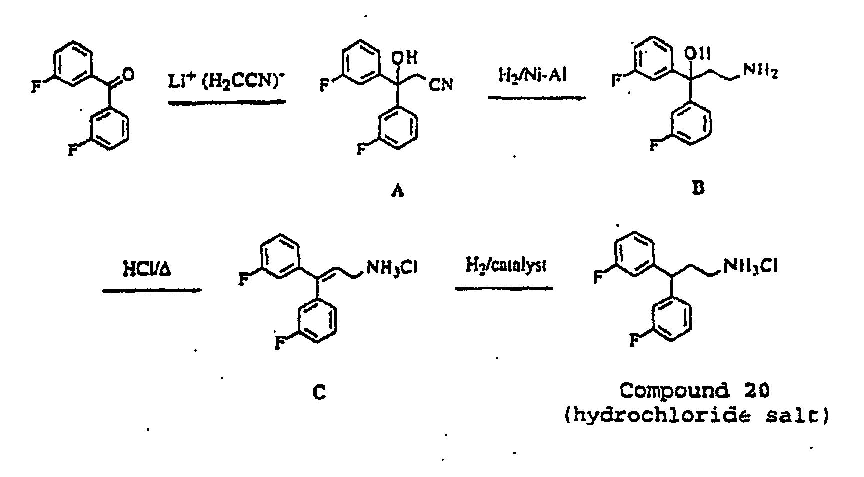

Synthesis of Compound **21**, Compound **33** and Reference Compounds was accomplished as follows.

A 100 ml round-bottomed flask equipped with stir bar, septa, and argon source was charged with compound **1** (2.43 g, 10 mmol) in 30 ml THF. The solution was cooled to -78°C and treated dropwise with 11 ml lithium bis(trimethylsilyl)amide (1M in THF) (11 mmol). The reaction was stirred at -78°C for 30 min and created dropwise with excess iodomethane (3.1 ml, 50 mmol). The reaction was stirred 30 min at -58°C. GC/EI-MS analysis of an aliquot from the reaction showed consumption of the starting nitrile **1**. The reaction was quenched with water, diluted with diethyl ether and transferred to a separatory funnel. The ether layer was washed with 10% HCl (3X), brine (1X), dried with anhydrous MgSO₄, and concentrated to a brown oil. This material was distilled (Kugelrohr, 100°C) at reduced pressure to afford 1.5 g of a clear oil. GC/EI-MS of this material showed it to contain the desired product **2** , (Rₜ=7.35 min) *m*/*z* (rel. int.) 257 (M⁺, 3), 203 (100), 183 (59), 170 (5), 133 (4), 109 (3) ; ¹H-NMR (CDCl₃) d 7.4-6.9 (8H, m), 4.01 (1H, d, J=10 Hz), 3.38 (1H, dq, J=7, 10 Hz), 1.32 (3H, d, J=7 Hz); ¹³C-NMR (CDCl₃) d 19.4, 30.5, 54.2, 114.5, 114.6, 114.7, 114.9, 115.0, 115.3, 123.3, 123.4, 123.6, 123.7, 130.5, 130.6, 131.7.

Product **3** was synthesized by the catalytic reduction of **2** using Raney nickel in 95:5 EtOH:aqueous sodium hydroxide (2 Eq.) under 60 psi hydrogen. GC/EI-MS (Rₜ=7.25 min) *m*/*z* (rel. int.) 261 (M', 20), 244 (35), 229 (16), 215 (17), 201 (80), 183 (100), 133 (42), 115 (27), 109 (47), 95 (20); ¹H-NMR (CDCl₃) d 7.3-6.8 (8H, m), 3.62 (1H, d, J=10 Hz), 2.70 (1H, M), 2.40 (2H, m), 1.73 (2H, m), 0.91 (3H, d, J=7 Hz). Note that product **3** in this reaction sequence corresponds to Compound **21**.

Product **2** in 10% IPA-hexane (100 mg/ml was chromatographed, in 500 µl aliquots, through Chiral Cel OD (2.0 x 25 cm) using 10% IPA-hexane at 10 ml/min measuring optical density at 254 nm. This afforded the two optically pure enantiomers **4** and **5** (as determined by analytical chiral HPLC; Note, the stereochemistry of these two compounds has not been assigned at this time). These two compounds were identical in their GC/EI-MS and ¹H-NMR spectra as product **2** (data above)

Each of the enantiomers **4** and **5** was reduced separately using dimethyl sulfideborane complex in the following manner. A solution of compound (**4** or **5** ) in THF was heated to reflux and treated with excess (2 Eq.) 1M (in THF). dimethyl sulfideborane complex and the reaction refluxed 30 min. After this time the reaction was cooled to 0°C and treated with 6 N HCl. The reaction was set to reflux for 30 min. After this time the reaction was transferred to a separatory funnel, basified to pH > 12 with 10N NaOH; and the product (**6**) extracted into ether. The ether layer was washed with brine, dried over anhydrous MgSO₄ and concentrated to an oil. The product was purified by prep-TLC using 5% methanol-chlorform. Each of the individual enantiomers (**6** and **7**) were found to be identical in their GC/EI-MS and ¹H-NMR spectra as product **3** (data above). Note that product **6** in this scheme corresponds to Compound 33 . Compound 33-HCl: mp = 260-270°C (dec), [a]₃₆₉26 = +6.6 (c 1.0 in EtOH), [a]_{D}26 = +0.4 (c 1.0 in EtOH). Compound 33·HI: The free base of Compound 33 was dissolved in EtOH and 47% hydriodic acid (1.1 equvt.) was added. The solvent was evaporated under vacuum and the resulting solid hydroiodide was recrystallized twice from heptane/EtOAc by slow evaporation: mp = 195-197°C. The absolute configuration of compound 33·HI was determined to be R by single crystal (monoclinic colorless needle, 0.50 x 0.05 x 0.03 mm) X-ray diffraction analysis using a Siemens R3*m*/*V* diffractometer (3887 observed reflections).

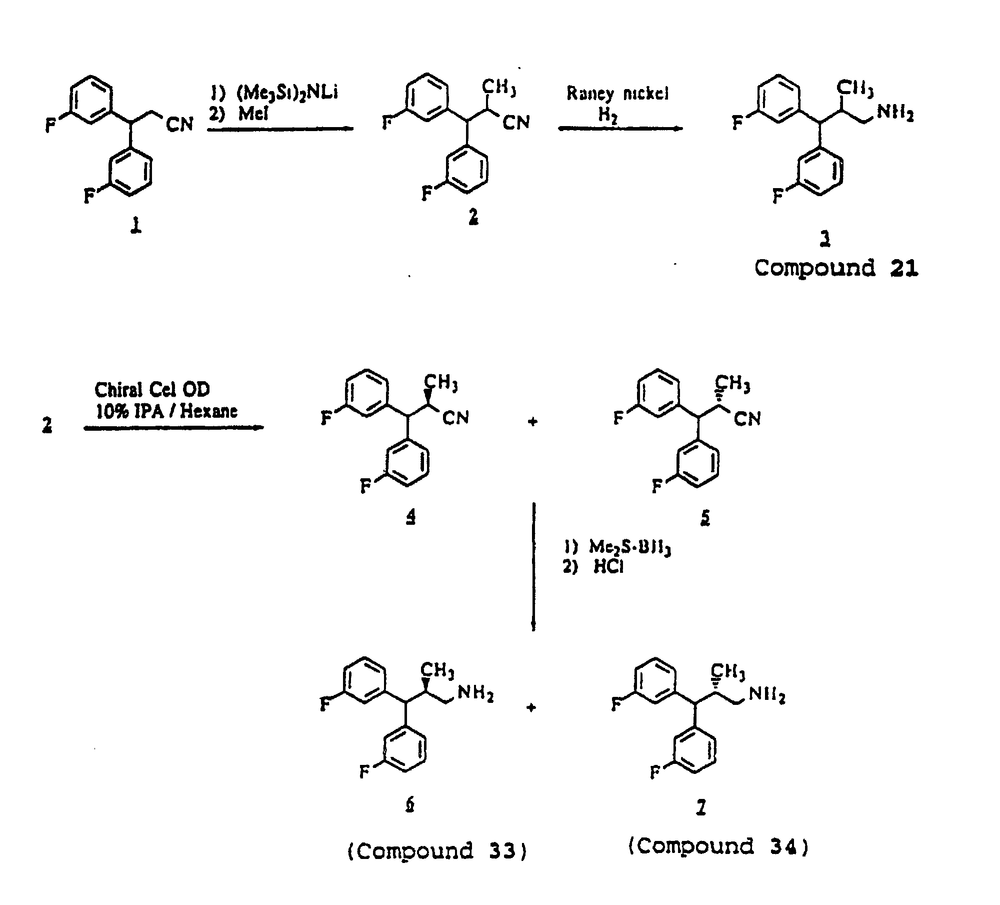

Synthesis of Compound **24** (Reference Compound) was accomplished as described below. Compounds **25** (Reference Compound) **65, 76-78, 83, 90, 96-97, 115,** and **135-136** were prepared in a similar manner.

A suspension of magnesium turnings (0.95 g, 39.2 mmol) in 150 ml anhydrous diethyl ether was treated with 1-bromo-3-fluorobenzene (6.83 g, 39.2 mmol) dropwise via syringe. After 1.5 hr the solution was transfered via cannula to a flask containing o-anisaldehyde (5.0 g, 36.7 mmol) in 100 ml anhydrous diethyl ether at 0°C and stirred 2hr. The reaction mixture was quenched with water and partitioned between water and ether. The combined organic layers were washed with brine and dried over anhydrous magnesium sulfate to afford 7.90g (93% yield) of product **A**.

Pyridinium dichromate (16.0 g, 42.5 mmol) was added to a solution of the alcohol **A** (7.90 g, 34.0 mmol) in dichloromethane (100 ml), and the reaction was stirred 12 hr. Diethyl ether (300 ml) was added to the reaction mixture and the black solution was filtered through a silica gel plug (30 cm) and washed with an additional 500 ml ether. After evaporation of the solvent *in vacuo*, the solid was recrystallized from acetone to give 7.45 g (95% yield) of product **B**.

Diethyl cyanomethylphosphonate (7.0 g, 39.5 mmol) was slowly added to a suspension of sodium hydride (1.58 g, 39.5 mmol) in 100 ml N,N-dimethylformamide. After 30 minutes the ketone **B** was added to the solution and stirred an additional 2 hr. The reaction mixture was quenches with water, and partitioned between water and ether. The combined organic layers were washed with brine and dried over anhydrous magnesium sulfate. The solvent was evaporated *in vacuo* to give a pale yellow oil.

In a glass bomb, the oil was dissolved in 100 ml ethanol and 20 ml 10N NaOH. A catalytic amount of Raney Nickel suspened in water (ca. 15 mol percent) was added to the solution. The reaction mixture was shaken under 60 psi H₂ for 12 hr on a Parr Hydrogenator. After filtering off excess Raney Nickel, the solution was extracted with chloroform. The combined organic layers were washed with brine and dried over anhydrous magnesium sulfate. After filtration, the oil was run through a silica gel column in chloroform and methanol. The solvent was evaporated *in vacuo* to give a pale yellow oil. GC/EI-MS (Rₜ=8.10 min) *m*/*z* (rel. intensity) **259** (100), **242** (44), **213** (48), **183** (42), **136** (50), **109** (94), **91** (60), **77** (25). The oil was then acidified with hydrogen chloride in diethyl ether. Evaporation of the ether afforded a pale yellow solid that was recrystallized in hot acetonitrile to afford 3.45 g (42.1% yield) white needles of Compound **24**, as the hydrochloride salt.

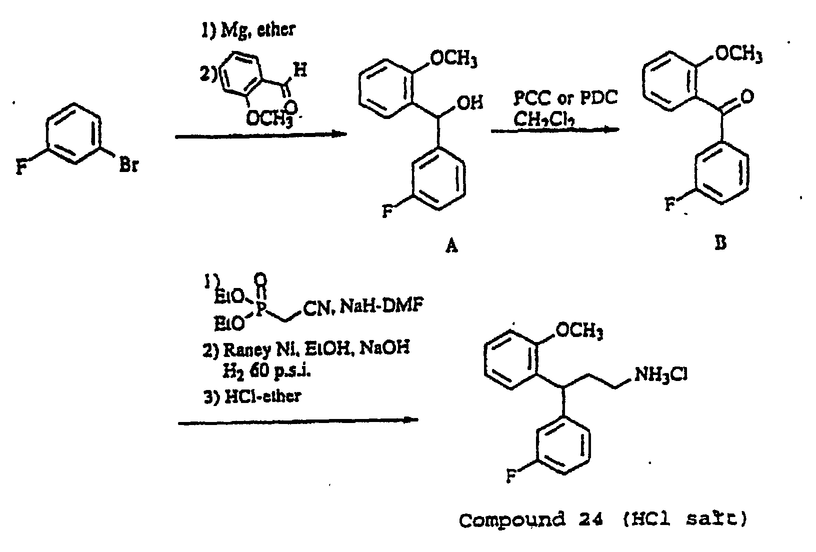

Compounds **101** and **103** were synthesized from compounds **25** and **24**, respectively, by cleavage of their *O*-methyl ethers with borane tribromide in the normal manner.

Compound **32** (Reference compound) was synthesized according to standard procedures as described above.

Compounds **107, 116, 139,** and **143** were prepared as synthetic intermediates used in the preparation of Compounds **32, 115, 20,** and **25,** respectively.

Compound **50** (Reference Compound) was also prepared using the chiral synthesis described below.

To an ice cold solution of *N*-benzyl-(*S*)-a-methylbenzylamine (18.0 g, 85.2 mmol) in THF (75 ml) was added butyl lithium (2.5 M in hexane) (37.5 ml, 93.8 mmol) via a syringe over a period of 10 min at such a rate as to keep the reaction temperature below 10°C during the addition. The reaction was then stirred at 0°C for 15 min. The reaction was cooled to -78°C in a dry ice/isopropanol bath and then a solution of benzyl crotonate (15.0 g, 85.2 mmol) in THF (100 ml) was added dropwise over a period of 45 min. The reaction was stirred at -78°C for 15 min, and then saturated NH₄Cl (50 ml) was added. The reaction mixture was then quickly transferred to a separatory funnel containing saturated NaCl (500 ml) and ether (200 ml). The layers were separated and the aqueous layer extracted with ether (200 ml). The combined organic layers were dried, evaporated, and chromatographed on silica gel (50 mm x 30 cm) (hexane/ethyl acetate [20:1]) to yield 21.0 g, 63.7% of product A. ¹H-NMR showed that the diastereoselectivity of the reaction is > 90%.

A mixture of magnesium (2.58 g, 106 mmol), THF (200 ml), and 1-bromo-3-fluorobenzene (18.60 g, 106.3 mmol) was refluxed for 45 min. While still under reflux, product **A** (16.45 g, 42.45 mmol) was added via syringe with THF (25 ml) over a 2 min period. The reaction was refluxed for 1 hr, and then allowed to cool to room temperature. Saturated NH₄Cl(aq) (200 ml) was added. The reaction mixture was then transferred to a separatory funnel containing saturated NaCl(aq) (500 ml) and diethyl ether (200 ml). The layers were separated and the aqueous layer extracted with ether (200 ml). The combined organic layers were dried over sodium sulfate and evaporated to give 21.41 g of product **B** as a yellow liquid.

Product **B** (20.02 g, 42.45 mmol, theoretical) was dissolved in acetic acid (120 ml) and sulfuric acid (30 ml). The reaction was stirred at 90°C for 1 hr. The acetic acid was rotary evaporated giving a brown sludge. This material was placed in an ice bath and cold water (400 ml) was added. The product immediately precipitated. To the reaction was slowly added 10 N NaOH (150 ml) to neutral pH. Diethyl ether (200 ml) was added to this mixture. The mixture was shaken until there was no undissolved material. The ether layer was separated, washed with water (2 x 100 ml), dried over sodium sulfate, and rotary evaporated yielding 13.14 g (68.2% based on ester) of a thick brown oil. This oil was taken up in ether and converted to the hydrochloride salt with hydrogen chloride in diethyl ether to give product **C** as a yellow-white solid.

Product **C** (7.17 g, 14.6 mmol) was taken up in absolute ethanol (200 ml). Pearlman's catalyst (Pd(OH)₂/C; 2.00 g) was added. The reaction was shaken under 70 psi hydrogen gas at 70°C for 20 hr, and the reaction mixture was filtered through Celite. The filtrate was rotary evaporated to give 3.54 g of a light yellow glass. This material was taken up in diethyl ether (100. ml) and was basified with 1 N MaOH (25 ml). The ether layer was washed with water (1 x 25 ml), dried over sodium sulfate, and rotary evaporated to give 2.45 g of a light yellow oil. This material was Kugelrohr distilled (90-100°C, 1 mm Hg) to give 1.17 g of a colorless liquid. This material was taken up in diethyl ether and converted to the hydrochloride salt with ethereal hydrogen chloride. After rotary evaporation, the salt was recrystallized from 0.12 N HCl (200 mg/ml). The crystals were filtered off and were washed with cold 0.12 N HCl yielding 0.77 g (18%) of Compound 50 as silvery white crystals (as the hydrochloride salt).

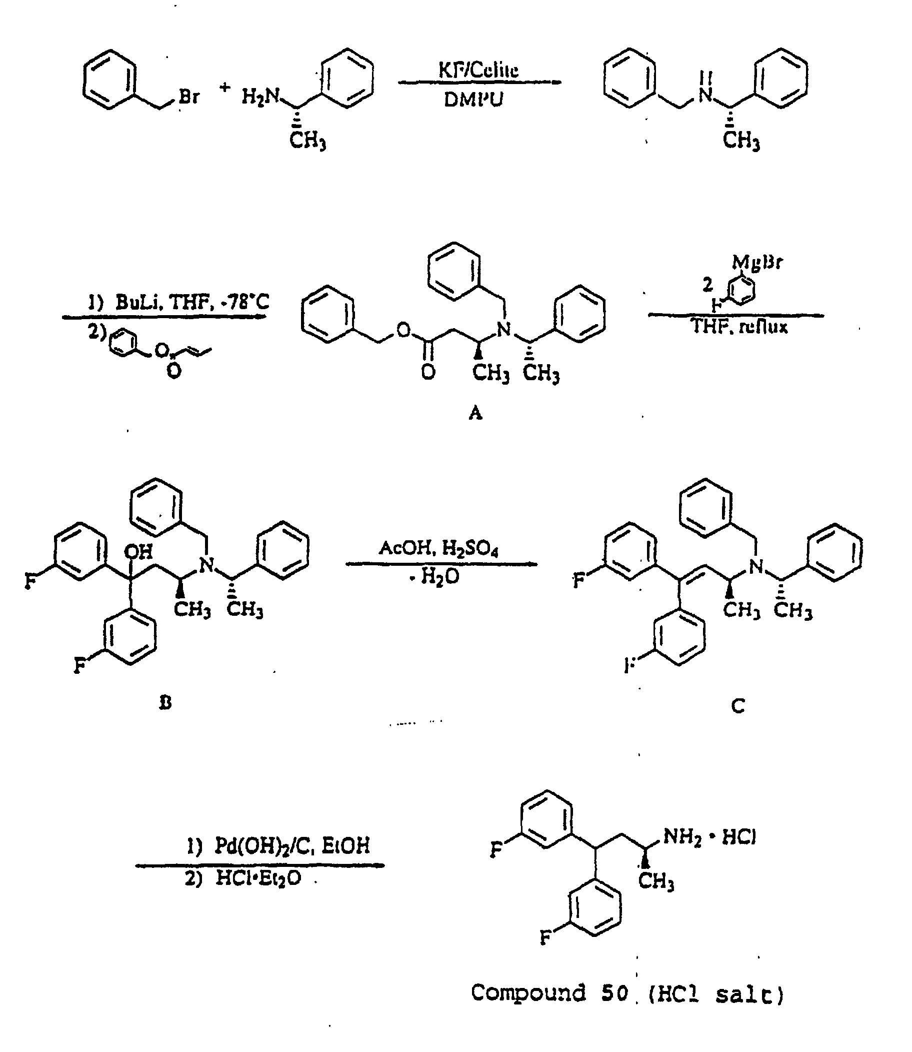

Synthesis of Compound **54** was accomplished as described below.

To a solution of 3,3'-difluorobenzophenone (5 g, 22.9 mmol) and methyl cyanoacetate (3.4 g, 34.4 mmol) in 15 ml of ether was added titanium isopropoxide (16.9 g ml, 57.25 mmol). This solution was stirred for 6 days at room temperature then quenched with 0.5 mol of HCl in 300 ml of water. The mixture was diluted with 100 ml of ether, and the layers separated. The ether layer was washed with 5% HCl and saturated brine, then dried over sodium sulfate. The solvents were evaporated *in vacuo* to give 8 g of product **A**.

Compound A was dissolved in 50 ml of isopropanol, followed by the addition of a small amount of bromocresol green. Sodium cyanoborohydride (1.52 g, 24.2 mmol) was added all at once followed immediately with the dropwise addition of concentrated HCl, added at such a rate as to keep the solution yellow. After 2 hours the reaction was worked up by partitioning between ether and water. The ether layer was washed with water and saturated brine, dried over sodium sulfate, and concentrated to give the product **B**.

To a solution of lithium aluminum hydride (30.4 ml, 30.4 mmol) in THF was added product **B** (1 g, 3.04 mmol) in 2 ml of THF over a period of 30 seconds. This solution was stirred overnight at room temperature, then quenched with the addition of 20 ml of ethyl acetate. The solvents were then removed *in vacuo,* and the resulting oil was dissolved in aqueous HCl and acetonitrile. The product was then purified on a C-18 column with a gradient of 0.1% HCl to acetonitrile to give 82 mg of Compound **54**, as the hydrochloride salt. EI-MS *m*/*z* (relative intensity) 277 (M⁺, 100), 260 (2.4), 242 (8.6), 229 (28), 215 (11.7), 204 (16), 183 (12), 133 (9.5), 124 (14), 109 (6.8), 30 (22).

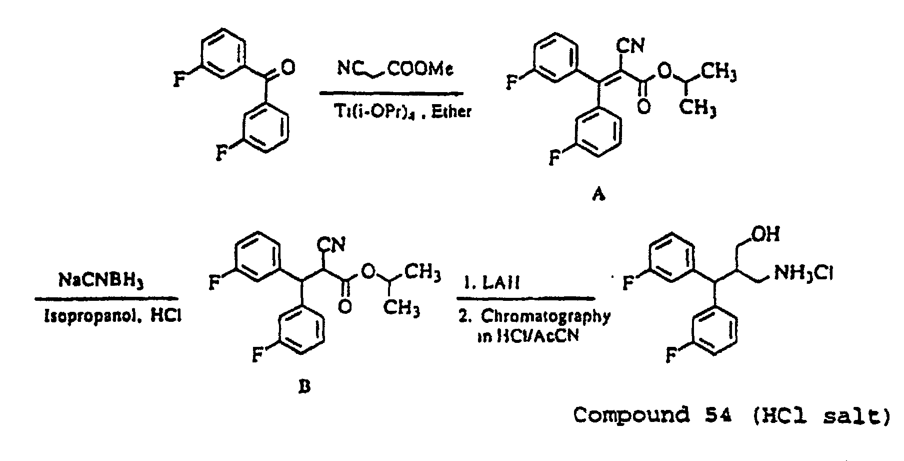

Compound 55 was synthesized analogously to Compound **21** except that ethyl iodide was used in the alkylation step. GC/EI-MS (Rₜ = 7.43 min) *m*/*z* (relative intensity) 275 (M⁺, 100), 258 (66), 229 (63), 204 (57), 201 (72), 183 (84 , 134 (57), 124 (68), 109 (98), 72 (72).

The synthesis of Compound 56 was accomplished as follows.

The alcohol **A** was synthesized from 3-fluorobromobenzene and 3-fluoro-2-methylbenzaldehyde as described for product **A** in the synthesis of Compound **24.**

The alcohol A (8.4 g, 36.2 mmol) was stirred with manganese dioxide (12.6 g, 144.8 mmol) in 100 ml of dichloromethane for 4 days. The reaction mixture was then diluted with ether and filtered through a 0.2 micron teflon membrane filter. The filtrate was concentrated to give 7.6 g of the ketone **B**.

The substituted acrylonitrile C was synthesized as described for product **A** in the Compound **20** synthesis.

To the nitrile C (4 g, 15.7 mmol) in 240 ml of ethanol was added 2 g of 10% palladium dihydroxide on carbon. This mixture was hydrogenated at 60-40 psi for 3 days. The reaction, mixture was then filtered and concentrated. The resulting oil was dissolved in chloroform and chromatographed on silica gel (30% methanol/5% isopropylamine in chloroform) to give the amine. This amine was dissolved in aqueous HCl/acetonitrile and purified *via* HPLC on C-18 (10% acetonitrile/0.1% HCl to 50% acetonitrile/0.1% HCl over 60 min) then lyophilized to give 800 mg of Compound **56**, as the hydrochloride salt. GC/EI-MS (Rₜ = 7.39 min) *m*/*z* (relative intensity) 261 (M⁺, 64), 244 (56), 229 (57), 215 (100), 203 (53), 183 (21), 133 (39), 122 (31), 109 (32).

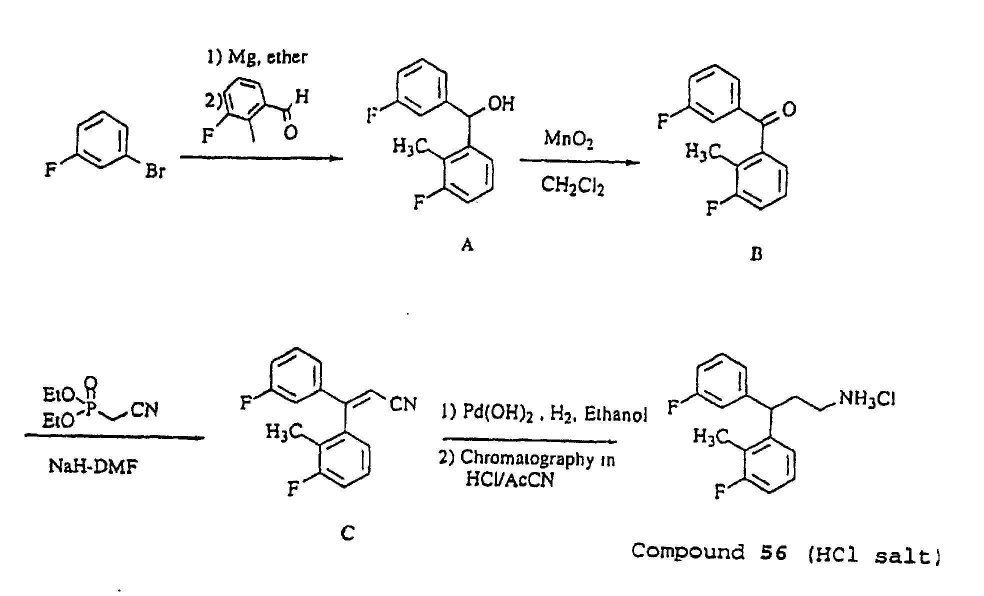

The synthesis of Compound **57** was accomplished as follows.

To a solution of 5-fluoro-2-methylbenzonitrile (5 g, 37 mmol) in 50 ml of THF was added 3-fluorophenylmagnesium bromide (46 ml, 40 mmol) and copper (I) cyanide (0.072 g, 0.8 mmol). This solution was refluxed for 4 hours, then poured into ether/20% HCl and stirred for a further 2 hours. The layers were separated, and the ether layer washed with water and saturated brine. The solution was dried over sodium sulfate and concentrated. The crude oil was purified on silica (hexane to 50% dichloromethane in hexane over 60 min) to give 6.7 g of the ketone **A**.

The ketone **A** was converted to Compound **57** as described for Compound **56**. GC/EI-MS (Rₜ = 7.35 min) *m*/*z* (relative intensity) 261 (M⁺, 52), 244 (41), 229 (67), 215 (100), 203 (42), 201 (42), 183 (21), 133 (45), 122 (28), 109 (26).

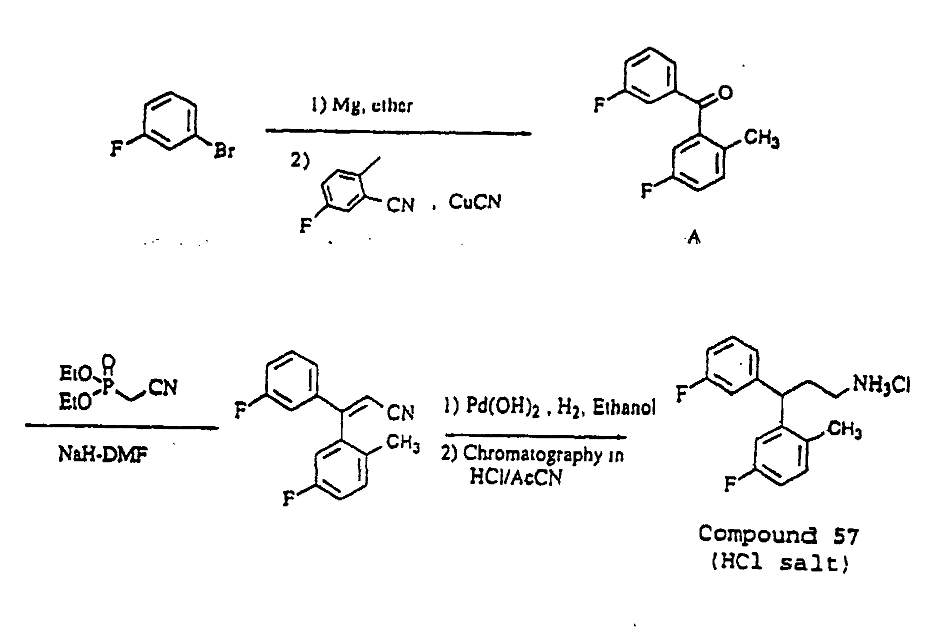

The synthesis of Compound 58 was accomplished as follows.

To a solution of 5-fluoro-2-methylbenzoyl chloride (2.24 g, 13 mmol) in 10 ml of dry THF was added iron III acetylacetonate (0.16 g, 0.44 mmol). The solution was cooled to 0°C, and a THF solution of 5-fluoro-2-methylphenylmagnesium bromide (20 ml, 15.5 mmol) was added by syringe over a period of 30 min. The reaction was stirred for another 30 min, then poured slowly into ether/5% HCl. The ether layer was separated, washed with saturated brine, dried over sodium sulfate, and concentrated to give 3.2 g of ketone **A**.

Dry THF (30 ml) was cooled to -78°C followed by the addition of butyl lithium (5.85 ml, 14.6 mmol, 2.5 M solution in hexanes). Acetonitrile (0.76 ml, 14.62 mmol) was then added over a period of 2 min, then allowed to stir at -78°C for 15 min. To this solution was added ketone **A** (3 g, 12.2 mmol) in 5 ml of THF. The solution was stirred for 30 min at -78°C then allowed to warm to room temperature and stirred overnight. The reaction mixture was partitioned between ether and 5% HCl. The ether layer was separated, washed with saturated brine, dried over sodium sulfate, and concentrated to give 2.2 g of the nitrile **B**.

The nitrile **B** (1 g, 3.48 mmol) was dissolved in 30 ml of ethanol and 3 ml of 10 N sodium hydroxide. To this solution was added 1 g of a 50% aqueous slurry of Raney nickel, and the mixture was hydrogenated at 60 psi for 2C hours. The reaction was filtered and concentrated to a white solid. This residue was taken up in ether/water and the ether layer separated. The ether solution was dried over sodium sulfate and concentrated to give 0.96 g of the hydroxy amine **C**.

The hydroxy amine C (0.96 g, 3.3 mmol) was taken up in concentrated HCl and heated to 70°C which caused brief solution, and then precipitation of the alkene **D**. The alkene was collected by filtration and dissolved in 30 ml of ethanol and 1 ml of conc. HCl. Palladium dihydroxide on carbon (0.4 g) was added to the solution, and the mixture hydrogenated at 60 psi for 24 hours. The product was isolated by filtering off the catalyst and evaporating the solvent. The residue was dissolved in 0.1% HCl and acetonitrile, and purified on C-18 (15% acetonitrile/0.1% HCl to acetonitrile) to give 0.6 g of Compound **58**, as the hydrochloride salt. GC/EI-MS (Rₜ = 7.82 min) m/z (relative intensity) 275 (M⁻, 100), 258 (20), 243 (74), 229 (38), 214 (65), 201 (31), 196 (32), 183 (20), 148 (35), 138 (42), 133 (48), 122 (69), 109 (41).

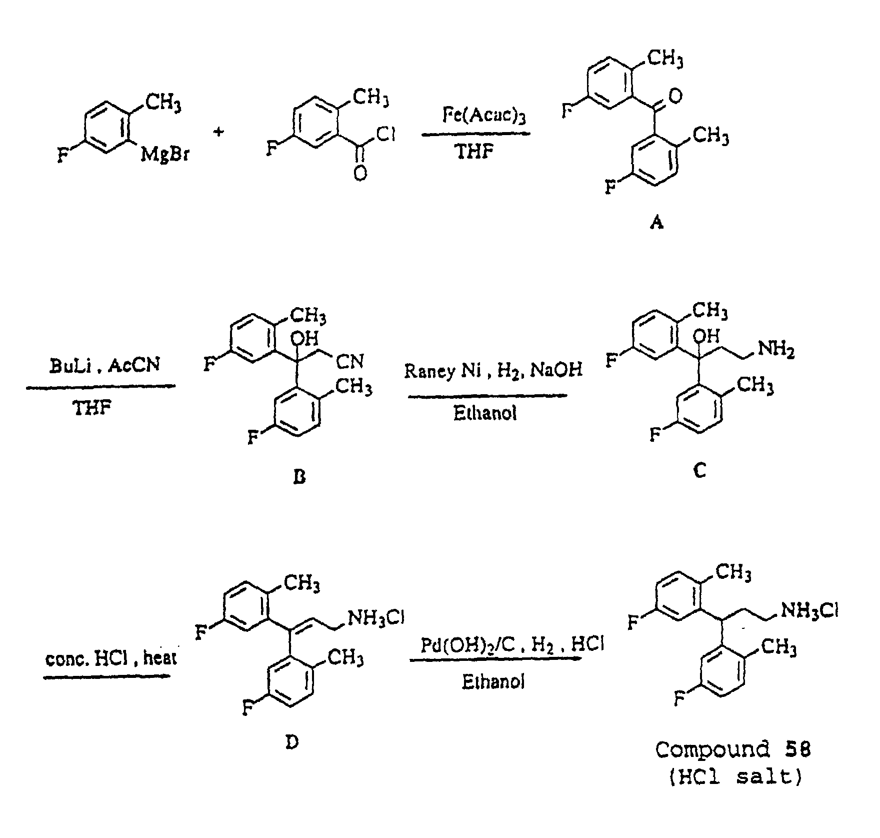

Synthesis of Compound **59** was accomplished as follows.

Compound **20** (2.0 g, 7.05 mmol) was dissolved in abs. EtOH (200 ml) and cooled to 5-10°C in an ice bath. Acetaldehyde (0.395 ml, 7.05 mmol, cooled to -4°C) was added followed by nickel-aluminum alloy (200 mg, Fluka Chemika), and the reaction was hydrogenated on a Parr apparatus at 50 psi for 2 hr. GC/MS showed 75% yield of the product and 2% of the *N,N*-diethyl side-reaction product. The reaction mixture was filtered through diatomaceous earth and the filtrate was evaporated under reduced pressure. The crude product was dissolved in isopropanol (5 ml)/ether (60 ml)/ethereal HCl (1 M), and then hexane (5 ml) was added to the cloud point. The cloudy mixture was filtered through paper, then hexane (10 ml) was added to the cloud point, and the solution was filtered again. The filtrate-was stoppered and the product was allowed to crystallize at room temperature. The crystals were collected and dried to provide 0.325 g (14.8% yield) of Compound 59, as the hydrochloride salt (colorless needles).

The synthesis of Compound **60** was accomplished as follows. Compounds **66, 69, 108, 123, 142,** and **145** can be synthesized in a similar manner starting from Compounds **33, 50, 32, 60, 25** and **119**, respectively.

Compound **20** (as the free base) (1.0 g, 4.0 mmol was refluxed in ethyl formate (150 ml) for 2 hr. The solvent was then removed under reduced pressure to provide 1.1 g, 99% yield of formamide **A** as a colorless oil. GC/MS showed the product to be 100.0% pure and was used in the following step without further purification.

The formamide **A** (1.1 g, 4.0 mmol) was dissolved in dry THF (100 ml) and heated to reflux (no condenser). Borane-methyl sulfide complex (1.2 ml, 12 mmol, 10.5 M) was added dropwise over a period of 3 min to the refluxing solution. Reflux was maintained for approximately 15 min, open to the air, until the reaction volume was reduced to approximately 30 ml. The reaction was then cooled in an ice bath, and ice (5 g, small pieces) was carefully added followed by H₂O (25 ml) and conc. HCl (25 ml). The acidic solution was refluxed for 30 min. The reaction mixture was then cooled in an ice bath, basified with NaOH (10N), extracted with ether (3 X 100 ml), dried (Na₂SO₄, anhydrous), and evaporated under reduced pressure. The crude product was dissolved in ether (10 ml)/hexane (50 ml) and ethereal HCL (1 M) was added dropwise to precipitate the hydrochloride salt. The salt was collected and recrystallized from isopropanol (3 ml)/ether (40 ml) to provide 0.5 g of Compound **60**, as the hydrochloride salt.

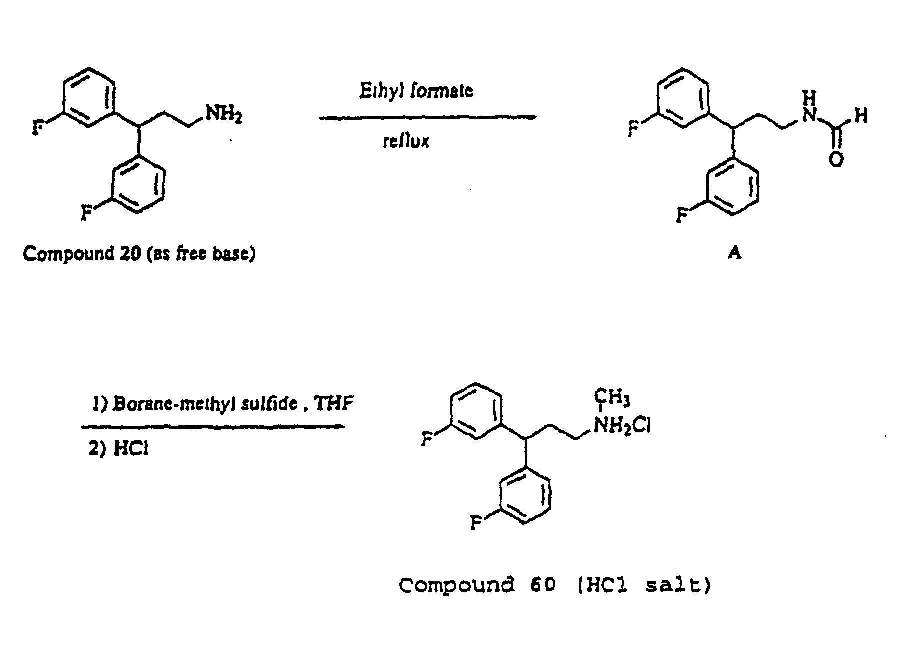

Alternatively, Compound **60** was synthesized from commercially available starting materials in the following four step reaction sequence. The first intermediate in this synthetic route, ethyl-*N*-benzyl-*N*-methyl-3-aminopropionate. was prepared by conjugate addition of *N*-benzylmethylamine to ethyl acrylate. The ester functionality of the first intermediate was then reacted with two equivalents of Griguard reagent (prepared from 1-bromo-3-fluorobenzene) to provide *N*-benzyl-*N*-methyl-3-hydroxy-3- (bis-3-fluorophenyl) propylamine. The Grignard reaction product was then dehydrated in a mixture of 6N HCl/acetic acid to yield *N*-benzyl-*N*-methyl-3-(bis-3-fluorophenyl)-2-propenamine. Catalytic hydrogenation of this material as its hydrochloride salt in ethanol over Pearlman's catalyst [Pd(OH₂)/C] provided, after recrystallization from ethyl acetate, colorless, needles of Compound 60 as the hydrochloride salt.

In a 500-mL, 3-necked flask equipped with thermometer, reflux condenser, and a 125-mL addition funnel [charged with ethyl acrylate (88.3 mL, 81.5 g, 0.815 mol)] was placed *N*-benzylmethylamine (100 mL, 94.0 g, 0.776 mol). The ethyl acrylate was added dropwise to the stirring reaction mixture over a period of 80 min. After stirring for 18 h at room temperature, the product was vacuum distilled and the fraction containing product was collected at 78-95°C (0.12-0.25 mm Hg), (138 g, 80% yield): Bp 78-95°C (0.12-0.25 mm Hg) ; TLC, R_{*f*} = 0.23 [hexane-EtOAc (5:1)], R_{*f*} = 0.57 [MeOH-CHCl₃ (100:5)]; GC, t_{*R*} = 6.06 min; MS, 221 (M⁻), 206 (M-CH₃), 192 (M-C₂H₅), 176 (M-OC₂H₅), 144 (M-C₆H₅), 134 [CH₂N(CH₃)CH₂Ph], 120 [N(CH₃)CH₂Ph], 91 (CH), 77 (CH), 42 (CH₂CH₂N); ¹H NMR (free base, CDCl₃) d 1.25 ppm (t, J = 7.1, 3H, CH₂CH₃), 2.20 (s, 3H, NCH₃), 2.51 (t, *J* = 7.3, 2H, COCH₂), 2.74 (t, *J =* 7.2, 2H, CH₂N), 3.51 (s, 2H, NCH₂Ph), 4.13 (q, *J* = 7.1, 2H, OCH₂CH₃), 7.18-7.35 (m, 5H, ArH); ¹³C NMR (free base, CDCl₃ d 15.2 (CH₂CH₃), 34.0 (COCH₂), 42.9 (NCH₃), 53.8 (NCH₂), 61.4 (OCH₂CH₃), 63.1 (CH₂Ph), 128.0 (CH), 129.2 (CH), 130.0 (CH), 139.9 (q), 173.7 (q).

In a 5-L, four-necked, round-bottom flask, under nitrogen, was placed Mg [51.5 g, 2.12 mol, turnings, washed with THF (2 x 300 mL)] and THF (2 L). An addition funnel was charged with 1-bromo-3-fluorobenzene (neat, 392.8 g, 2.24 mol). One-twentieth of the bromide was added to the magnesium suspension followed by one crystal of iodine. After initiation of the Grignard reaction the remaining 1-bromo-3-fluorobenzene was then added to the refluxing mixture over a period of 50 min. The reaction was refluxed for an additional 45 min. To the refluxing solution of Grignard reagent was added a solution of ethyl N-benzyl-*N*-methyl-3-aminopropionate (187.5 g, 0.847 mol) in THF (100 mL) over a period of 20 min. After the ester addition was complete, the reaction was refluxed for 1h. The reaction was then cooled in an ice bath. Saturated NH₄Cl (aq., 400 mL) and H₂O (400 mL) were added and the mixture was transferred to a separatory funnel. The organic layer was separated and the aqueous layer was extracted once with THF (400 mL). The combined organic layers were washed with said. NaCl (2 x 200 mL, aq.), dried (anh. Na₂SO₄), filtered through paper, and rotary evaporated vacuum to yield 281.6 g (90%) of crude product as an orange, viscous oil. This material (281.6 g, 0.766 mol) was dissolved in acetonitrile (1.4 L). Concentrated hydrochloric acid (65.0 mL, 0.786 mol, 12N) was added to the stirring filtrate. The crystallizing mixture was then cooled to -20 °C for 17 h. The product was collected, washed with cold acetonitrile (800 mL), and dried to provide a white solid, 235.6 g (69% yield from the ester). For analytical purposes, the hydrochloride salt was further purified by recrystallization from acetonitrile: Mp 194-197 °C (uncorr.); TLC, R_{f} = 0.23 [hexane-EtOAc (5:1)], R_{f} = 0.85 [MeOH-CHCl₃ (100:5)], R_{f} = 0.72 [MeOH-CHCl₃ (100:3)]; GC, t _{R} = 10.93 min; MS, 367 (M⁺), 272 (M-C₆H₄F), 258 (M-CH₂Ph-H₂O), 219 [(C₆H₄F)₂CH], 148 [CH₂CH₂N(CH₃)CH₂Ph], 134 [CH₂N(CH₃)CH₂Ph], 91 (C₇H₇), 42 (CH₂CH₂N); ¹H NMR (free base, CDCl₃) d 2.18 (s, 3H, NCH₃), 2.41 (m, 2H, CHCH₂), 2.58 (m, 2H, CH₂N), 3.42 (s, 2H, CH₂Ph), 6.86 (dt, *J*₁ = 8.5, *J*₂ = 1.8, 2H, Ar-H), 7.18-7.30 (m, 10H. Ar-H), 8.33 (bs, 1H, OH); ¹³C NMR (free base, CDCl₃) d 35.6 (CHCH₂), 41.5 (CH₃, NCH₃), 54.3 (CH₂, CH₂N), 62.6 (CH₂, CH₂Ph), 113.1 (d, *J* = 23, CH, Ar-C_{5,5'}), 113.5 (d, *J* = 23, CH), 121.2 (d, *J* = 3, CH), 127.5 (CH), 128.5 (CH), 129.2 (CH), 129.5 (CH), 129.6 (CH), 137.0 (q), 150.2 (q), 162.8 (d, *J* = 243, q, Ar-C_{3,3'}).

In a 5-L, 3-necked reaction vessel, equipped with an overhead mechanical stirrer, reflux condenser, and thermometer, was placed *N*-benzyl-*N*-methyl-3-hydroxy-3-bis (3-fluorophenyl)propylamine hydrochloride (225.4 g, 0.559 mol), 6N HCl (1392 mL) and glacial HOAc (464 mL). The suspension was heated in a water bath (80-85 °C) and stirred for 18 h. After 18 h of heating, the reaction mixture was cooled in an ice/MeOH bath. Ethyl acetate (500 mL) was added to the cooled reaction mixture. NaOH (10*N*, 1.7 L) was then added to the cooled mixture over a period of 25 min at such a rate as to keep the temperature below 40 °C. The mixture was transferred to a 6-L separatory funnel. The organic layer was separated and the aqueous layer was extracted with ethyl acetate (2 x 500 mL). The combined organic layers were washed with satd. NaCl (2 x 100 mL, aq.), dried Na₂SO₄ (250 g), rotary evaporated, and then dried under vacuum to provide 185.6 g (95% yield) of the free base as a fluid, brownish-colored oil.

The material above was stirred with hexane (1.5 L). The resulting solution was filtered through paper. 4M HCl in dioxane (146 mL) was added dropwise with stirring to the filtrate over a period of 5 min. The semi-translucent solvent was then decanted away from the light-yellow colored, semisolid precipitate. The crude hydrochloride salt was dissolved in refluxing ethyl acetate (600 mL) and was filtered. The filtrate was then thoroughly cooled in an ice bath, and hexane (110 mL) was slowly added, with vigorous stirring. After cooling in an ice bath for 2 h, the entire flask filled with a white crystalline solid. This material was collected on a filter funnel, washed with ice-cold hexane/ethyl acetate [(1:4), 400 mL], and dried to yield 128.7 g, 59.7% of a white solid. On standing the mother liquor precipitated another 14.8 g of an off-white solid. Total yield 128.7 g + 14.8 g = 143.5 (67%). Mp 141-142 °C (uncorr.); TLC, R_{*f*} = 0.20 [hexane-EtOAc (5:1)], R_{*f*} = 0.75 [MeOH-CHCl₃ (100:5)], R_{*f*} = 0.49 [MeOH-CHCl₃ (100:3)]; GC, t_{*R*} = 10.40 min; MS, 349 (M⁺), 330, 301, 281, 258 (M-CH₂Ph), 240, 229 [M-N(CH₃)CH₂Ph], 201, 183, 146, 133, 109, 91 (CH₂C₆H₅), 65, 42 (CH₂NHCH₃); ¹H NMR (free base, CDCl₃) d 2.20 ppm (s, 3H, NCH₃), 3.08 (d, *J* = 6.8, 2H, CH₂N), 3.47 (d, *J* < 1, 2H, CH₂Ph), 6.29 (t, *J* = 6.8, 1H, CH), 6.85-7.04 (m, 6H, ArH), 7.19-7.35 (m, 7H, ArH).

*N*-Benzyl-*N*-methyl-3-bis(3-fluorophenyl) allylamine hydrochloride (120.0 g, 0.311 mol) was dissolved in abs. EtOH (1250 mL). Pd(OH)₂/charcoal (10,0 g, -20% Pd, Fluka Chemical) was added. The reaction mixture was stirred under a steady flow of hydrogen gas for 18 h at 25 °C (atmospheric pressure). The mixture was then filtered through Celite®/fritted glass, the catalyst was washed with EtOH (2 x 50 mL), and the solvent was removed under reduced pressure to yield 95.4 g, 103% of crude product. This material was dissolved in refluxing ethyl acetate (300 mL) with vigorous stirring and filtered. The flask was allowed to stand for 2 h at 25 °C, during which time the hydrochloride salt began to crystallize as needles. The flask was then cooled, the product was collected, washed with ice-cold ethyl acetate (20 mL), and dried to yield 73.7 g, 80%, of Compound 60 as a white, crystalline solid. Mp 129-130 °C; UV/Vis, e = 2.1 x 10³ L·mol⁻¹cm⁻¹ (264 nm, EtOH, 25 °C, linear range: 0.05-0.20 mg/mL); TLC, R_{*f*} = 0.00 [hexane-EtOAc (5:1)], R_{*f*} = 0.07 [MeOH-CHCl₃ (100:5)], R_{*f*} = 0.19 [MeOH-CHCl₃-NH₄OH (100:5:1)]; GC, t_{*N*} = 7.45 min; MS, 261 (M⁺), 229, 215, 201, 183, 164, 150, 138, 122, 101, 83, 75, 57, 42 [CH₂NHCH₃]; ¹H NMR (HCl salt, CDCl₃ + 1 gtt MeOD) δ 2.56 (m, 2H, NCH₂), 2.60 (s, 3H, NCH₃), 2.85 (t, *J* = 8.0, 2H, CHCH₂), 4.11 (t, *J =* 8.0, 1H, CH), 6.87-6.98 (m, 4H, ArH), 7.06 (d, *J* = 7.7, 2H, Ar_{2.2'}H), 7.25 (dd, *J*_{*1*} = 6, *J*_{*2*} = 8, ArH); ¹³C NMR (HCl salt, CDCl₃ + 1 gt MeOD) δ 30.9 (CH₂, CHCH₂), 32.7 (CH₃, NCH₃), 47.6 (CH, CHCH₂), 47.8 (CH₂, CH₂N), 113.9 (*J* = 21, ArC_{2,2'} or ArC_{4,4'}), 114.5 (d, *J* = 22, ArC_{2,2'} or ArC_{4,4'}), 123.2 (d, *J* = 3, Ar-C_{6,6'}), 130.3 (d, *J* = 9, Ar-C_{5,5'}), 144.7 (d, *J* = 7, Ar-C_{1,1'}), 162.9 (d, *J* = 245, Ar-C_{3,3'}); IR: KBr pellet (cm⁻¹), 3436.9, 2963.4, 2778.5, 2453.7, 1610.6, 1589.3, 1487.0, 1445.3, 1246.0, 764.5; solubility: 2 g/mL (H₂O), 1 g/mL (EtOH); anal. calcd. for C₁₆H₁₇NF₂.HCl (Karl Fischer: 0.26% H₂O): C, 64.37; H, 6.11; N, 4.69; found: C, 64.14; H, 6.13; N, 4.69.

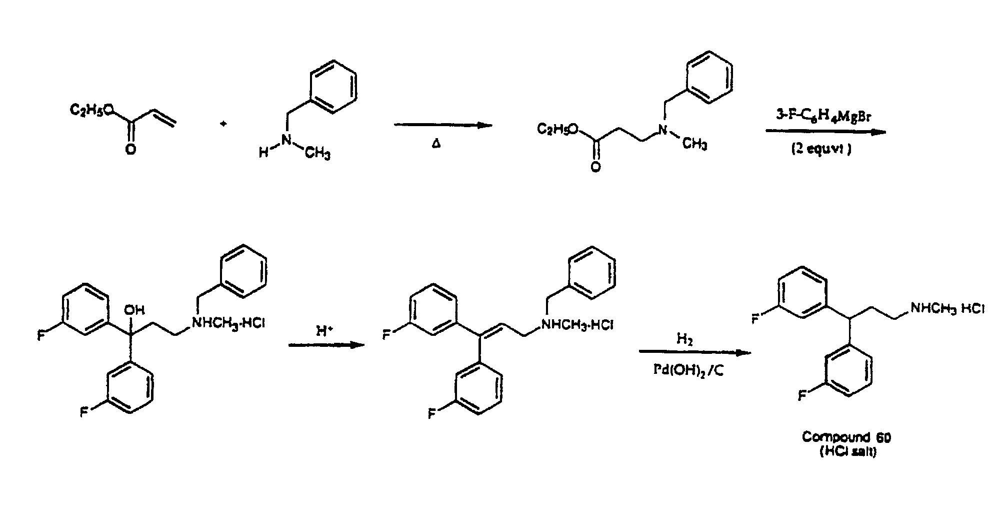

Compound **105** was prepared by selective reduction of its corresponding alkene by catalytic hydrogenation over Pd/C.

Compound **61** was prepared from 2-bromo-4-fluoroanisole and 3-fluorobenzaldehyde as described for Compound **24**. GC/EI-MS (Rₜ = 9.22 min) *m*/*z* (relative intensity) 277 (M⁺, 74), 260 (46), 245 (35), 231 (44), 229 (34), 217 (24), 203 (28), 201 (31), 183 (28), 154 (24), 133 (19), 109 (100).

Compound 62 was prepared from 2-bromoanisole and 2-methoxybenzaldehyde as described for Compound **24.** GC/EI-MS (Rₜ = 9.30 min) *m*/*z* (relative intensity) 271 (M', 100), 254 (17), 240 (23), 225 (40), 223 (45), 207 (22), 181 (32), 165 (31), 136 (48), 121 (98), 91 (83).

The synthesis of Compound **63** was accomplished as follows.

Alcohol **A** was obtained from 3-fluorobenzaldehyde as described for product **A** of the Compound **24** synthesis.

To alcohol **A** (10.275 g, 47 mmol) in 200 ml of ethanol was added 1.6 g of 10% Pd/C and 1 ml of concentrated HCl. This mixture was hydrogenated for 3 hr at 60 psi, then filtered and concentrated to give the diphenylmethane **B**.

Product **B** (2.01 g, 9.86 mmol) was dissolved in 20 ml of THF and cooled to -78°C. Butyl lithium (4.4 ml, 10.8 mmol, 2.5 M in hexanes) was added slowly by syringe, and then the reaction stirred for another 30 min at -7.8°C. To this orange solution was added cyclopentene oxide (0.9 ml, 10.3 mmol). The reaction was allowed to stir 3 hours while warming slowly to room temperature. The reaction was quenched with 150 ml of 10% HCl and extracted 3 times with ether. The ether layer was dried over sodium sulfate and concentrated to give 2.5 g of the alcohol **C**.

To the alcohol C (1 g, 3.5 mmol) in 10 ml of dry THF was added triphenylphosphine (1.37 g, 5.2 mmol) in 5 ml of THF and p-nitrobenzoic acid (0.87 g, 5.2 mmol) in 5 ml of THF. This solution was cooled to 0°C followed by the addition of DEAD (0.82 ml, 5.2 mmol), and allowed to stir overnight. The reaction was partitioned between water and ether. The ether was removed *in vacuo* and the resulting oil was chromatographed on silica gel in hexane/ethyl acetate to yield 365 mg of the cis-ester. This ester was hydrolyzed in methanol with potassium carbonate by stirring overnight. After removal of the methanol, the residue was taken up in ether, washed with water, dried over sodium sulfate and concentrated to give 250 mg of the *cis* alcohol **D**.

To the alcohol D (.25 g, 0.9 mmol) in 5 ml of dry THF was added triphenylphosphine (342 mg, 1.3 mmol) in 5 ml of THF and phthalimide (191.3 mg, 1.3 mmol) in 5 ml of THF. This solution was cooled to 0°C followed by the addition of DEAD (0.205 ml, 1.3 mmol), and allowed to stir overnight. The reaction was partitioned between water and ether. The ether was removed *in vacuo* and the resulting oil was chromatographed on silica gel in hexane/ethyl acetate to yield 100 mg of the phthalimide **E**.

To a solution of the phthalimide **E** (100 mg) in 20 ml of ethanol was added 8.8 mg of hydrazine hydrate. The solution was refluxed for 5 hours then stirred at room temperature overnight. The reaction was worked up by adding 1 ml of conc. HCl and filtering off the white solid. The resulting solution was concentrated to dryness and the solid taken up in ether and aqueous sodium hydroxide. The ether layer was dried over sodium sulfate and concentrated to a white solid. This was taken up in a small amount of ether and treated with 10 drops of 1M HCl in ether. After stirring overnight, the white solid was collected by filtration and dried to give 50 mg of Compound **63**, as the hydrochloride salt. GC/EI-MS (Rₜ = 9.22 min) *m*/*z* (relative intensity) 287 (M⁺, 45), 270 (12), 201 (63), 183 (81), 133 (38), 109 (43), 83 (44), 56 (100), 43 (37).

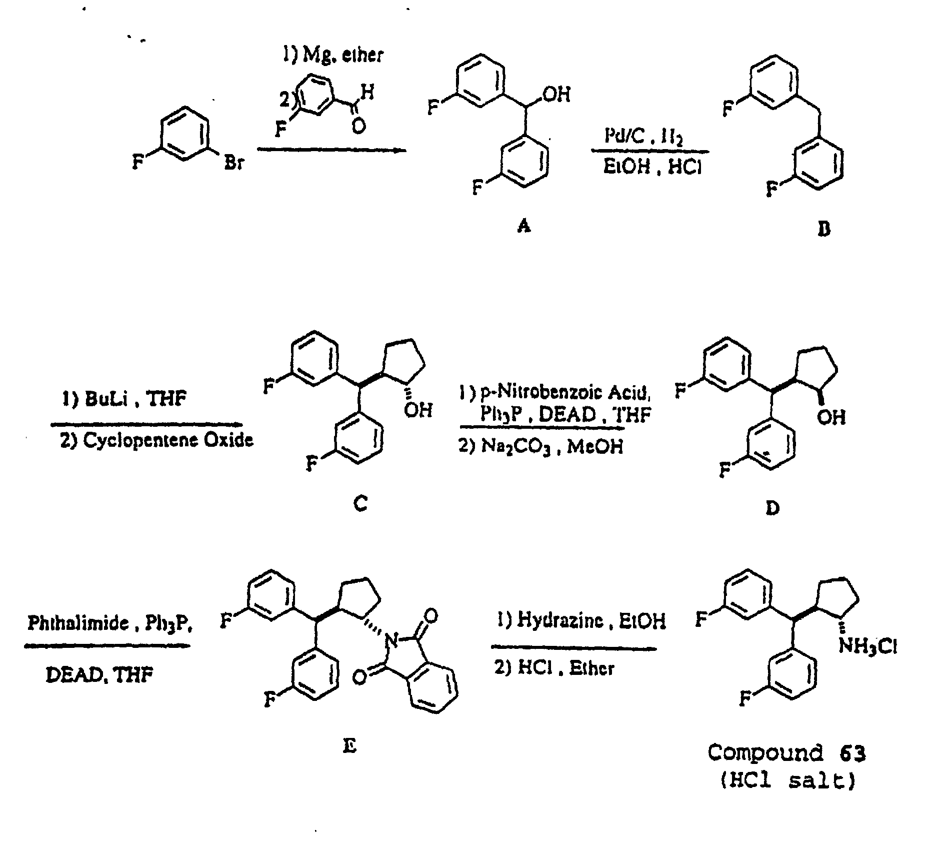

The synthesis of Compound **64** was done as described for Compound **63** except that the inversion step (product **C** to **D**) was omitted in order to obtain the cis amine as the final product. GC/EI-MS (Rₜ = 8.28 min) *m*/*z* (relative intensity) 287 (M⁺, 15), 270 (4), 201 (13), 183 (15), 133 (11), 109 (16), 84 (43), 56 (100), 43 (32).

The synthesis of Compound **65** was accomplished as follows.

The ketone **A** was synthesized similarly to ketone **B** in the Compound **24** synthesis using 2-methylphenylmagnesium bromide and 2-methylbenzaldehyde as starting materials. This ketone was converted to the final product using the procedure outlined for Compound **58.** GC/EI-MS (Rₜ = 7.84 min) m/z (relative intensity) 239 (M⁺, 88), 222 (14), 207 (100), 193 (46), 178 (71), 165 (60), 130 (39), 120 (40), 115 (51), 104 (40), 91 (38), 77 (21). 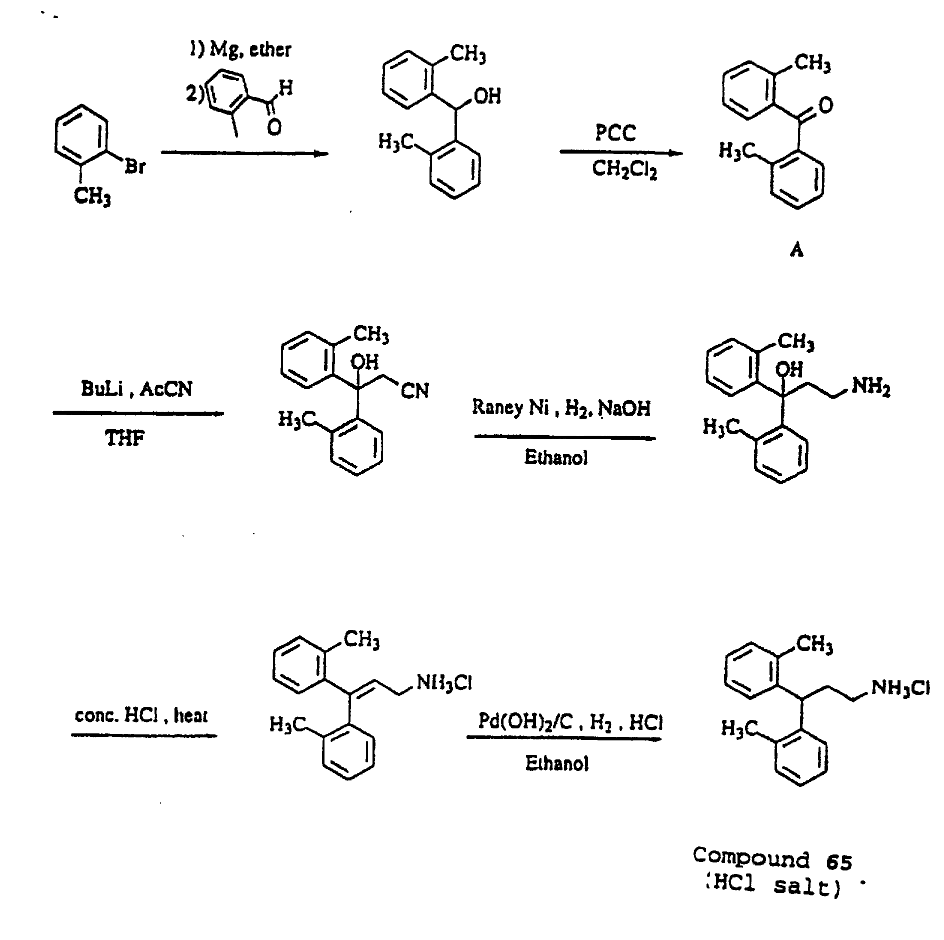 Compound **119** was synthesized in a seven-step reaction sequence starting from commercially-available *trans*-3-fluorocinnamic acid. This synthetic route is conceptually similar to that reported in the literature [U.S. Patent 4,313,896 (1982)] for related analogs. However, the three final steps were performed using a significantly different reaction sequence than that reported. The cinnamic acid was reduced and chlorinated in three steps to the corresponding 3- (3-fluorophenyl)propylchloride. This compound was brominated with NBS (*N*-bromosuccinimide) and the resulting trihalide was then reacted with 3-fluorophenol. The resulting ether was converted to the final product using a Gabriel synthesis.

*Trans*-3-fluorocinnamic acid (25.0 g, 150.4 mmol) was dissolved in abs. EtOH (250 mL) and hydrogenated over 10% Pd/C (2.5 g) in a Parr apparatus at 60 psig, 50°C, for 1 h (hydrogen uptake: calcd. 245 psig; found 260 psig). The reaction mixture was filtered and evaporated to yield a crystalline product (23.0 g, 89%). GC, t_{f} = 4.43 min; MS, 168 (M⁺).

Under a stream of dry nitrogen, at 0-10°C, a solution of 3-fluorohydrocinnamic acid (22.0 g, 131 mmol) in THF (100 mL) was added dropwise, over a period of 15 min, to a suspension of LiAlH₄ (4.23 g, 111 mmol) in THF (200 mL). The reaction was heated to reflux for a period of 1 h and then worked-up according to Fieser & Fieser's Reagents for Organic Synthesis (Vol. 1, 1967) to provide a white solid (20.1 g, 99%). GC, t_{*R*} = 3.74 min; MS, 154 (M⁺).

A solution of 3-(3-fluorophenyl)-1-propanol (15.0 g, 97.4 mmol) and triphenylphosphine (36.0 g, 137.3 mmol) in CCl₄ (150 mL) was refluxed for 19 h. Additional P(C₆H₅)₃ (3 x 3.0 g, 3 x 11.4 mmol) was added periodically over a period of 24 h. The resulting precipitate was removed by filtration and the solids were washed with hexane. The filtrate was evaporated under vacuum and the residue was suspended in hexane (200 mL) and then filtered. Evaporation of the filtrate provided 16.0 g (95.1%) of crude product which was purification by silica gel flash chromatography, elution with hexane, to provide 14.7 g (87%) of a colorless liquid. GC, t_{*R*} = 3.63 min; MS, 172/174 (M⁺).

A solution of the above chloride (12.0 g, 69.5 mmol), *N*-bromosuccinimide (17.3 g, 97.2 mmol), and dibenzoyl peroxide (0.06 g) in CCl₄ (75 mL) was refluxed for 1 h. The reaction mixture was then cooled in an ice bath, filtered, and the solids were washed with hexane. The filtrate was evaporated to provide 17.9 g (100%) of product. GC, t_{*R*} = 5.21 min; MS, 251/253 (M⁺).

A mixture of 3-bromo-3-(3-fluorophenyl) -1-propylchloride (4.0 g, 15.9 mmol), 3-fluorophenol (1.98 g, 17.7 mmol), and K₂CO₃ (2.65 g, 19.2 mmol) suspended in acetone (80 mL) was refluxed for 15 h. The volatiles were then removed under vacuum and the resulting residue was suspended in a mixture of hexane (200 mL) and NaOH (0.1N, 100 mL). The layers were separated and the organic layer washed, 0.1N NaOH (100 mL and H₂O (100 mL), dried (anh. Na₂SO₄), and evaporated *in vacuuo.* The resulting residue was chromatographed on silica gel, elution with hexane followed by hexane/EtOAc (100:1] then [40:1] to provide 1.64 g (37%) of product as a colorless oil. GC, t_{*R*} = 7.28 min; MS, 282/283 (M⁺); TLC r _{*f*}= 0.3, hexane/EtOAc [40:1].

A solution of 3-(3-fluorophenyl)-3-(3-fluorophenoxy)-1-propylchloride (1.52 g, 5.38 mmol) and potassium phthalate (1.20 g, 6.48 mmol) was heated to 90°C in DMF (30 mL) for a period of 2 h in a nitrogen atmosphere. The reaction mixture was then cooled and poured into H₂O (100 mL). The resulting solution was extracted with Et₂O (2 x 100 mL). The organic extract was washed, sat. NaCl (100 mL) and H₂O (2 x 100 mL), dried (anh. Na₂SO₄), and evaporated under vacuum to provide 2.17 g of crude product. The material was chromatographed on silica gel, elution with hexane/EtOAc [40:1] and then [20:1] to provide after evaporation 1.81 g (86%) of product as a glass.

A solution of *N*-phthaloyl-3-(3-fluorophenyl)-3 -(fluorophenoxy)-1-propylamine (1.74 g, 4.42 mmol) and anh. hydrazine (1.43 g, 44.6 mmol) in abs. EtOH (30 mL) was refluxed for 1 h. The reaction was cooled and evaporated under vacuum. The resulting material was suspended in Et₂O (75 mL) and washed with 0.2N NaOH (2 x 25 mL). The organic layer was dried (anh. Na₂SO₄), and evaporated under vacuum to provide 1.04 g (89.3%) which was purified by reverse-phase chromatography [Vydac Prep. C18; 264 nm; 50 mL/min; gradient elution ACN/0.1% HCl aq., 10%-50% over 20 min; rₜ = 17.4 min], to yield 0.89 g (67%) of Compound **119** as a hygroscopic hydrochloride salt.

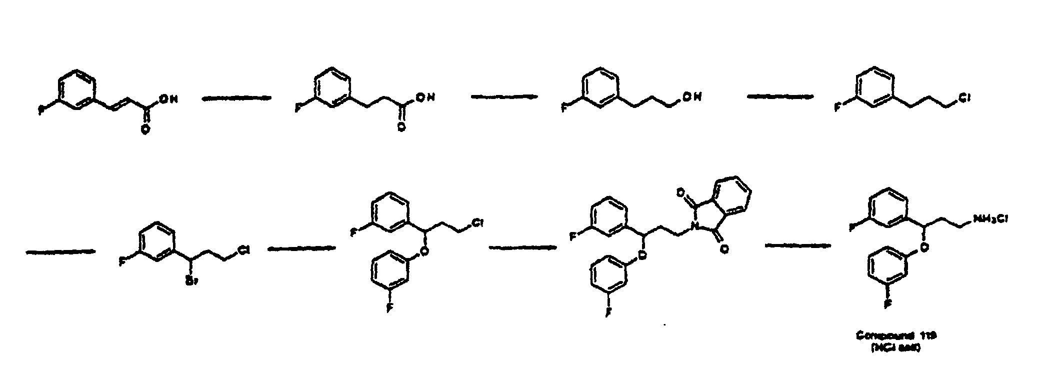

Compounds **118, 120-122** and **137** were prepared in a manner similar to the procedures used for the preparation of Compound **119.**

Compound **113** was synthesized from commercially available 4,4-diphenylcyclohexenone in three steps. First, the alkene in the starting material was reduced by means of catalytic hydrogenation. Methoxylamine formation followed by reduction using standard procedures.

Compounds **67-68, 70-75, 79-82, 84-89, 91-95, 98-100, 102, 105-106, 109-114, 117, 124-134, 138,** and **141-150** were synthesized by standard procedures known to those skilled in the art, as described above.

### Gas Chromatography of Simplified Arylalkylamines

Gas chromatographic and mass spectral data were obtained on a Hewlett-Packard 5890 Series II Gas Chromatograph equipped with a 5971 Series Mass Selective Detector [Ultra-2 Ultra Performance Capillary Column (cross-linked 5% phenyl methyl silicone); column length, 25 m, column i.d., 0.20 mm; The flow rate, 60 mL/min; injector temp., 250°C; gradient temperature program, 20°C/min from 125 to 325°C for 1C min, then held constant at 325°C for 6 min].

Compound **20**. (Rt = 7.34 min), m/z (rel. int.) 247 (M+,27), 231 (16). 230 (100), 229 (45), 215 (29), 214 (14), 204 (43), 203 (37), 202 (13), 201 (47), 184 (14), 183 (58), 181 (8), 151 (9), 135 (13), 134 (31), 133 (25), 124 (18), 122 (16), 121 (19), 109 (15), 101 (29), 96 (18), 95 (11), 83 (11), 75 (20), 57 (10), 42 (9)

Compound **24.** (Rt = 8.21 min), m/z (rel. int.) 259 (M+,122), 260 (23), 242 (44), 241 (15), 228 (15), 227 (49), 216 (15), 213 (56), 212 (16), 211 (55), 195 (32), 196 (22), 185 (34), 184 (19), 183 (67), 171 (16), 170 (38), 165 (44), 151 (20), 150 (16), 146 (13), 136 (46), 134 (17), 133 (37), 123 (15), 121 (22), 120 (13), 109 (100), 91 (34), 77 (29), 51 (15)

Compound **25**. (Rt = 8.49 min), m/z (rel. int.) 259 (M+,39), 243 (16), 242 (95), 241 (25), 227 (27), 217 (15), 216 (100), 215 (27), 212 (13), 211 (50), 201 (14), 200 (11), 199 (15), 196 (15), 185 (20), 184 (19), 183 (50), 171 (24), 170 (28), 165 (15), 146 (10), 136 (11), 134 (12), 133 (23), 121 (21), 77 (9)

Compound **32**. (Rt = 7.30 min), m/z (rel. int.) 229 (M+,21), 213 (16), 212 (100), 211 (61), 197 (33), 196 (19), 194 (14), 186 (26), 185 (30), 184 (19), 183 (69), 170 (17), 166 (16), 165 (77), 134 (25), 133 (23), 116 (17), 115 (17), 103 (18), 101 (11), 78 (13), 77 (23), 75 (13), 51 (18), 43 (13), 42 (13)

Compound **33**. (Rt = 7.56 min), m/z (rel. inc.) 261 (M+,68), 245 (18), 244 (100), 229 (43), 215 (16), 214 (15), 204 (57), 203 (43), 202 (15), 201 (64), 184 (14), 183 (73), 148 (16), 136 (13), 135 (46), 133 (60), 124 (51), 115 (27), 111 (14), 109 (96), 107 (16), 96 (14), 83 (27), 75 (20), 58 (96), 57 (33), 56 (23), 41 (35)

Compound **50**. (Rt = 7.37 min), m/z (rel. int.) 261 (M+,2), 244 (9), 229 (4), 204 (7), 203 (11), 201 (8), 183 (11), 101 (5). 58 (7), 44 (100), 42 (7)

Compound **55**. (Rt = 7.86 min), m/z (rel. int.) 275 (M+,98), 276 (20), 258 (59), 229 (58), 216 (31), 215 (22), 214 (19), 204 (49), 203 (41), 202 (21), 201 (82), 184 (18), 183 (100), 181 (14), 150 (21), 135 (33), 133 (55), 124 (41), 115 (13), 109 (90), 101 (15), 83 (20), 75 (16), 72 (23), 57 (13), 56 (24)

Compound **56**. (Rt = 7.79 min), m/z (rel. int.) 261 (M+,67), 262 (12), 244 (54), 229 (56), 218 (27), 217 (16), 216 (19), 215 (100), 214 (45), 203 (50), 202 (32), 201 (51), 197 (16), 196 (26), 183 (24), 138 (17), 135 (20), 134 (17), 133 (39), 122 (26), 121 (13), 109 (30), 101 (17), 96 (14), 83 (16), 75 (13)

Compound **57.** (Rt = 7.65min), m/z (rel. int.) 261 (M+,62), 244 (50), 229 (50), 218 (24), 217 (13), 216 (18), 215 (100), 214 (36), 203 (42), 202 (19), 201 (33), 197 (14), 196 (19), 183 (17), 138 (19), 135 (16), 134 (12), 133 (29), 122 (29), 109 (25), 101 (13)

Compound **58.** (Rt = 8.15 min), m/z (rel. int.) 275 (M+,134), 276 (26), 258 (23), 244 (19), 243 (100), 232 (25), 229 (53), 217 (51), 216 (23), 215 (67), 214 (97), 201 (44), 197 (21), 196 (43), 183 (23), 148 (38), 147 (21), 138 (46), 135 (46), 134 (18), 133 (64), 125 (25), 123 (28), 122 (81), 115 (27), 109 (54), 107 (17), 83 (27), 44 (19), 43 (19)

Compound **59**. (Rt = 7.61 min), m/z (rel. int.) 275 (M+,27), 204 (8), 203 (10), 201 (19), 183 (25), 109 (8), 101 (7), 58 (100), 57 (8), 56 (8), 44 (9)

Compound **60.** (Rt = 7.34 min), m/z (rel. int.) 261 (M+,55), 262 (10), 204 (16), 203 (15), 201 (31), 183 (35), 133 (11), 122 (11), 121 (10), 109 (9), 101 (16), 96 (11), 75 (10), 57 (9), 44 (100), 42 (11)

Compound **61**. (Rt = 8.07min;, m/z (rel. int., 277 (M+,68), 278 (13), 260 (31), 246 (11), 245 (25), 234 (12), 231 (32), 229 (26), 217 (20), 203 (23), 201 (24), 188 (12), 183 (22), 154 (24), 151 (15), 150 (10), 133 (18), 124 (10), 109 (100), 95 (11), 44 (14)

Compound **62.** (Rt = 8.93 min), m/z (rel. int.) 271 (M+,115), 272 (22), 254 (16), 239 (22), 225 (36), 223 (40), 181 (33), 165 (34), 153 (13), 152 (24), 136 (39), 132 (13), 131 (16), 123 (20), 122 (13), 121 (89), 119 (13), 115 (23), 105 (17), 91 (100), 77 (22)

Compound **63.** (Rt = 8.47min), m/z (rel. int.) 287 (M+,31., 241 (9), 204 (27), 203 (20), 202 (9), 201 (30), 183 (38), 150 (13), 133 (20), 109 (27), 84 (45), 83 (43), 82 (11), 57 (18), 56 (100), 43 (25)

Compound **64**. (Rt = 8.57 min), m/z (rel. int.) 287 (M+,63,, 288 (13), 270 (14), 242 (16), 241 (17), 215 (17), 214 (18), 204 (35), 203 (27), 202 (18), 201 (70), 183 (86), 150 (18), 147 (16), 146 (17), 135 (16), 133 (45), 109 (45), 84 (31), 83 (38), 82 (13), 75 (15), 57 (21), 56 (100), 43 (44)

Compound **65**. (Rt = 8.18 min), m/z (rel. int.) 239 (M+,88), 240 (17), 222 (12), 208 (18), 207 (100), 195 (24), 193 (48), 192 (11), 181 (33), 180 (32), 179 (57), 178 (72), 166 (16), 165 (60), 152 (13), 130 (36), 129 (17), 120 (40), 117 (34), 116 (14), 115 (53), 107 (20), 105 (19), 104 (42), 103 (11), 91 (37), 77 (20), 65 (17)

Compound **66.** (Rt = 7.46 min), m/z (rel. int.) 275 (M+,7), 201 (5), 183 (6), 133 (3), 109 (6), 71 (3), 45 (3), 44 (100), 42 (3)

Compound **67.** (Rt = 7.56 min), m/z (rel. int.) 225 (M+,24), 194 (8), 193 (12), 179 (6), 168 (10), 167 (12), 166 (6), 165 (20), 152 (9), 120 (8), 116 (6), 115 (7), 103 (7), 77 (8), 51 (5), 44 (100)

Compound **68.** (Rt = 7.85 min), m/z (rel. int.) 239 (M+,22), 194 (5), 193 (10), 168 (10), 167 (12), 166 (6), 165 (19), 152 (9), 134 (6), 116 (5), 115 (7), 91 (7), 77 (6), 59 (5), 58 (100), 44 (8)

Compound **69**. (Rt = 7.35min), m/z (rel. int.) 275 (M+,11), 203 (24), 202 (7), 201 (23), 183 (35), 122 (6), 121 (6), 101 (9), 58 (100), 57 (8), 56 (10)

Compound **72.** (Rt = 7.90 min), m/z (rel. int.) 253 (M+,25), 238 (9), 193 (7), 168 (8), 167 (14), 165 (17), 152 (9), 115 (7), 91 (11), 73 (8), 72 (100), 58 (45), 56(7), 44 (6), 43 (9), 42 (8)

Compound **73**. (Rt = 7.29 min), m/z (rel. int.) 239 (M+,9), 240 (2), 167 (2), 165 (5), 152 (2), 115 (2), 77 (2), 59 (5), 58 (100), 44 (3), 42 (5)

Compound **79**. (Rt = 7.89 min), m/z (rel. int.) 230 (M+,37), 214 (15), 213 (100), 212 (62), 201 (26), 200 (72), 198 (21), 195 (12), 188 (17), 187 (85), 186 (46), 185 (42), 184 (9), 157 (12), 135 (9), 133 (24), 109 (10), 107 (20), 106 (62), 80 (14), 79 (32), 78 (9), 51 (20)

Compound **81**. (Rt = 7.40 min), m/z (rel. int.) 209 (M+, 89), 210 (14), 208 (100), 193 (17), 192 (56), 191 (42), 189 (12), 178 (20), 166 (11), 165 (45), 152 (12), 132 (86), 131 (10), 130 (53), 117 (22), 115 (48), 106 (22), 105 (10), 104 (12), 103 (16), 91 (16), 77 (22), 51 (15)

Compound **82.** (Rt = 7.93min), m/z (rel. int.) 275 (M+, 124), 276 (25), 232 (33), 215 (12), 214 (16), 204 (14), 203 (100), 201 (24), 196 (8), 183 (20), 150 (14), 138 (9), 136 (14), 135 (44), 133 (26), 125 (9), 124 (71), 123 (29), 121 (14), 115 (14), 111 (72), 110 (9), 109 (84), 101 (14), 83 (9), 75 (8)

Compound **83.** (Rt = 7.22 min), m/z (rel. int.) 235 (M+,10), 219 (17), 218 (100), 217 (62), 203 (20), 192 (10), 191 (38), 190 (7), 189 (14), 185 (17), 183 (7), 171 (9), 165 (8), 147 (10), 146 (11), 134 (12), 133 (17), 121 (8), 109 (8), 97 (8), 45 (7)

Compound **85**. (Rt = 7.73 min), m/z (rel. int.) 239 (M+, 7), 222 (15), 179 (8), 178 (9), 168 (16), 167 (33), 166 (12), 165 (43), 161 (9), 152 (20), 146 (17), 129 (7), 120 (15), 118 (7), 117 (19), 115 (25), 91 (25), 77 (7), 72 (9), 44 (100), 42 (6)

Compound **86**. (Rt = 7.66 min), m/z (rel. int.) 239 (M+,3), 222 (4), 168 (4), 167 (11), 166 (4), 165 (14), 152 (7), 120 (6), 117 (6), 115 (8), 91 (9), 72 (5), 44 (100), 42 (3)

Compound **87**. (Rt = 7.33 min), m/z (rel. int.) 239 (M+,4), 222 (9), 179 (9), 178 (11), 168 (11), 167 (27), 166 (13), 165 (48), 161 (7), 152 (22), 146 (14), 128 (7), 120 (11), 118 (8), 117 (21), 115 (31), 91 (29), 77 (9), 72 (8), 51 (7), 44 (100), 42 (9)

Compound **88**. (Rt = 7.4 min), m/z (rel. int.) 227 (M+,.0), 183 (10), 168 (18), 167 (100), 166 (32), 165 (83), 164 (10), 163 (6), 153 (6), 152 (35), 139 (6), 115 (8), 105 (9), 77 (12), 51 (7), 45 (23)

Compound **89.** (Rt = 8.74 min), m/z (rel. int.) 260 (M+,220), 261 (39), 259 (89), 242 (18), 203 (17), 202 (16), 201 (61), 183 (58), 165 (100), 150 (20), 148 (25), 138 (24), 137 (61), 122 (73), 121 (31), 111 (47), 101 (23), 96 (16), 75 (16), 44 (17), 43 (29)

Compound **90**. (Rt = 7.32min), m/z (rel. int.) 235 (M+,9), 219 (16), 218 (100), 217 (42), 206 (17), 205 (9), 204 (7), 203 (21), 202 (8), 193 (12), 192 (71), 191 (62), 190 (9), 189 (19), 185 (13), 171 (14), 159 (9), 147 (14), 146 (16), 134 (10), 133 (17), 121 (14), 109 (11), 101 (8), 97 (17), 45 (15)

Compound **91**. (Rt = 10.67 min), m/z (rel. int.) 329 (M+,6), 301 (20), 300 (81), 167 (18), 166 (6), 165 (18), 152 (10), 132 (5), 120 (45), 119 (21), 118 (11), 117 (9), 115 (11), 106 (6), 105 (5), 104 (12), 103 (5), 92 (8), 91 (100), 77 (10), 41 (6)

Compound **92.** (Rt = 10.37min), m/z (rel. int.) 337 (M+,30), 338 (7), 204 (7), 203 (7), 201 (7), 183 (10), 133 (6), 121 (8), 120 (70), 106 (6), 92 (9), 91 (100)

Compound **93**. (Rt = 10.25 min), m/z (rel. int.) 351 (M+,28), 352 (7), 337 (9), 336 (39), 203 (10), 201 (11), 183 (17), 135 (6), 134 (20), 133 (6), 132 (6), 120 (11), 118 (5), 109 (18), 106 (12), 105 (100), 104 (13), 103 (8), 91 (14), 79 (11), 77 (12)

Compound **94**. (Rt = 10.48 min), m/z (rel. int.) 365 (M+,2), 337 (25), 336 (100), 203 (8), 201 (8), 183 (14), 133 (5), 132 (6), 120 (14), 119 (13), 118 (9), 115 (5), 109 (20), 106 (5), 104 (10), 91 (52)

Compound **95.** (Rt = 6.68min), m/z (rel. int.) 283 (M+,59), 284 (11), 267 (11), 266 (71), 265 (19), 251 (24), 250 (9), 241 (14), 240 (100), 239 (48), 237 (30), 232 (10), 220 (17), 219 (65), 199 (9), 152 (12), 151 (18), 142 (20), 140 (13), 139 (20), 127 (22), 119 (24), 114 (12), 101 (10), 63 (10), 44 (9)

Compound **96.** (Rt = 6.93 min), m/z (rel. int.) 265 (M+,46), 249 (16), 248 (100), 247 (34), 233 (27), 232 (11), 223 (9), 222 (65), 221 (39), 220 (10), 219 (36), 202 (14), 201 (54), 152 (15), 151 (14), 133 (9), 124 (12), 119 (9), 109 (9), 101 (14), 75 (9)

Compound **97.** (Rt = 8.10 min), m/z (rel. int.) 241 (M+,101), 242 (18), 224 (50), 223 (19), 210 (11), 209 (37), 197 (12), 196 (10), 195 (55), 194 (16), 193 (60), 181 (29), 178 (20), 167 (38), 166 (16), 165 (52), 153 (12), 152 (36), 136 (27), 133 (12), 132 (14), 116 (12), 115 (25), 103 (13), 91 (100), 77 (18)

Compound **98.** (Rt = 6.69 min), m/z (rel. int.) 232 (M+,3), 204 (11), 203 (37), 202 (30), 201 (100), 188 (9), 184 (14), 183 (84), 182 (10), 181 (15), 170 (9), 109 (17), 107 (10), 83 (10), 75 (8), 57 (7)

Compound **100.** (Rt = 7.66 min). m/z (rel. int.) 261 (M+,150), 262 (29), 217 (11), 216 (70), 215 (28), 214 (11), 203 (30), 202 (31), 201 (100), 196 (10), 184 (15), 183 (90), 181 (11), 133 (20), 124 (12), 122 (20), 109 (39), 101 (14), 83 (10), 75 (10), 45 (43)

Compound **101.** (Rt = 7.72 min), m/z (rel, int.) 245 (M+,20). 229 (16), 228 (100), 227 (36), 213 (21), 211 (22), 202 (57), 201 (30), 199 (21), 183 (50), 181 (14), 171 (15), 170 (26), 165 (12), 152 (21), 134 (19), 133 (35), 122 (28), 120 (19), 120 (13), 119 (12), 109 (20), 107 (20), 106 (18), 101 (15), 94 (15), 91 (20), 77 (18), 74 (15), 65 (20), 63 (14), 55 (14), 51 (15), 44 (27), 43 (17), 42 (14)

Compound **102.** (Rt = 8.33 min), m/z (rel. int.) 273 (M+,19), 204 (16), 203 (16), 201 (15), 183 (18), 177 (9), 133 (8), 109 (13), 70 (41), 69 (100), 68 (20), 43 (25), 42 (5), 41 (5)

Compound **103.** (Rt = 8.59 min), m/z (rel. int.) 245 (M+,118), 246 (20), 229 (15), 228 (100), 227 (85), 213 (27), 211 (23), 209 (15), 207 (12), 202 (19), 201 (32), 200 (17), 199 (84), 196 (10), 183 (38), 181 (15), 171 (13), 170 (23), 152 (19), 151 (15), 150 (10), 134 (18), 133 (32), 131 (12), 122 (36), 119 (15), 109 (24), 107 (10), 106 (12), 91 (19), 77 (12)

Compound **105.** (Rt = 10.24 min), m/z (rel. int.) 351 (M+,7), 201 (5), 183 (7), 135 (9), 134 (79), 133 (4), 109 (5), 92 (8), 91 (100), 65 (8), 42 (7)

Compound **106.** (Rt = 7.52 min), m/z (rel. int.) 259 (M-,77:, 260 (14), 258 (31), 244 (30), 228 (13), 227 (28), 214 (14), 201 (24), 165 (12), 164 (100), 162 (29), 133 (56), 109 (44), 75 (13), 44 (80), 42 (56)

Compound **107**. (Rt = 7.45 min), m/z. (rel. int.) 227 (M+,101), 228 (16), 226 (100), 211 (22), 210 (68), 209 (49), 207 (13), 196 (22), 184 (15), 183 (62), 150 (50), 148 (31), 133 (44), 132 (53), 130 (45), 117 (15), 115 (29), 106 (14), 77 (18), 75 (13), 51 (14)

Compound **108**. (Rt = 7.46 min), m/z (rel. int.) 243 (M+,34), 244 (6), 212 (6), 211 (9), 197 (6), 186 (12), 185 (10), 184 (5), 183 (19), 165 (15), 133 (6), 120 (6), 103 (5), 77 (6), 44 (100), 42 (6)

Compound **109**. (Rt = 8.68 min), m/z (rel. int.) 285 (M+,110), 286 (22), 284 (27), 256 (16), 228 (37), 227 (27), 225 (10), 220 (11), 207 (15), 201 (27), 191 (14), 190 (100), 163 (11), 162 (85), 161 (10), 147 (11), 146 (11), 133 (32), 109 (20), 83 (12), 82 (36)

Compound **111.** (Rt = 8.81 min), m/z (rel. int.) 287 (M+,29), 214 (9), 204 (15), 203 (18), 202 (9), 201 (34), 183 (42), 135 (9), 133 (28), 109 (28), 84 (47), 83 (100), 82 (19), 75 (5), 70 (16), 68 (13), 57 (18), 56 (28), 44 (16), 43 (25), 42 (14)

Compound **114.** (Rt = 8.71 min), m/z (rel. int.) 237 (M+,197), 238 (37), 236 (67), 193 (15), 179 (30), 178 (40), 165 (41), 159 (43), 158 (26), 132 (24), 130 (16), 116 (17), 115 (37), 106 (21), 103 (34), 91 (50), 77 (48), 57 (68), 56 (100), 51 (32), 43 (50), 42 (34)

Compound **115.** (Rt = 9.45 min), m/z (rel. int.) 271 (M+,34), 255 (12), 254 (67), 253 (14), 239 (23), 229 (16), 228 (100), 227 (18), 224 (16), 223 (68), 213 (9), 212 (10), 211 (10), 197 (34), 196 (17), 195 (11), 181 (18), 169 (10), 165 (22), 153 (19), 152 (27), 146 (16), 145 (13), 141 (12), 139 (10), 136 (22), 134 (11), 133 (41), 122 (16), 121 (31), 115 (30), 91 (18), 77 (15), 65 (11), 63 (10), 44 (10)

Compound **116**. (Rt = 9.50 min), m/z (rel. int.) 269 (M-,41), 268 (32), 254 (8), 253 (21), 252 (100), 251 (14), 238 (23), 237 (18), 221 (10), 209 (9), 178 (8), 165 (19), 162 (22), 160 (19), 152 (18), 147 (11), 146 (8), 145 (18), 110 (9), 130 (11), 115 (10)

Compound **117.** (Rt = 7.64 min), m/z (rel. int.) 212 (M+, 13), 183 (16), 182 (100), 180 (7), 167 (7), 152 (3), 104 (27), 91 (7), 78 (4), 77 (41), 51 (13)

Compound **118.** (Rt = 7.46 min), m/z (rel. int.) 245 (M+,4), 153 (8), 152 (43), 150 (9), 135 (6), 133 (10), 124 (5), 123 (36), 122 (38), 121 (17), 109 (16), 101 (14), 96 (24), 95 (16), 94 (100), 93 (7), 83 (7), 77 (21), 75 (11), 66 (15), 65 (30), 63 (10), 51 (14), 50 (6)

Compound **119**. (Rt = 7.39 min), m/z (rel. int.) 263 (M+,7), 171 (14), 170 (14), 152 (74), 151 (13), 150 (20), 141 (55), 135 (10), 133 (23), 123 (20), 122 (100), 121 (49), 120 (11), 113 (9), 112 (92), 111 (9), 109 (41), 107 (12), 103 (13), 102 (11), 101 (40), 97 (9), 96 (66), 95 (51), 94 (9), 84 (28), 83 (88), 82 (8), 81 (16), 77 (14), 75 (54), 74 (10), 70 (10), 69 (10), 64 (10), 63 (23), 57 (62), 56 (13), 51 (15), 50 (12), 42 (8)

Compound **120.** (Rt = 8.48 min), m/z (rel. int.) 279 (M+,4), 159 (16), 157 (49), 153 (11), 152 (100), 150 (12), 133 (11), 130 (27), 128 (73), 123 (12), 122 (57), 121 (23), 111 (10), 109 (25), 101 (23), 99 (16), 96 (26), 95 (10), 83 (9), 75 (28), 73 (10), 65 (12), 64 (11), 63 (22), 51 (9), 50 (8)

Compound **121**. (Rt = 8.30 min), m/z (rel. int.) 275 (M+,2), 152 (15), 125 (B), 124 (100), 122 (14), 121 (7), 109 (35), 96 (7), 95 (10), 81 (14), 77 (9), 65 (7), 52 (11)

Compound **122.** (Rt = 7.39 min), m/z (rel. int.) 263 (M+,.1), 170 (12), 152 (66), 151 (10), 150 (18), 141 (68), 135 (10), 133 (19), 123 (16), 122 (76), 121 (39), 112 (100), 111 (18), 109 (36), 107 (11), 103 (11), 102 (9), 101 (33), 96 (56), 95 (32), 92 (11), 83 (96), 81 (13), 77 (13), 75 (43), 64 (25), 63 (26), 57 (61), 56 (14), 51 (14), 50 (11)

Compound **123**. (Rt = 5.88 min), m/z (rel. int.) 275 (M+,46), 276 (9), 202 (8), 201 (30), 183 (28), 133 (8), 109 (9), 101 (9), 71 (9), 59 (12), 58 (100), 44 (8), 42 (26)

Compound **124.** (Rt = 7.05 min), m/z (rel. int.) 229 (M+,15), 213 (15), 212 (89), 211 (13), 196 (20), 197 (100), 196 (24), 186 (12), 185 (21), 184 (29), 183 (87), 179 (7), 178 (8), 177 (13), 176 (5), 171 (7), 170 (18), 169 (4), 166 (5), 165 (20), 152 (5), 133 (7), 75 (4), 63 (4), 57 (9), 56 (4)

Compound **125.** (Rt = 7.54 min), m/z (rel. int.) 225 (M+, 57), 226 (13), 209 (13), 208 (75), 193 (13), 180 (14), 179 (21), 178 (20), 165 (22), 130 (34), 117 (59), 115 (28), 105 (18), 104 (94), 103 (45), 91 (100), 78 (30), 77 (38), 65 (36), 63 (13), 51 (20), 45 (17)

Compound **126**. (Rt = 7.81 min). m/z (rel. int.) 261 (M+,12), 244 (31), 152 (27), 151 (17), 150 (9), 136 (11), 135 (100), 133 (21), 122 (24), 115 (5), 110 (13), 109 (90), 107 (6), 96 (7), 83 (27), 56 (7)

Compound **127**. (Rt = 7.93 min), m/z (rel. int.) 225 (M+,23), 208 (20), 207 (6), 193 (13), 181 (7), 180 (37), 179 (100), 178 (36), 167 (9), 166 (12), 165 (36), 152 (9), 134 (30), 130 (26), 129 (9), 117 (16), 115 (22), 104 (6), 91 (38), 77 (7), 65 (7)

Compound **128.** (Rt = 7.42 min), m/z (rel. int.) 211 (M+,83), 212 (15), 194 (36), 193 (18), 182 (62), 181 (20), 180 (17) 179 (53), 178 (60), 176 (11), 167 (57), 166 (44), 165 (100), 152 (24), 120 (39), 116 (12), 115 (28), 104 (22), 103 (15), 91 (46), 89 (16), 78 (10), 77 (20), 65 (15), 63 (12), 51 (12)

Compound **129.** (Rt = 7.39 min), m/z (rel. int.) 229 (M+,104), 230 (19), 212 (28), 211 (14), 201 (13), 200 (85), 199 (22), 198 (14), 197 (50), 196 (58), 185 (73), 184 (45), 183 (100), 179 (43), 178 (55), 177 (17), 176 (17), 170 (18), 165 (33), 152 (12), 133 (22), 120 (57), 115 (17), 109 (44), 104 (23), 103 (17), 91 (32), 89 (16), 83 (20), 78 (12), 77 (22), 63 (16), 51 (13)

Compound **130.** (Rt = 7.38 min), m/z (rel. int.) 229 (M+,133), 230 (24), 212 (27), 211 (14), 200 (54), 199 (17), 198 (16), 197 (53), 196 (64), 185 (49), 184 (43), 183 (100), 179 (28), 178 (29), 177 (14), 170 (19), 165 (26), 133 (22), 120 (35), 115 (19), 109 (32), 104 (17), 103 (18), 91 (38), 89 (17), 83 (18), 77 (24), 63 (16)

Compound **131.** (Rt = 7.40 min), m/z (rel. int.) 229 (N+,146), 230 (26), 212 (48), 211 (23), 200 (51), 199 (17), 198 (16), 197 (61), 196 (70), 185 (50), 184 (43), 183 (100), 179 (28), 178 (28), 170 (20), 165 (23), 133 (21), 120 (35), 115 (20), 109 (59), 104 (25), 103 (17), 91 (27), 89 (17), 83 (22), 77 (22)

Compound **132**. (Rt = 7.03 min), m/z (rel. int.) 0 (M+,.0), 185 (14), 184 (100), 183 (23), 181 (17), 165 (18), 155 (12), 153 (14), 152 (12), 120 (85), 119 (67), 115 (10). 106 (16), 91 (19), 89 (14), 78 (12), 77 (25), 51 (16)

Compound **133**. (Rt = 7.09 min), m/z (rel. int.) 211 (M+,13), 195 (16), 194 (100), 181 (27), 180 (70), 179 (31), 178 (28), 166 (25), 165 (40), 152 (9), 120 (14), 119 (14), 118 (12), 115 (10), 104 (26), 103 (53), 102 (12). 91 (62), 89 (10), 78 (13), 77 (42), 65 (17), 51 (13)

Compound **134**. (Rt = 7.45 min), m/z (rel. int.) 211 (M⁺,14), 183 (15), 182 (100), 181 (14), 179 (13), 178 (18), 167 (27), 166 (18), 165 (46), 152 (10), 115 (8), 104 (8), 103 (6), 91 (29), 89 (7), 78 (5), 77 (7), 65 (7)

Compound **135**. (Rt = 8.60 min), m/z (rel. int.) 273 (M+,34), 257 (14), 256 (76), 231 (16), 230 (100), 228 (18), 227 (57), 213 (14), 211 (37), 202 (30), 201 (40), 199 (26), 184 (13), 183 (50), 181 (12), 171 (17), 170 (20), 152 (15), 150 (19), 134 (15), 133 (31), 122 (14), 121 (29), 109 (16), 107 (13), 106 (17), 91 (12), 65 (12)

Compound **136**. (Rt = 9.26 min), m/z (rel. int.) 275 (M+,44), 277 (15), 260 (28), 259 (19), 258 (81), 257 (13), 243 (15), 234 (33), 233 (19), 232 (100), 231 (13), 229 (15), 227 (42), 224 (15), 223 (86), 208 (13), 197 (45), 196 (26), 195 (13), 182 (14), 181 (33), 179 (11), 178 (18), 166 (22), 165 (60), 164 (12), 163 (10), 153 (32), 152 (55), 151 (18), 149 (10), 139 (11), 137 (17), 136 (19), 121 (13), 115 (25), 102 (11), 91 (16), 77 (17)

Compound **137**. (Rt = 7.42 min), m/z (rel. int.) 245 (M+,1), 153 (8), 152 (7), 141 (64), 135 (10), 134 (100), 132 (11), 117 (6), 115 (12), 112 (56), 105 (15), 104 (55), 103 (32), 95 (8), 91 (16), 84 (8), 83 (15), 78 (24), 77 (24), 75 (9), 65 (6), 63 (8), 57 (10), 51 (9)

Compound **138**. (Rt = 9.24 min), m/z (rel. int.) 289 (M+,77), 290 (16), 230 (20), 229 (21), 215 (15), 203 (22), 201 (32), 183 (36), 134 (10), 133 (13), 124 (10), 121 (9), 109 (10), 101 (10), 73 (100), 43 (23)

Compound **139**. (Rt = 7.25 min), m/z (rel. int.) 245 (M+,92), 246 (15), 244 (67), 229 (16), 228 (63), 227 (46), 225 (10), 224 (15), 214 (13), 201 (39), 183 (13), 151 (13), 150 (100), 149 (14), 148 (58), 135 (22), 133 (54), 124 (14), 122 (12), 109 (18), 101 (15), 75 (13) (20). 131 (10)

Compound **141**. (Rₜ = 8.44 min), m/z (rel. int.) 257 (M+,48), 258 (8), 256 (36), 241 (21), 240 (100), 239 (19), 226 (2-2), 225 (20), 209 (11), 197 (14), 196 (18), 183 (25), 170 (16), 162 (19), 160 (10), 150 (28), 148 (26), 147 (9), 146 (8), 145 (13), 133 (20), 130 (8), 121 (10)

Compound **142**. (Rₜ = 8.47 min) , m/z (rel. int.) 273 (M+,14), 217 (5), 216 (31), 215 (5), 183 (8), 170 (4), 150 (5), 121 (4), 58 (5), 45 (5), 44 (100)

Compound **143**. (Rₜ = 9.39 min), m/z (rel. int.) 273 (M+,47), 275 (16), 274 (19), 272 (36), 258 (39), 257 (26), 256 (100), 255 (17), 242 (25), 241 (15), 221 (23), 178 (25), 177 (11), 176 (14), 168 (14), 167 (11), 166 (54), 165 (34), 164 (34), 163 (16), 162 (45), 160 (19), 152 (28), 151 (22), 149 (19), 147 (18), 145 (24), 139 (11), 136 (15), 131 (15), 130 (35), 121 (15), 115 (14), 111 (11), 103 (13), 102 (19), 89 (11), 77 (16), 75 (14), 63 (16), 51 (12)

Compound **148.** (Rt = 8.43 min), m/z (rel. int.) 261 (M+, 3), 170 (14), 169 (5), 168 (44), 153 (4), 151 (4), 140 (6), 139 (4), 138 (15), 132 (6), 125 (7), 123 (40), 115 (6), 103 (24), 102 (8), 101 (5), 95 (7), 94 (100), 89 (5), 77 (22), 75 (6), 66 (8), 65 (16), 63 (7), 51 (10), 50 (4).

Compound **149.** (Rt = 9.28 min), m/z (rel. int.) 295 (M+,4), 170 (32), 169 (12), 169 (100), 166 (8), 159 (22), 157 (66), 152 (11), 140 (16), 139 (11), 138 (41), 132 (11), 130 (32), 129 (8), 128 (82), 127 (10), 125 (16), 115 (12), 111 (15), 103 (55), 102 (18), 101 (15), 99 (19), 89 (10), 77 (26), 76 (8), 75 (27), 73 (11), 65 (11), 64 (10), 63 (22), 51 (11).

Compound **150**. (Rt = 8.32 min), m/z (rel. int.) 279 (M+,4), 171 (9), 170 (37), 169 (13), 168 (100), 166 (8), 142 (8), 141 (88), 140 (19), 139 (12), 138 (42), 132 (12), 130 (7), 125 (16), 115 (12), 113 (10), 112 (89), 111 (11), 104 (8), 103 (60), 102 (19), 101 (12), 95 (14), 89 (11), 84 (11), 83 (24), 77 (29), 76 (6), 75 (24), 63 (13), 57 (17), 51 (11).

### Example 30: Biological properties of synthesized arylalkylamines

Compounds synthesized as described in Example 28 and Example 29 were tested for various biological properties detailed in the examples.

**Table 1**

| **Compound** | IC₅₀ (µm)vs. NMDA^{a} | IC₅₀ (µm)vs. [¹H]MK-801^{c} |
|---|---|---|
| Compound **54** | 0.036 (4) | 0.046 (3) |
| Compound **55** | 0.035 (3) | 0.153 (2) |
| Compound **56** | 0.218 (4) | 0.955 (2) |
| Compound **57** | 0.028 (4) | 0.063 (2) |
| Compound **58** | 0.028 (2) | 0.203 (3) |
| Compound **59** | 0.272 (2) | 0.453 (3) |
| Compound **60** | 0.416 (11) | 0.641 (9) |
| Compound **61** | 0.134 (4) | 0.324 (2) |
| Compound **62** | 0.177 (5) | 0.617 (1) |
| Compound **63** | 0.093 (6) | 0.245 (3) |
| Compound **64** | 0.309 (3) | 0.851 (2) |
| Compound **65** | 0.167 (3) | 2.0 (1) |
| Compound **66** | 0.236 (4) | 1.2 (2) |
| Compound **67** | 10.95 (2) | 2.9 (1) |
| Compound **68** | 2.9 (1) | not tested |
| Compound **69** | 0.224 (2) | 0.366 (1) |
| Compound **70** | 1.7 (1) | not tested |
| Compound **71** | 6.35 (2) | not tested |
| Compound **72** | 7.4 (1) | not tested |
| Compound **73** | 12.6 (1) | not tested |
| Compound **75** | 0.94 (2) | not tested |
| Compound **76** | 0.73 (2) | not tested |
| Compound **77** | 5.5 (2) | not tested |
| Compound **78** | 10.2 (1) | not tested |
| Compound **79** | 12.6 (4) | 10.2 (2) |
| Compound **81** | 1.4 (1) | 6.1 (2) |
| Compound **82** | 0.106 (5) | 0.794 (1) |
| Compound **83** | 0.342 (4) | 0.794 (1) |
| Compound **84** | 7.9 (2) | 23.4 (1) |
| Compound **85** | 1.2 (3) | 3.5 (1) |
| Compound **86** | 1.2 (3) | 6.0 (1) |
| Compound **87** | 0.657 (4) | 3.0 (1) |
| Compound **88** | 2.5 (3) | 10.6 (2) |
| Compound **89** | 0.240 (3) | 1.2 (2) |
| Compound **90** | 0.270 (4) | 1.4 (2) |
| Compound **91** | 0.162 (3) | 14.1 (2) |
| Compound **92** | 1.3 (3) | 20.2 (2) |
| Compound **93** | 0.486 (3) | 26.9 (2) |
| Compound **94** | 0.248 (4) | 22.6 (2) |
| Compound **95** | 0.311 (3) | 3.0 (2) |
| Compound **96** | 0.187 (5) | 1.1 (2) |
| Compound **97** | 0.410 (3) | 2.6 (1) |
| Compound **98** | 7.9 (1) | 52.5 (2) |
| Compound **100** | 0.602 (2) | 3.2 (1) |
| Compound **101** | 0.912 (2) | 2.0 (1) |
| Compound **102** | 1.01 (2) | 3.3 (1) |
| Compound **103** | 0.380 (4) | 0.661 (2) |
| Compound **105** | 1.03 (1) | > 3 (1) |
| Compound **106** | 0.767 (1) | 1.31 (1) |
| Compound **107** | 2.67 (1) | 3.83 (1) |
| Compound **108** | 1.06 (1) | 0.942 (1) |
| Compound **109** | 2.0 (2) | 0.882 (1) |
| Compound **111** | 0.790 (3) | 0.137 (1) |
| Compound **114** | 5.25 (1) | not tested |
| Compound **115** | 1.9 (1) | not tested |
| Compound **116** | 4.47 (1) | not tested |
| Compound **117** | 15.83 (3) | 5.73 (1) |
| Compound **118** | 0.498 (2) | 0.336 (1) |
| Compound **119** | 0.122 (2) | 0.137 (1) |
| Compound **120** | 0.112 (2) | 0.128 (1) |
| Compound **121** | 0.835 (2) | 0.773 (1) |
| Compound **122** | 0.275 (1) | not tested |
| Compound **123** | 9.6 (7) | > 3 (2) |
| Compound **124** | 3.5 (1) | 14.3 (3) |
| Compound **125** | 1.7 (1) | 6.7 (2) |
| Compound **126** | 0.398 (3) | 6.0 (1) |
| Compound **127** | 1.2 (3) | 17.5 (2) |
| Compound **128** | 0.646 (4) | 5.5 (1) |
| Compound **129** | 1.26 (2) | not tested |
| Compound **130** | 0.851 (2) | not tested |
| Compound **131** | 1.23 (2) | not tested |
| Compound **132** | 1.3 (1) | 6.4 (1) |
| Compound **133** | 0.760 (1) | 3.0 (1) |
| Compound **134** | 2.5 (1) | > 10 (1) |
| Compound **135** | 0.403(2) | not tested |
| Compound **136** | 0.226(2) | not tested |
| Compound **137** | 0.346(2) | not tested |
| Compound **138** | 138.0(1) | not tested |
| Compound **139** | 1.97(2) | not tested |
| Compound **141** | 5.2(1) | not tested |
| Compound **142** | not tested | not tested |
| Compound **143** | 3.1(1) | not tested |
| Compound **144** | not tested | not tested |
| Compound **145** | not tested | not tested |
| Compound **146** | 1.1(1) | 0.372(1) |
| Compound **147** | 0.894(2) | not tested |
| Compound **148** | not tested | not tested |
| Compound **149** | not tested | not tested |
| Compound **150** | not tested | not tested |

| | | |
|---|---|---|
| ^{a}:Inhibition of NMDA/glycine-induced increases in intracellular calcium in cultured rat cerebellar granule cells (RCGC's) (see Example 1). (# in parentheses indicates the number of experiments). ^{b}:TFA salt. | | |
| ^{c}:Inhibition of [³H]MK-801 binding in rat cortical/ hippocampal washed membrane preparations (see Example 4). ^{d}:IC₅₀ study incomplete. % inhibition at the stated concentration. | | |

The simplified arylalkylamines exemplified by Compounds 54 - 150 bind to the site labeled by [³H]MK-801 at concentrations ranging approximately 1 to 400-fold higher than those which antagonize NMDA receptor-mediated function in the rat cerebellar granule cell assay.

Some of the simplified arylalkylamines disclosed have structural features similar to portions of other compounds which are utilized as, for example, anticholinergics, antiparkinsonians, antihistamines, antidepressants, calcium channel blockers, coronary vasodilators, opiate analgesics, and antiarrhythmics. However, when certain of these compounds were evaluated for NMDA receptor antagonist potency (Example 1), as can be seen in Table 2, none of the compounds tested, with the exception of (R)- and (S)-fendiline and nisoxetine, had IC₅₀ values less than 1 µM. These data are summarized in Table 2.

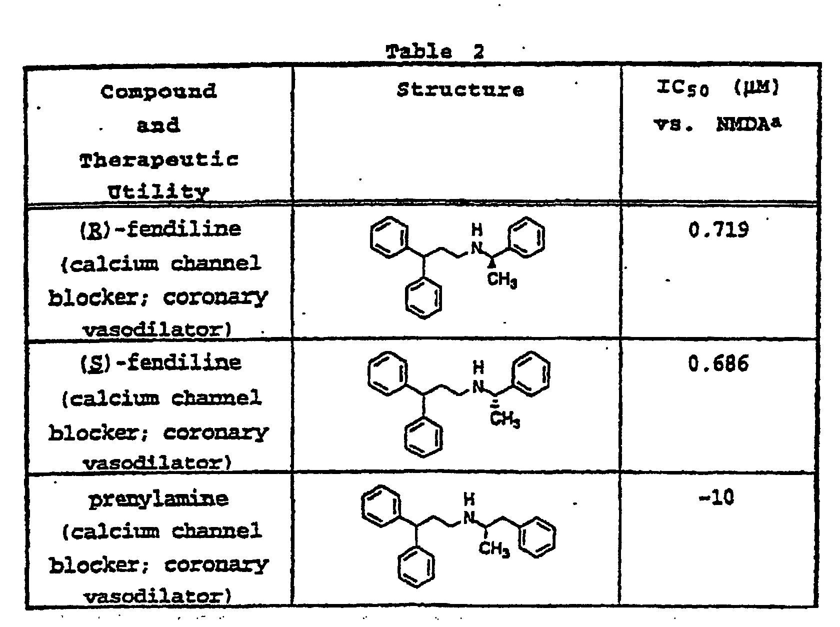

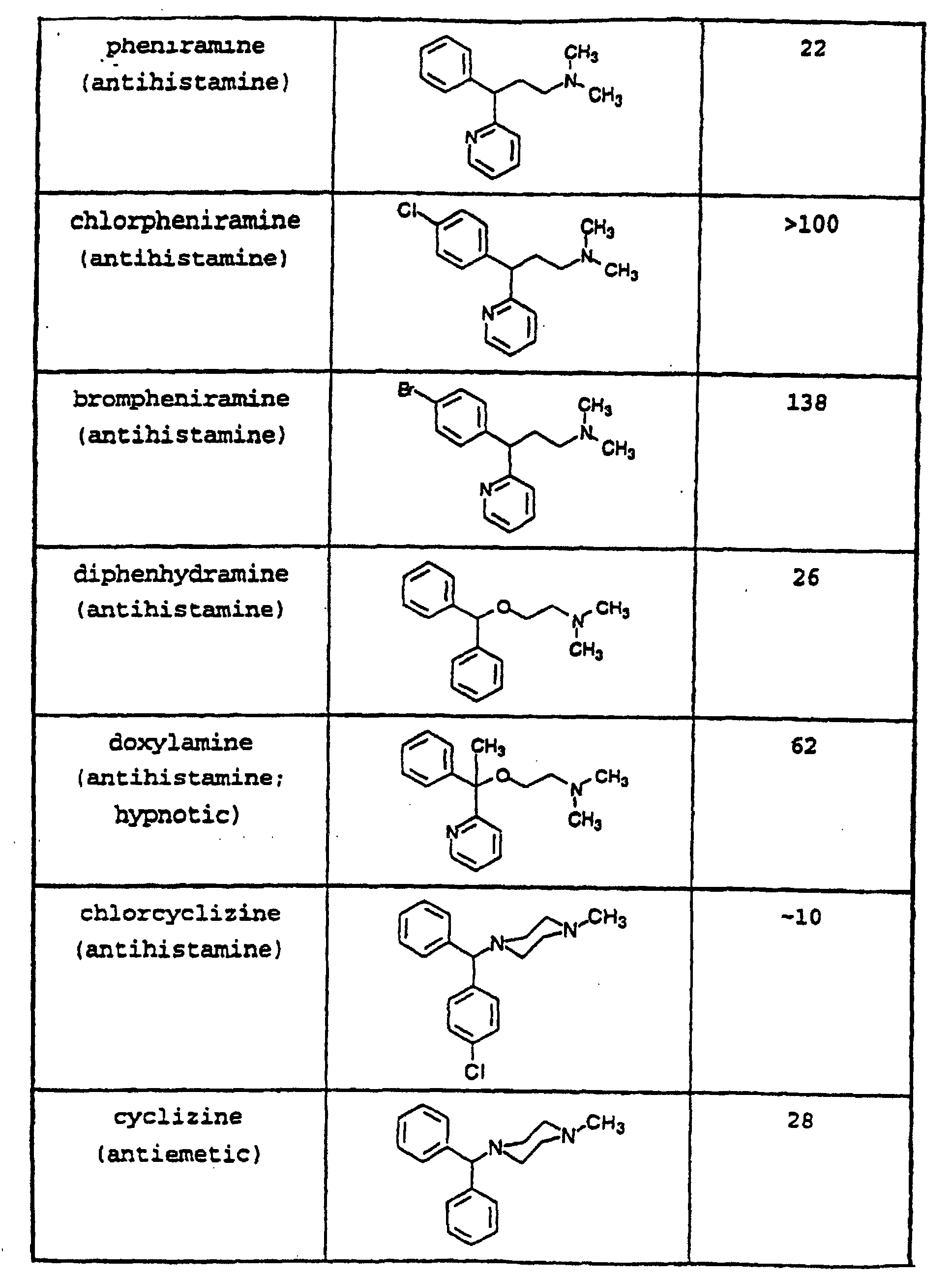

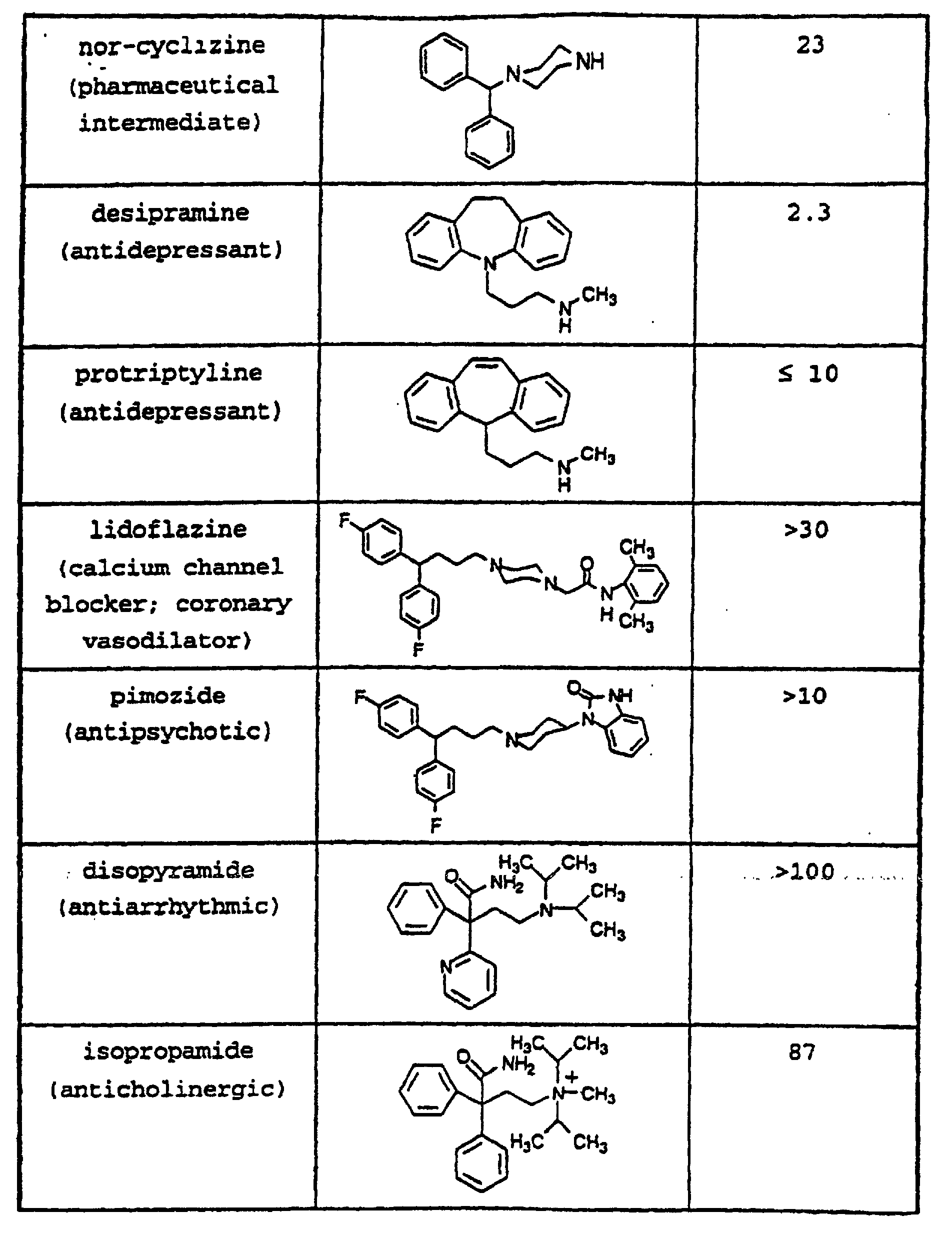

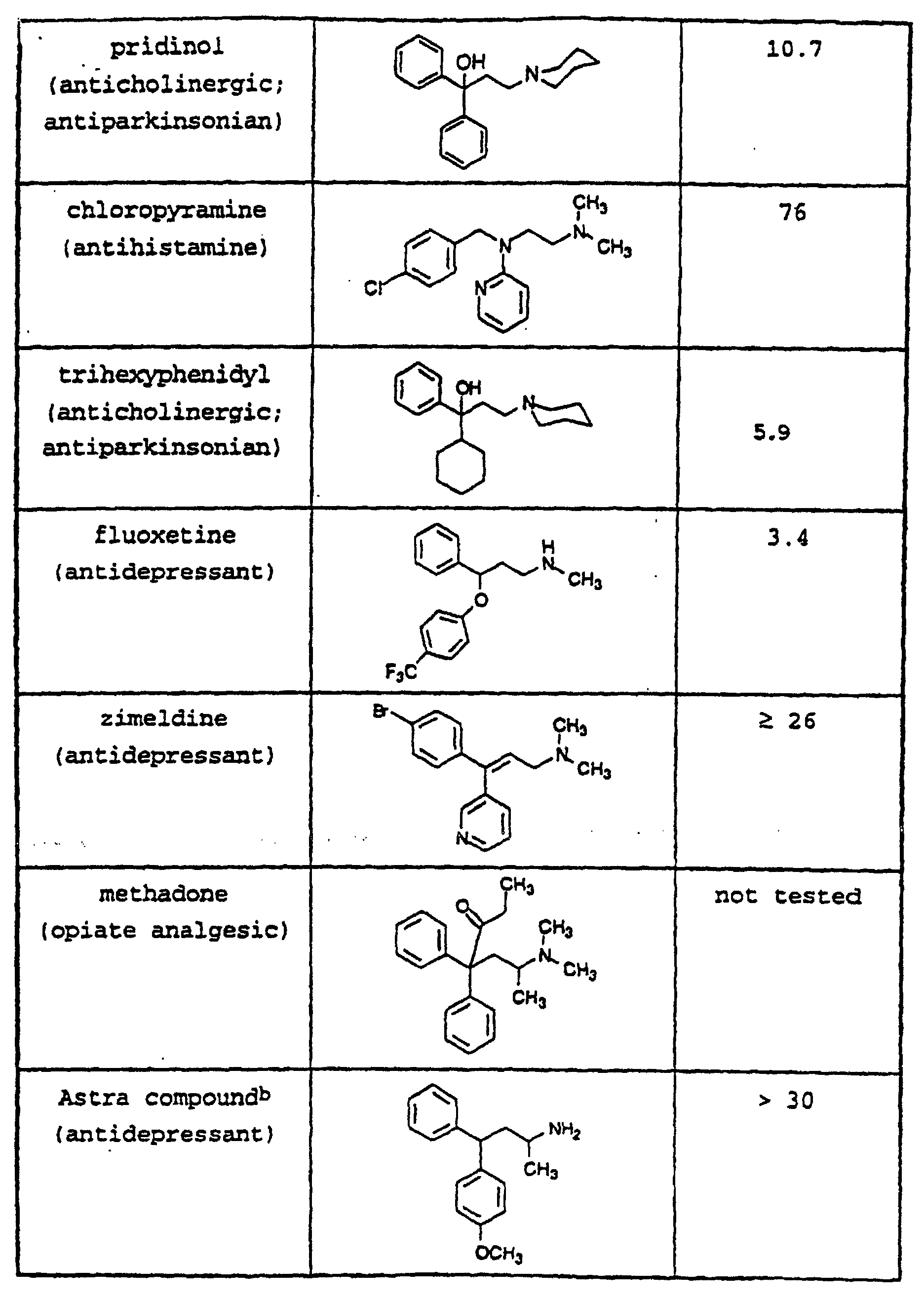

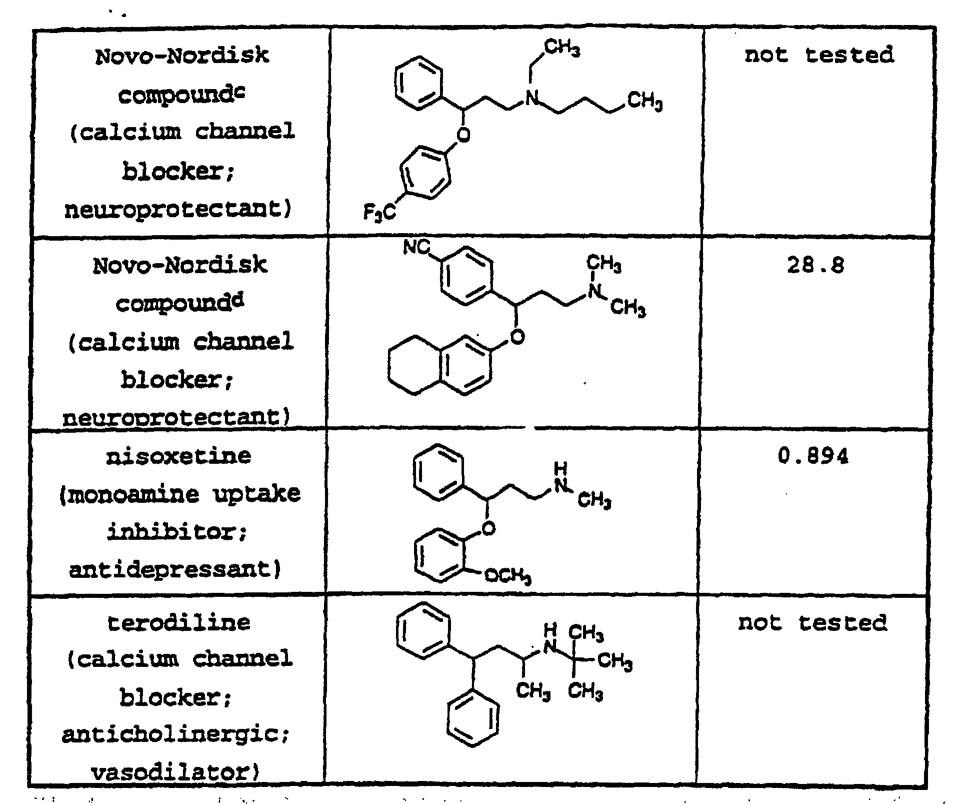

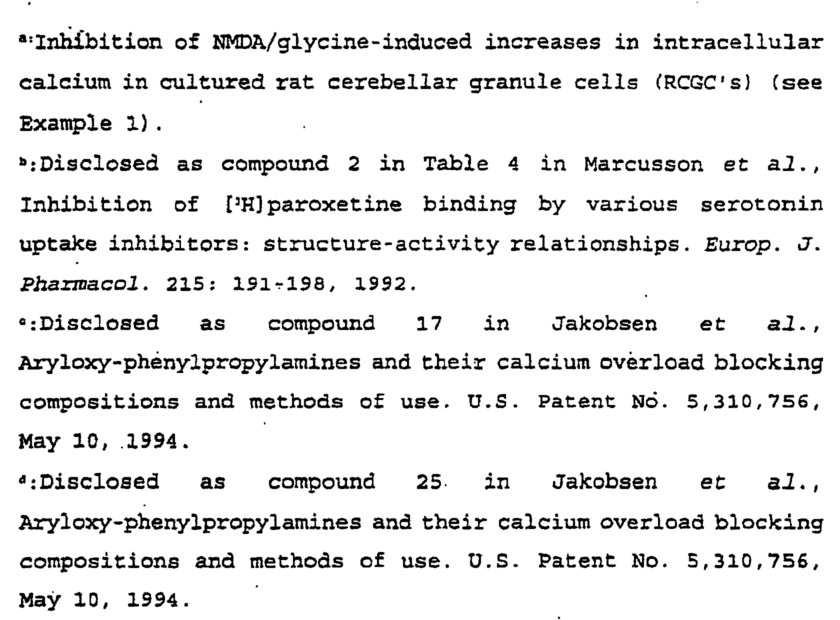

Structure-activity relationship studies were initiated using Compound **19** (Reference compound) as the lead structure. An examination of the side chain demonstrated that the propyl side chain was optimal for NMDA receptor antagonist potency (Table 3).

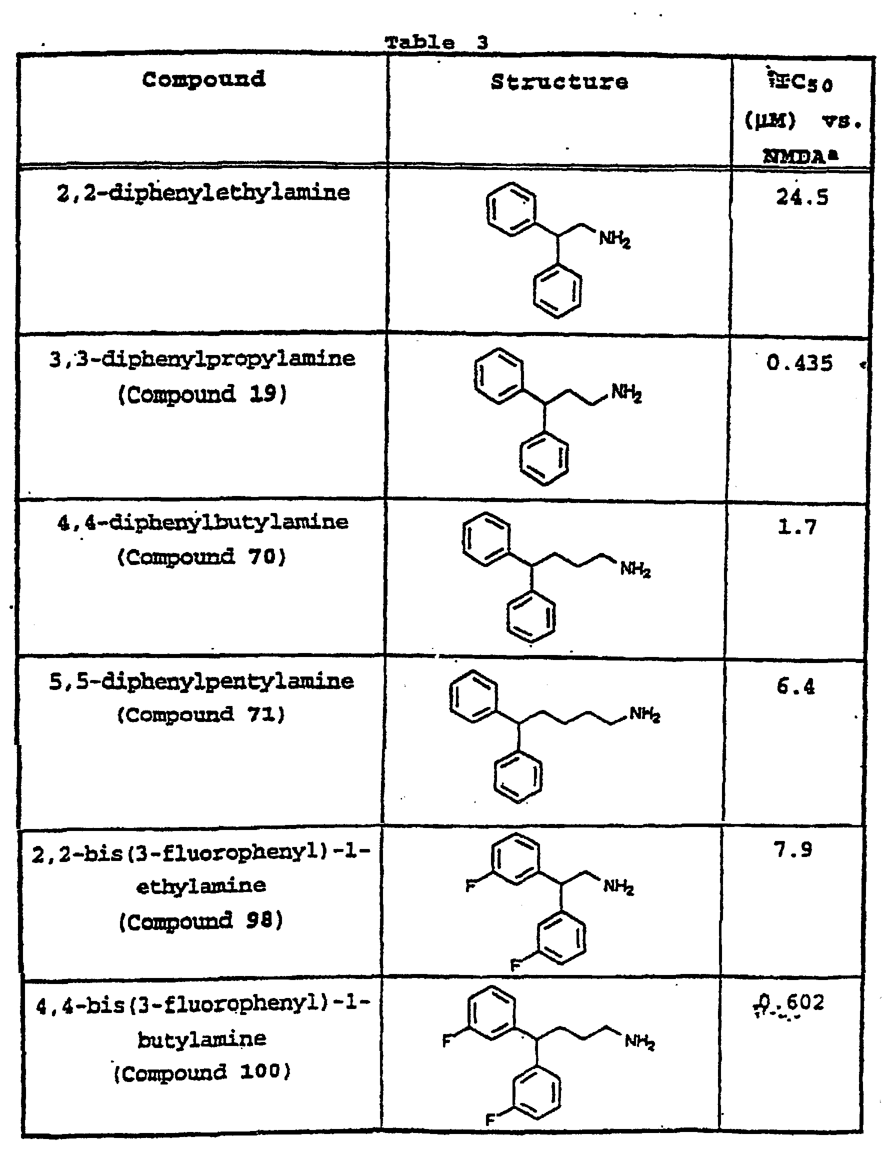

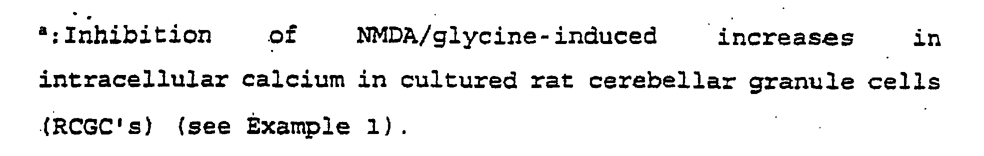

Further SAR studies examined the optimal pattern of phenyl ring substitution. Initial studies demonstrated that substitution of a halogen group (fluoro or chloro) at the meta position was optimal for NMDA receptor antagonist potency (Table 4). Increasing the number of fluoro substituents led to an apparent decrease in potency (Table 4).

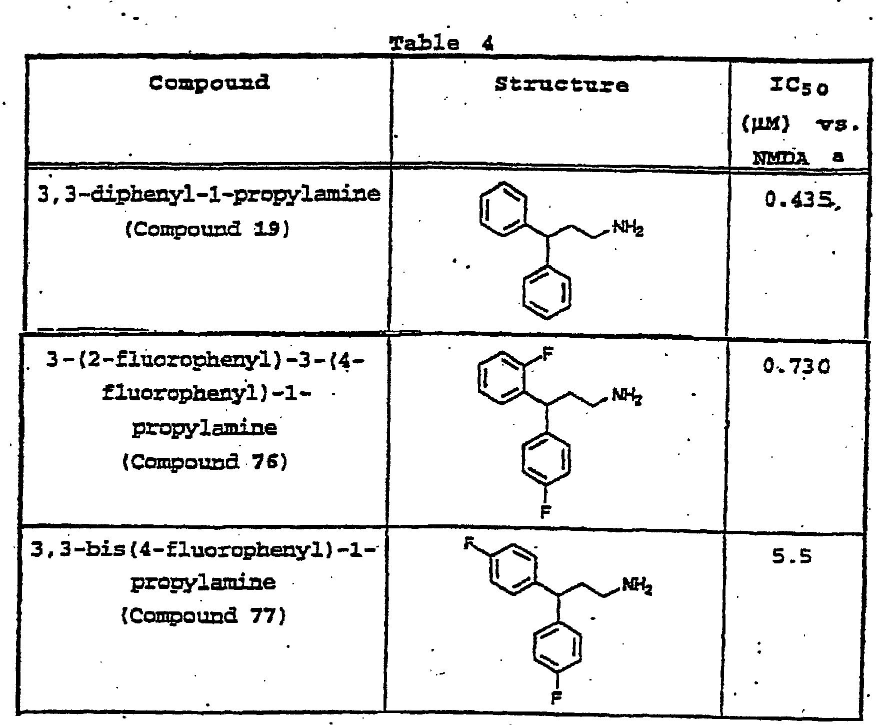

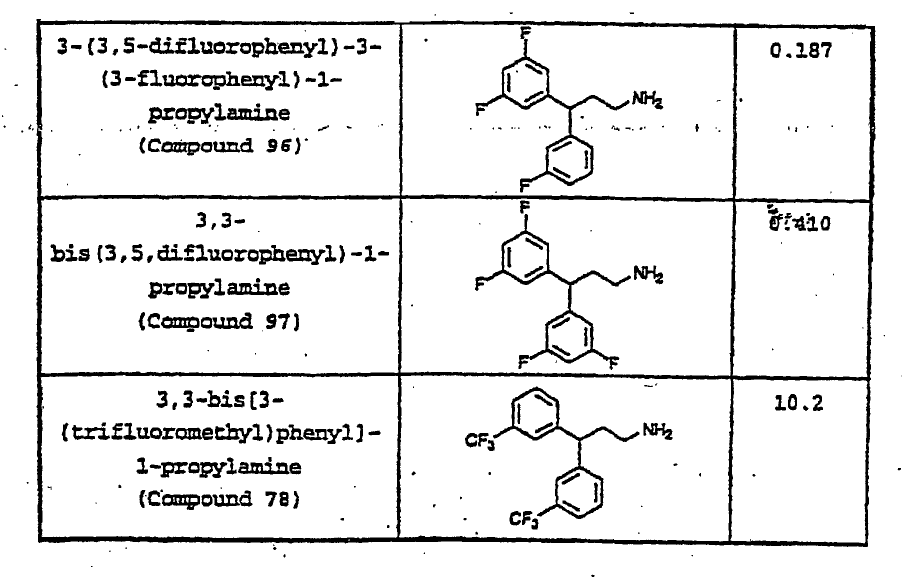

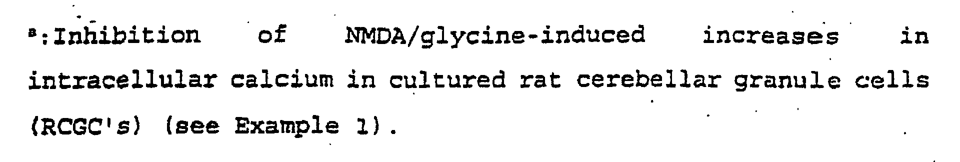

Replacement of one of the fluoro groups on one phenyl ring with a methyl, methoxy or hydroxy group led to no change or a decrease in the in vitro NMDA receptor antagonist potency. The *ortho* position was optimal for this methyl, methoxy or hydroxy group, and the rank order of potency for this substitution was methyl > methoxy > hydroxy (Table 5). Also illustrated in Table 5 are those compounds possessing the 3,3-bis(3-fluorophenyl) moiety with additional methyl or methoxy substitutions on the phenyl rings, often leading to an increase in NMDA receptor antagonist potency. Table 5 also illustrates those compounds possessing the 3,3-bis(2-methylphenyl) or 3,3-bis(2-methoxyphenyl) moiety in place of the 3,3-bis(3-fluorophenyl) moiety; these substitutions are acceptable, although a decrease in potency is noted.

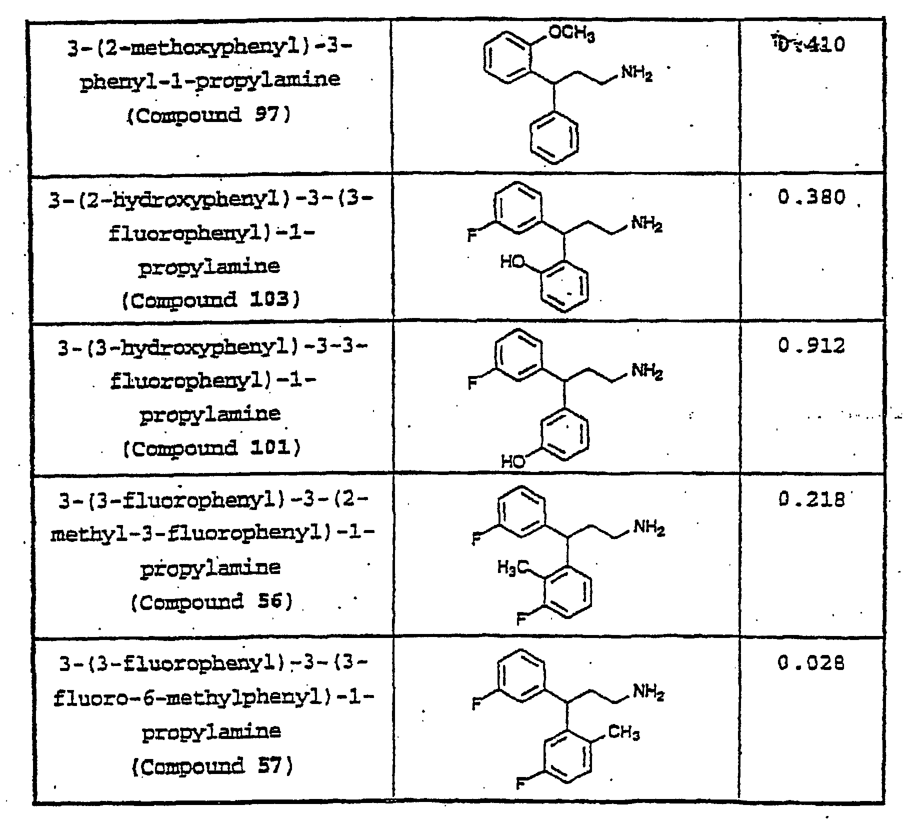

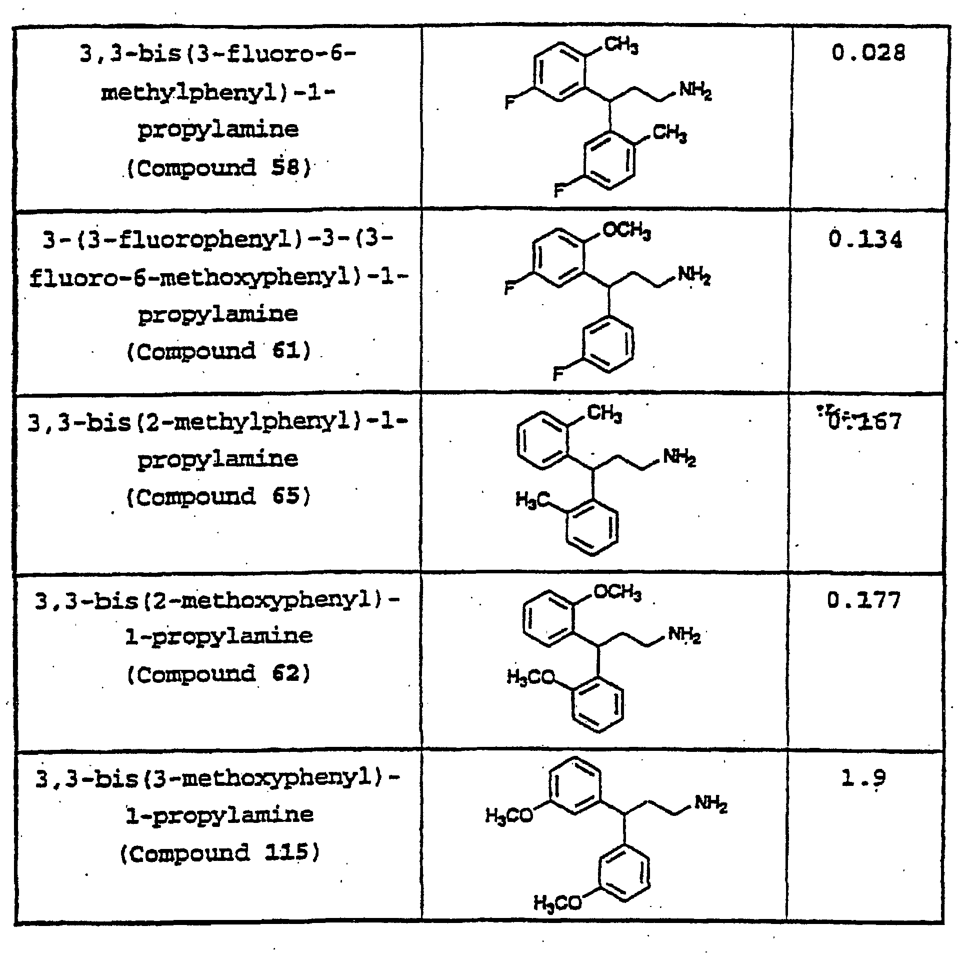

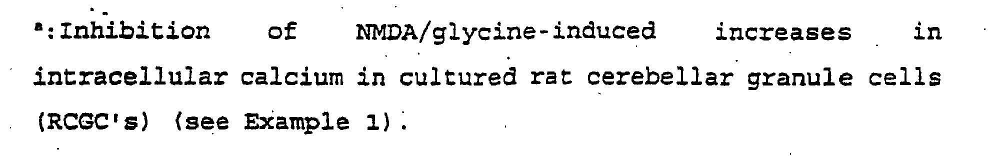

The next series of SAR experiments investigated the effect of alkyl chain substitutions (branching patterns) on NMDA receptor antagonist potency *in vitro*. The addition of a methyl group on either the α or β carbon on the propyl side chain led to a decrease or no change in potency, respectively (Table 6).

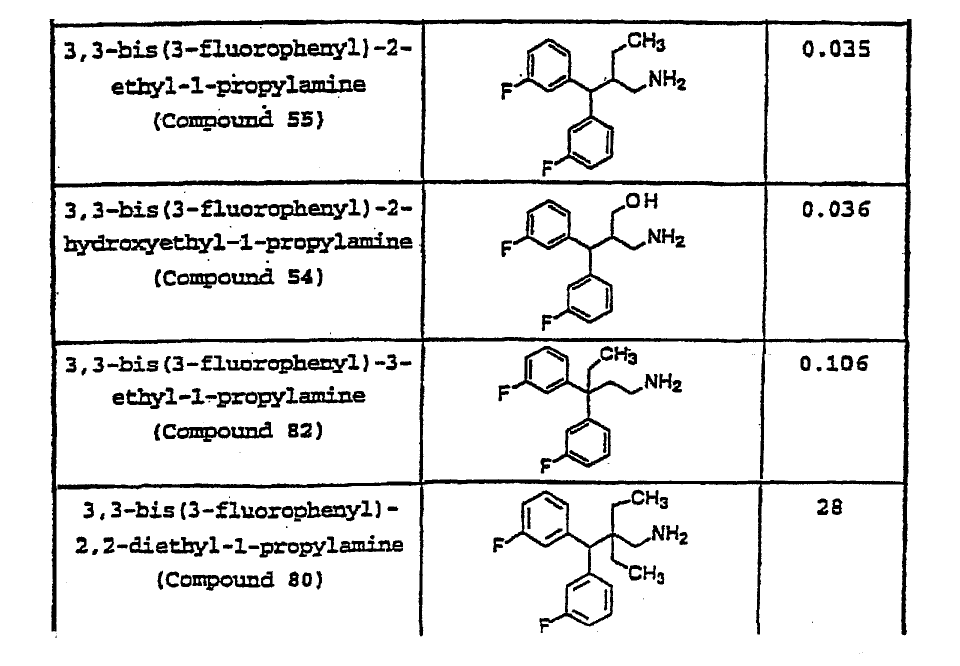

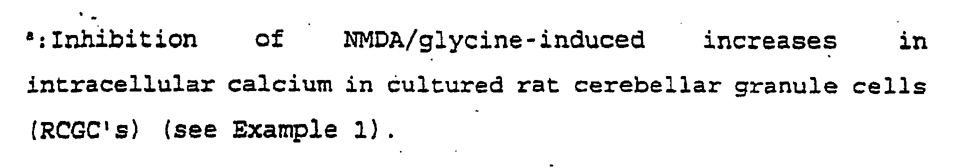

The next series of SAR experiments investigated the effect of incorporation of a double bond within the propyl chain on HMDA receptor antagonist potency in vitro (Table 7). As can be seen in Table 7, the incorporation of a double bond decreased potency in a consistent manner.

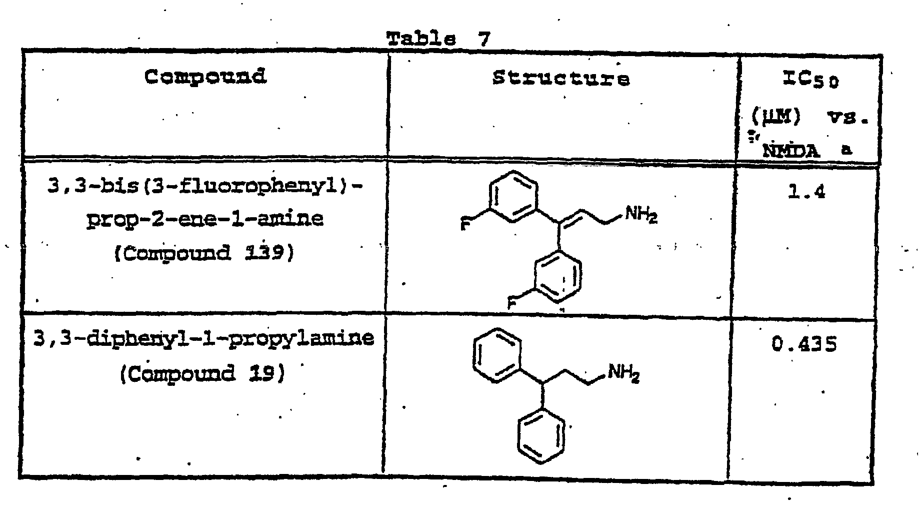

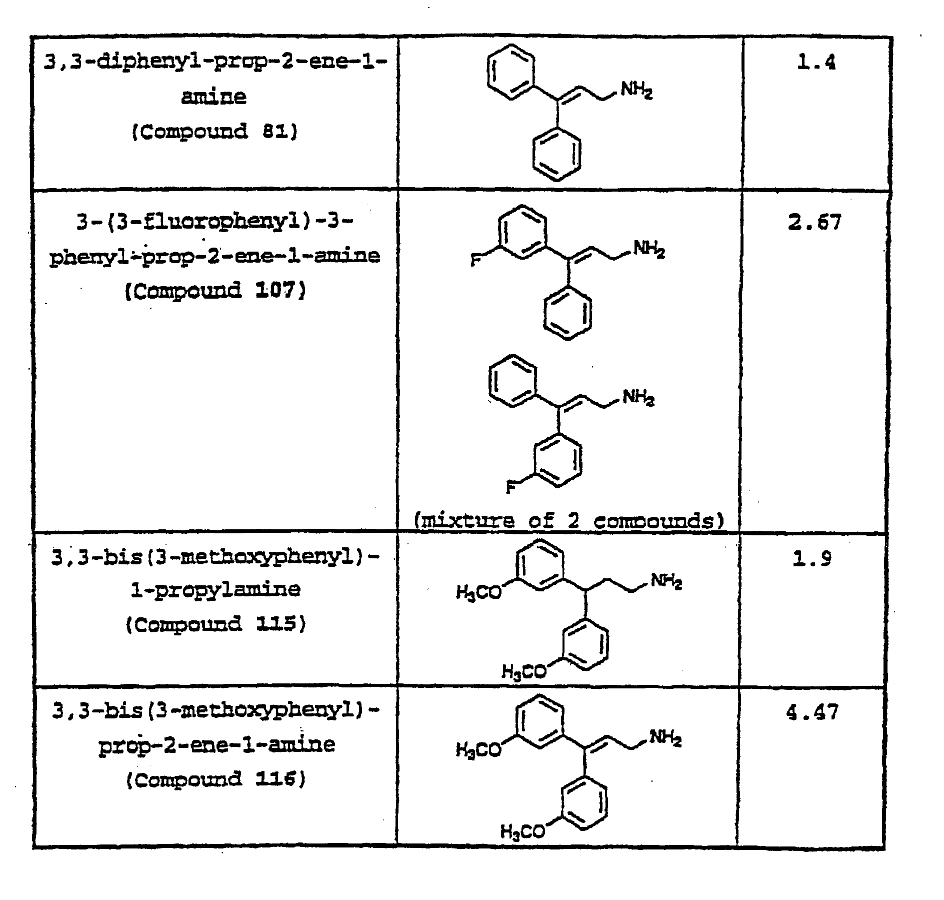

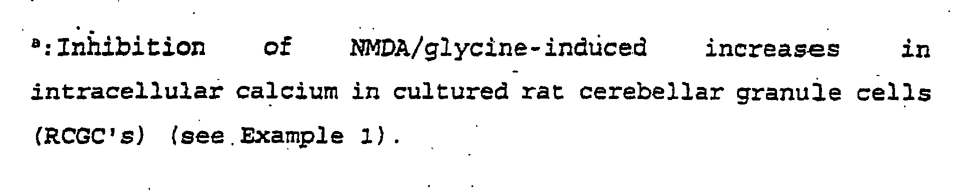

The next series of SAR experiments investigated the effect of incorporation of the propylamine chain into a ring structure on NMDA receptor antagonist potency *in vitro* (Table 8).

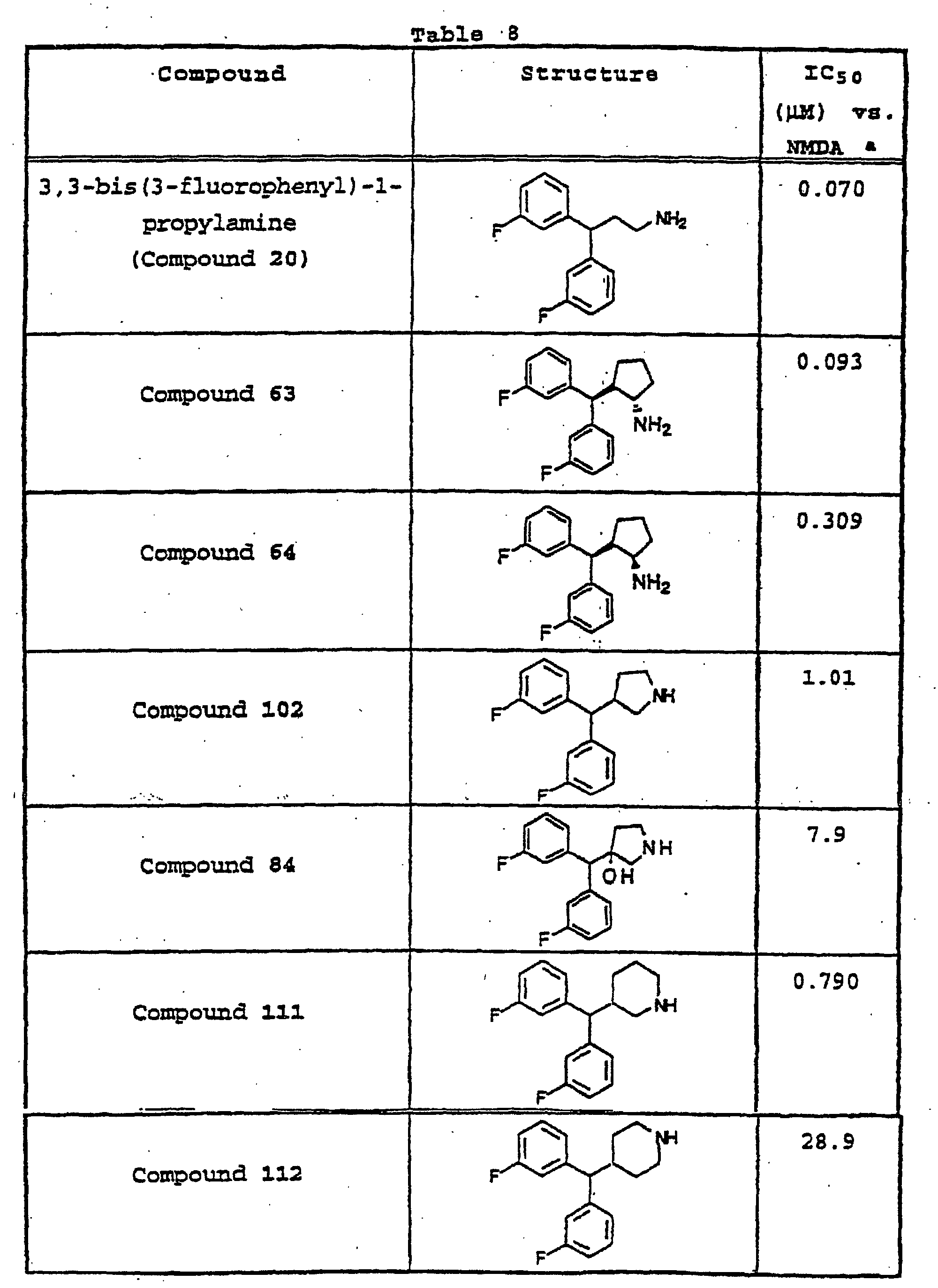

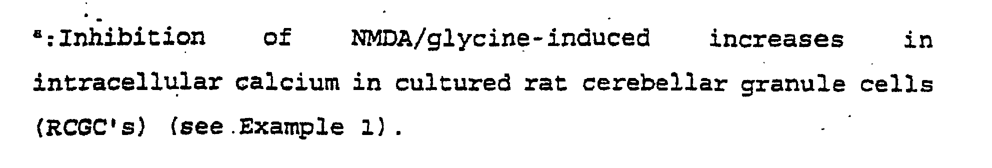

The next series of SAR experiments investigated the effect of simple alkyl substitution on the nitrogen on NMDA receptor antagonist potency *in vitro* (Table 9).

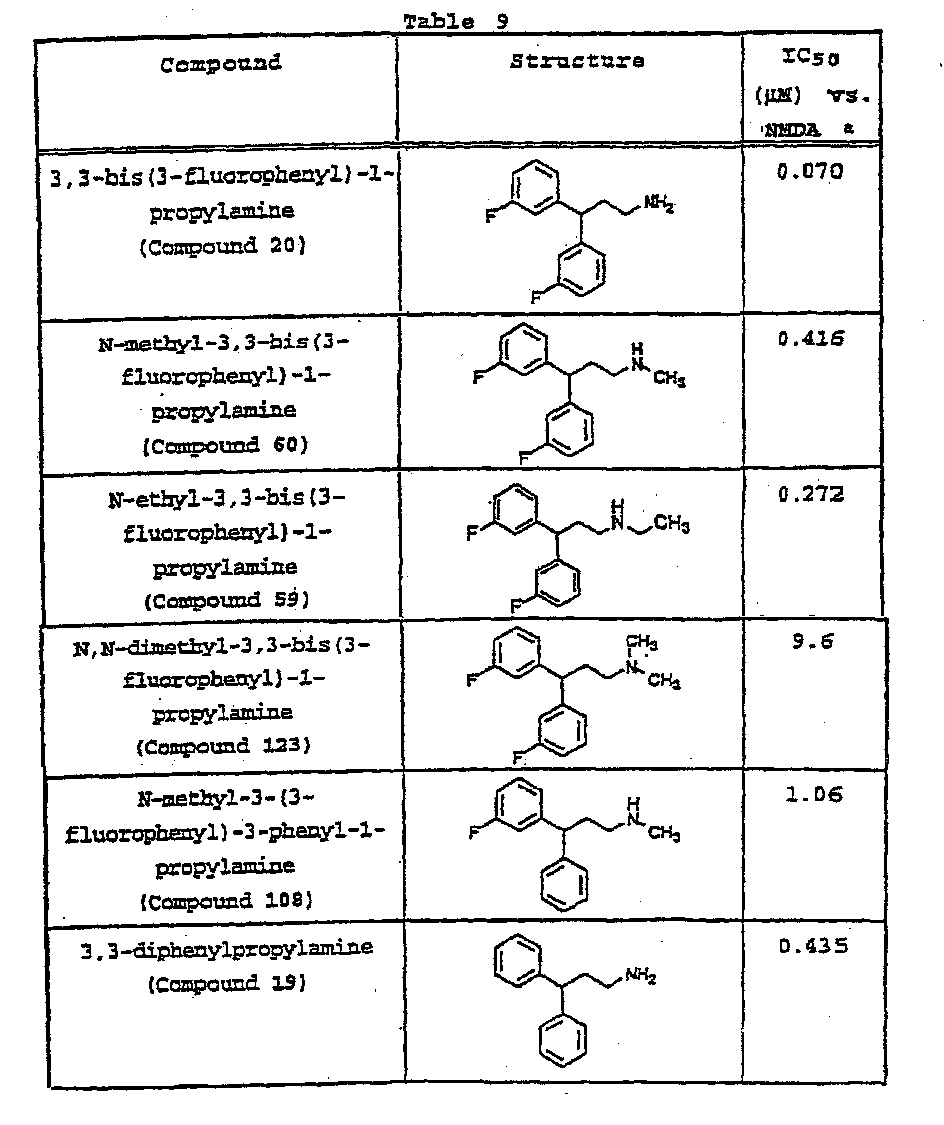

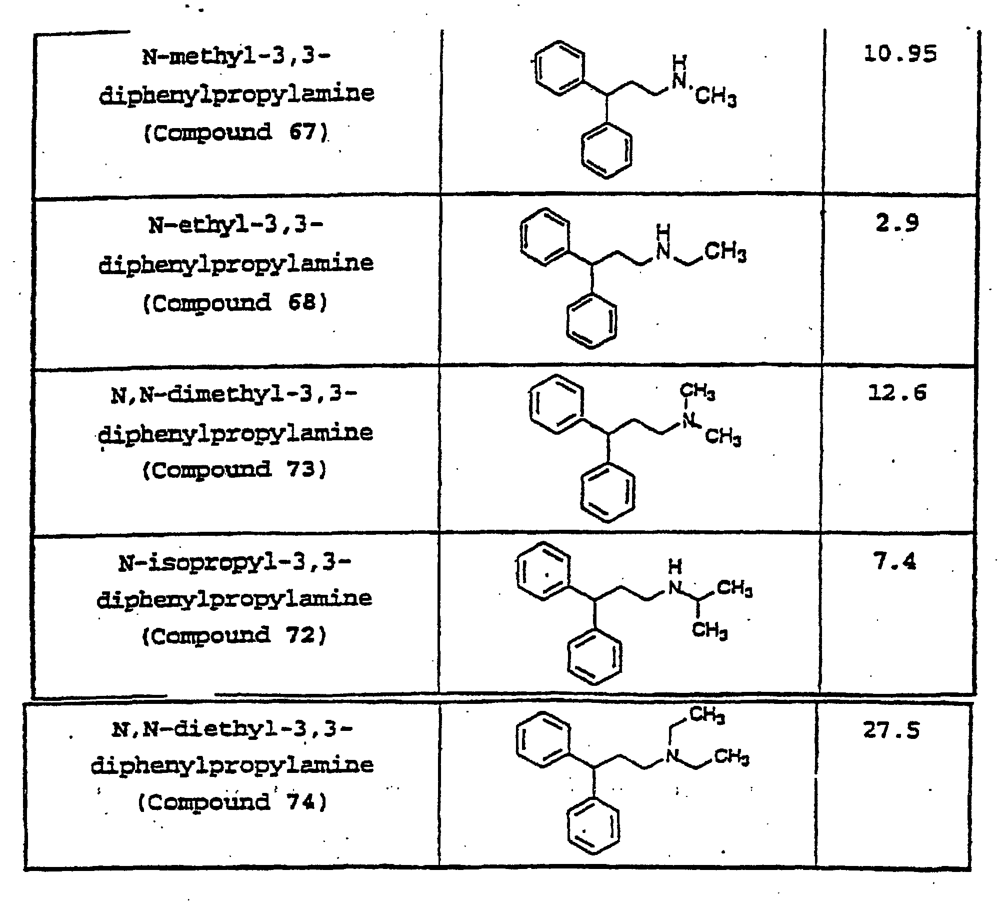

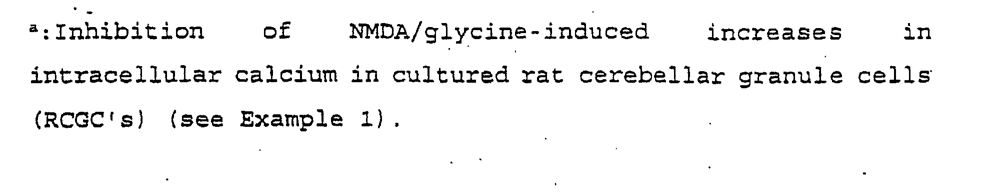

Certain simplified arylalkylamine compounds were selected for evaluation of activity in a battery of neurotransmitter receptor binding assays, and for activity against the L-type calcium channel and delayed rectifier potassium channel. The compounds were inactive (less than 50% inhibition at concentrations up to 10 µM) in the following assays: nonselective α2 adrenergic receptor ([³H]RX 821002 binding in rat cortex), H1 histamine receptor ([³H]pyrilamine binding in bovine cerebellum), nonselective sigma receptor ([³H]DTG binding in guinea pig brain), nonselective opiate receptor ([³H]naloxone binding in rat forebrain), monoamine oxidase (MAO) activity, both MAO-A ([¹⁴C] serotonin metabolism in rat liver mitochondria) and MAO-B ([¹⁴C]phenylethylamine metabolism in rat liver mitochondria).

As can be seen in Table 10, activity was noted for several compounds at concentrations below 10 µM in the following assays: L-type calcium channel, delayed rectifier potassium channel, central muscarinic cholinergic receptor binding, and monoamine (dopamine, norepinephrine, and serotonin) uptake binding assays. This profile of activity in the central muscarinic cholinergic receptor and monoamine uptake binding assays is not unexpected, given the chemical structures of our simplified arylalkylamines (refer to Table 2 above). With the exceptions, however, of the activity of Compounds **63** and **64** in the dopamine uptake binding assay, and the activity of Compound **60** in the dopamine and serotonin uptake binding assays, the simplified arylalkylamine compounds were most potent at the NMDA receptor.

**Table 10**

| Compound | IC₅₀ (µM) vs. NMDA^{a} | L-type calcium channel^{b} | Delayed rectifier potassium channel^{c} | Central miscarinic cholinergic receptor^{d} | Monoamine uptake binding assays^{e} |
|---|---|---|---|---|---|
| Compound **63** | 0.093 | 1.9 | not tested | 11% at 0.1B1% at 10 | 64% at 0.1^{f}98% at 10^{f}7% at 0.1^{g}76% at 10^{g}13% at 0.1^{h}85% at 10^{h} |
| Compound **64** | 0.309 | not tested | not tested | 11% at 0.183% at 10 | 50% at 0.1^{f}99% at 10^{f} 8% at 0.1^{g}65% at 10^{g}29% at 0.1^{h}68% at 10^{h} |
| Compound **58** | 0.028 | 1.6 | not tested | 1% at 0.148% at 10 | 0% at 0.1^{f}45% at 10^{f}1% at 0.1^{g}67% at 10^{g}27% at 0.1^{h}95% at 10^{h} |
| Compound **59** | 0.272 | not tested | not tested | 9% at 0.187% at 10 | 2% at 0.1^{f}78% at 10^{f}7% at 0.1^{g}51% at 10^{g}14% at 0.1^{h}86% at 10^{h} |
| Compound **60** | 0.416 | 2.3 | not tested | 13% at 0.193% at 10 | 0.914^{f}16% at 0.1^{g}64% at 10^{g} 0.068^{h} |

| | | | | | |
|---|---|---|---|---|---|
| ^{a}:Inhibition of NMDA/glycine-induced increases in intracellular calcium in cultured rat cerebellar granule cells (RCGC's) (see Example 1). | | | | | |
| ^{b}:Inhibition of KCl depolarization-induced increases in intracellular calcium in cultured rat cerebellar granule cells (RCGCs); estimated IC₅₀ value in µM. | | | | | |
| ^{c}:Inhibition of delayed rectifier potassium channel in cultured N1E-115 neuroblastoma cells; estimated IC₅₀ value in µM. | | | | | |
| ^{d}:Inhibition of the binding of [³H]quinuclidinylbenzilate (QNB) to rat cortical membranes; percent block at indicated concentration in µM. | | | | | |
| ^{e}:Inhibition of the binding of [³H]WIN-35,428 to guinea pig striatal membranes (dopamine uptake binding assay), [³H]desipramine to rat cortical membranes (norepinephrine uptake binding assay), or [³H]citalopram to rat forebrain membranes (serotonin uptake binding assay); percent block at indicated concentration in µM, or IC₅₀ when available. ^{f}:dopamine uptake binding assay ^{g}:norepinephrine uptake binding assay ^{h}:serotonin uptake binding assay | | | | | |

Advantageous properties of the arylalkylamine compounds of the present invention are illustrated by the fact that concentrations which suppress NMDA receptor-mediated synaptic transmission fail to inhibit LTP.

### Formulation and Administration

As demonstrated herein, useful compounds of this invention and their pharmaceutically acceptable salts may be used to treat neurological disorders or diseases. While these compounds will typically be used in therapy for human patients, they may also be used to treat similar or identical diseases in other vertebrates such as other primates, farm animals such as swine, cattle and poultry, and sports animals and pets such as horses, dogs and cats.

In therapeutic and/or diagnostic applications, the compounds of the invention can be formulated for a variety of modes of administration, including systemic and topical or localized administration. Techniques and formulations generally may be found in *Remington's Pharmaceutical Sciences,* Mack Publishing Co., Easton PA.

Pharmaceutically acceptable salts are generally well known to those of ordinary skill in the art, and may include, by way of example but not limitation, acetate, benzenesulfonate, besylate, benzoate, bicarbonate, bitarcrace, calcium edetate, camsylate, carbonate, citrate, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionace, malate, maleate, mandelate, mesylate, mucate, napsylate, nitrate, pamoate (embonate), pantothenate, phosphate/disphosphate, polygalacturonate, salicylate, stearate, subacetate, succinate, sulfate, tannate, tartrate, or teoclate. Other pharmaceutically acceptable salts may be found in, for example, *Remington's Pharmaceutical Sciences,* Mack Publishing Co., Easton, PA (18th ed, 1990).

Preferred pharmaceutically acceptable salts include, for example, acetate, benzoate, bromide, carbonate, citrate, gluconate, hydrobromide, hydrochloride, maleate, mesylate, napsylate pamoate (embonate), phosphate, salicylate, succinate, sulfate, or tartrate.

The useful compounds of this invention may also be in the form of pharmaceutically acceptable complexes. Pharmaceutically acceptable complexes are known to those of ordinary skill in the art and include, by way of example but not limitation, B-chlorotheophyllinate (teoclate).

The exact formulation, route of administration and dosage can be chosen by the individual physician in view of the patient's condition. (See e.g. Fingl et al., in The Pharmacological Basis of Therapeutics, 1975, Ch. 1 p. 1).

It should be noted that the attending physician would know how and when to terminate, interrupt, or adjust administration due to toxicity or organ dysfunction. Conversely, the attending physician would also know to adjust treatment to higher levels if the clinical responses were not adequate (precluding toxicity). The magnitude of an administered dose in the management of the disorder of interest will vary with the severity of the condition to be treated and to the route of administration. The severity of the condition may, for example, be evaluated in part, by standard prognostic evaluation methods. Further, the dose and perhaps dose frequency, will also vary according to the age, body weight, and response of the individual patient. A program comparable to that discussed above may be used in veterinary medicine.

Depending on the specific conditions being treated, such agents may be formulated into liquid or solid dosage forms and administered systemically or locally. The agents may be delivered, for example, in a timed or sustained-release form as is known to those skilled in the art. Techniques for formulation and administration may be found in *Remington's Pharmaceutical Sciences,* Mack Publishing Co., Easton, PA. Suitable routes may include oral, buccal, sublingual, rectal, transdermal, vaginal, transmucosal, nasal or intestinal administration; parenteral delivery, including intramuscular, subcutaneous, intramedullary injections, as well as intrathecal, direct intraventricular, intravenous, intraperitoneal, intranasal, or intraocular injections, just to name a few.

For injection, the agents of the invention may be formulated in aqueous solutions, preferably in physiologically compatible buffers such as Hank's solution, Ringer's solution, or physiological saline buffer. For such transmucosal administration, penetrants appropriate to the barrier to be permeated are used in the formulation. Such penetrants are generally known in the art.

Use of pharmaceutically acceptable carriers to formulate the compounds herein disclosed for the practice of the invention into dosages suitable for systemic administration is within the scope of the invention. With proper choice of carrier and suitable manufacturing practice, the compositions of the present invention, in particular, those formulated as solutions, may be administered parenterally, such as by intravenous injection. The compounds can be formulated readily using pharmaceutically acceptable carriers well known in the art into dosages suitable for oral administration. Such carriers enable the compounds of the invention to be formulated as tablets, pills, capsules, liquids, gels, syrups, slurries, suspensions and the like, for oral ingestion by a patient to be treated.

Agents intended to be administered intracellularly may be administered using techniques well known to those of ordinary skill in the art. For example, such agents may be encapsulated into liposomes, then administered as described above. Liposomes are spherical lipid bilayers with aqueous interiors. All molecules present in an aqueous solution at the time of liposome formation are incorporated into the aqueous interior. The liposomal contents are both protected from the external microenvironment and, because liposomes fuse with cell membranes, are efficiently delivered into the cell cytoplasm. Additionally, due to their hydrophobicity, small organic molecules may be directly administered intracellularly.

Pharmaceutical compositions suitable for use in the present invention include compositions wherein the active ingredients are contained in an effective amount to achieve its intended purpose. Determination of the effective amounts is well within the capability of those skilled in the art, especially in light of the detailed disclosure provided herein.

In addition to the active ingredients, these pharmaceutical compositions may contain suitable pharmaceutically acceptable carriers comprising excipiencs and auxiliaries which facilitate processing of the active compounds into preparations which can be used pharmaceutically. The preparations formulated for oral administration may be in the form of tablets, dragees, capsules, or solutions.

The pharmaceutical compositions of the present invention may be manufactured in a manner that is itself known, e.g., by means of conventional mixing, dissolving, granulating, dragee-making, levigating, emulsifying, encapsulating, entrapping or lyophilizing processes.

Pharmaceutical formulations for parenteral administration include aqueous solutions of the active compounds in water-soluble form. Additionally, suspensions of the active compounds may be prepared as appropriate oily injection suspension. Suitable lipophilic solvents or vehicles include fatty oils such as sesame oil, or synthetic fatty acid ester, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Pharmaceutical preparations for oral use can be obtained by combining the active compounds with solid excipients, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations, for example, maize starch, wheat starch, rice starch, potato starch, gelatin, gum tragacanth, methyl cellulose, hydroxypropylmethyl-cellulose, sodium carboxymethylcellulose (CMC), and/or polyvinylpyrrolidone (PVP: povidone). If desired, disintegrating agents may be added, such as the cross-linked polyvinylpyrrolidone, agar, or alginic acid or a salt thereof such as sodium alginate.

Dragee cores are provided with suitable coatings. For this purpose, concentrated sugar solutions may be used, which may optionally contain gum arabic, talc, polyvinylpyrrolidone, carbopol gel, polyethylene glycol (PEG., and/or titanium dioxide, lacquer solutions, and suitable organic solvents or solvent mixtures. Dye-stuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin, and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable, liquids, such as fatty oils, liquid paraffin, or liquid polyethylene glycols (PEGS). In addition, stabilizers may be added.

Other embodiments are within the following claims.

## Claims

1. Use of a compound which is selected from the group consisting of 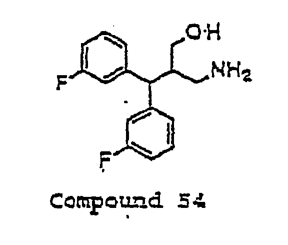 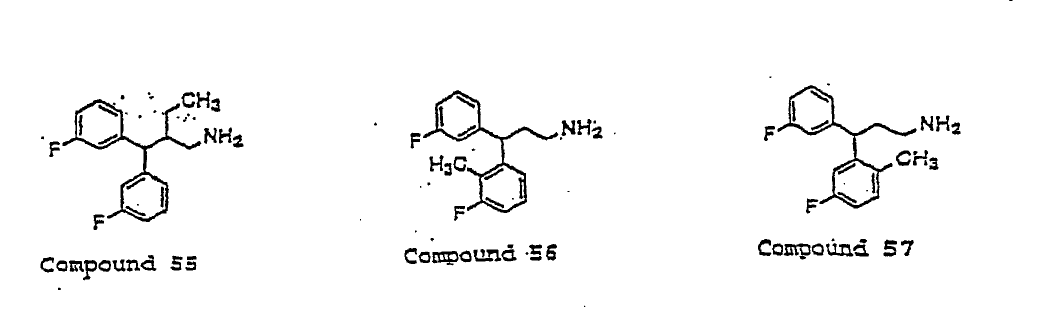 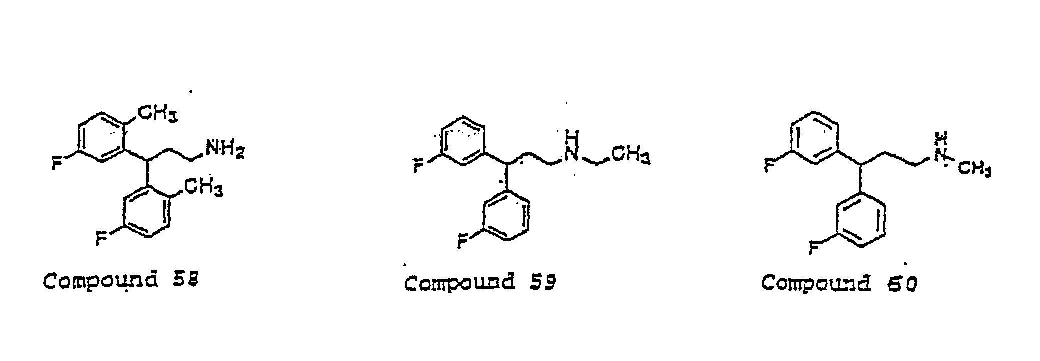 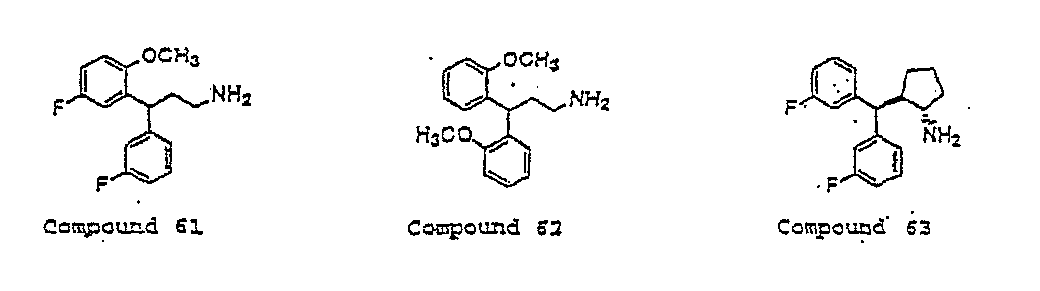 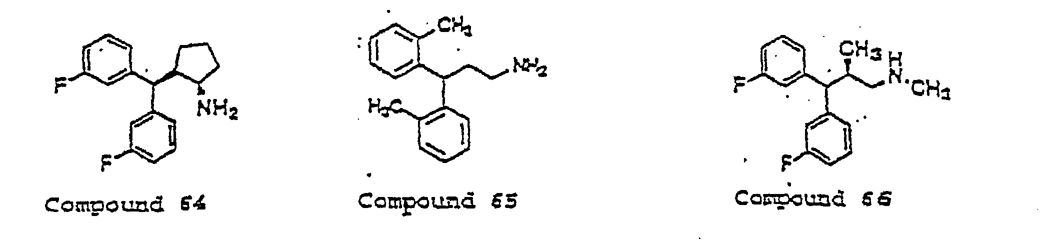 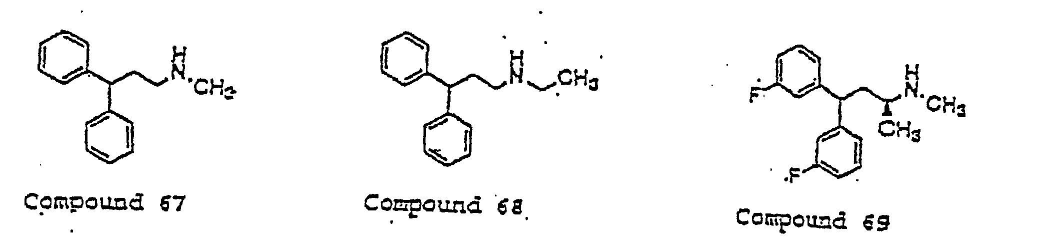 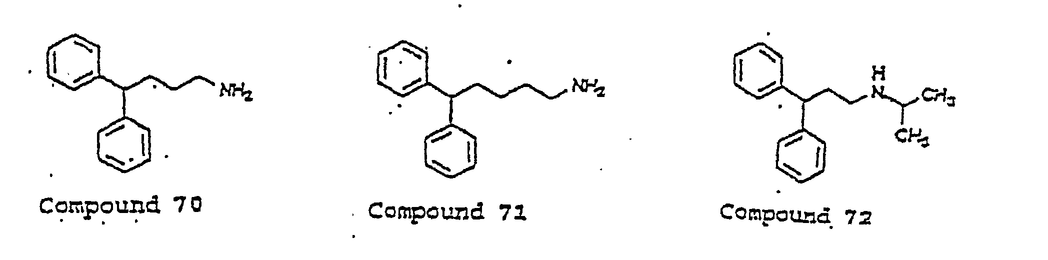 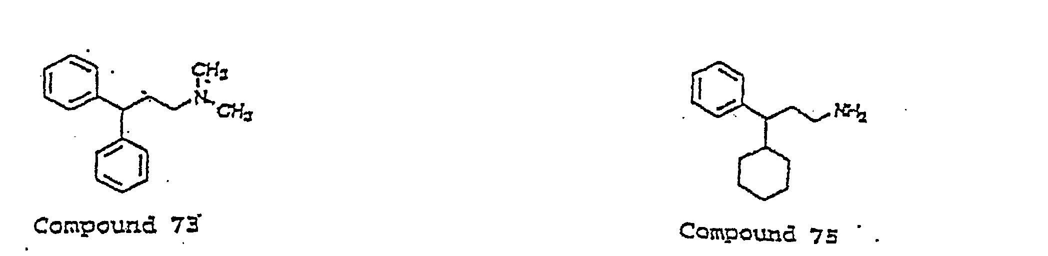 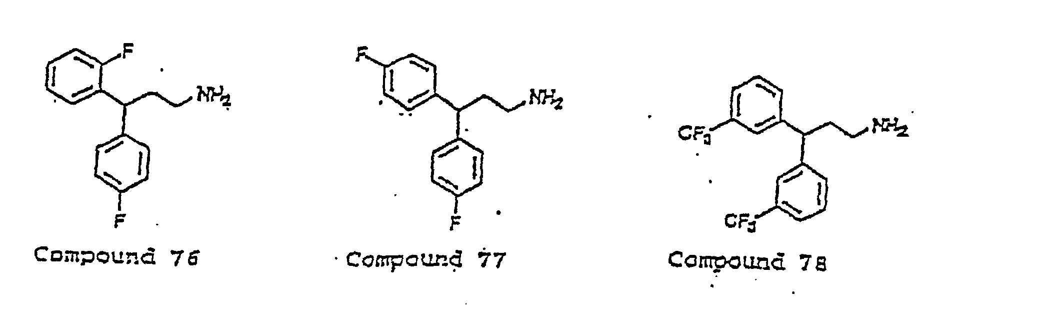 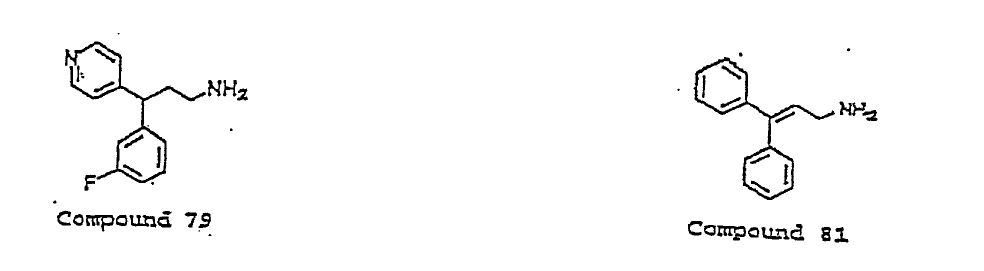 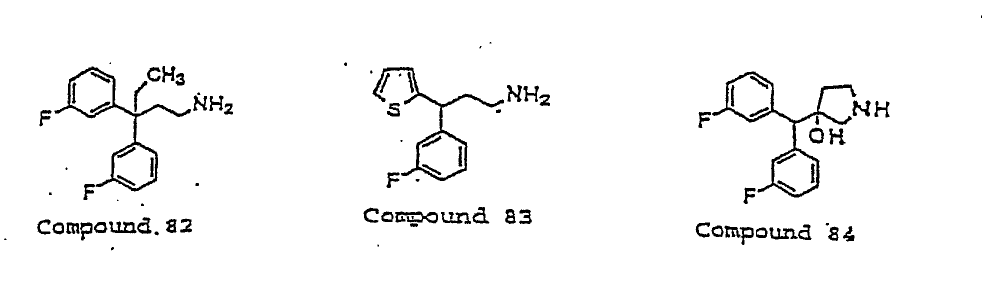 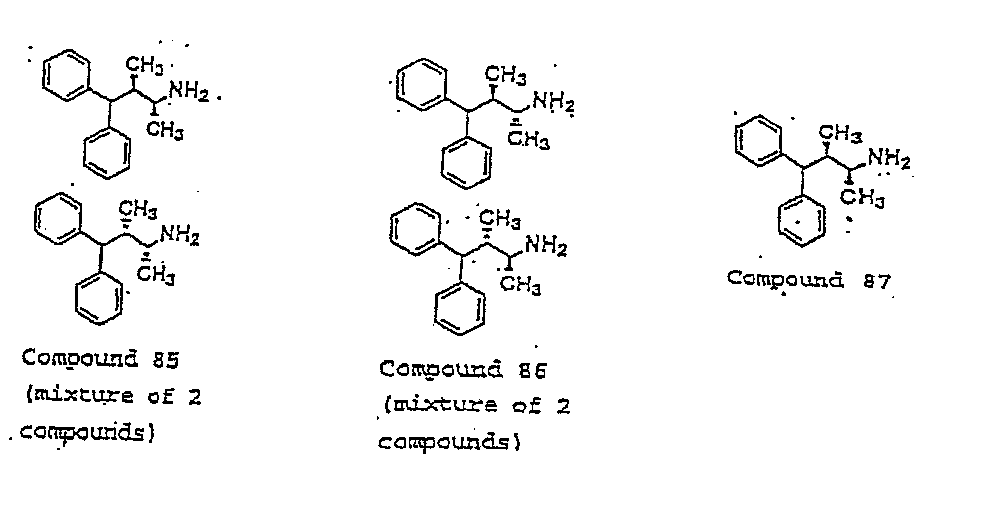 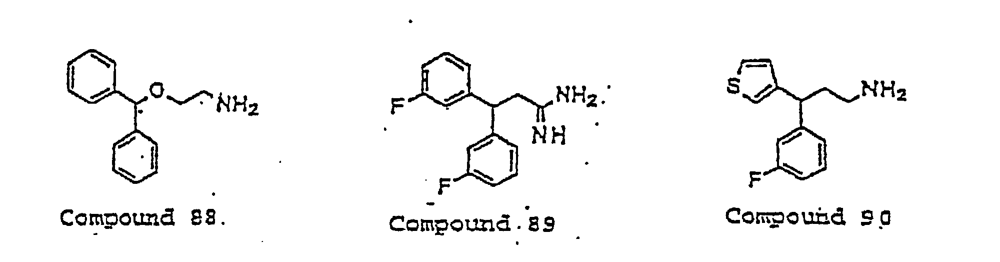 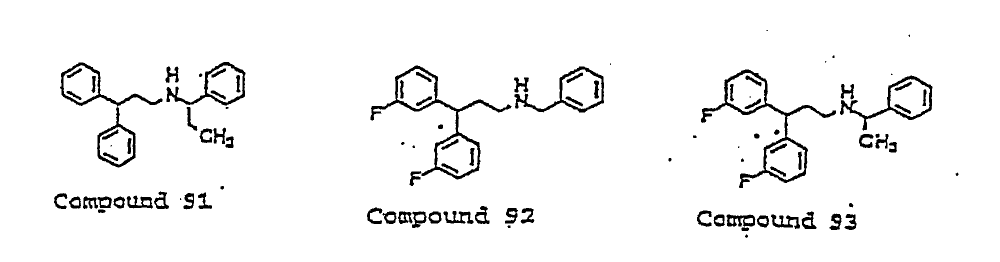 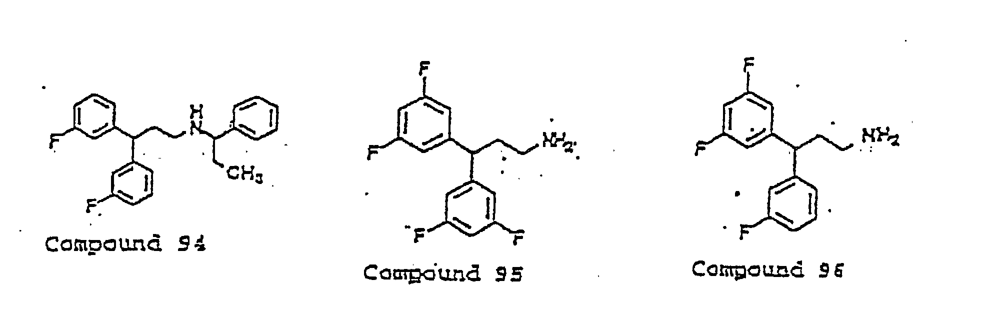 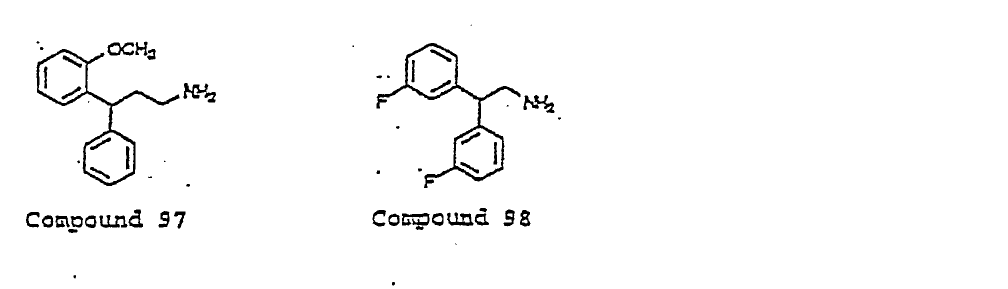 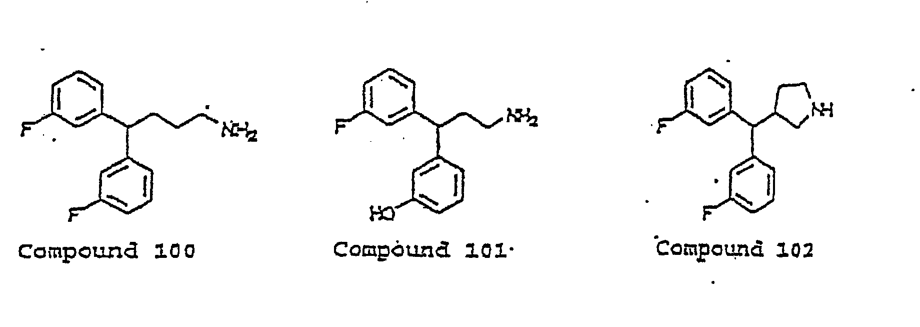 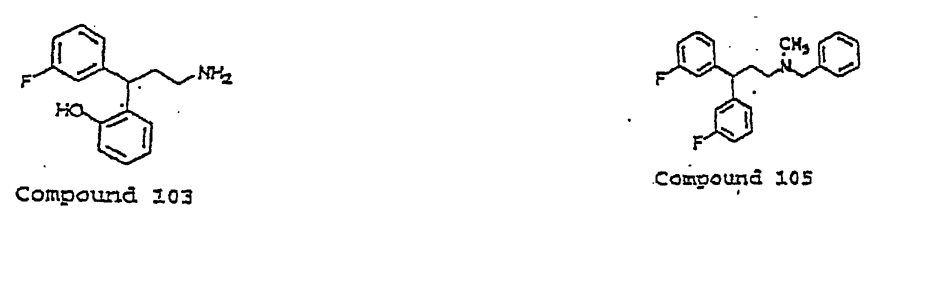 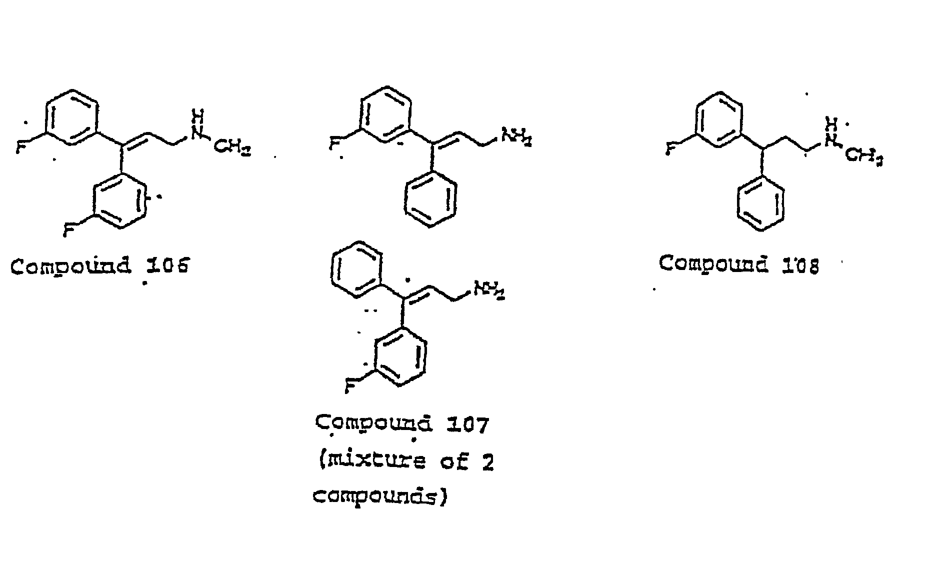 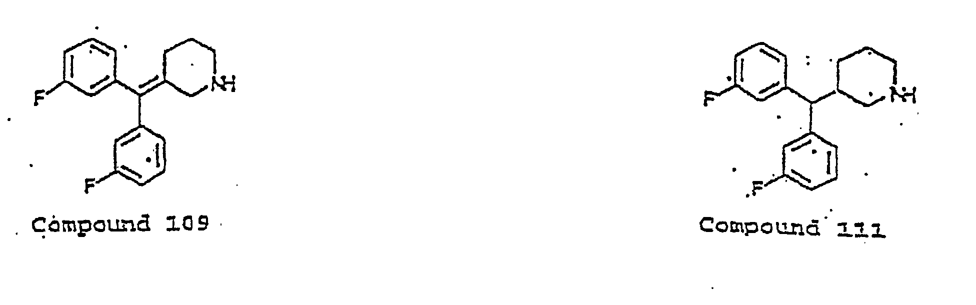 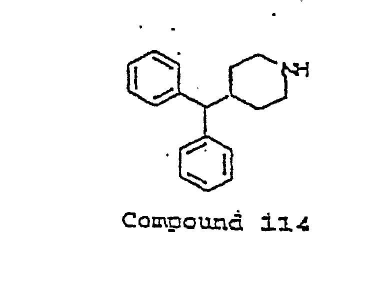 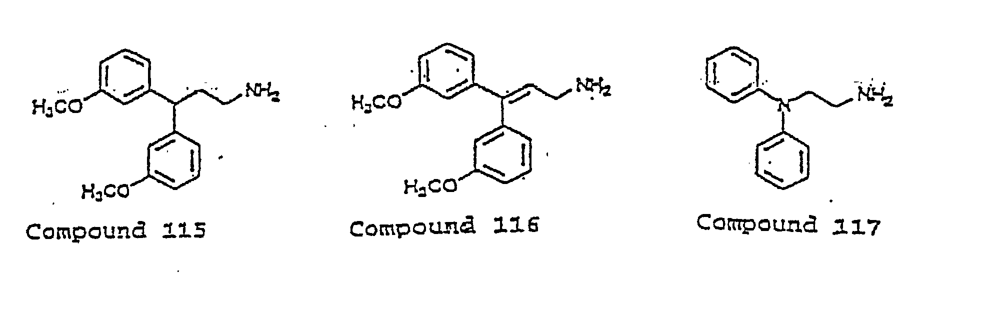 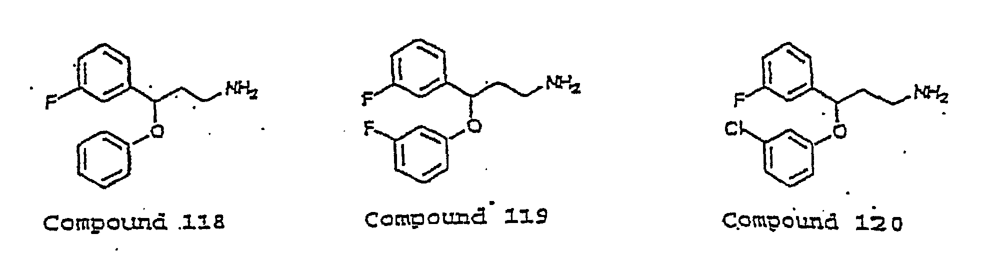 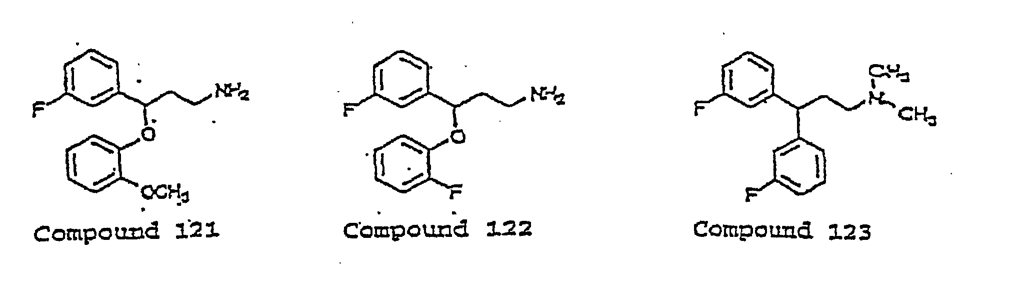 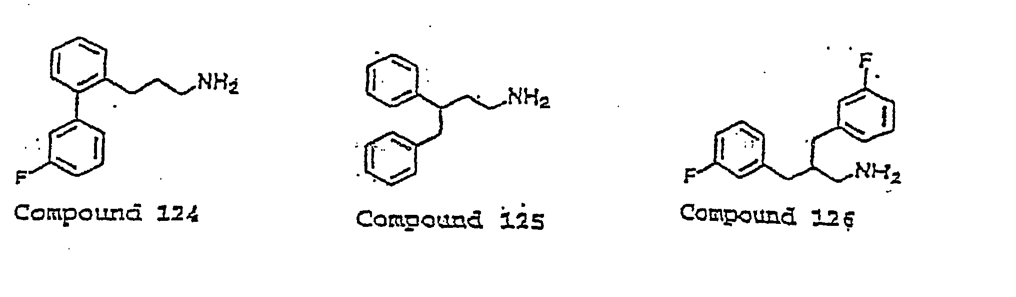 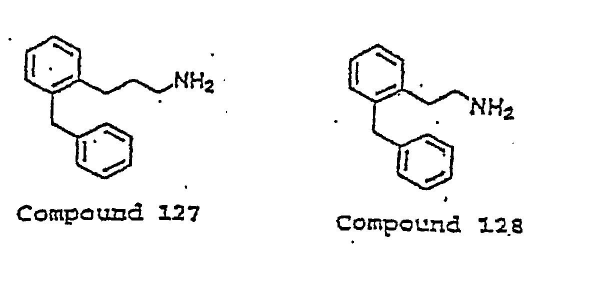 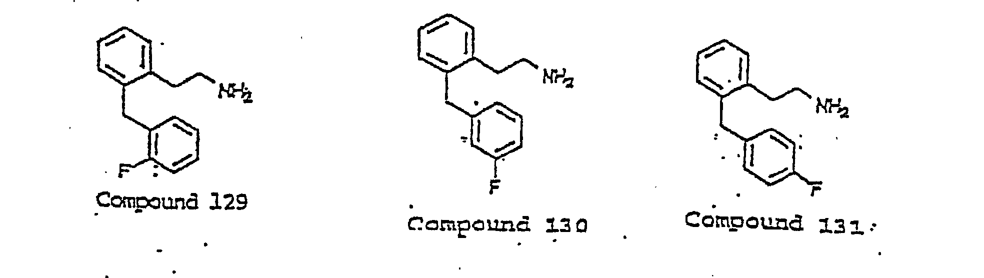 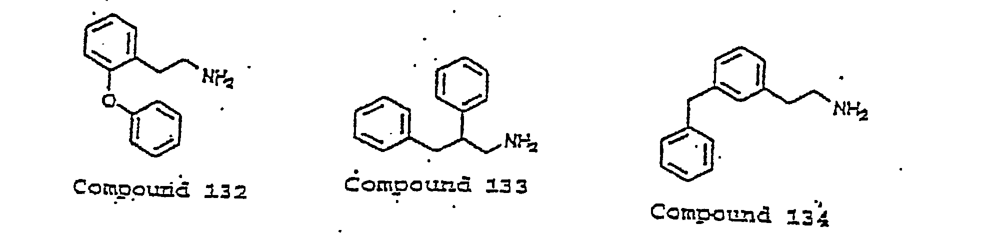 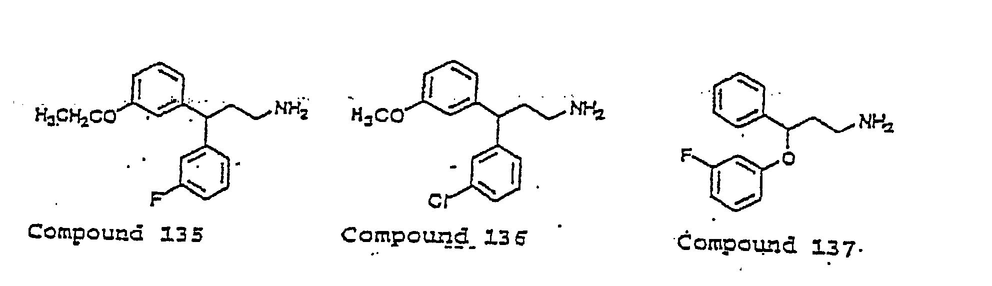 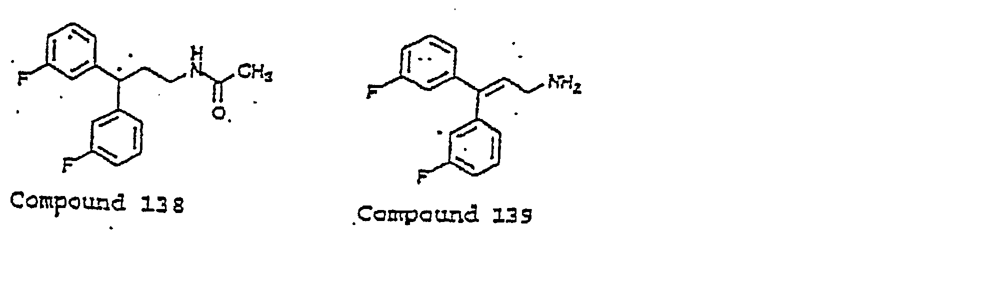 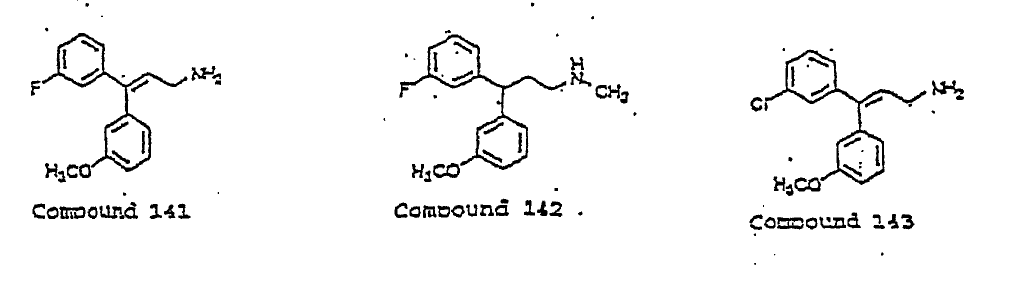 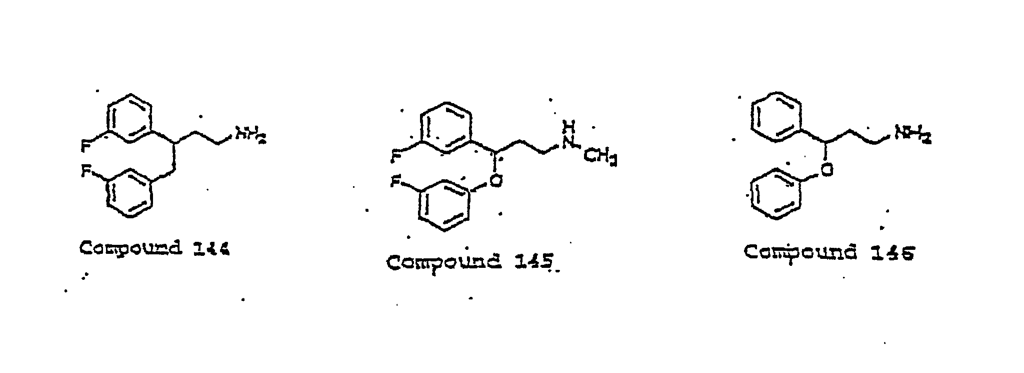 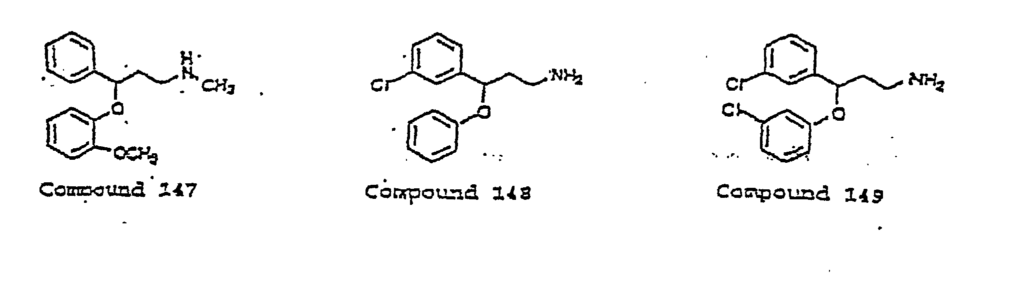 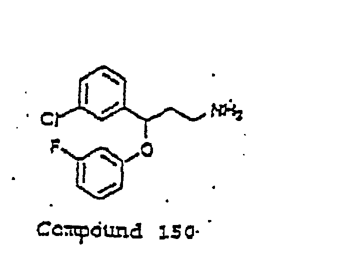 , and pharmaceutically acceptable salts and complexes thereof for the preparation of a pharmaceutical composition for the treatment of a neurological disease or disorder.

2. The use according to claim 1, wherein the compound is selected from the group consisting of compounds 54-66, 68-71, 75, 76, 78, 79, 81-90, 92-98, 100, 101, 103, 105, 106, 108, 109, 111, 114-122, 124-136, 138, 139, 141-144, 148-150, and pharmaceutically acceptable salts and complexes thereof.

3. The use according to claim 1, wherein the compound is selected from the group consisting of compounds 54-66, 69, 70, 75, 76, 81-83, 85-97, 100- 103, 105, 106, 108, 109, 111, 115, 118-122, 125-133, 135-139, 142, 144-150, and pharmaceutically acceptable salts and complexes thereof.

4. The use according to claim 1, wherein the compound is selected from the group consisting of compounds 54-66, 69, 70, 75, 76, 81-83, 85-90, 92-97, 100, 101, 103, 105, 106, 108, 109, 111, 115, 118-122, 125-133, 135, 136, 138, 139, 142, 144, 148-150, and pharmaceutically acceptable salts and complexes thereof.

5. The use according to claim 1, wherein the compound is selected from the group consisting of compounds 54-66, 69, 82, 83, 89-97, 103, 111, 118-120, 122, 126, 135-138, 142, 144, 145, 147-150, and pharmaceutically acceptable salts and complexes thereof.

6. The use according to claim 1, wherein the compound is selected from the group consisting of compounds 54-66, 69, 82, 83, 89-90, 92-97, 103, 111, 118-120, 122, 126, 135, 136, 138, 142, 144, 148-150, and pharmaceutically acceptable salts and complexes thereof.

7. The use according to claim 1, wherein the compound is selected from the group consisting of compounds 60, 66, 69, 103, 111, 118-120, 122, 136, 138, 142, 144, 148-150, and pharmaceutically acceptable salts and complexes thereof.

8. The use according to claim 1, wherein the compound is selected from the group consisting of compounds 118-122, 137, 145, 148-150, and pharmaceutically acceptable salts and complexes thereof.

9. The use according to claim 1, wherein the compound is selected from the group consisting of compounds 118-122, 148-150, and pharmaceutically acceptable salts and complexes thereof.

10. The use according to claim 1, wherein the compound is selected from the group consisting of compounds 63 and 64 and pharmaceutically acceptable salts and complexes thereof.

11. The use according to claim 1, wherein the compound is selected from compound 119, and pharmaceutically acceptable salts and complexes thereof.

12. The use according to claim 1, wherein the compound is selected from compound 144, and pharmaceutically acceptable salts and complexes thereof.

13. The use of compound 60, and pharmaceutically acceptable salts and complexes thereof for the preparation of a pharmaceutical composition for the treatment of a neurological disease or disorder.

14. Use of a compound having the formula: 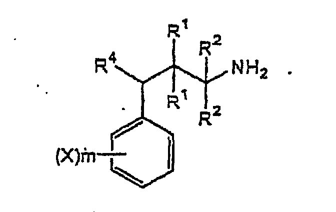 wherein:
X is independently selected from the group consisting of -Br, -Cl, -F, -I,-CF₃, alkyl, -OH, -OCF₃, -O-alkyl, and -O-acyl;
R₁ is independently selected from the group consisting of -H, C₁-C₄ alkyl, and -O-acyl;
R₂ is independently selected from the group consisting of -H, alkyl, and hydroxyalkyl, or both R₂s together are imino;
R₄ is phenoxy which is optionally substituted with -F, -Cl, -Br, -I, -CF₃, alkyl, -OH,- OCF₃-O-alkyl, or -O-acyl; and
m is independently an integer from 0 to 5; and pharmaceutically acceptable salts and complexes thereof provided that said compound is not:
3-(p-isopropoxyphenoxy)-3-phenylpropylamine
3-(2'-methyl-4',5'-dichlorophenoxy)-3-phenylpropylamine
3-(p-t-butylphenoxy)-3-phenylpropylamine
3-(2',4'-dichlorophenoxy)-3-phenyl-2-methyl propylamine
3-(o-ethylphenoxy)-3-phenylpropylamine
3-(o-methoxyphenoxy)-3-phenylpropylamine
3-phenoxy-3-phenylpropylamine
for the preparation of a pharmaceutical composition for the treatment of a neurological disease or disorder.

15. Use of a compound having the formula 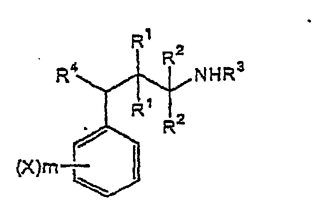 wherein:
X is independently selected from the group consisting of-F, -Cl, -Br, -I, -CF₃ alkyl, -OH, -OCF₃, -O-alkyl, and -O-acyl;
R₁ is independently selected from the group consisting of -H, C₁-C₄ alkyl, and -O-acyl;
R₂ is independently selected from the group consisting of -H, C₁-C₄ alkyl, and hydroxyalkyl, or both R₂s together are imino;
R₃ is selected from the group consisting of methyl and ethyl;
R₄ is phenoxy which is optionally substituted with -F, -Cl, -Br, -I, -CF₃, alkyl, -OH, -OCF₃, -O-alkyl, or -O-acyl;
and m is independently an integer from 0 to 5; and pharmaceutically acceptable salts and complexes thereof provided that said compound is not
N-methyl 3-(o-chloro-p-tolyloxy)-3-phenyl-1-methylpropylamine
N-methyl 3-(p-tolyloxy)-3-phenylpropylamine
N-methyl 3-(o-chloro-p-isopropylphenoxy)-3-phenyl-2-methylpropylamine
N-methyl 3-(p-iodophenoxy)-3-phenyl-propylamine
N-methyl 3-(3-n propylphenoxy)-3-phenyl-propylamine
N-methyl 3-(p-trifluoromethylphenoxy)-3-phenylpropylamine
N-methyl 3-(m-chlorophenoxy)-3-phenylpropylamine
N-methyl 3-(p-fluorophenoxy)-3-phenylpropylamine
N-methyl 3-(o-methoxyphenoxy)-3-phenylpropylamine
N-methyl 3-(o-methoxyphenoxy)-3-phenylpropylamine
N-methyl 3-(o-fluorophenoxy)-3-phenylpropylamine
N-methyl 3-(m-fluorophenoxy)-3-phenylpropylamine
N-methyl 3-(p-chlorophenoxy)-3-phenylpropylamine
N-methyl 3-(m-fluorophenoxy)-3-phenylpropylamine
N-methyl 3-phenoxy-3-phenyl-2-methylpropylamine
N-methyl 3-phenoxy-3-phenyl-1-methylpropylamine
N-methyl 3-phenoxy-3-phenylpropylamine
N-methyl 3-(o-trifluoromethylphenoxy)-3-phenylpropylamine
N-methyl 3-(m-methoxyphenoxy)-3-phenylpropylamine
N-methyl 3-(o,p-difluorophenoxy)-3-phenylpropylamine
N-ethyl-3-(o-iodophenoxy)-3-phenylpropylamine
N-methyl-3-(o-chlorophenoxy)-3-phenylpropylamine
N-methyl-3-(o-bromophenoxy)-3-phenylpropylamine
for the preparation of a pharmaceutical composition for the treatment of a neurological disease or disorder.

16. Use of a compound having the formula: 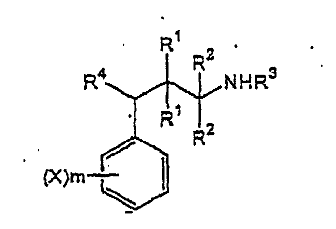 wherein:
(X)m is selected from the group consisting of meta-fluoro, meta-chloro, Ortho-O-C₁-C₄ alkyl, ortho-methyl, ortho-fluoro, ortho-chloro, meta-O-C₁-C₄ alkyl, meta-methyl, ortho-OH, and meta-OH;
R₁ is H;
R₂ is H;
R₃ is selected from the group consisting of methyl and ethyl;
R₄ is phenoxy which is optionally substituted with -F, -Cl, -Br, -I, -CF₃, alkyl, -OH, -OCF₃, -O-alkyl, or -O-acyl; and pharmaceutically acceptable salts and complexes thereof, for the preparation of a pharmaceutical composition for the treatment of a neurological disease or disorder.

17. The use of any one of claims 1 to 16, wherein the neurological disease or disorder comprises stroke, head trauma, spinal cord injury, spinal cord ischemia, ischemia- or hypoxia-induced nerve cell damage, epilepsy, pain, anxiety, neuropsychiatric or cognitive deficits due to ischemia or hypoxia such as those that frequently occur as a consequence of cardiac surgery under cardiopulmonary bypass, Alzheimer's disease, Huntington's disease, Parkinson's disease, or amyotyrophic lateral sclerosis.

18. The use of claim 17, wherein said stroke is global ischemic.

19. The use of claim 17, wherein said stroke is focal ischemic.

20. The use of claim 17, wherein said stroke is hemorrhagic.

21. The use of claim 17, wherein the neurological disease or disorder comprises Parkinson's disease.

22. A compound selected from the group consisting of 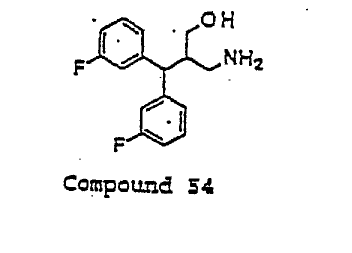 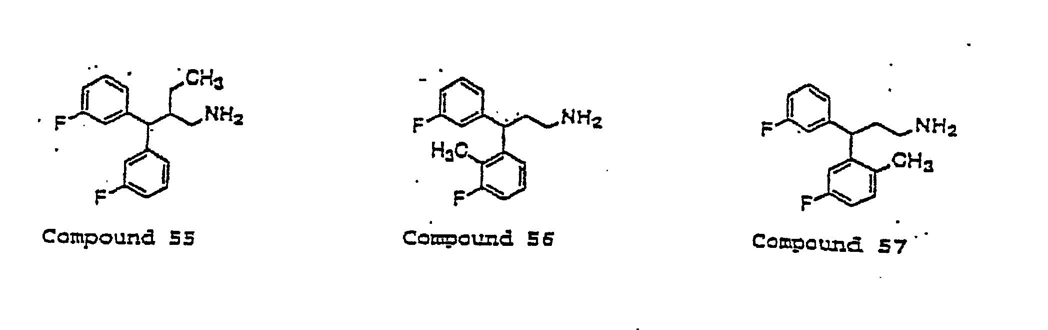 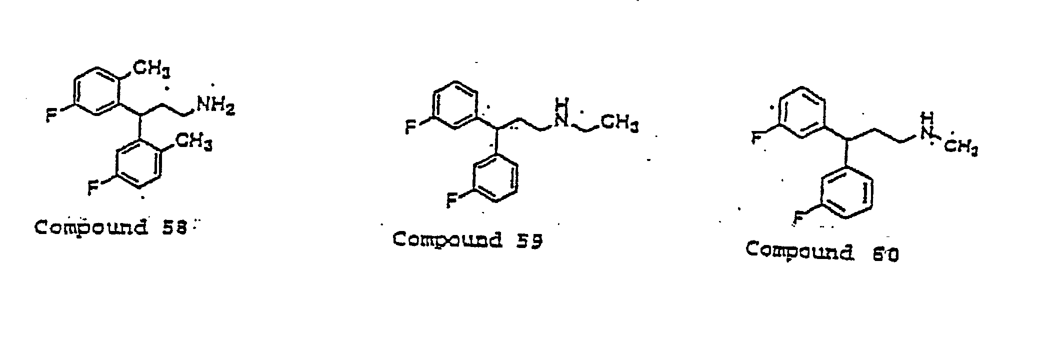 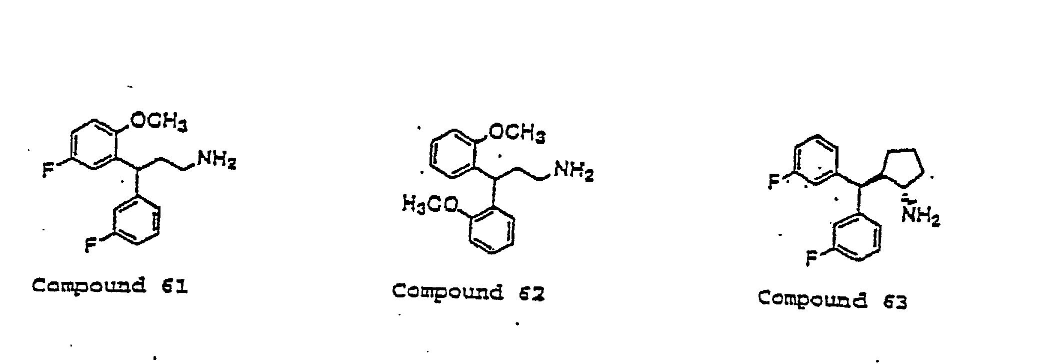 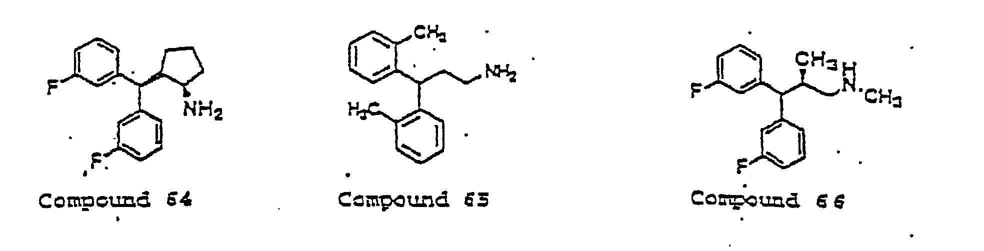 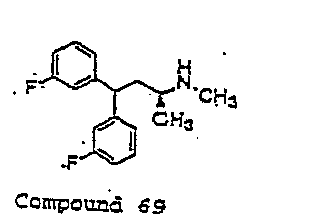 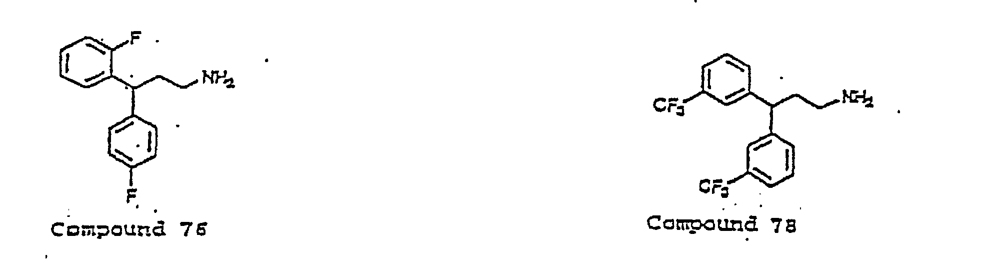 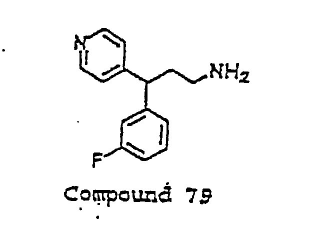 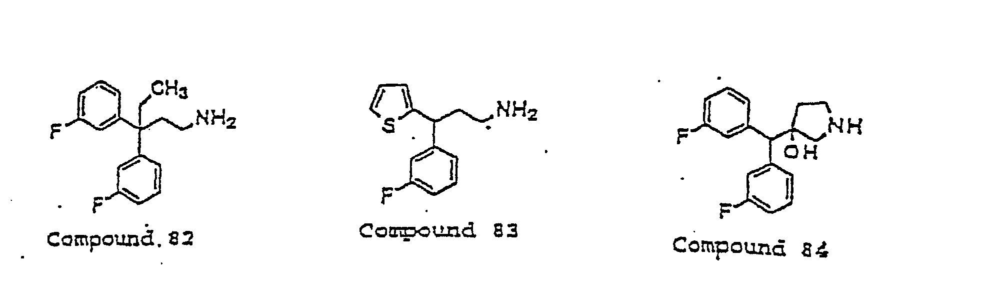 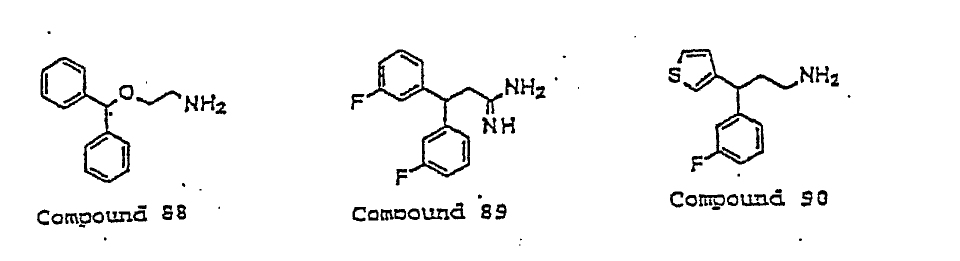 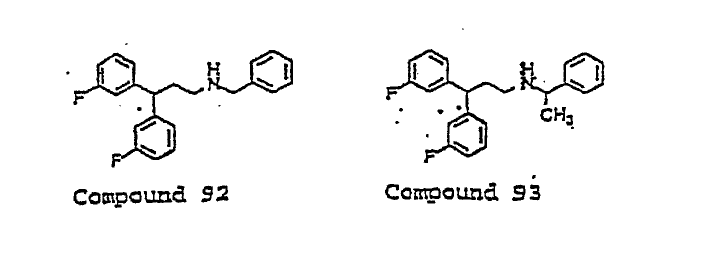 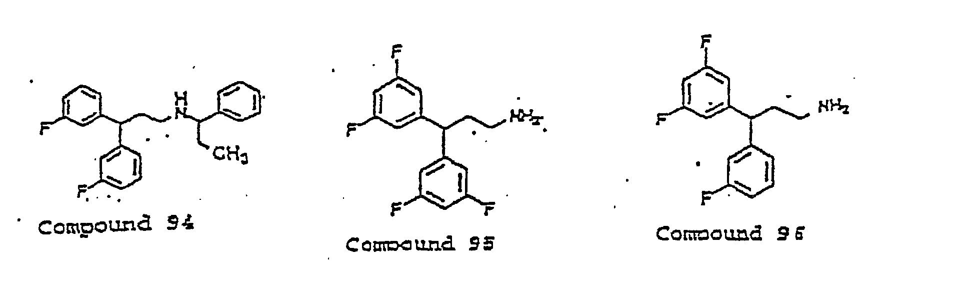 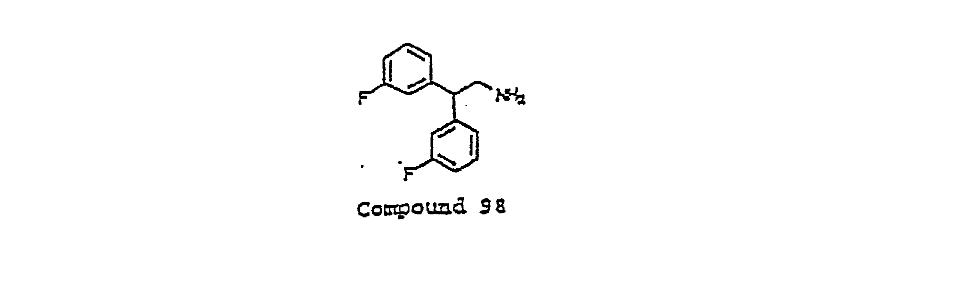 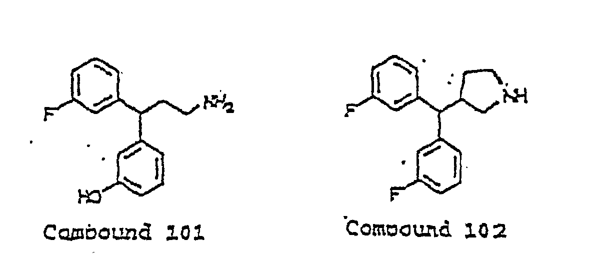 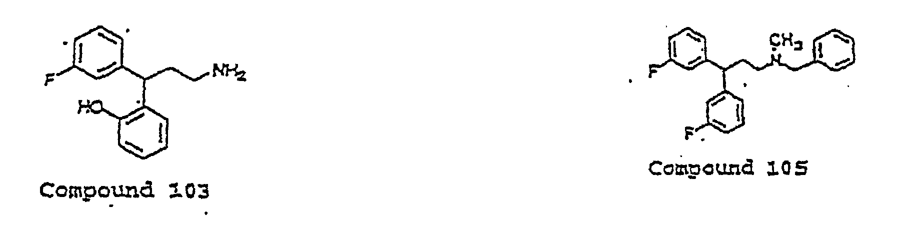 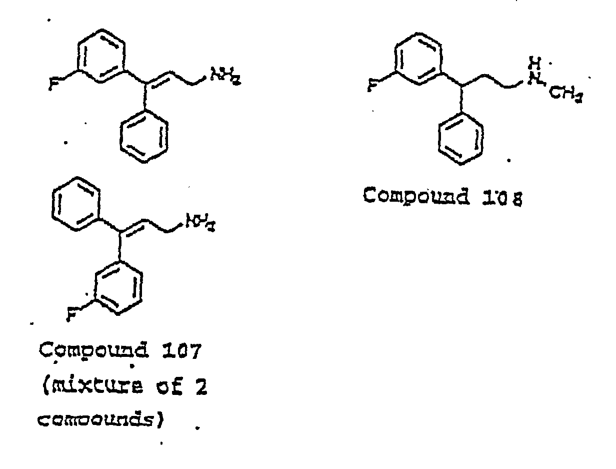 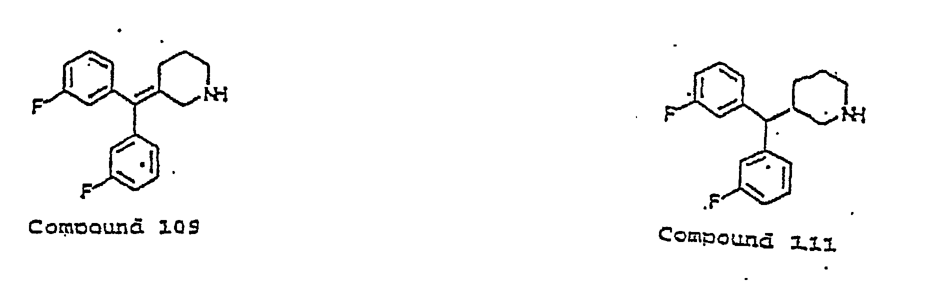 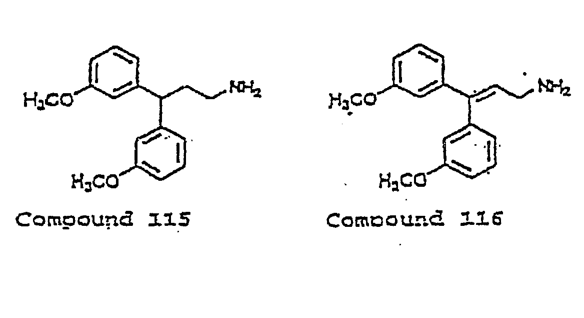 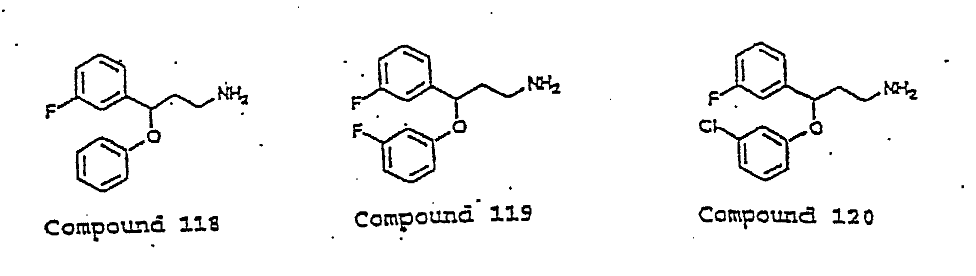 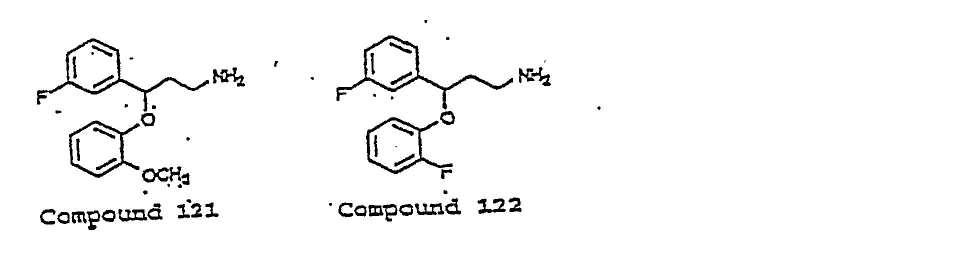 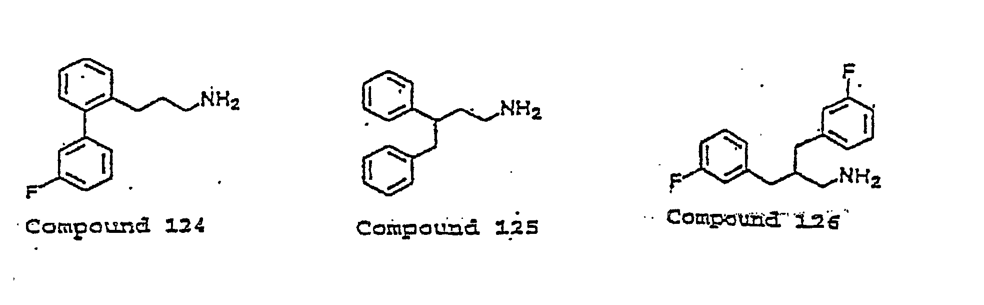 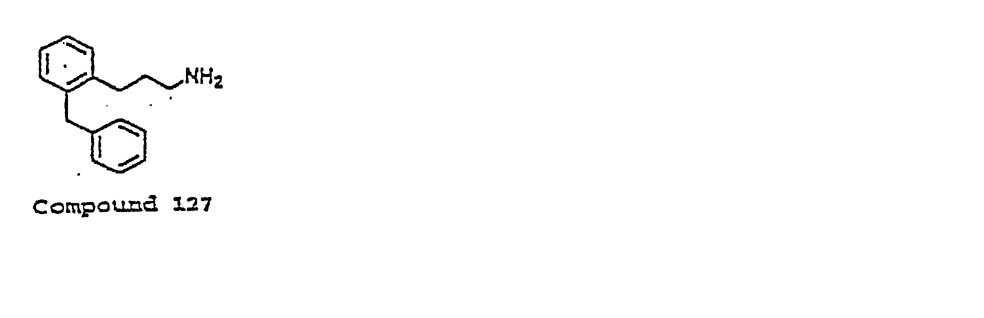 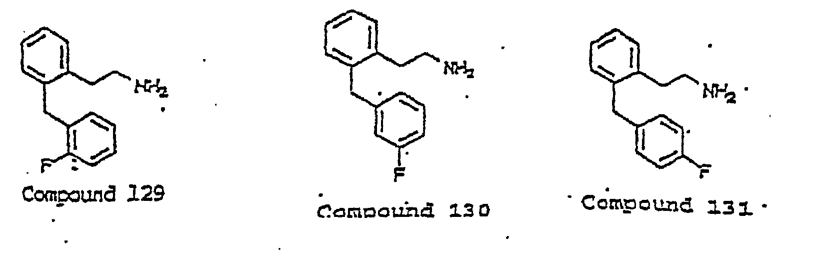 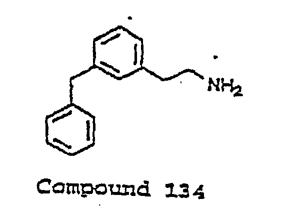 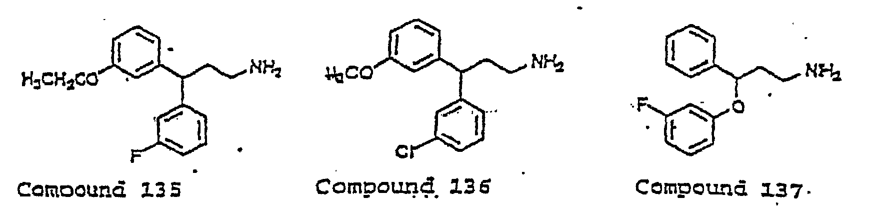 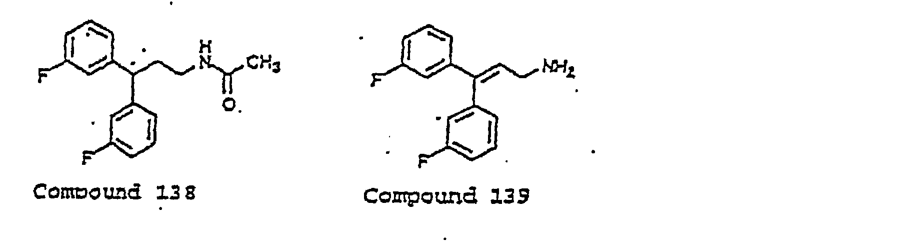 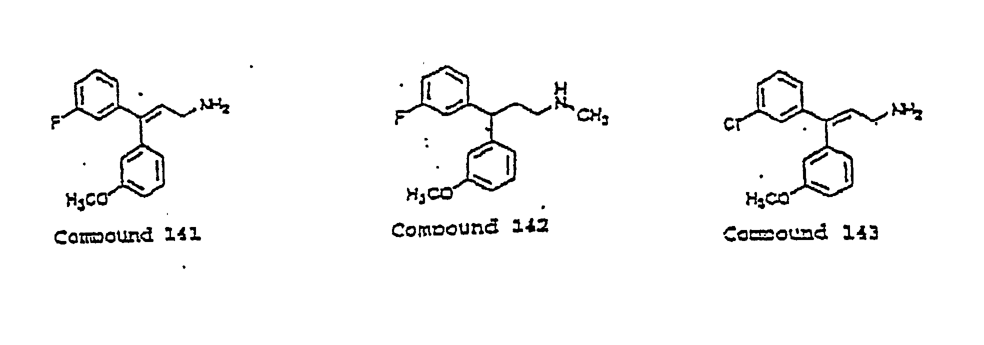 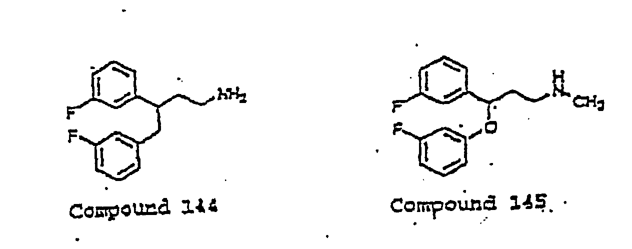 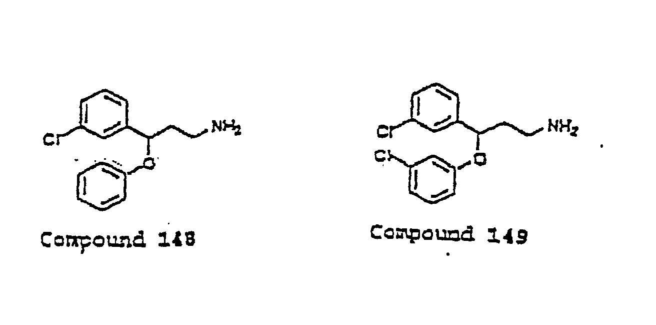 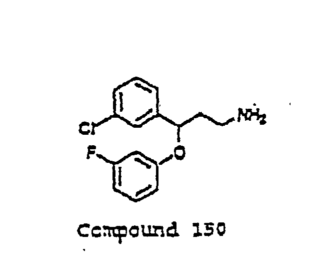 and pharmaceutically acceptable salts thereof.

23. The compound according to claim 22, selected from the group consisting of compounds 54-66, 69, 76, 82, 83, 88-90, 92-96, 101, 102, 103, 105, 108, 109, 111, 115, 118-122, 125-127, 129-131, 135-139, 142, 144, 145, 148-150, or pharmaceutically acceptable salts thereof.

24. The compound according to claim 22, selected from the group consisting of compounds 54-66, 69, 82, 83, 89, 90, 93-96, 103, 111,118-120, 122, 126, 135-138, 142,144,145,148-150, or pharmaceutically acceptable salts thereof.

25. The compound according to claim 22, selected from the group consisting of compounds 60, 66, 69, 103, 111, 118-120, 122, 136-138, 142, 144, 145, 148-150, or pharmaceutically acceptable salts thereof.

26. The compound according to claim 22, selected from the group consisting of compounds 118-122, 137, 145, 148-150, or pharmaceutically acceptable salts thereof.

27. The compound according to claim 22, selected from the group consisting of compounds 118-122,148-150, or pharmaceutically acceptable salts thereof.

28. The compound according to claim 22, selected from the group consisting of compounds 63 and 64, or pharmaceutically acceptable salts thereof.

29. The compound according to claim 22, which is compound 119, or pharmaceutically acceptable salts thereof.

30. The compound according to claim 22, which is compound 144, or pharmaceutically acceptable salts thereof.

31. Compound 60, or pharmaceutically acceptable salts thereof.

32. A compound of the formula: 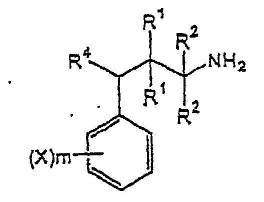 wherein:
X is independently selected from the group consisting of -Br, -Cl, -F, -I,-CF₃, alkyl, -OH, -OCF₃, -O-alkyl, and -O-acyl;
R₁ is independently selected from the group consisting of -H, C₁-C₄ alkyl, and -O-acyl;
R₂ is independently selected from the group consisting of -H, alkyl, and hydroxyalkyl, or both R₂s together are imino;
R₄ is phenoxy which is optionally substituted with -F, -Cl, -Br, -I, -CF₃, alkyl, -OH,- OCF₃ -O-alkyl, or -O-acyl; and
m is independently an integer from 1 to 5; and pharmaceutically acceptable salts and complexes thereof:

33. A compound of the formula: 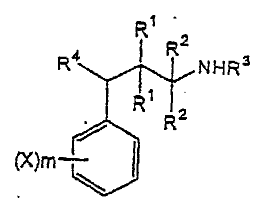 wherein:
X is independently selected from the group consisting of -F, -Cl, -Br, -I, -CF₃ alkyl, -OH, -OCF₃, -O-alkyl, and -O-acyl;
R₁ is independently selected from the group consisting of-H, C₁-C₄ alkyl, and -O-acyl;
R₂ is independently selected from the group consisting of-H, C₁-C₄ alkyl, and hydroxyalkyl, or both R₂s together are imino;
R₃ is selected from the group consisting of methyl and ethyl;
R₄ is phenoxy which is optionally substituted with -F, -Cl, -Br, -I, -CF₃, alkyl, -OH, -OCF₃, -O-alkyl, or -O-acyl;
and m is independently an integer from 1 to 5; and pharmaceutically acceptable salts and complexes thereof provided that said compound is not N-methyl 3-(m-trifluoromethylphenoxy)-3-(4-fluorophenyl)propylamine.

34. A compound of the formula: 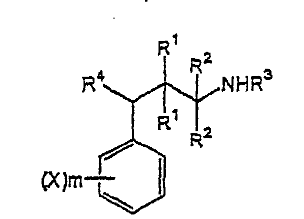
(X)m is selected from the group consisting of meta-fluoro, meta-chloro, ortho-O-C₁-C₄ alkyl, ortho-methyl, ortho-fluoro, ortho-chloro, meta-O- C₁-C₄ alkyl, meta-methyl, ortho-OH, and meta-OH;
R₁ is H;
R₂ is H;
R₃ is selected from the group consisting of methyl and ethyl;
R₄ is phenoxy which is optionally substituted with -F, -Cl, -Br, -I, -CF₃, alkyl, -OH, -OCF₃, -O-alkyl, or -O-acyl; and pharmaceutically acceptable salts and complexes thereof.

35. A pharmaceutical composition comprising a compound which is selected from the group consisting of 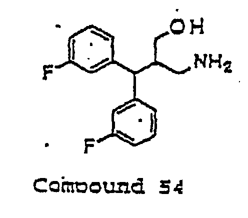 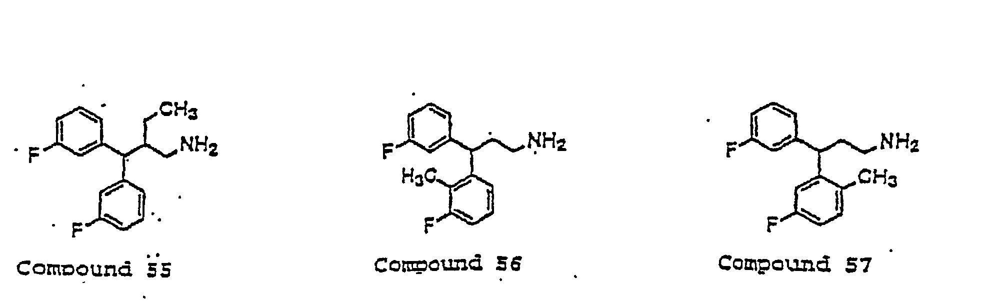 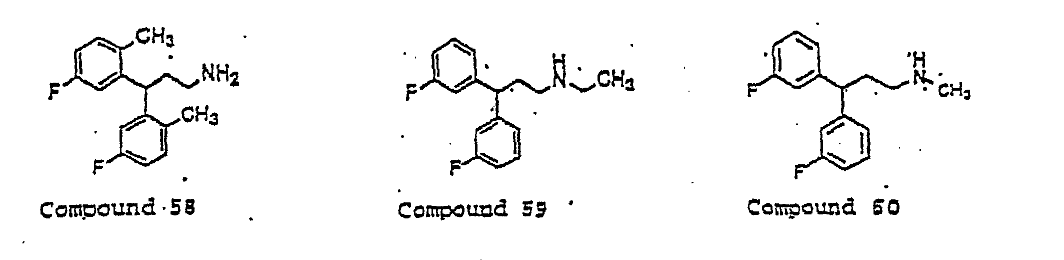 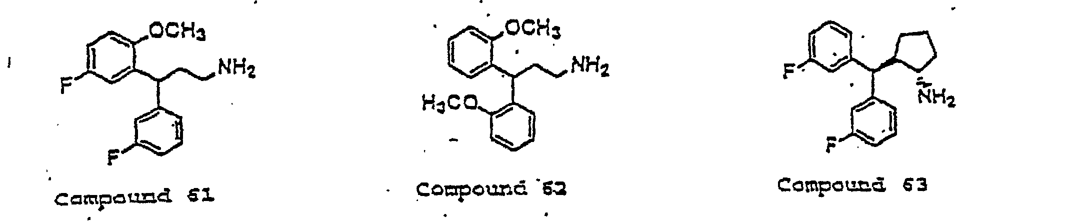 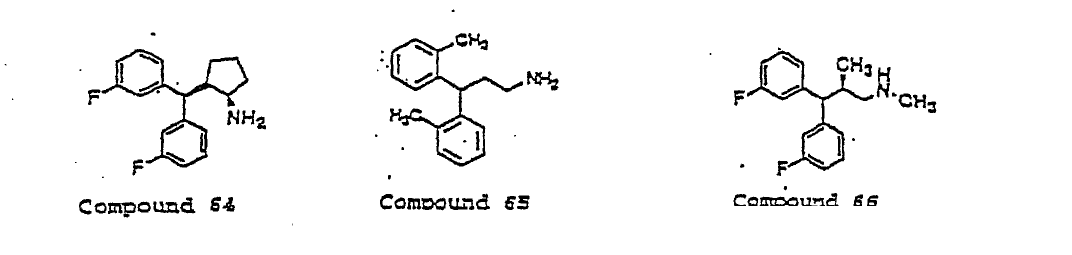 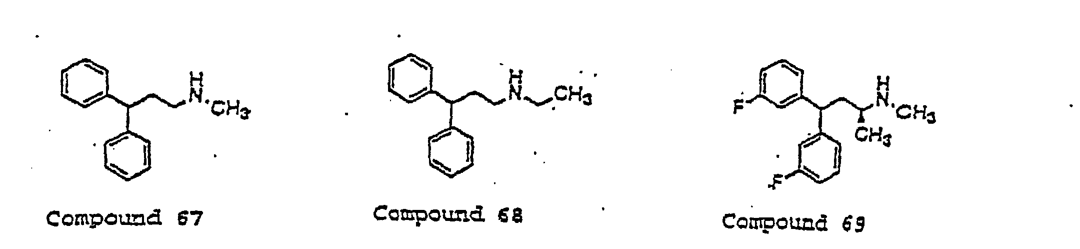 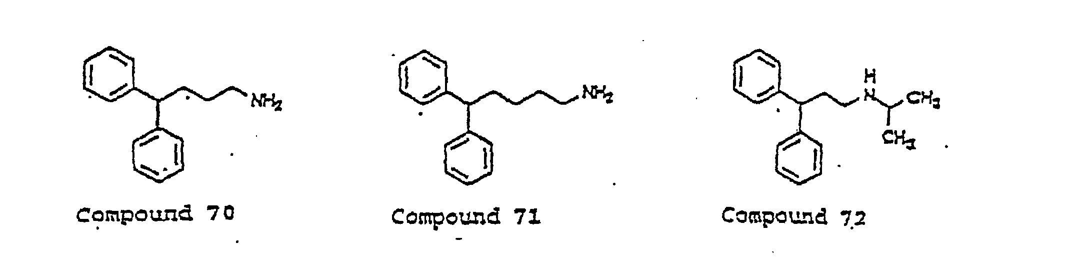 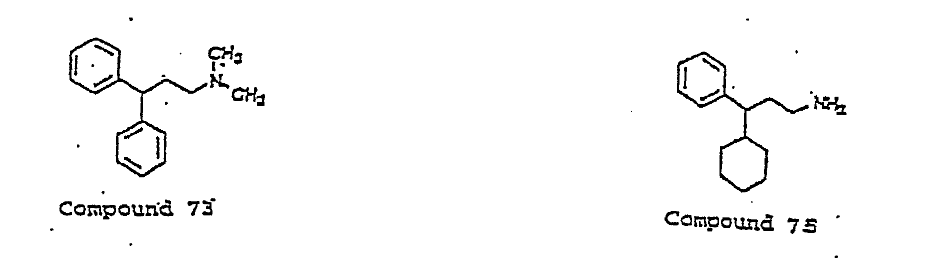 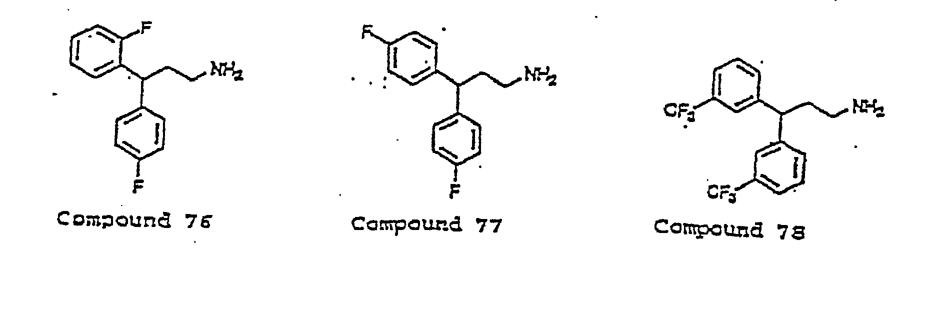 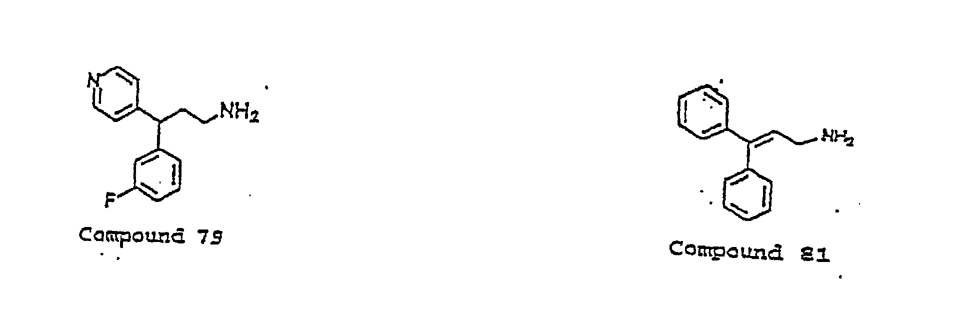 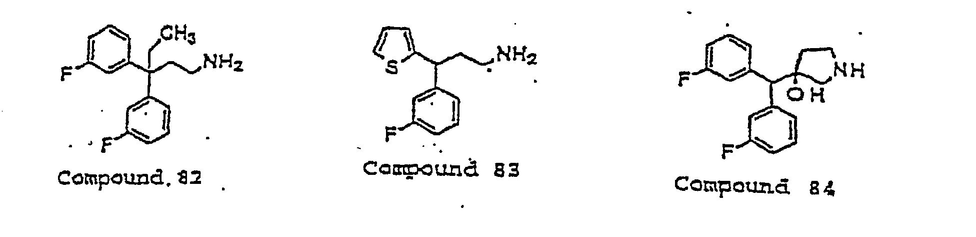 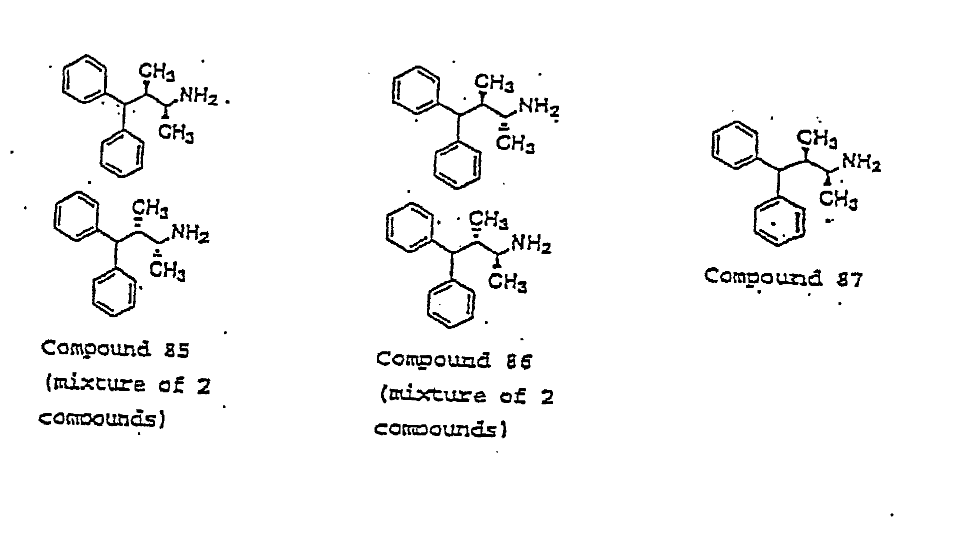 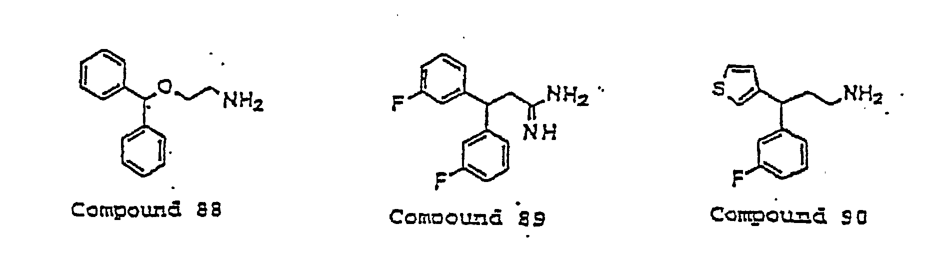 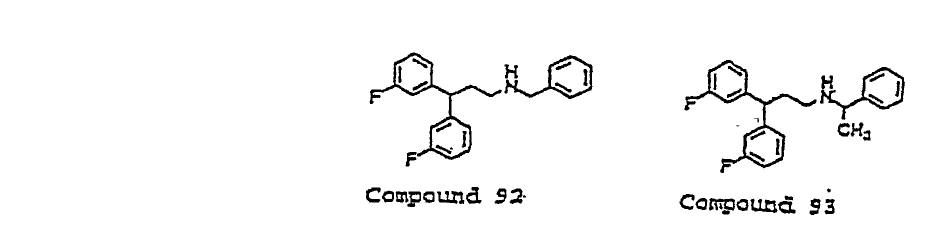 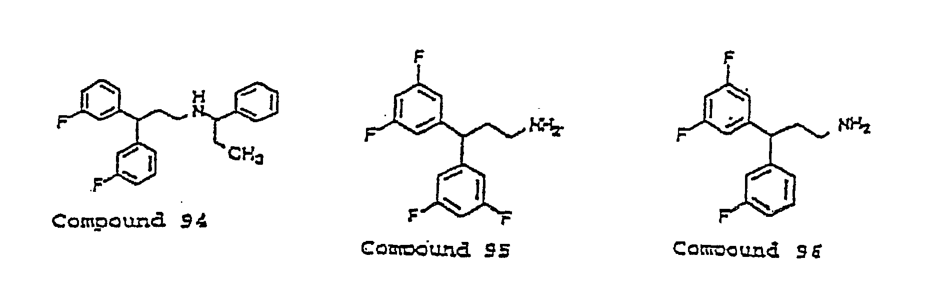 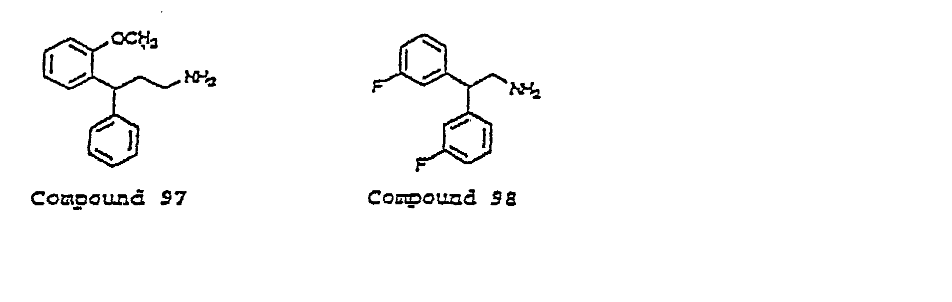 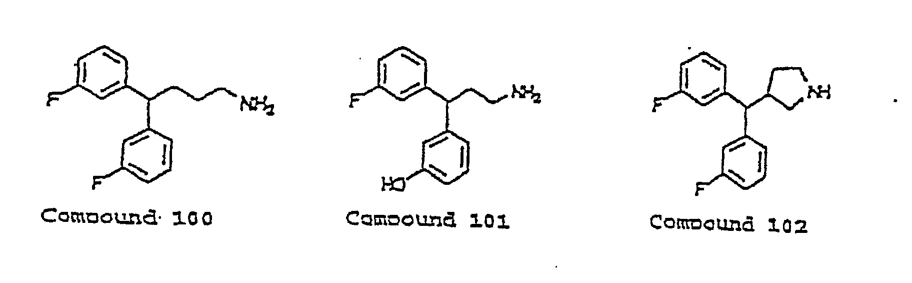 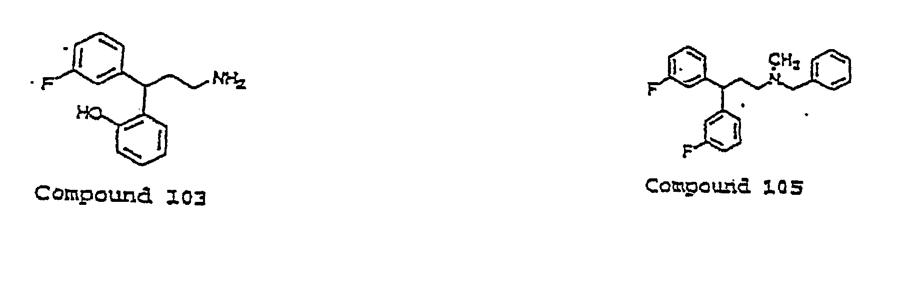 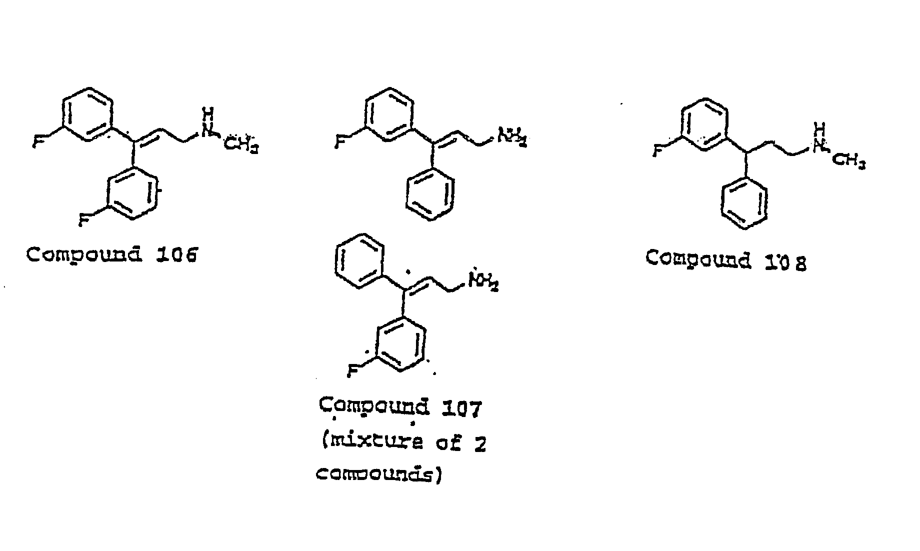 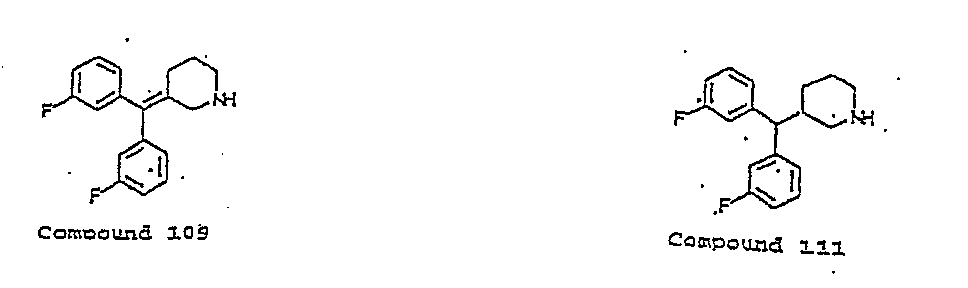 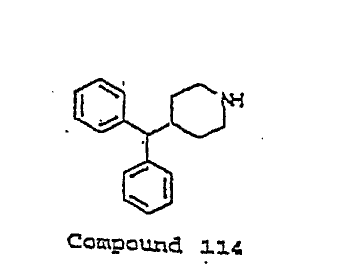 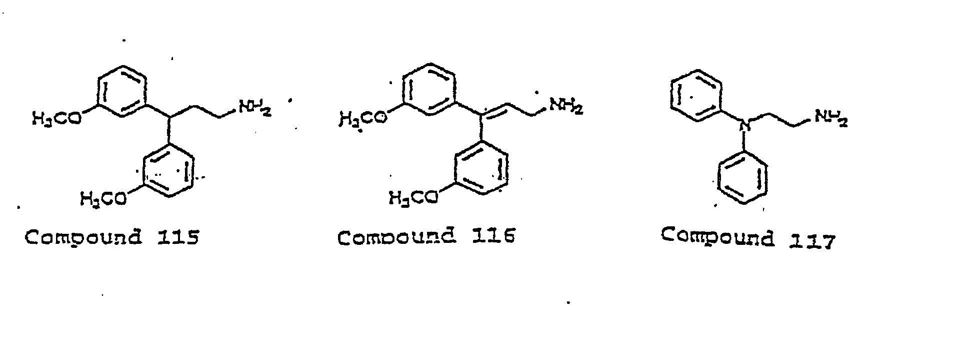 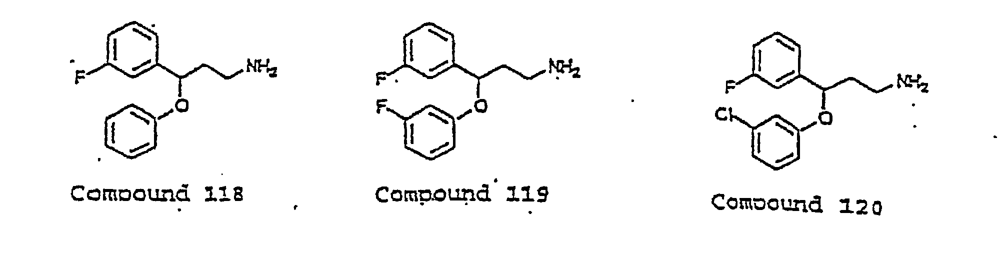 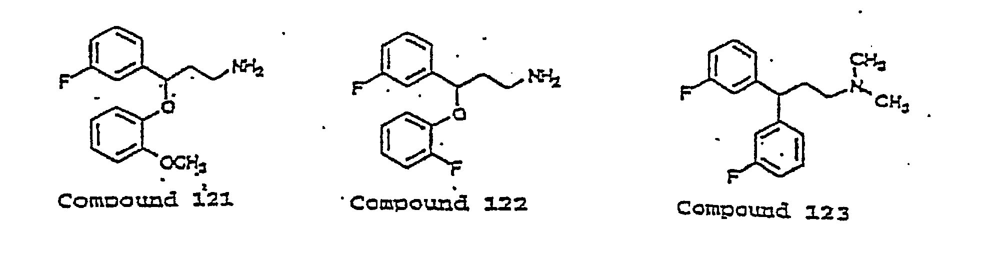 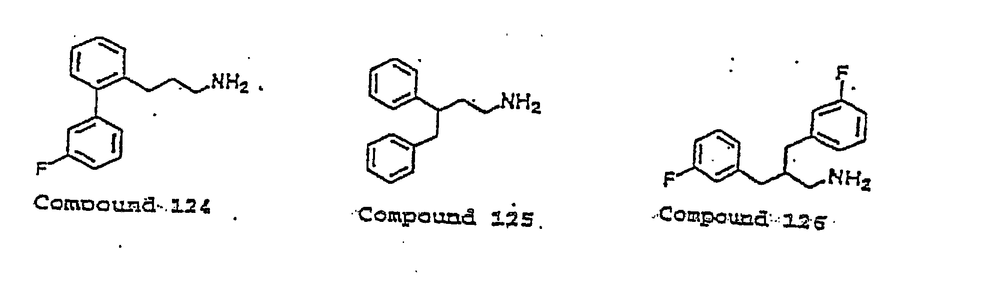 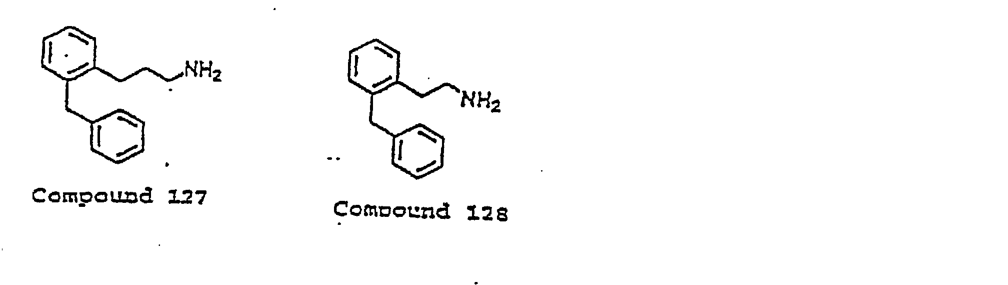 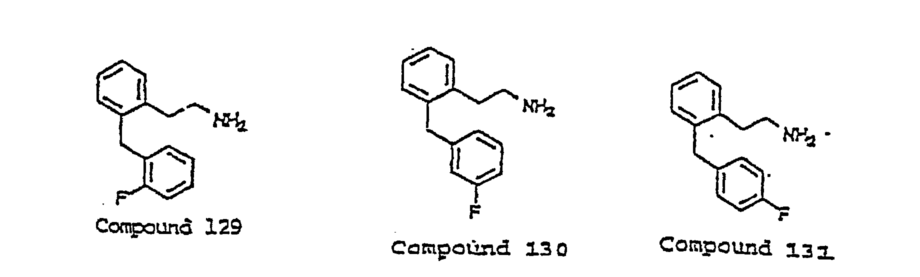 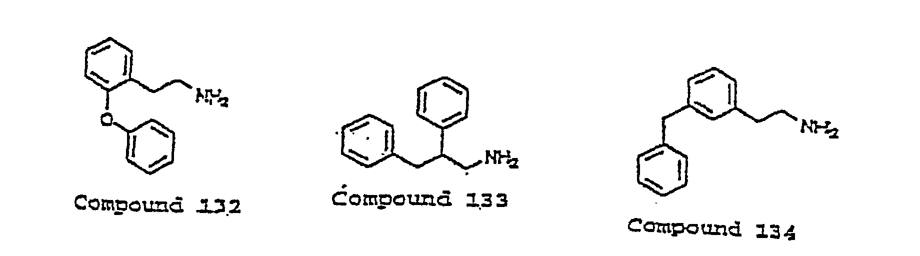 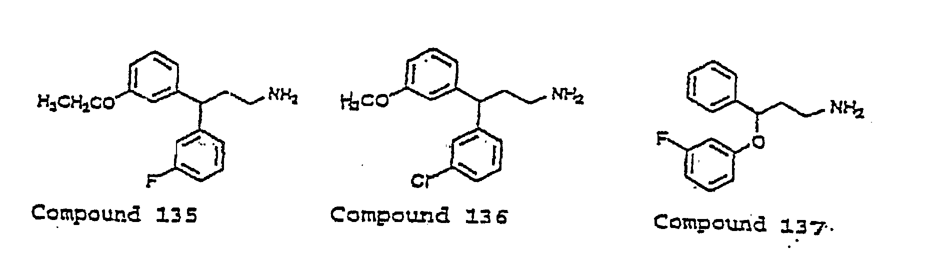 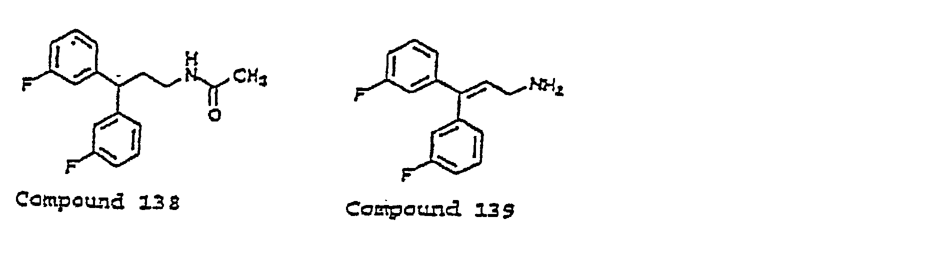 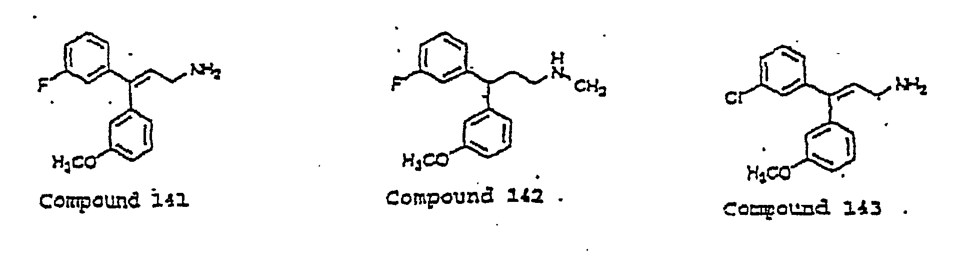 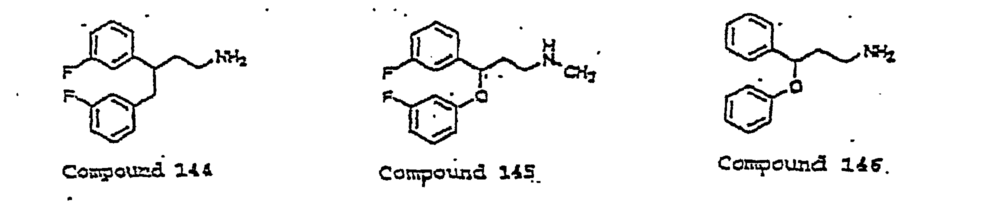 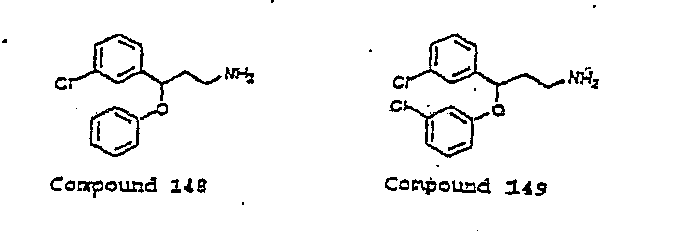 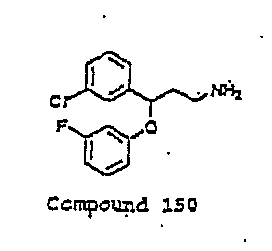 and pharmaceutically acceptable salts thereof in a pharmaceutically acceptable carrier.

36. The pharmaceutical composition according to claim 35, comprising a compound selected from the group consisting of compounds 54-71, 73, 76-79, 81-84, 88-90, 92-98, 101-103, 105, 107-109, 111, 115, 117-123, 125-127, 129-136, 138, 139, 142, 144-146, 148-150, and pharmaceutically acceptable salts thereof, in a pharmaceutically acceptable carrier.

37. The pharmaceutical composition according to claim 35, comprising a compound selected from the group consisting of compounds 54-66, 69, 70, 75, 76, 81-83, 85-90, 92-97, 100-103, 105, 106, 108, 109, 111, 115, 118-122, 125-133, 135-139, 142, 144-146, 148-150, and pharmaceutically acceptable salts thereof, in a pharmaceutically acceptable carrier.

38. The pharmaceutical composition according to claim 35, comprising a compound selected from the group consisting of compounds 54-66, 69, 70, 76, 81-83, 88-90, 92-97, 101-103, 105, 106, 108, 109, 111, 115, 118-122, 125-127, 129-133, 135, 136, 138, 139, 142, 144-146, 148-150, and pharmaceutically acceptable salts thereof, in a pharmaceutically acceptable carrier.

39. The pharmaceutical composition according to claim 35, comprising a compound selected from the group consisting of compounds 54-66, 69, 82, 83, 89, 90, 93-97, 103, 111, 118-120, 122, 126, 135- 138, 142, 144, 145, 148-150, and pharmaceutically acceptable salts thereof, in a pharmaceutically acceptable carrier.

40. The pharmaceutical composition according to claim 35, comprising a compound selected from the group consisting of compounds 54-66, 69, 82, 83, 89, 90, 93-97, 103, 111, 118-120, 122, 126, 135, 136, 138, 142, 144, 145, 148-150, and pharmaceutically acceptable salts thereof, in a pharmaceutically acceptable carrier.

41. The pharmaceutical composition according to claim 35, comprising a compound selected from the group consisting of compounds 60, 66, 69, 103, 111, 118-120, 122, 136-138, 142, 144, 145, 148-150, and pharmaceutically acceptable salts thereof, in a pharmaceutically acceptable carrier.

42. The pharmaceutical composition according to claim 35, comprising a compound selected from the group consisting of compounds 118-122, 137, 145, 148-150, and pharmaceutically acceptable salts thereof, in a pharmaceutically acceptable carrier.

43. The pharmaceutical composition according to claim 35, comprising a compound selected from the group consisting of compounds 118-122, 148-150, and pharmaceutically acceptable salts thereof, in a pharmaceutically acceptable carrier.

44. The pharmaceutical composition according to claim 35, comprising a compound selected from the group consisting of compounds 63 and 64, and pharmaceutically acceptable salts thereof, in a pharmaceutically acceptable carrier.

45. The pharmaceutical composition according to claim 35, comprising a compound selected from compound 119, and pharmaceutically acceptable salts thereof, in a pharmaceutically acceptable carrier.

46. The pharmaceutical composition according to claim 35, comprising a compound selected from compound 144, and pharmaceutically acceptable salts thereof, in a pharmaceutically acceptable carrier.

47. A pharmaceutical composition comprising a compound selected from compound 60, and pharmaceutically acceptable salts thereof, in a pharmaceutically acceptable carrier.

48. A pharmaceutical composition comprising a compound of claim 32, in a phamiaceutically acceptable carrier.

49. A pharmaceutical composition comprising a compound of claim 33, in a pharmaceutically acceptable carrier.

50. A pharmaceutical composition comprising a compound of claim 34, in a pharmaceutically acceptable carrier.

51. The pharmaceutical composition of any one of claims 35-50 adapted for the treatment of a neurological disease or disorder.

52. The pharmaceutical composition of claim 51, wherein said neurological disease or disorder is selected from the group consisting of stroke, head trauma, spinal cord injury, epilepsy, anxiety, Alzheimer's disease, Huntington's disease, Parkinson's disease, or amyotrophic lateral sclerosis.

53. The pharmaceutical composition of claim 51, wherein said pharmaceutical composition has neuroprotectant activity.

54. The pharmaceutical composition of claim 52, wherein said stroke is global ischemic.

55. The pharmaceutical composition of claim 52, wherein said stroke is focal ischemic.

56. The pharmaceutical composition of claim 52, wherein said stroke is hemorrhagic.

57. The pharmaceutical composition of claim 52, wherein said neurological disease or disorder is Parkinson's disease.

## Patentansprüche

1. Verwendung einer Verbindung, die'aus 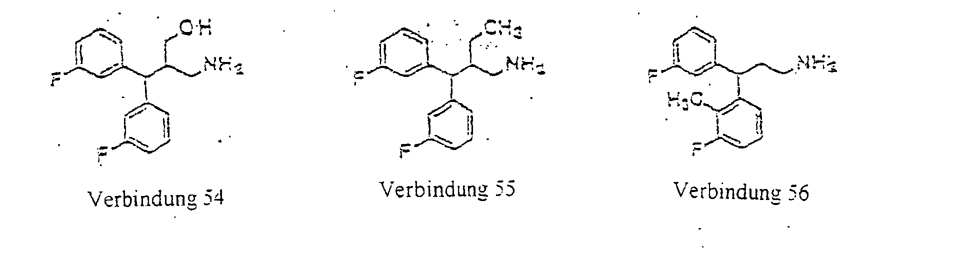 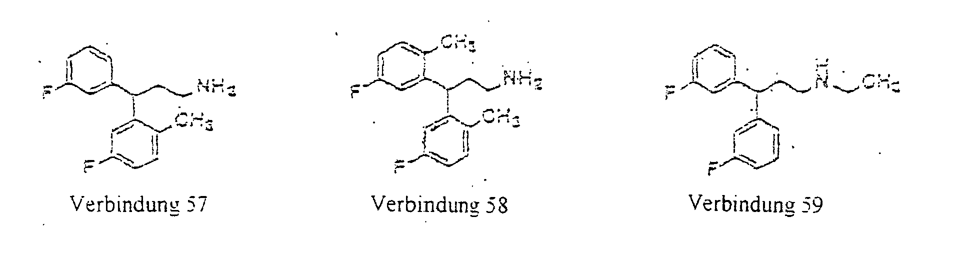 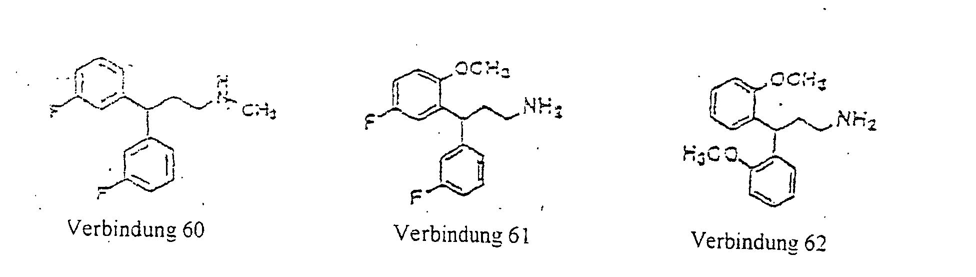 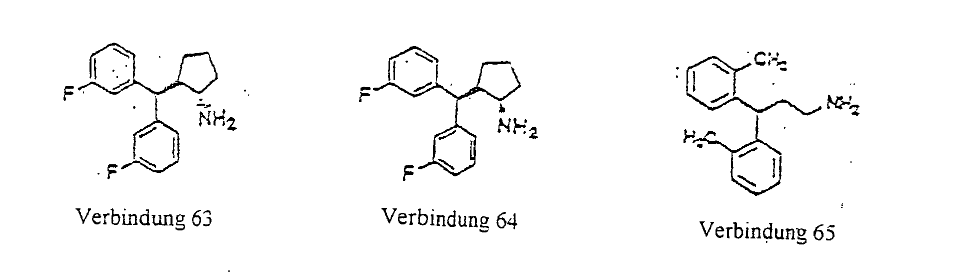 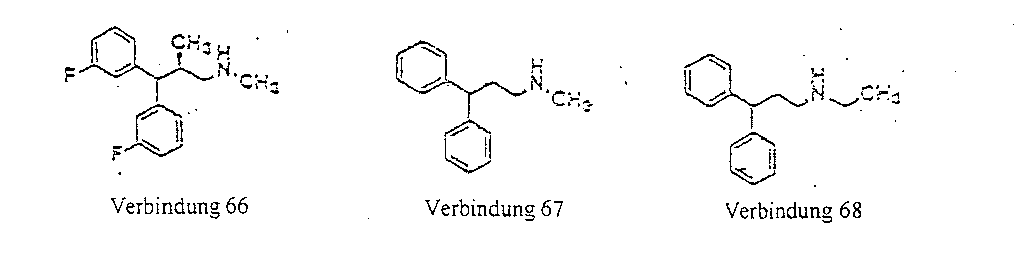 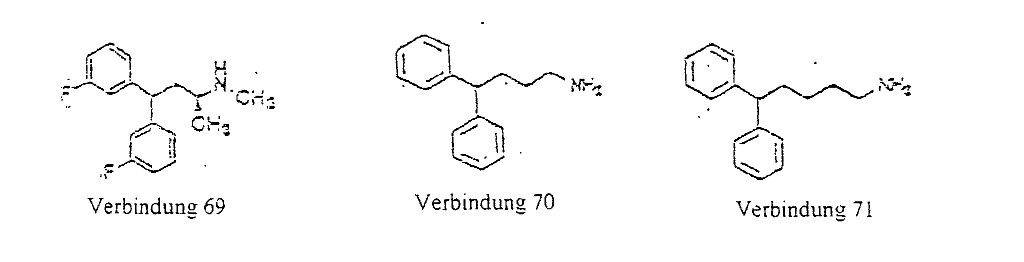 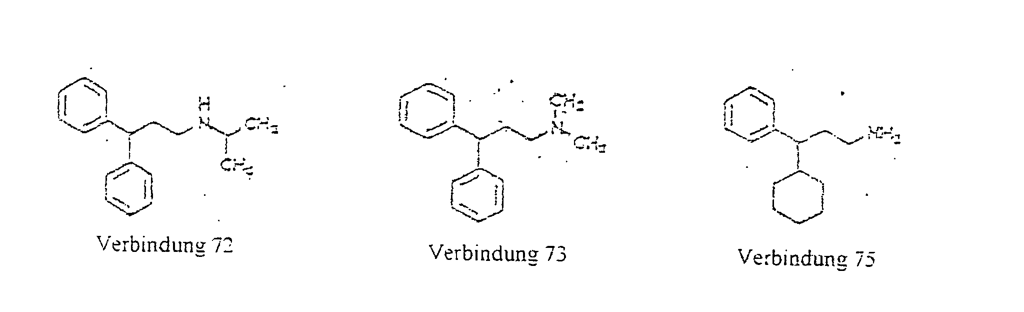 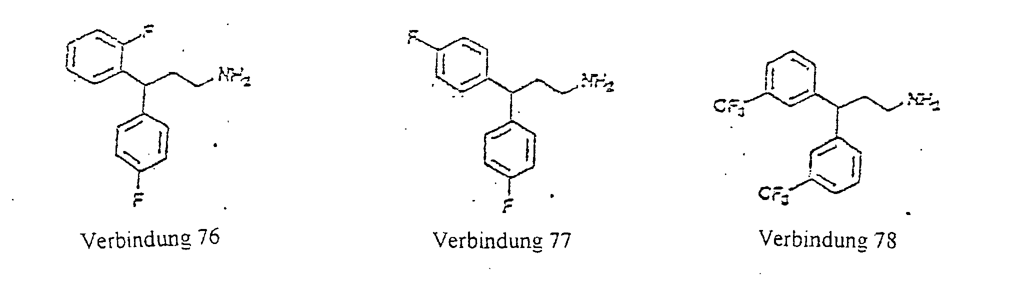 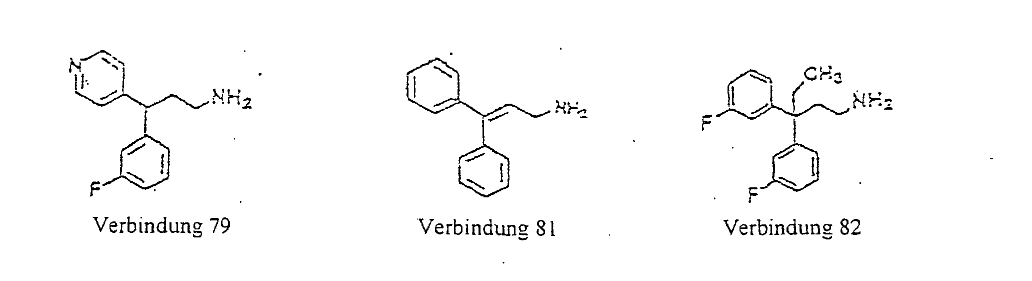 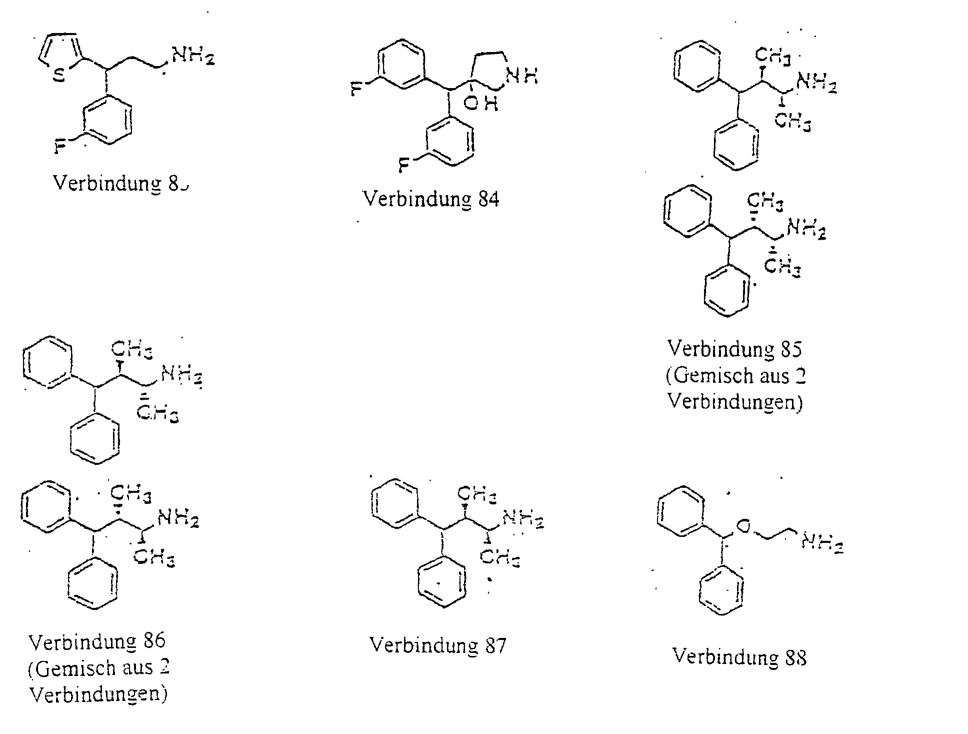 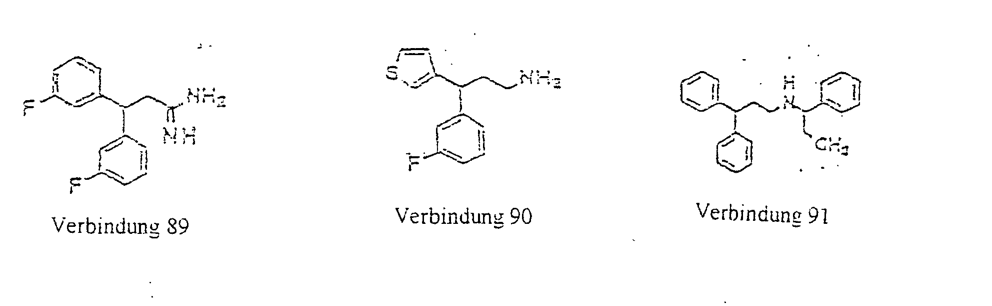 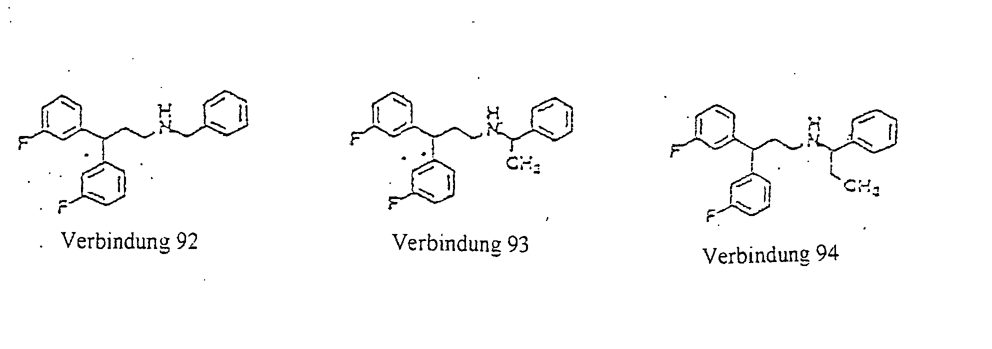 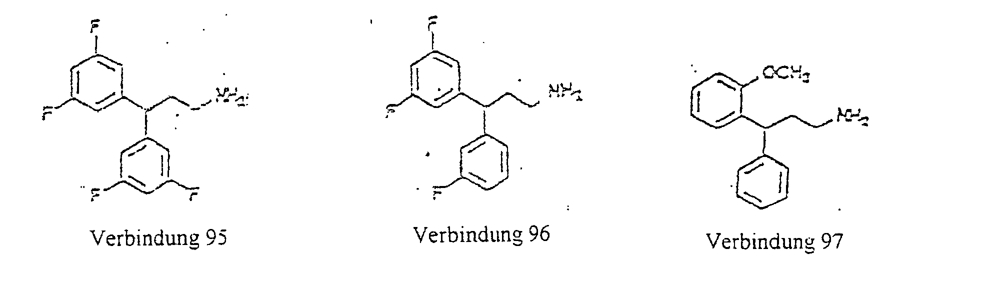 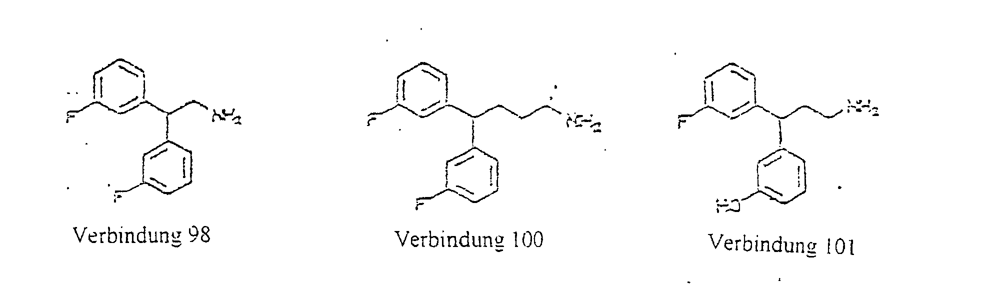 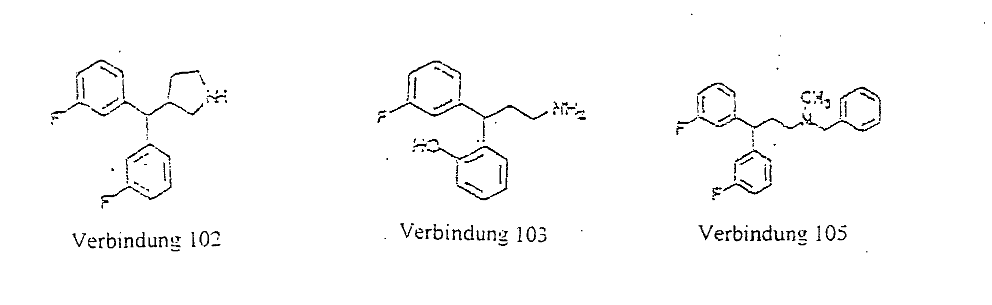 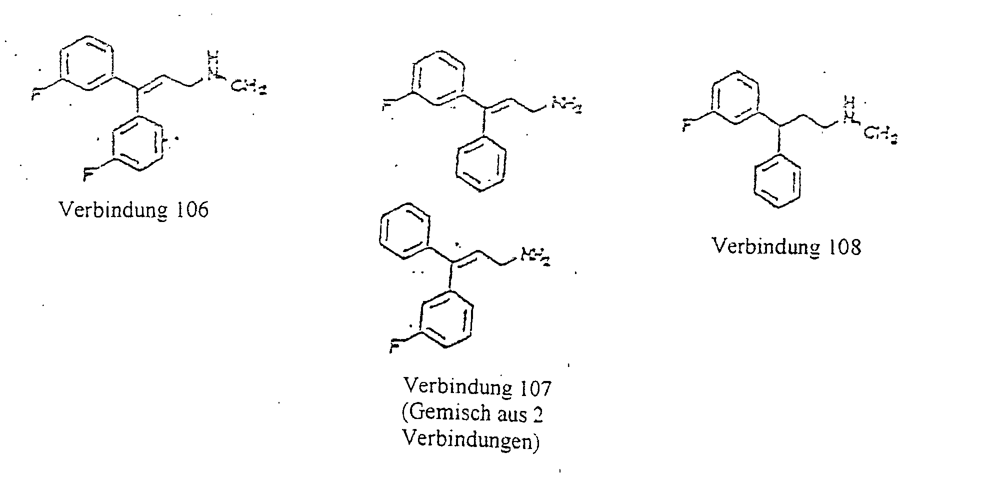 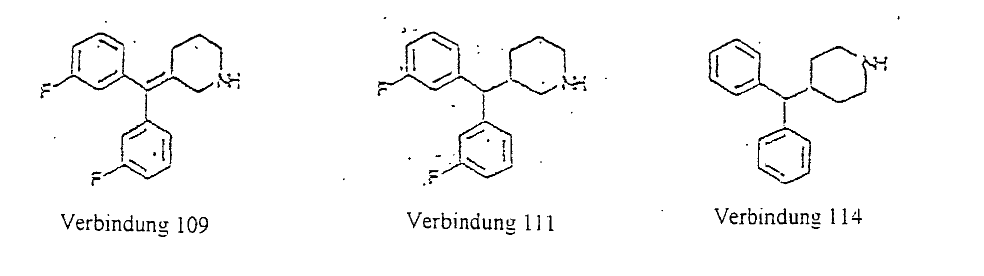 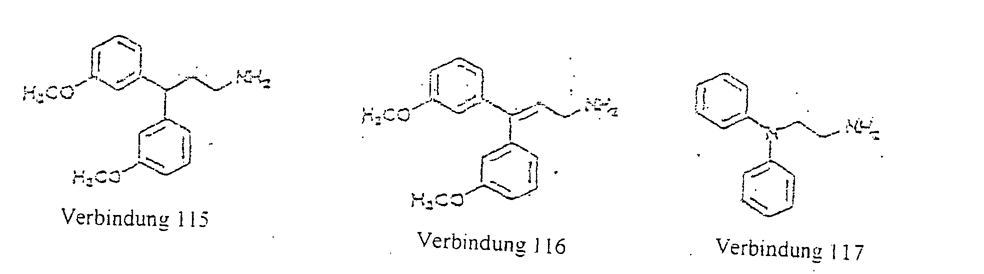 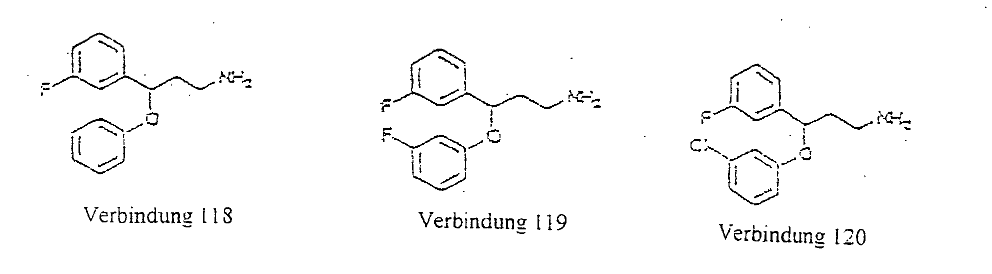 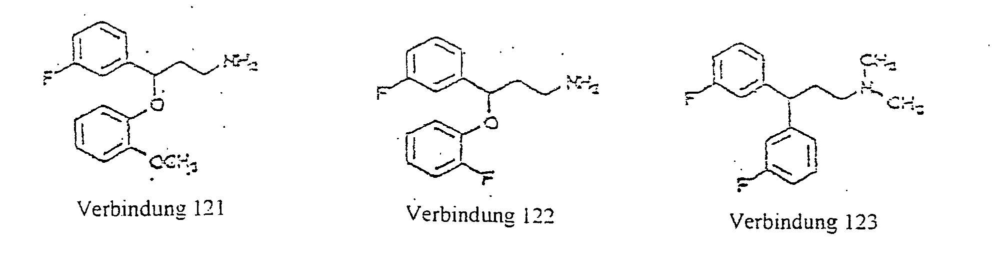 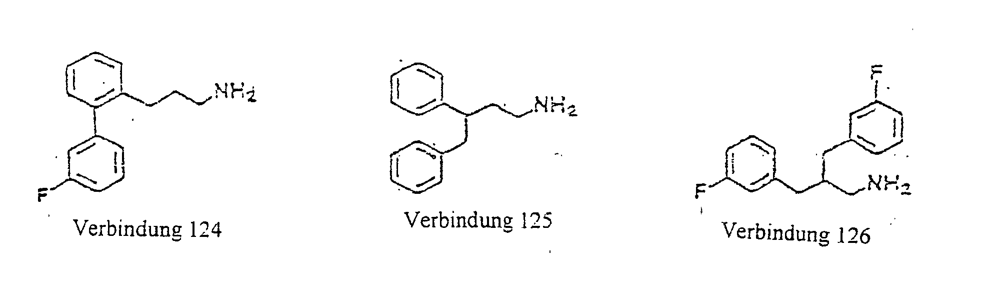 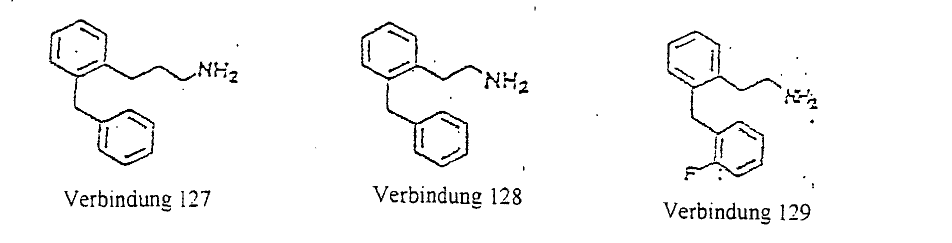 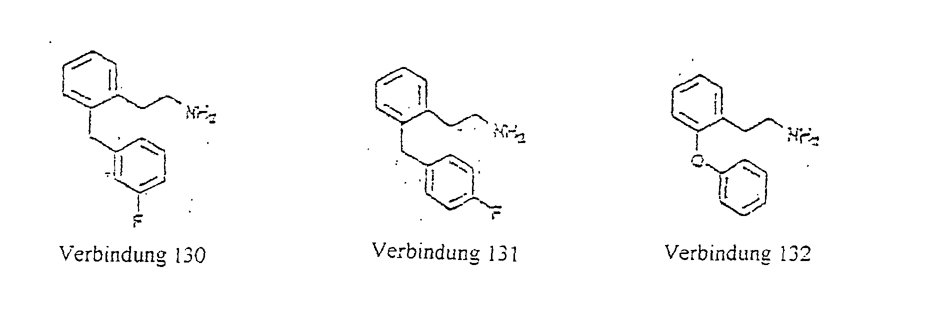 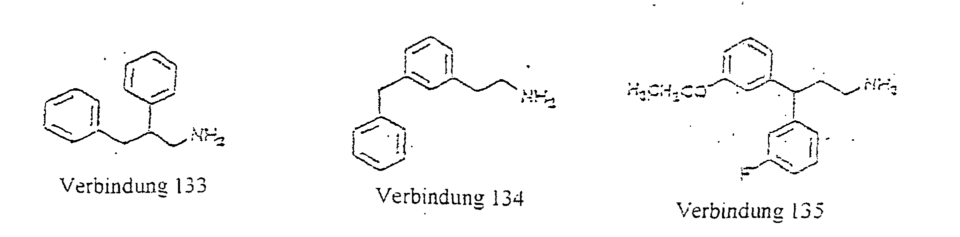 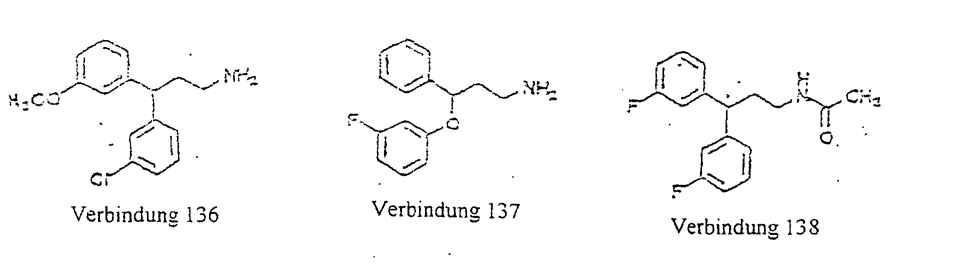 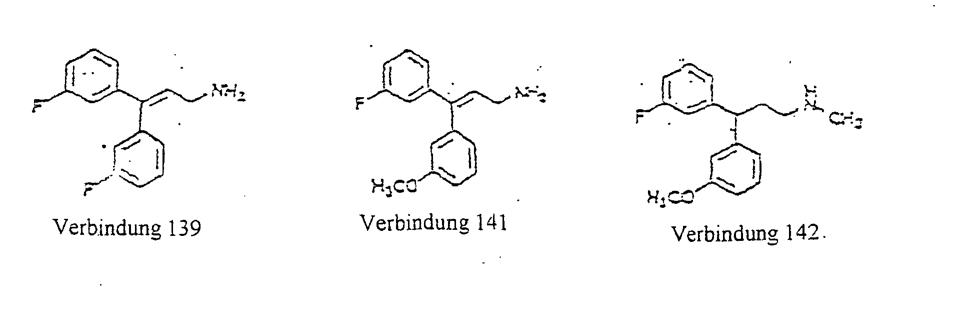 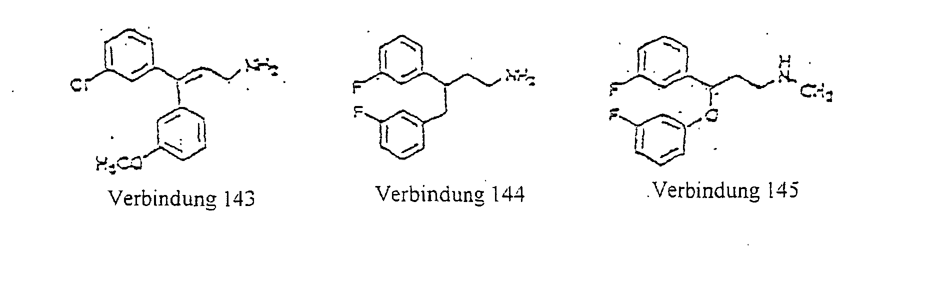 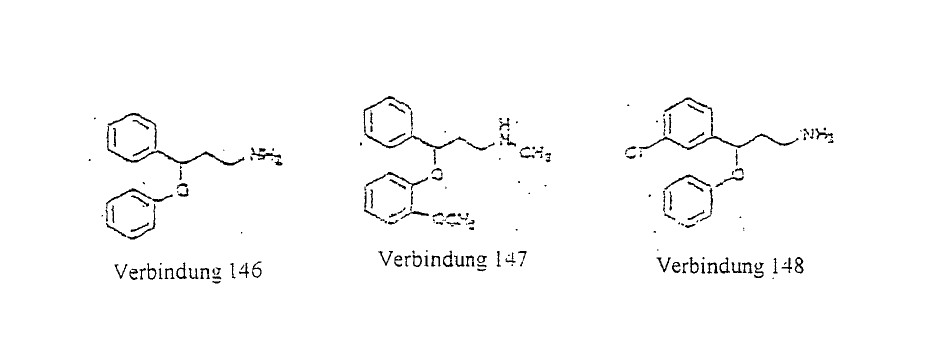 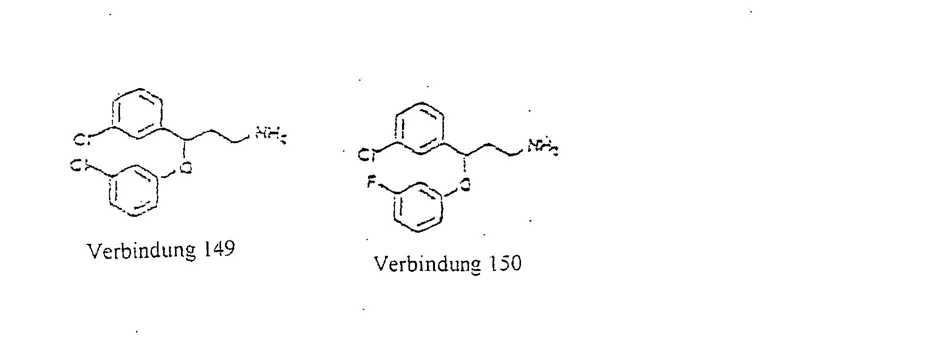 und pharmazeutisch verträglichen Salzen und Komplexen davon ausgewählt ist, zur Herstellung eines Arzneimittels zur Behandlung von neurologischen Krankheiten oder Störungen.

2. Verwendung gemäß Anspruch 1, wobei die Verbindung aus den Verbindungen 54-66, 68-71, 75, 76, 78, 79, 81-90, 92-98, 100, 101, 103, 105, 106, 108, 109, 111, 114-122, 124-136, 138, 139, 141-144, 148-150 und pharmazeutisch verträglichen Salzen und Komplexen davon ausgewählt ist.

3. Verwendung gemäß Anspruch 1, wobei die Verbindung aus den Verbindungen 54-66, 69, 70, 75, 76, 81-83, 85-97, 100-103, 105, 106, 108, 109, 111, 115, 118-122, 125-133, 135-139, 142, 144-150 und pharmazeutisch verträglichen Salzen und Komplexen davon ausgewählt ist.

4. Verwendung gemäß Anspruch 1, wobei die Verbindung aus den Verbindungen 54-66, 69, 70, 75, 76, 81-83, 85-90, 92-97, 100, 101, 103, 105, 106, 108, 109, 111, 115, 118-122, 125-133, 135, 136, 138, 139, 142, 144, 148-150 und pharmazeutisch verträglichen Salzen und Komplexen davon ausgewählt ist.

5. Verwendung gemäß Anspruch 1, wobei die Verbindung aus den Verbindungen 54-66, 69, 82, 83, 89-97, 103, 111, 118-120, 122, 126, 135-138, 142, 144, 145, 147-150 und pharmazeutisch verträglichen Salzen und Komplexen davon ausgewählt ist.

6. Verwendung gemäß Anspruch 1, wobei die Verbindung aus den Verbindungen 54-66, 69, 82, 83, 89, 90, 92-97, 103, 111, 118-120, 122, 126, 135, 136, 138, 142, 144, 148-150 und pharmazeutisch verträglichen Salzen und Komplexen davon ausgewählt ist.

7. Verwendung gemäß Anspruch 1, wobei die Verbindung aus den Verbindungen 60, 66, 69, 103, 111, 118-120, 122, 136, 138, 142, 144, 148-150 und pharmazeutisch verträglichen Salzen und Komplexen davon ausgewählt ist.

8. Verwendung gemäß Anspruch 1, wobei die Verbindung aus den Verbindungen 118-122, 137, 145, 148-150 und pharmazeutisch verträglichen Salzen und Komplexen davon ausgewählt ist.

9. Verwendung gemäß Anspruch 1, wobei die Verbindung aus den Verbindungen 118-122, 148-150 und pharmazeutisch verträglichen Salzen und Komplexen davon ausgewählt ist.

10. Verwendung gemäß Anspruch 1, wobei die Verbindung aus den Verbindungen 63 und 64 und pharmazeutisch verträglichen Salzen und Komplexen davon ausgewählt ist.

11. Verwendung gemäß Anspruch 1, wobei die Verbindung aus der Verbindung 119 und pharmazeutisch verträglichen Salzen und Komplexen davon ausgewählt ist.

12. Verwendung gemäß Anspruch 1, wobei die Verbindung aus der Verbindung 144 und pharmazeutisch verträglichen Salzen und Komplexen davon ausgewählt ist.

13. Verwendung von Verbindung 60 und pharmazeutisch verträglichen Salzen und Komplexen davon zur Herstellung eines Arzneimittels zur Behandlung von neurologischen Krankheiten oder Störungen.

14. Verwendung einer Verbindung der Formel: 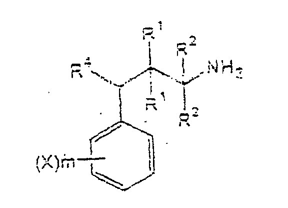 wobei:
X unabhängig aus -Br, -Cl, -F, -I, -CF₃, einem Alkylrest, -OH, -OCF₃, einem -O-Alkyl- und -O-Acylrest ausgewählt ist;
R₁ unabhängig aus -H, einem C₁₋₄-Alkyl- und -O-Acylrest ausgewählt ist;
R₂ unabhängig aus -H, einem Alkyl- und Hydroxyalkylrest ausgewählt ist oder beide Reste R₂ zusammen eine Iminogruppe sind;
R₄ ein Phenoxyrest ist, der gegebenenfalls mit -F, -Cl, -Br, -I, -CF₃, Alkyl,-OH, -OCF₃, -O-Alkyl oder -O-Acyl substituiert ist; und
m unabhängig eine ganze Zahl von 0 bis 5 ist; und pharmazeutisch verträglicher Salze und Komplexe davon mit der Maßgabe, dass die Verbindung nicht:
3-(p-Isopropoxyphenoxy)-3-phenylpropylamin
3-(2'-Methyl-4',5'-dichlorphenoxy)-3-phenylpropylamin
3-(p-t-Butylphenoxy)-3-phenylpropylamin
3-(2',4'-Dichlorphenoxy)-3-phenyl-2-methylpropylamin
3-(o-Ethylphenoxy)-3-phenylpropylamin
3-(o-Methoxyphenoxy)-3-phenylpropylamin
3-Phenoxy-3-phenylpropylamin ist,
zur Herstellung eines Arzneimittels zur Behandlung von neurologischen Krankheiten oder Störungen.

15. Verwendung einer Verbindung der Formel: 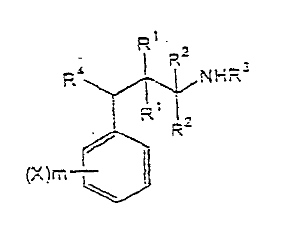 wobei:
X unabhängig aus -F, -Cl, -Br, -I, -CF₃, einem Alkylrest, -OH, -OCF₃, einem -O-Alkyl- und -O-Acylrest ausgewählt ist;
R₁ unabhängig aus -H, einem C₁₋₄-Alkyl- und -O-Acylrest ausgewählt ist;
R₂ unabhängig aus -H, einem C₁₋₄-Alkyl- und Hydroxyalkylrest ausgewählt ist oder beide Reste R₂ zusammen eine Iminogruppe sind;
R₃ aus einer Methyl- und Ethylgruppe ausgewählt ist;
R₄ ein Phenoxyrest ist, der gegebenenfalls mit -F, -Cl, -Br, -I, -CF₃, Alkyl,-OH, -OCF₃, -O-Alkyl oder -O-Acyl substituiert ist; und
m unabhängig eine ganze Zahl von 0 bis 5 ist; und pharmazeutisch verträglicher Salze und Komplexe davon, mit der Maßgabe, dass die Verbindung nicht:
N-Methyl-3-(o-chlor-p-tolyloxy)-3-phenyl-1-methylpropylamin
N-Methyl-3-(p-tolyloxy)-3-phenylpropylamin
N-Methyl-3-(o-chlor-p-isopropylphenoxy)-3-phenyl-2-methylpropylamin
N-Methyl-3-(p-iodphenoxy)-3-phenylpropylamin
N-Methyl-3-(3-n-propylphenoxy)-3-phenylpropylamin
N-Methyl-3-(p-trifluormethylphenoxy)-3-phenylpropylamin
N-Methyl-3-(m-chlorphenoxy)-3-phenylpropylamin
N-Methyl-3-(p-fluorphenoxy)-3-phenylpropylamin
N-Methyl-3-(p-methoxyphenoxy)-3-phenylpropylamin
N-Methyl-3-(o-methoxyphenoxy)-3-phenylpropylamin
N-Methyl-3-(o-fluorphenoxy)-3-phenylpropylamin
N-Methyl-3-(o-tolyloxy)-3-phenylpropylamin
N-Methyl-3-(p-chlorphenoxy)-3-phenylpropylamin
N-Methyl-3-(m-fluorphenoxy)-3-phenylpropylamin
N-Methyl-3-phenoxy-3-phenyl-2-methylpropylamin
N-Methyl-3-phenoxy-3-phenyl-1-methylpropylamin
N-Methyl-3-phenoxy-3-phenylpropylamin
N-Methyl-3-(o-trifluormethylphenoxy)-3-phenylpropylamin
N-Methyl-3-(m-methoxyphenoxy)-3-phenylpropylamin
N-Methyl-3-(o,p-difluorphenoxy)-3-phenylpropylamin
N-Ethyl-3-(o-iodphenoxy)-3-phenylpropylamin
N-Methyl-3-(o-chlorphenoxy)-3-phenylpropylamin
N-Methyl-3-(o-bromphenoxy)-3-phenylpropylamin ist,
zur Herstellung eines Arzneimittels zur Behandlung von neurologischen Krankheiten oder Störungen.

16. Verwendung einer Verbindung der Formel: 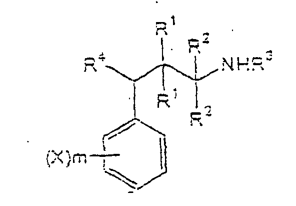 wobei:
(X)m aus einem meta-Fluor-, meta-Chloratom, ortho-O-C₁₋₄-Alkylrest, einer ortho-Methylgruppe, einem ortho-Fluor-, ortho-Chloratom, meta-O-C₁₋₄Alkylrest, einer meta-Methylgruppe, ortho-OH und meta-OH ausgewählt ist;
R₁ H ist;
R₂ H ist;
R₃ aus einer Methyl- und Ethylgruppe ausgewählt ist;
R₄ ein Phenoxyrest ist, der gegebenenfalls mit -F, -Cl, -Br, -I, -CF₃, Alkyl,-OH, -OCF₃, -O-Alkyl oder -O-Acyl substituiert ist; und
pharmazeutisch verträglicher Salze und Komplexe davon zur Herstellung eines Arzneimittels zur Behandlung von neurologischen Krankheiten oder Störungen.

17. Verwendung gemäß einem der Ansprüche 1 bis 16, wobei die neurologische Krankheit oder Störung Schlaganfall, Schädeltrauma, Rückenmarksverletzung, Rückenmarksischämie, eine durch Ischämie oder Hypoxie bedingte Schädigung von Nervenzellen, Epilepsie, Schmerz, Ängstlichkeit, von Ischämie oder Hypoxie ausgelöste neuropsychiatrische oder kognitive Defizite, wie diejenigen, die häufig als eine Konsequenz einer Herzchirurgie mit einem kardiopulmonalen Bypass auftreten, Alzheimer-Krankheit, Chorea Huntington, Parkinson-Krankheit oder amyotrophische Lateralsklerose umfasst.

18. Verwendung gemäß Anspruch 17, wobei der Schlaganfall totalischämisch auftritt.

19. Verwendung gemäß Anspruch 17, wobei der Schlaganfall als fokale Ischämie auftritt.

20. Verwendung gemäß Anspruch 17, wobei der Schlaganfall in hemorrhagischer Form auftritt.

21. Verwendung gemäß Anspruch 17, wobei die neurologische Krankheit oder Störung Parkinson-Krankheit umfasst.

22. Verbindung, ausgewählt aus 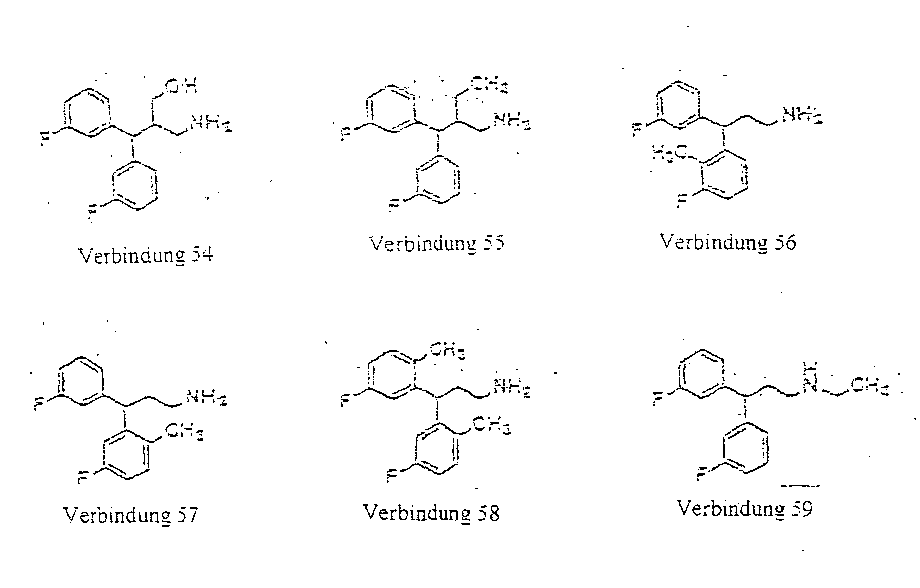 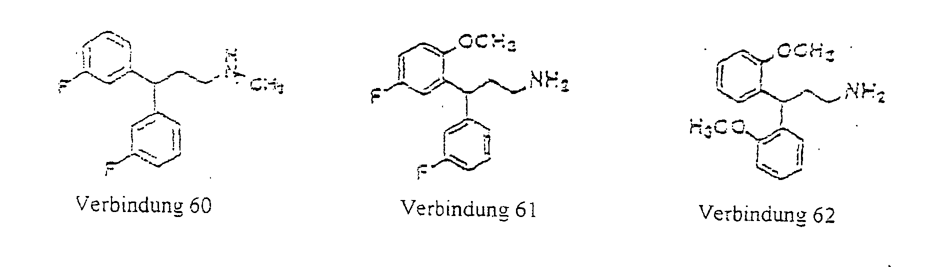 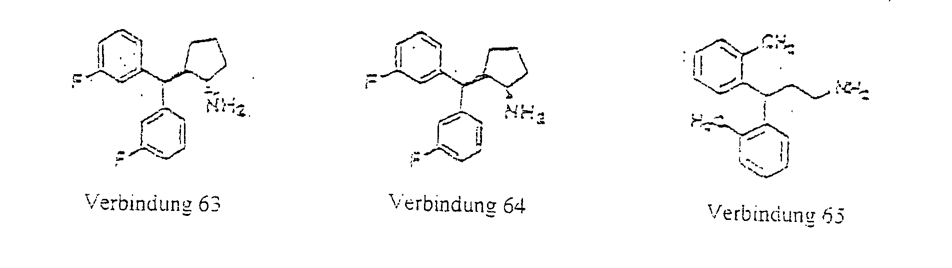 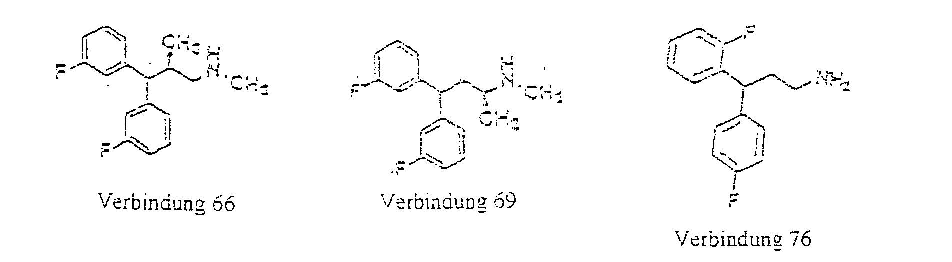 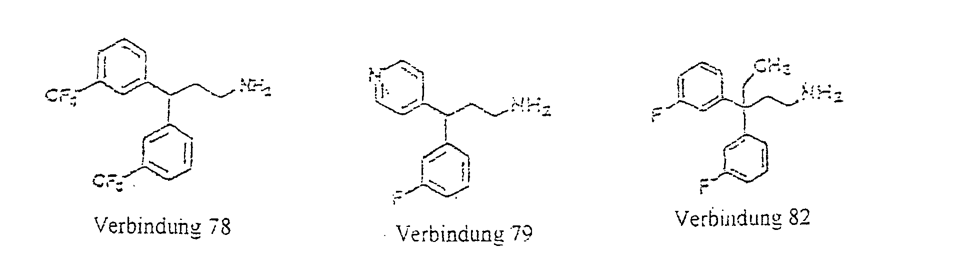 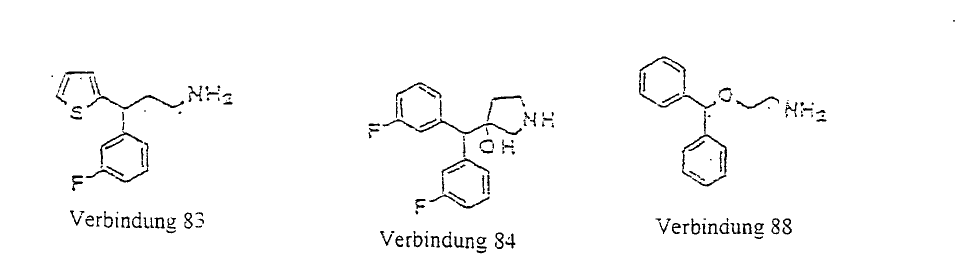 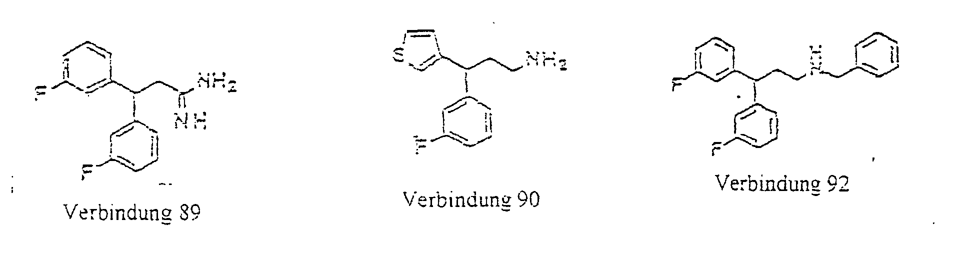 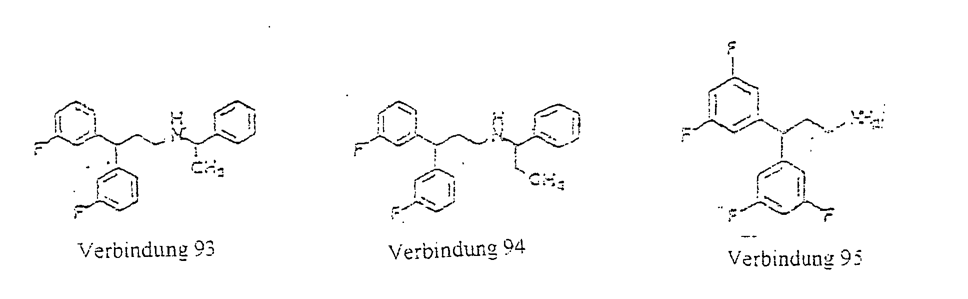 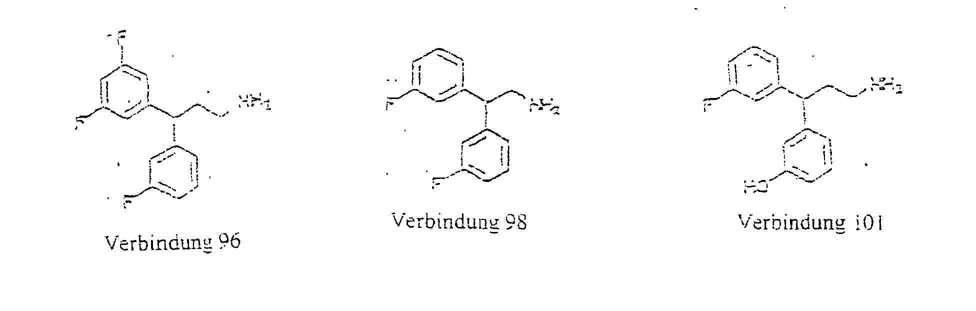 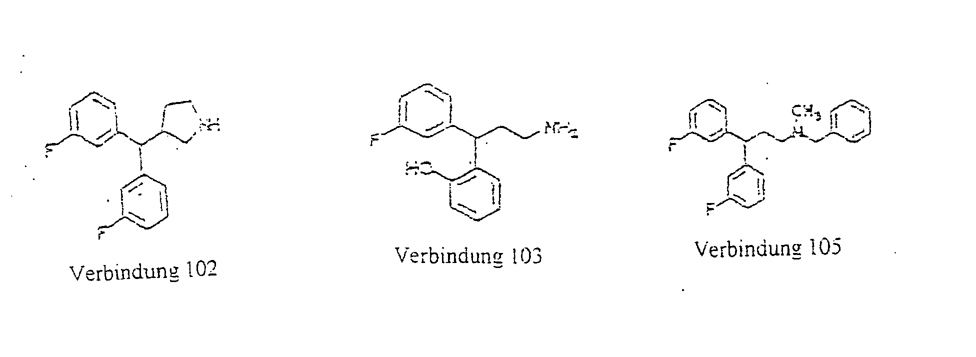 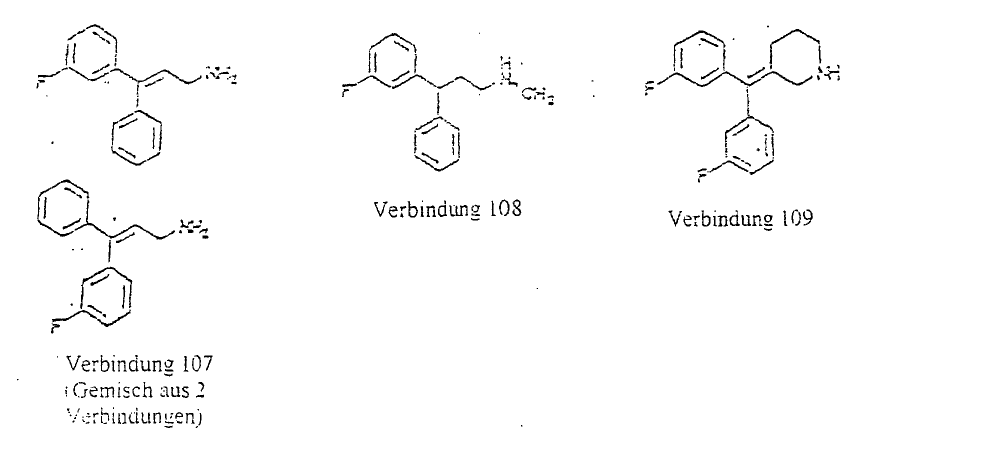 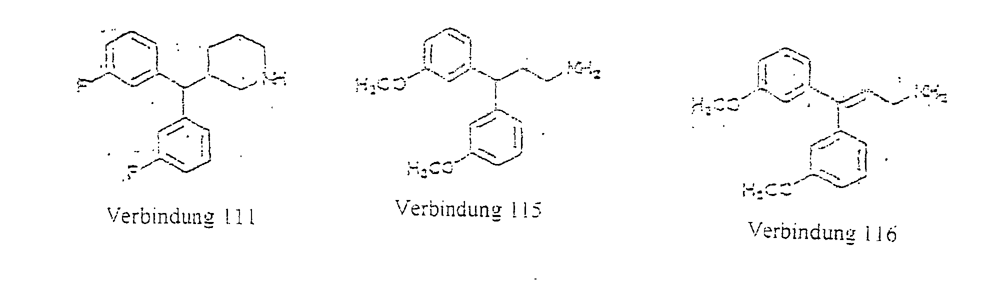 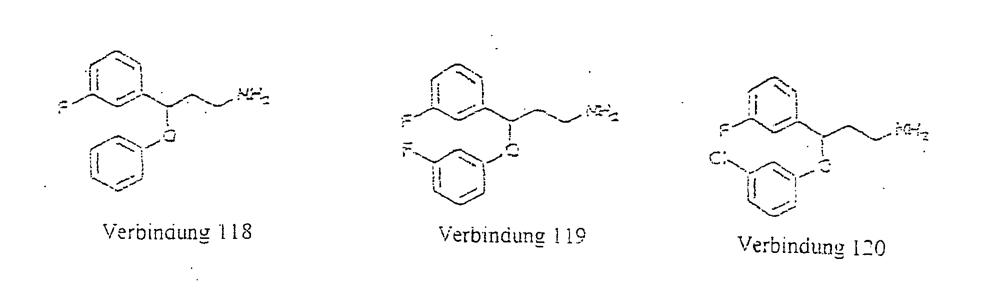 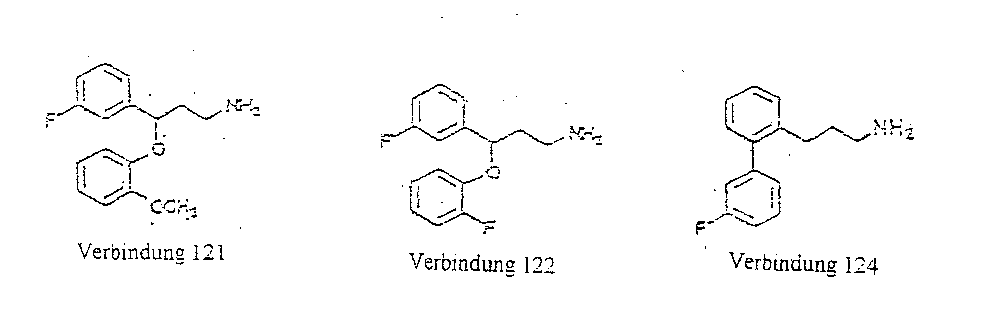 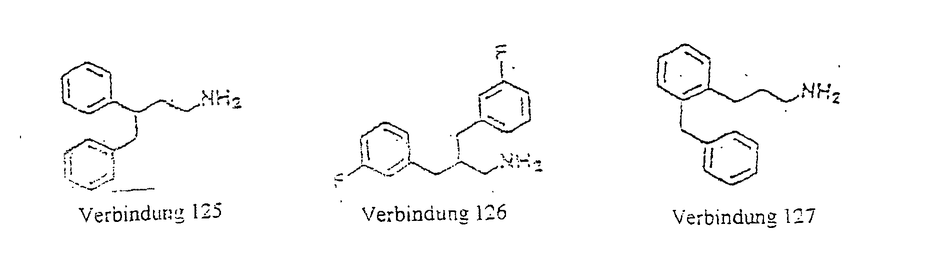 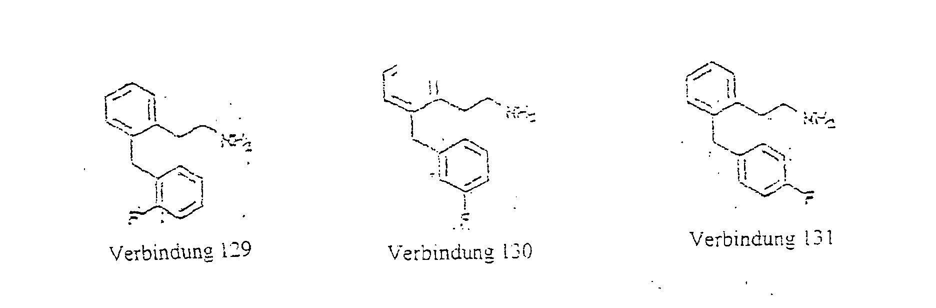 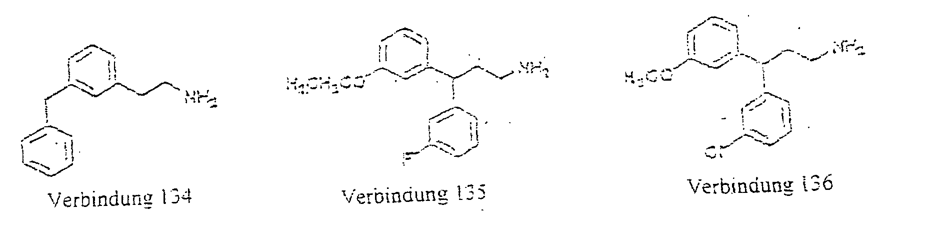 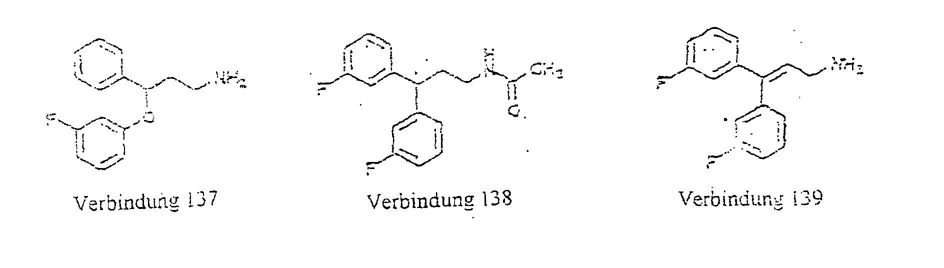 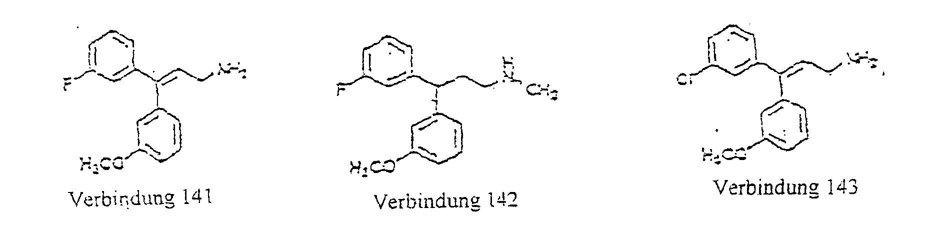 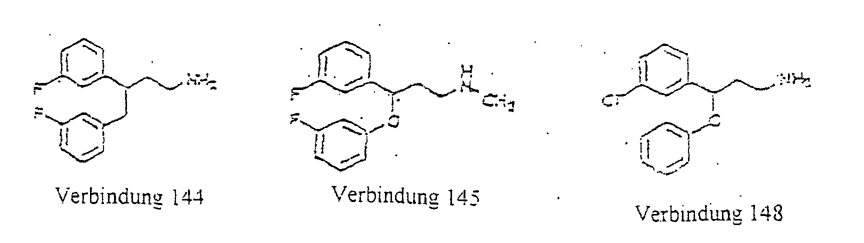 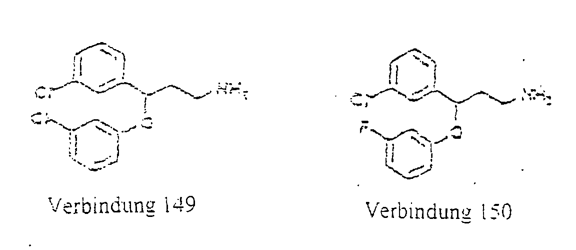 und pharmazeutisch verträglichen Salzen davon.

23. Verbindung gemäß Anspruch 22, die aus den Verbindungen 54-66, 69, 76, 82, 83, 88-90, 92-96, 101, 102, 103, 105, 108, 109, 111, 115, 118-122, 125-127, 129-131,135-139, 142, 144, 145, 148-150 oder pharmazeutisch verträglichen Salzen davon ausgewählt ist.

24. Verbindung gemäß Anspruch 22, die aus den Verbindungen 54-66, 69, 82, 83, 89, 90, 93-96, 103, 111, 118-120, 122, 126, 135-138, 142, 144, 145, 148-150 oder. pharmazeutisch verträglichen Salzen davon ausgewählt ist.

25. Verbindung gemäß Anspruch 22, die aus den Verbindungen 60, 66, 69, 103, 111, 118-120, 122, 136-138, 142, 144, 145, 148-150 oder pharmazeutisch verträglichen Salzen davon ausgewählt ist.

26. Verbindung gemäß Anspruch 22, die aus den Verbindungen 118-122, 137, 145, 148-150 oder pharmazeutisch verträglichen Salzen davon ausgewählt ist.

27. Verbindung gemäß Anspruch 22, die aus den Verbindungen 118-122, 148-150 oder pharmazeutisch verträglichen Salzen davon ausgewählt ist.

28. Verbindung gemäß Anspruch 22, die aus den Verbindungen 63 und 64 oder pharmazeutisch verträglichen Salzen davon ausgewählt ist.

29. Verbindung gemäß Anspruch 22, die aus der Verbindung 119 oder pharmazeutisch verträglichen Salzen davon ausgewählt ist.

30. Verbindung gemäß Anspruch 22, die aus der Verbindung 144 oder pharmazeutisch verträglichen Salzen davon ausgewählt ist.

31. Verbindung 60 oder pharmazeutisch verträgliche Salze davon.

32. Verbindung der Formel: 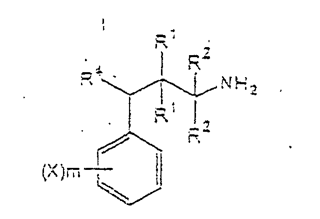 wobei:
X unabhängig aus -Br, -Cl, -F, -I, -CF₃, einem Alkylrest, -OH, -OCF₃, einem -O-Alkyl- und -O-Acylrest ausgewählt ist;
R₁ unabhängig aus -H, einem C₁₋₄-Alkyl- und -O-Acylrest ausgewählt ist;
R₂ unabhängig aus -H, einem Alkyl- und Hydroxyalkylrest ausgewählt ist oder beide Reste R₂ zusammen eine Iminogruppe sind;
R₄ ein Phenoxyrest ist, der gegebenenfalls mit -F,₋-Cl, -Br, -I, -CF₃, Alkyl,-OH, -OCF₃, -O-Alkyl oder -O-Acyl substituiert ist; und
m unabhängig eine ganze Zahl von 1 bis 5 ist; und pharmazeutisch verträgliche Salze und Komplexe davon.

33. Verbindung der Formel: 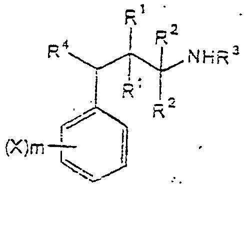 wobei:
X unabhängig aus -F, -Cl, -Br, -I, -CF₃, einem Alkylrest, -OH, -OCF₃, einem-O-Alkyl- und -O-Acylrest ausgewählt ist;
R₁ unabhängig aus -H, einem C₁₋₄-Alkyl- und -O-Acylrest ausgewählt ist;
R₂ unabhängig aus -H, einem C₁₋₄-Alkyl- und Hydroxyalkylrest ausgewählt ist oder beide Reste R₂ zusammen eine Iminogruppe sind;
R₃ aus einer Methyl- und Ethylgruppe ausgewählt ist;
R₄ ein Phenoxyrest ist, der gegebenenfalls mit -F, -Cl, -Br, -I, -CF₃, Alkyl,-OH, -OCF₃, -O-Alkyl oder -O-Acyl substituiert ist; und
m unabhängig eine ganze Zahl von 1 bis 5 ist; und pharmazeutisch verträgliche Salze und Komplexe davon mit der Maßgabe, dass die Verbindung nicht N-Methyl-3-(m-trifluormethylphenoxy)-3-(4-fluorphenyl)propylamin ist.

34. Verbindung der Formel: 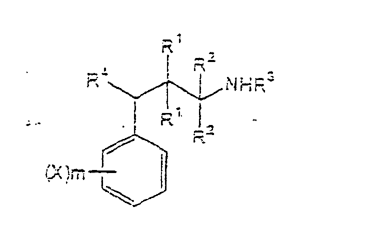 wobei:
(X)m aus einem meta-Fluor-, meta-Chloratom, ortho-O-C₁₋₄-Alkylrest, einer ortho-Methylgruppe, einem ortho-Fluor-, ortho-Chloratom, meta-O-C₁₋₄-Alkylrest, einer meta-Methylgruppe, ortho-OH und meta-OH ausgewählt ist;
R₁ H ist;
R₂ H ist;
R₃ aus einer Methyl- und Ethylgruppe ausgewählt ist;
R₄ ein Phenoxyrest ist, der gegebenenfalls mit -F, -Cl, -Br, -I, -CF₃, einem Alkyl, -OH. -OCF₃, -O-Alkyl oder -O-Acyl substituiert ist; und
pharmazeutisch verträgliche Salze und Komplexe davon.

35. Arzneimittel, umfassend eine Verbindung, welche aus 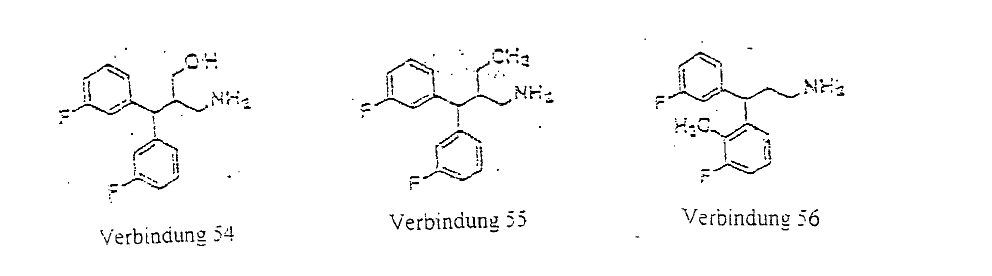 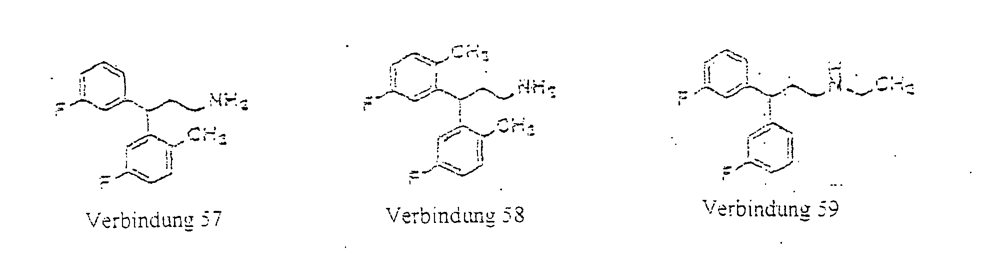 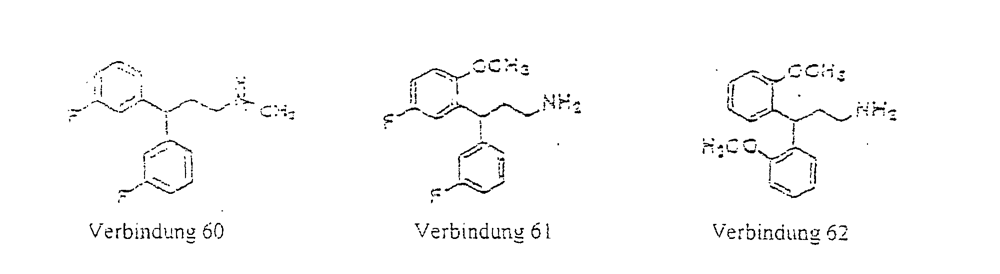 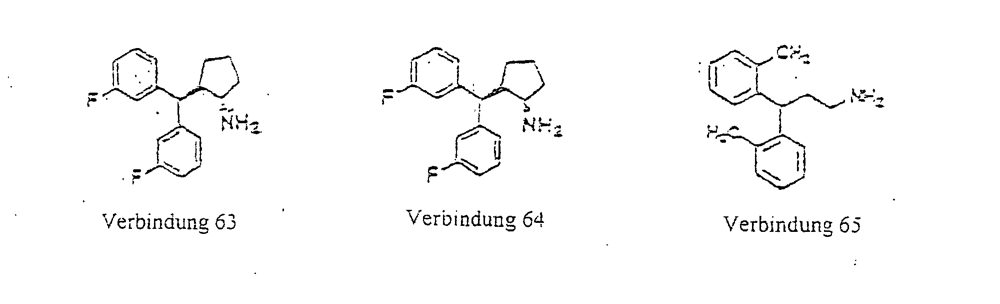 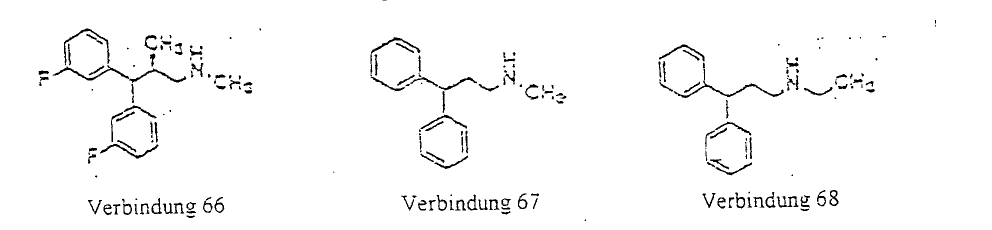 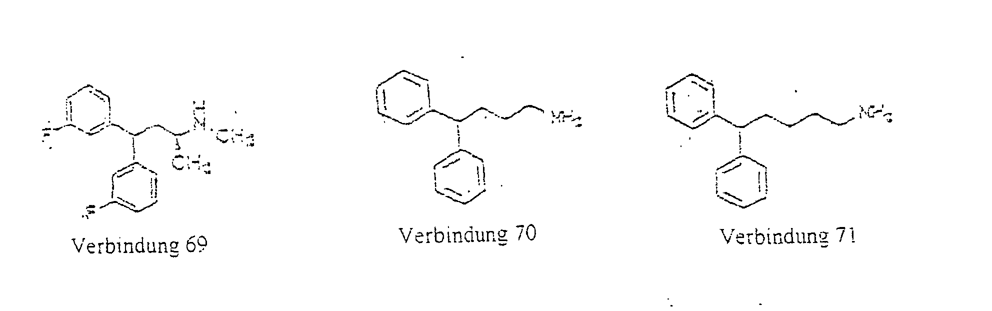 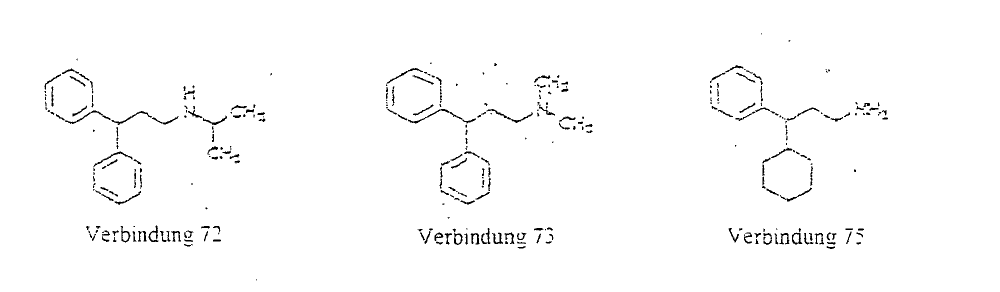 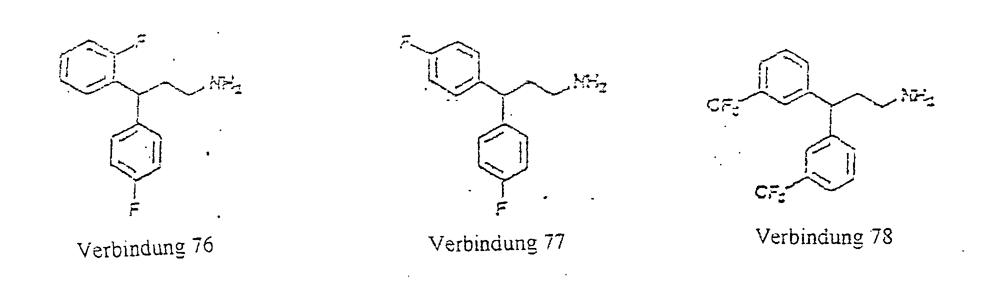 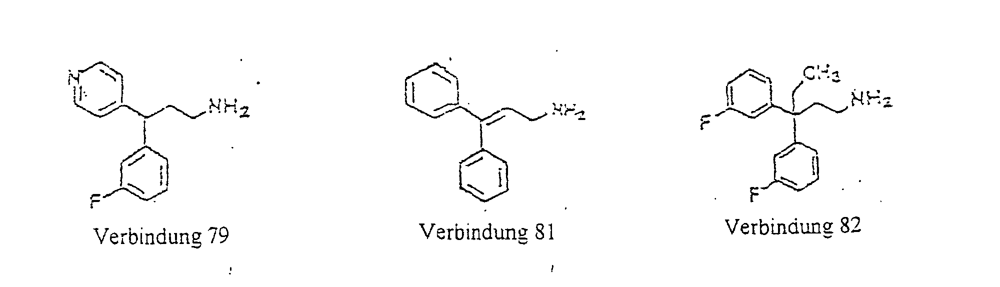 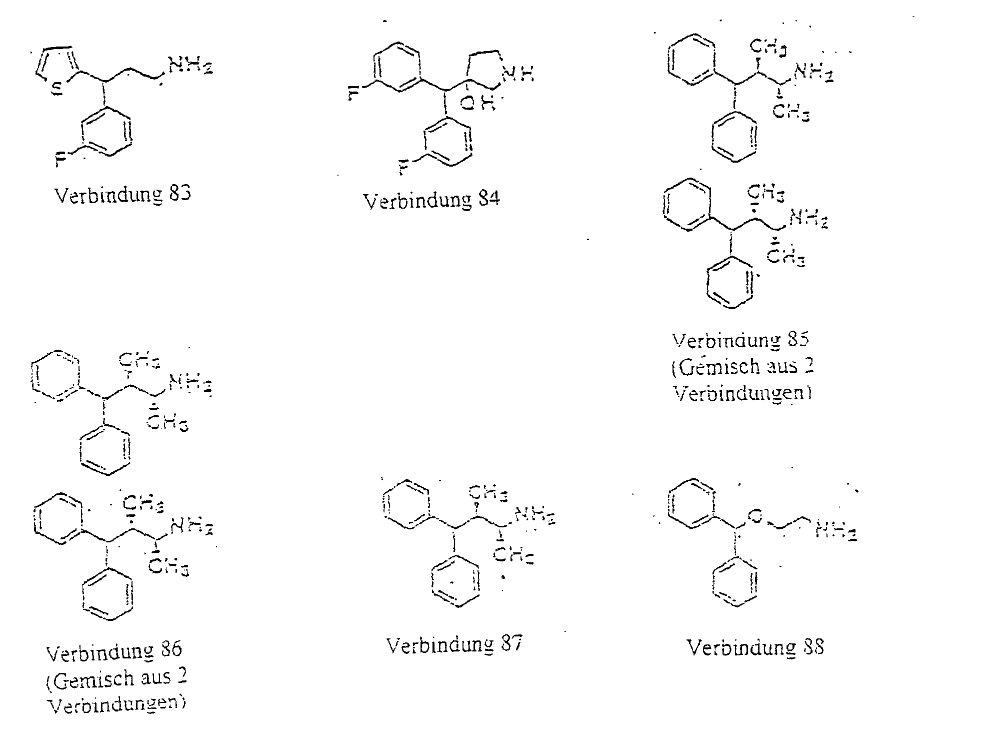 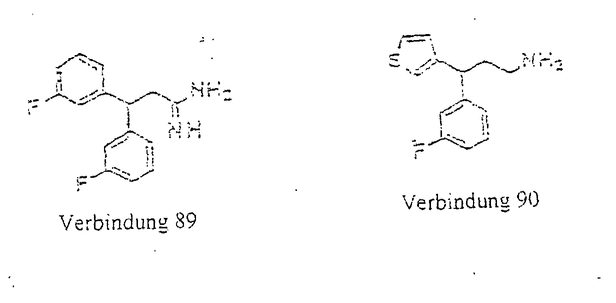 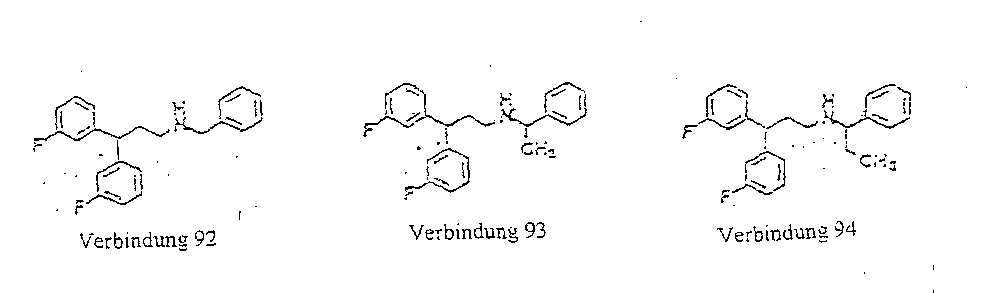 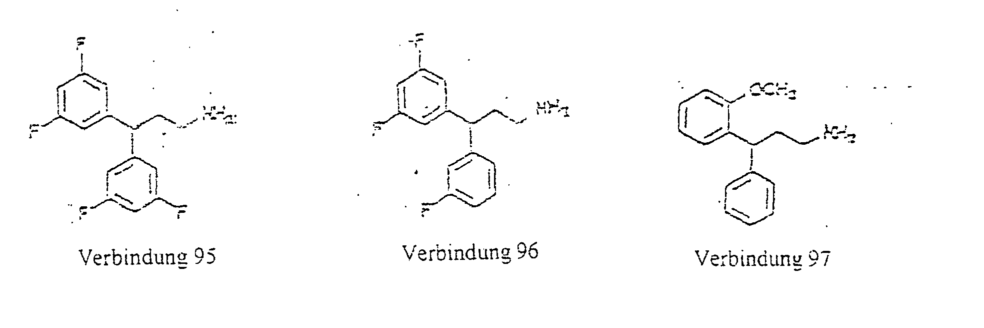 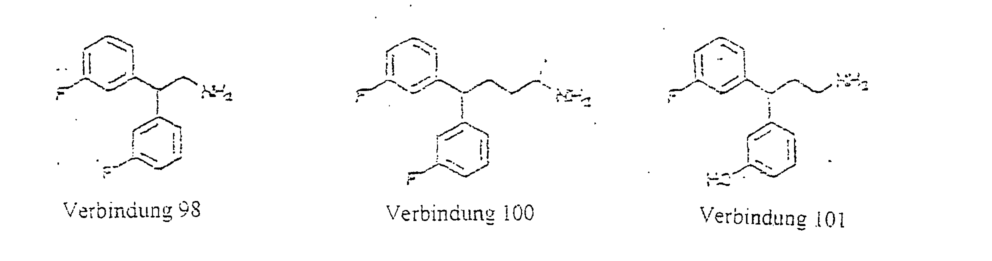 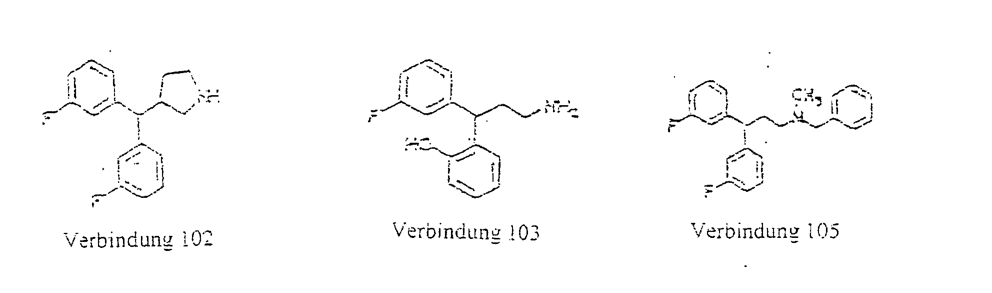 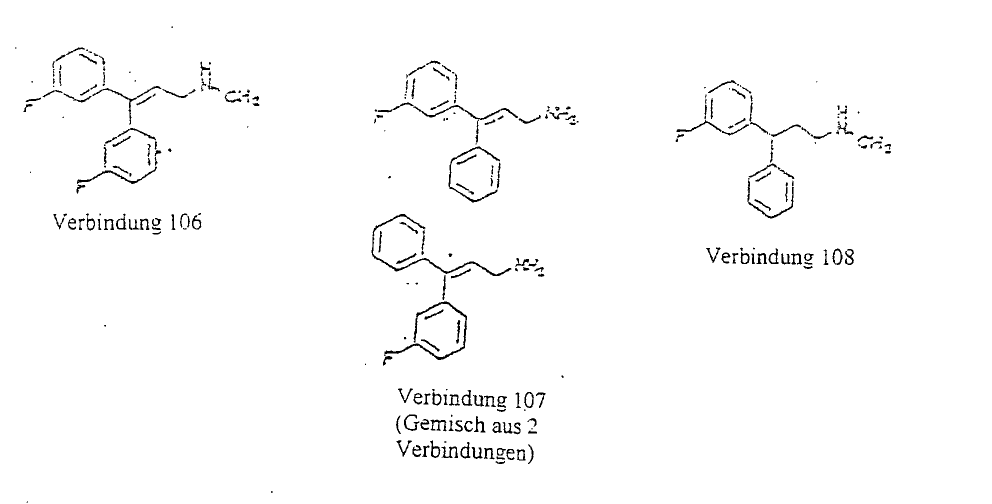 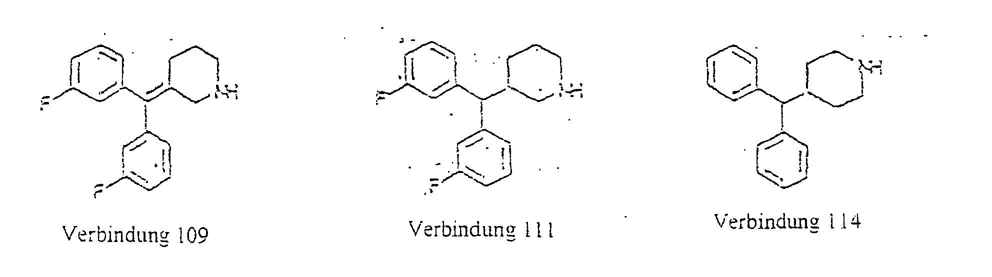 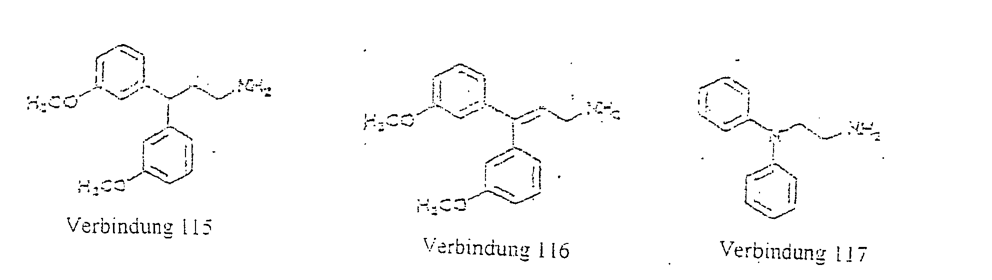 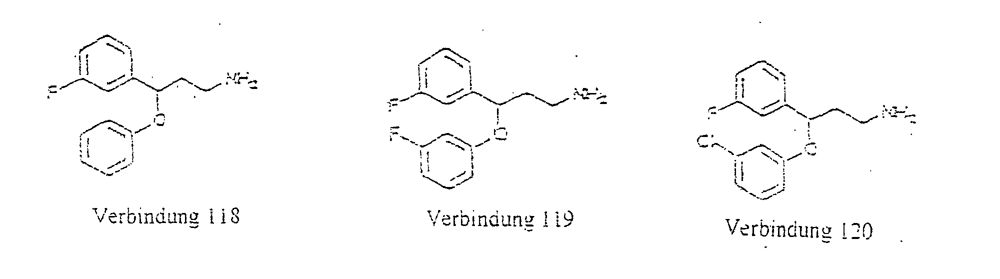 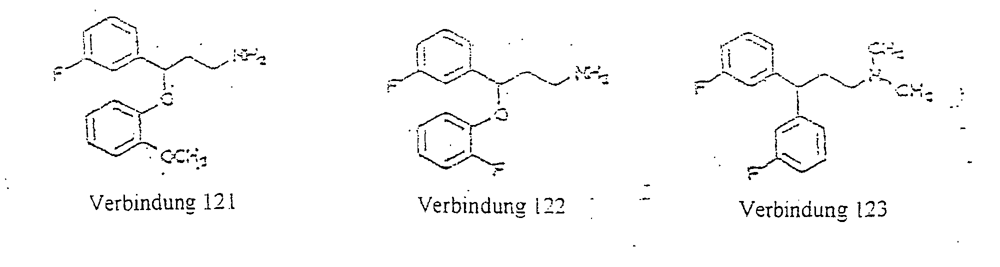 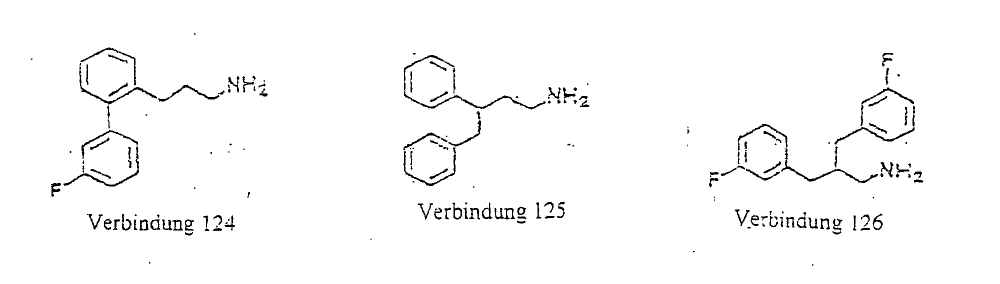 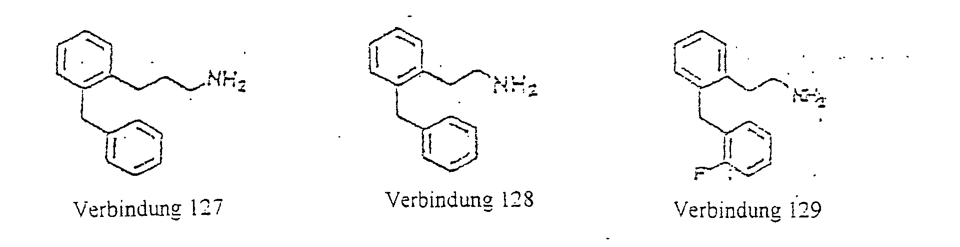 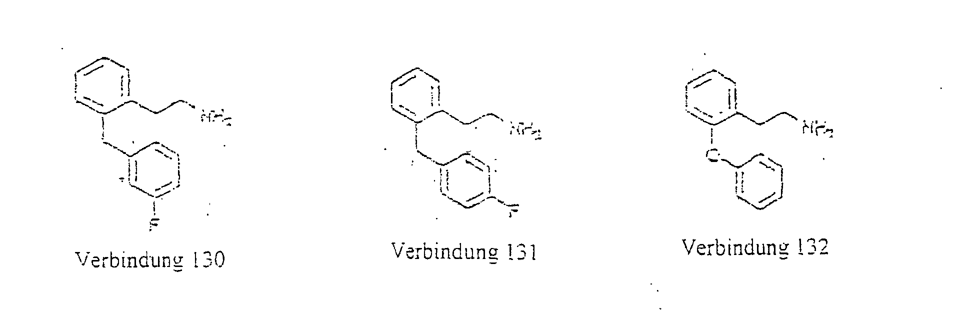 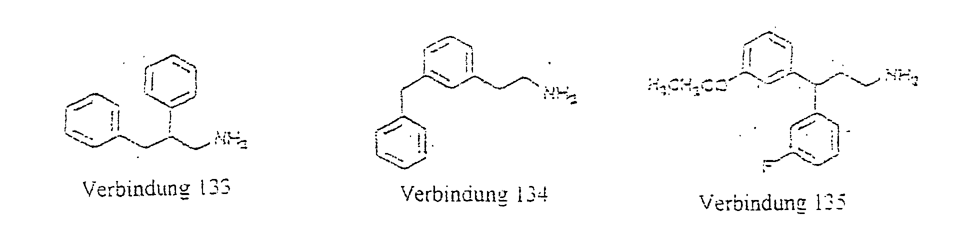 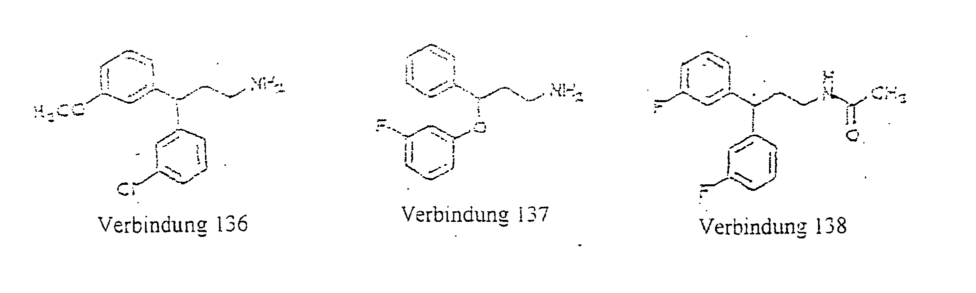 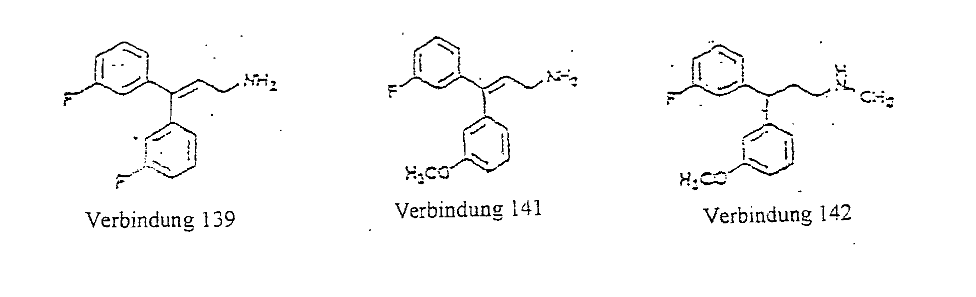 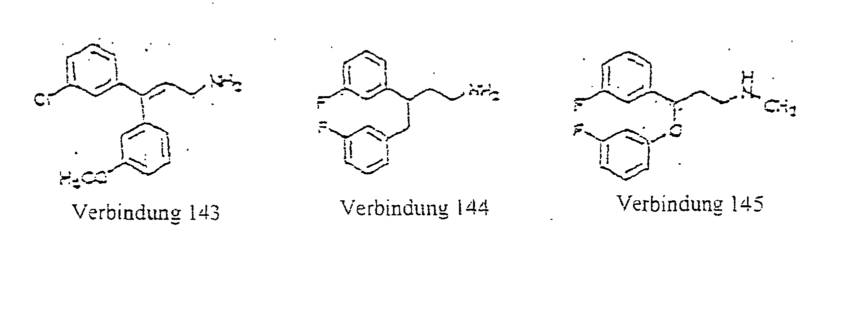 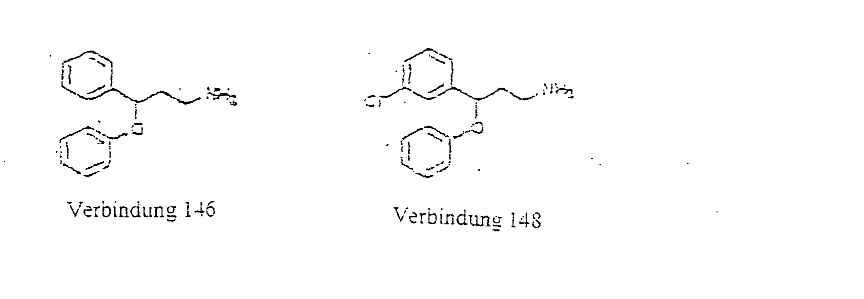 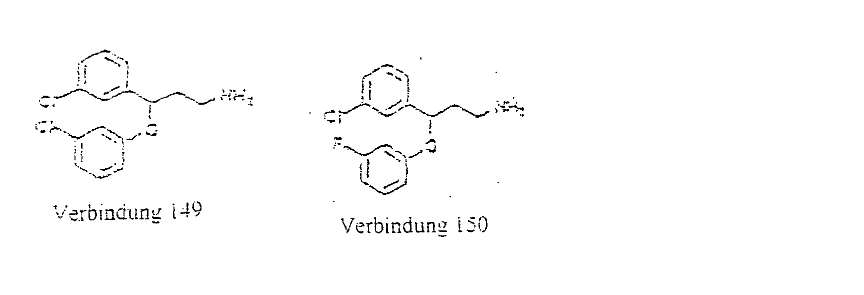 und pharmazeutisch verträglichen Salzen davon ausgewählt ist in einem pharmazeutisch verträglichen Träger.

36. Arzneimittel gemäß Anspruch 35, umfassend eine Verbindung, die aus den Verbindungen 54-71, 73, 76-79, 81-84, 88-90, 92-98, 101-103, 105, 107-109, 111, 115, 117-123, 125-127, 129-136, 138, 139, 142, 144-146, 148-150 und pharmazeutisch verträglichen Salzen davon ausgewählt ist, in einem pharmazeutisch verträglichen Träger.

37. Arzneimittel gemäß Anspruch 35, umfassend eine Verbindung, die aus den Verbindungen 54-66, 69, 70, 75, 76, 81-83, 85-90, 92-97, 100-103, 105, 106, 108, 109, 111, 115, 118-122, 125-133, 135-139, 142, 144-146, 148-150 und pharmazeutisch verträglichen Salzen davon ausgewählt ist, in einem pharmazeutisch verträglichen Träger.

38. Arzneimittel gemäß Anspruch 35, umfassend eine Verbindung, die aus den Verbindungen 54-66, 69, 70, 76, 81-83, 88-90, 92-97, 101-103, 105, 106, 108, 109, 111, 115, 118-122, 125-127, 129-133, 135, 136, 138, 139, 142, 144-146, 148-150 und pharmazeutisch verträglichen Salzen davon ausgewählt ist, in einem pharmazeutisch verträglichen Träger.

39. Arzneimittel gemäß Anspruch 35, umfassend eine Verbindung, die aus den Verbindungen 54-66, 69, 82, 83, 89, 90, 93-97, 103, 111, 118-120, 122, 126, 135-138, 142, 144, 145, 148-150 und pharmazeutisch verträglichen Salzen davon ausgewählt ist, in einem pharmazeutisch verträglichen Träger.

40. Arzneimittel gemäß Anspruch 35, umfassend eine Verbindung, die aus den Verbindungen 54-66, 69, 82, 83, 89, 90, 93-97, 103, 111, 118-120, 122, 126, 135, 136, 138, 142, 144, 145, 148-150 und pharmazeutisch verträglichen Salzen davon ausgewählt ist, in einem pharmazeutisch verträglichen Träger.

41. Arzneimittel gemäß Anspruch 35, umfassend eine Verbindung, die aus den Verbindungen 60, 66, 69, 103, 111, 118-120, 122, 136-138, 142, 144, 145, 148-150 und pharmazeutisch verträglichen Salzen davon ausgewählt ist, in einem pharmazeutisch verträglichen Träger.

42. Arzneimittel gemäß Anspruch 35, umfassend eine Verbindung, die aus den Verbindungen 118-122, 137, 145, 148-150 und pharmazeutisch verträglichen Salzen davon ausgewählt ist, in einem pharmazeutisch verträglichen Träger.

43. Arzneimittel gemäß Anspruch 35, umfassend eine Verbindung, die aus den Verbindungen 118-122, 148-150 und pharmazeutisch verträglichen Salzen davon ausgewählt ist, in einem pharmazeutisch verträglichen Träger.

44. Arzneimittel gemäß Anspruch 35, umfassend eine Verbindung, die aus den Verbindungen 63 und 64 und pharmazeutisch verträglichen Salzen davon ausgewählt ist, in einem pharmazeutisch verträglichen Träger.

45. Arzneimittel gemäß Anspruch 35, umfassend eine Verbindung, die aus der Verbindung 119 und pharmazeutisch verträglichen Salzen davon ausgewählt ist, in einem pharmazeutisch verträglichen Träger.

46. Arzneimittel gemäß Anspruch 35, umfassend eine Verbindung, die aus der Verbindung 144 und pharmazeutisch verträglichen Salzen davon ausgewählt ist, in einem pharmazeutisch verträglichen Träger.

47. Arzneimittel umfassend eine Verbindung, die aus der Verbindung 60 und pharmazeutisch verträglichen Salzen davon ausgewählt ist, in einem pharmazeutisch verträglichen Träger.

48. Arzneimittel umfassend eine Verbindung gemäß Anspruch 32 in einem pharmazeutisch verträglichen Träger.

49. Arzneimittel umfassend eine Verbindung gemäß Anspruch 33 in einem pharmazeutisch verträglichen Träger.

50. Arzneimittel umfassend eine Verbindung gemäß Anspruch 34 in einem pharmazeutisch verträglichen Träger.

51. Arzneimittel gemäß einem der Ansprüche 35-50, das an die Behandlung von neurologischen Krankheiten und Störungen angepasst ist.

52. Arzneimittel gemäß Anspruch 51, wobei die neurologische Krankheit oder Störung aus Schlaganfall, Schädeltrauma, Rückenmarksverletzung, Epilepsie, Ängstlichkeit, Alzheimer-Krankheit, Chorea Huntington, Parkinson-Krankheit oder amyotrophische Lateralsklerose ausgewählt ist.

53. Arzneimittel gemäß Anspruch 51, wobei das Arzneimittel neuroprotektive Wirkung aufweist.

54. Arzneimittel gemäß Anspruch 52, wobei der Schlaganfall als Totalischämie auftritt.

55. Arzneimittel gemäß Anspruch 52, wobei der Schlaganfall als fokale Ischämie auftritt.

56. Arzneimittel gemäß Anspruch 52, wobei der Schlaganfall in hemorrhagischer Form auftritt.

57. Arzneimittel gemäß Anspruch 52, wobei die neurologische Krankheit oder Störung Parkinson-Krankheit ist.

## Revendications

1. Utilisation d'un composé qui est choisi dans le groupe consistant en : 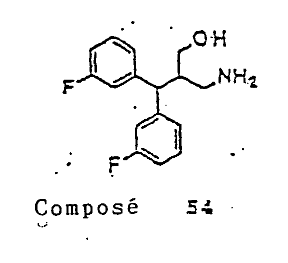 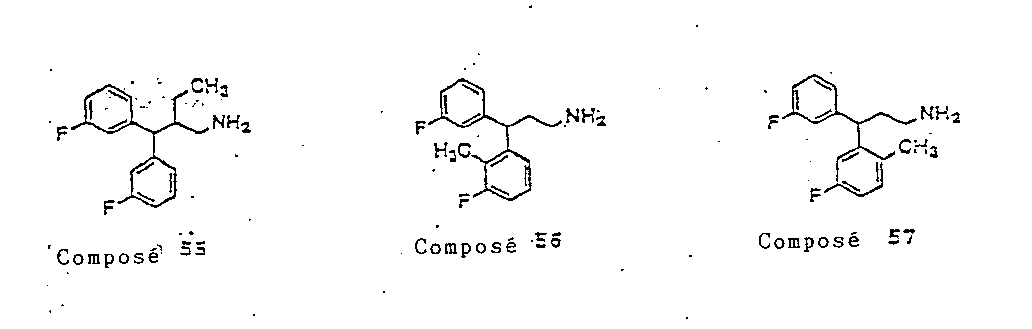 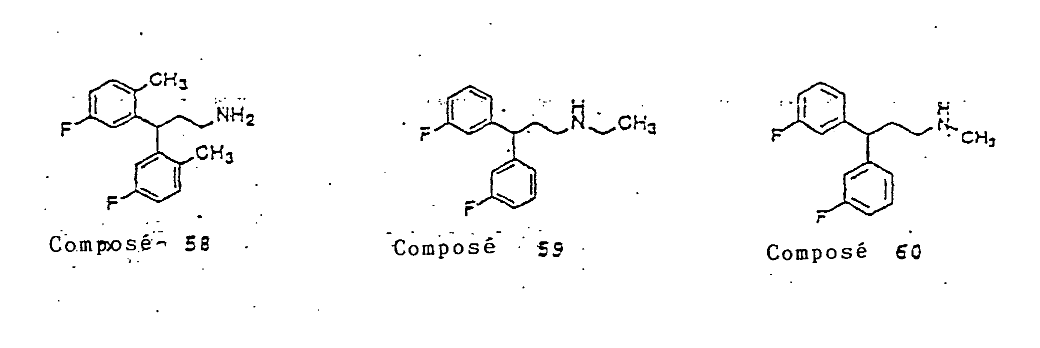 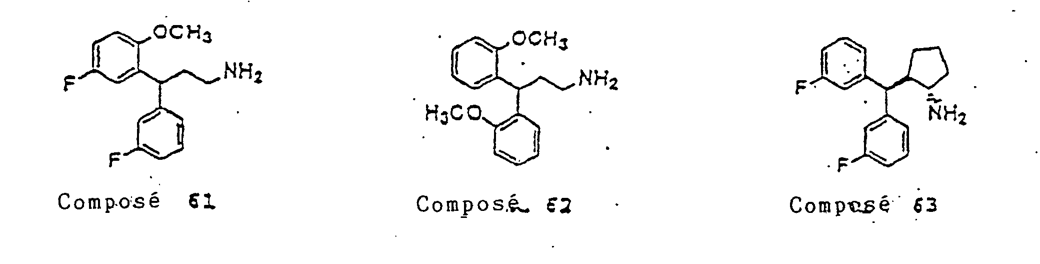 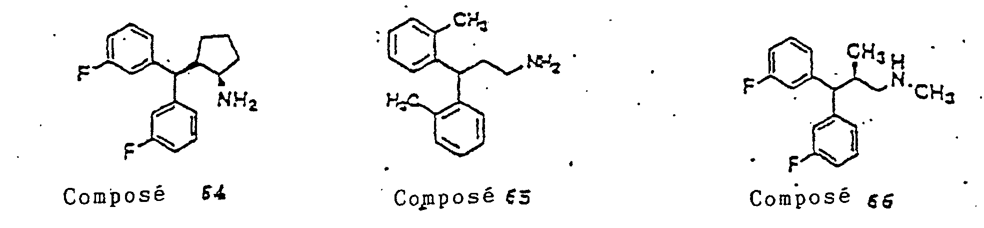 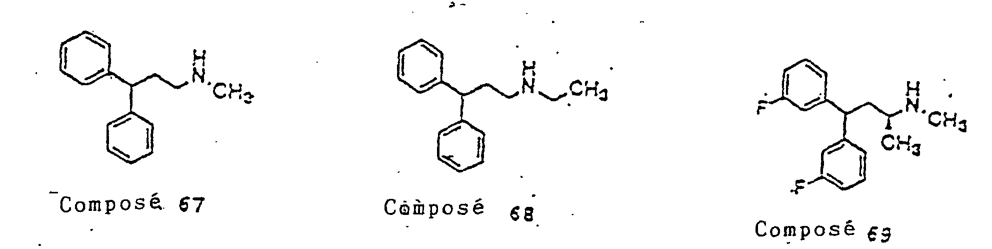 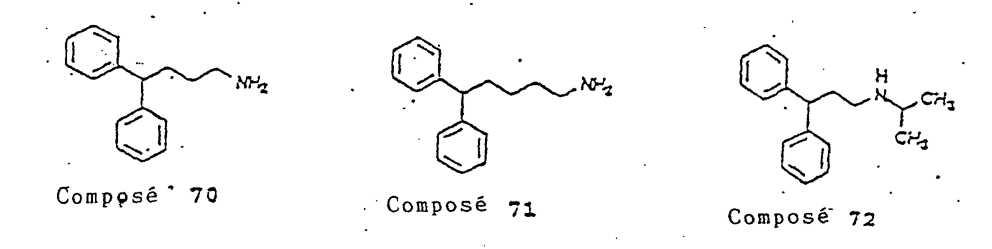 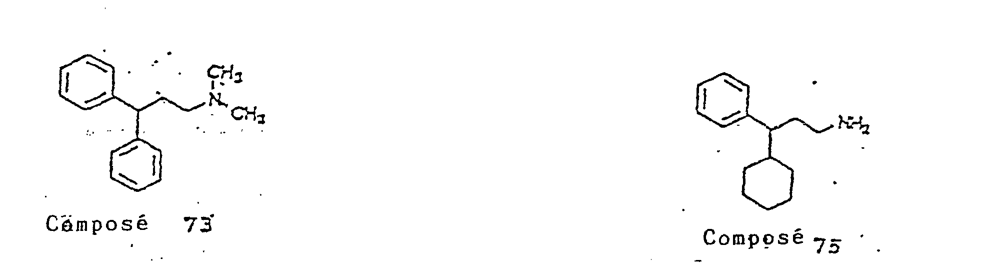 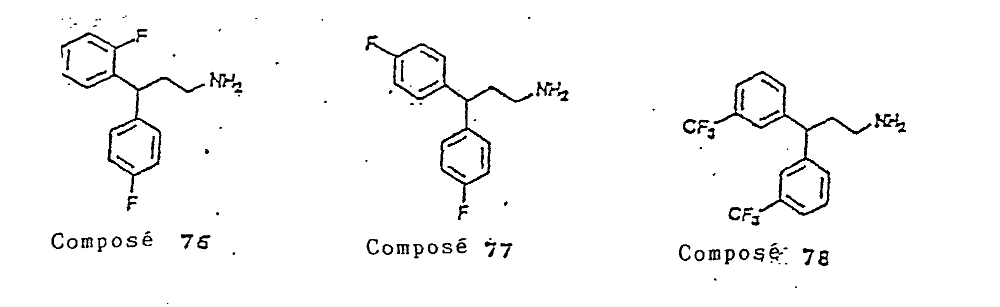 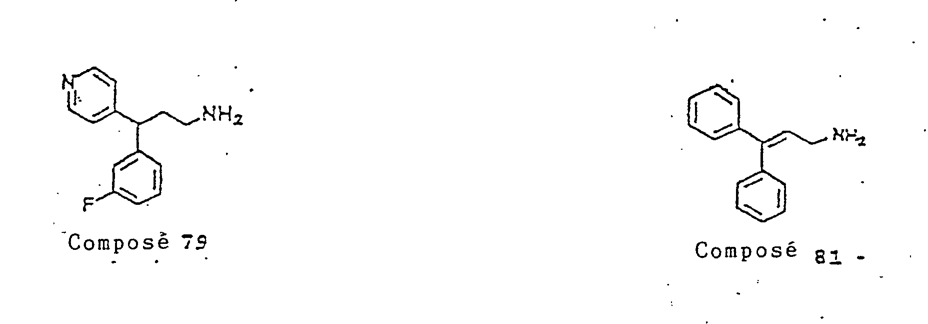 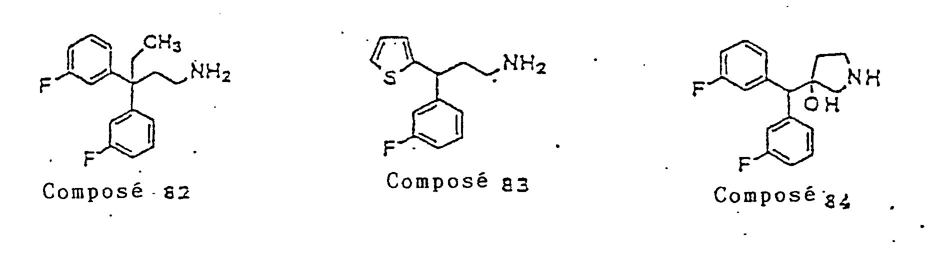 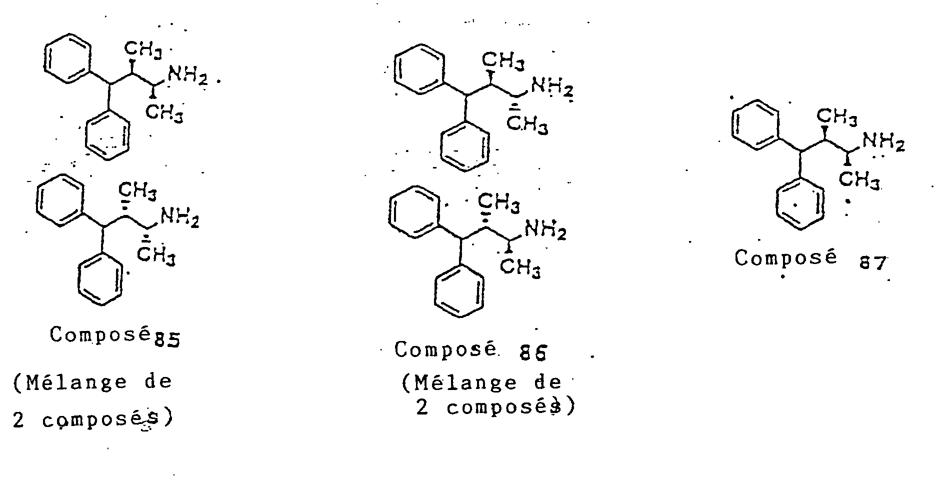 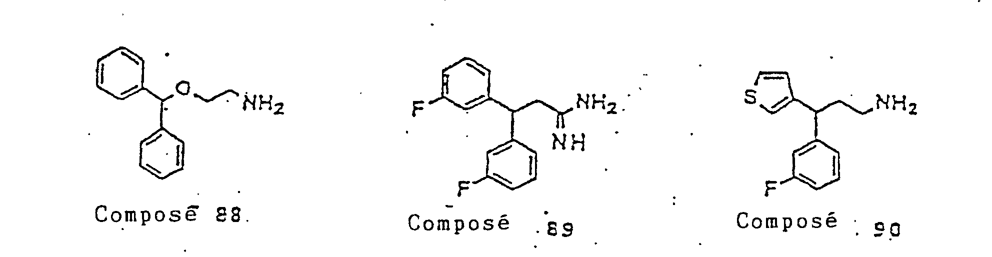 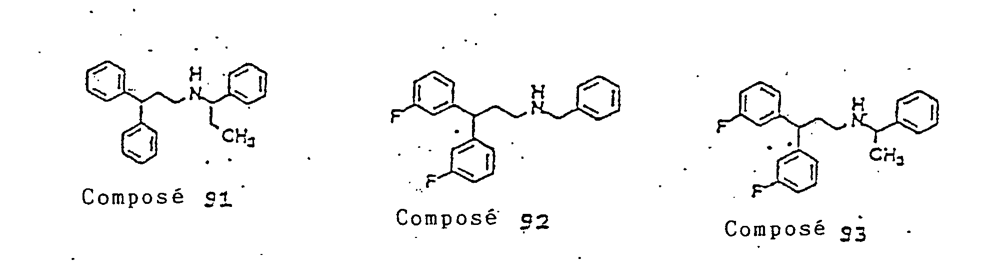 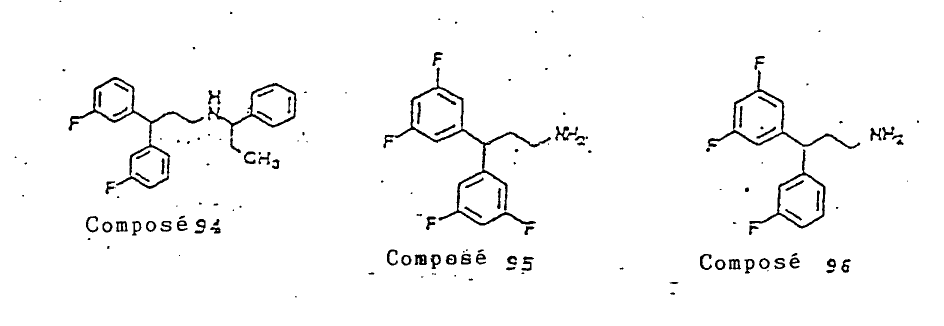 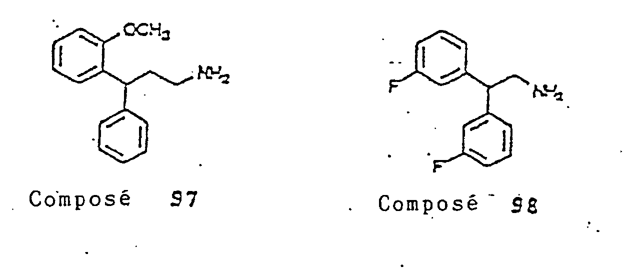 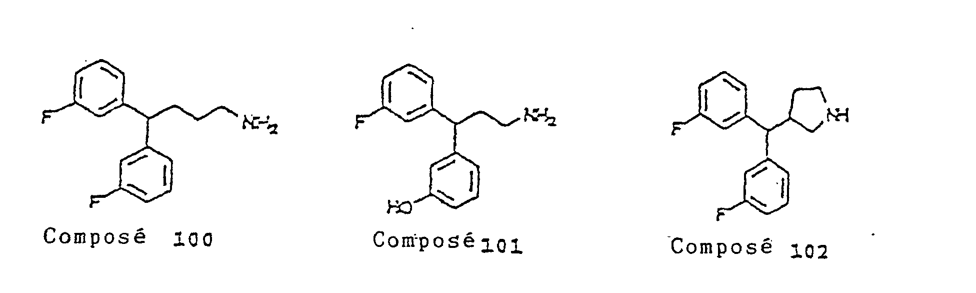 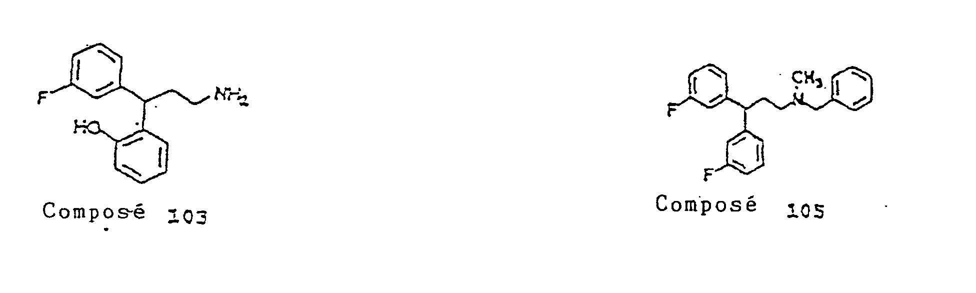 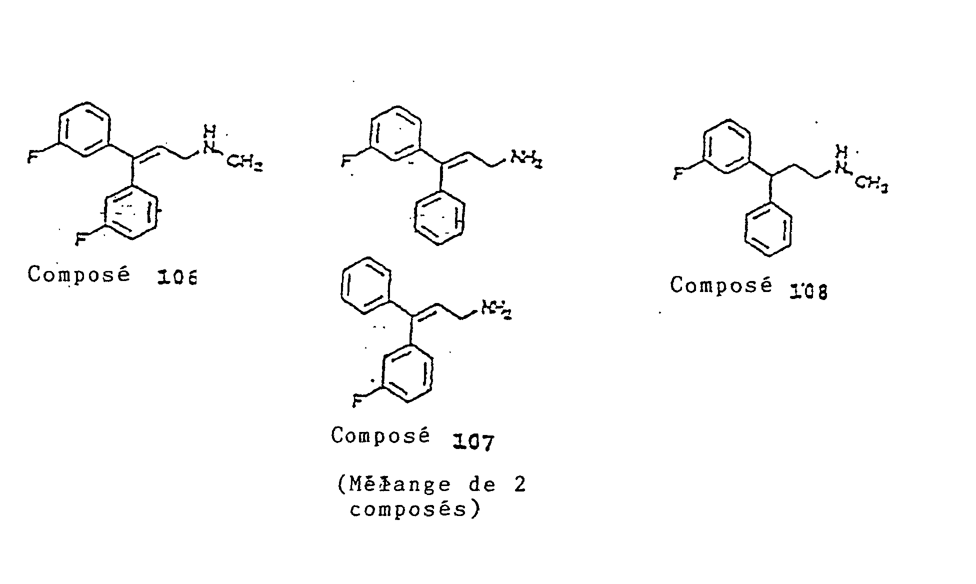 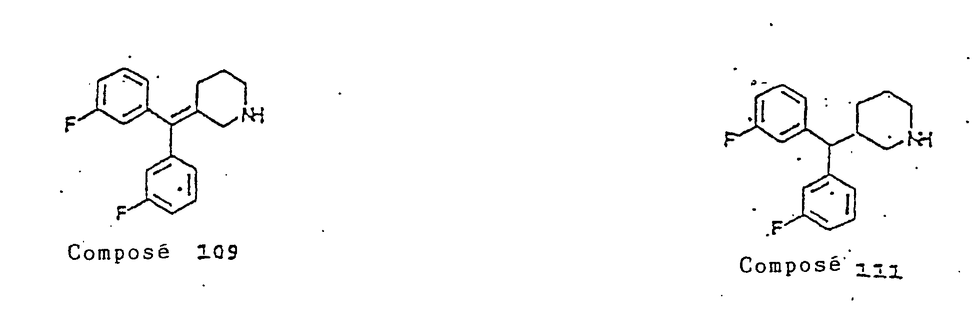 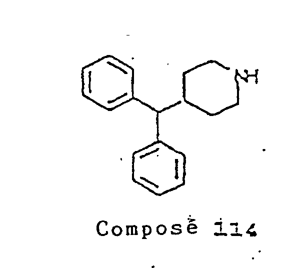 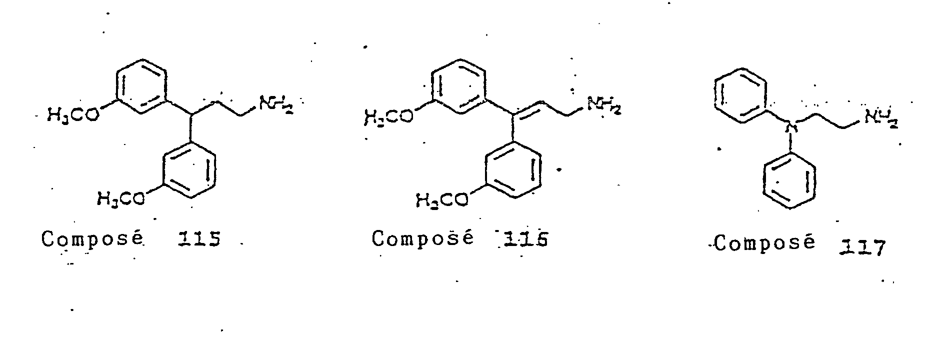 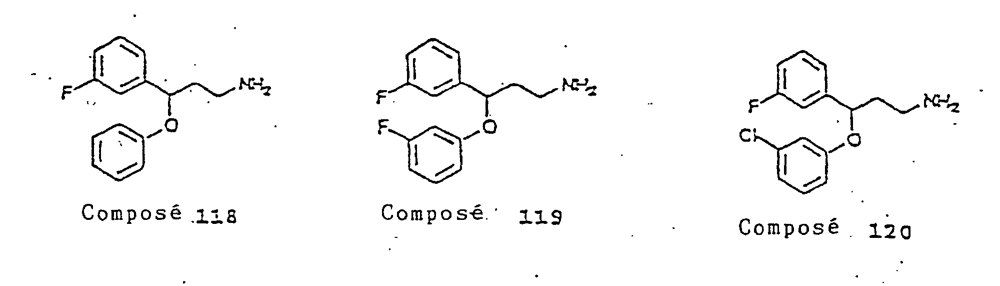 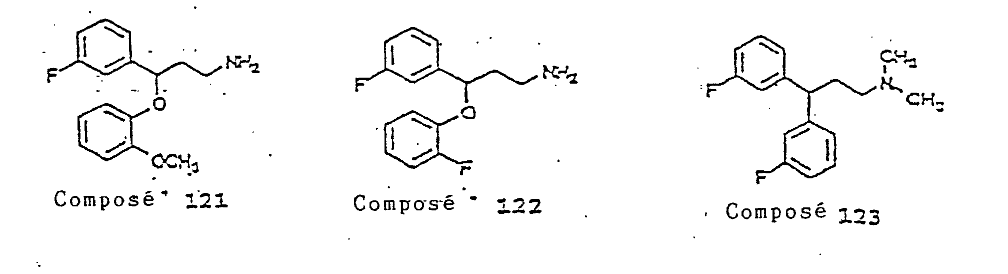 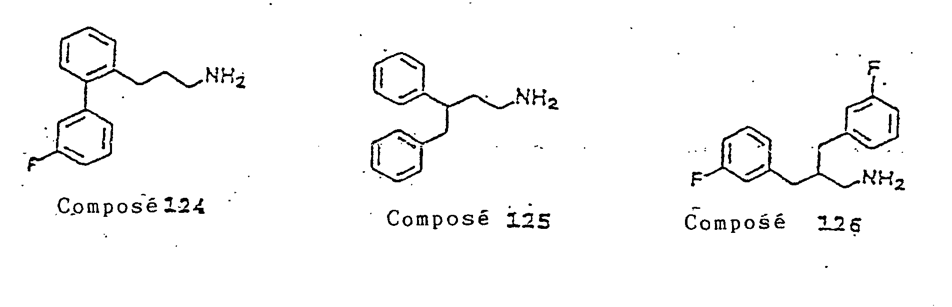 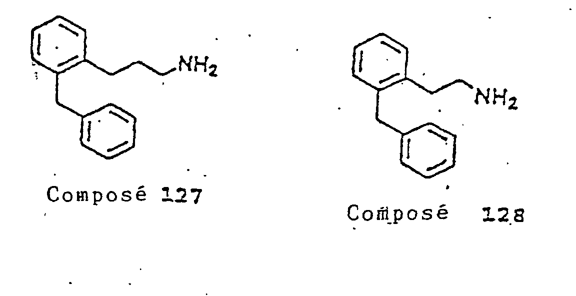 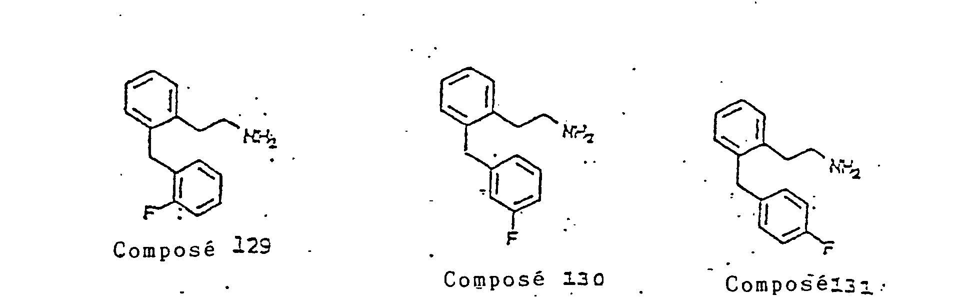 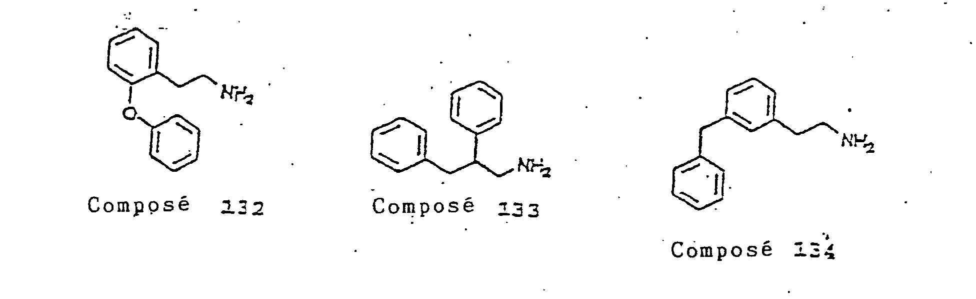 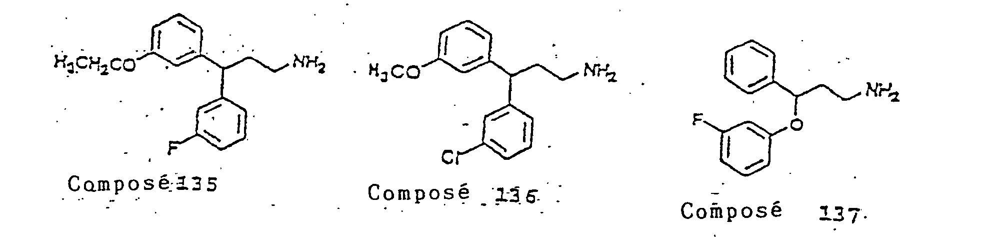 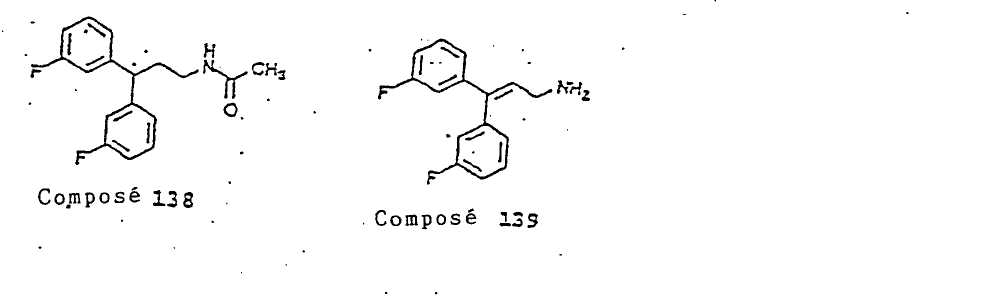 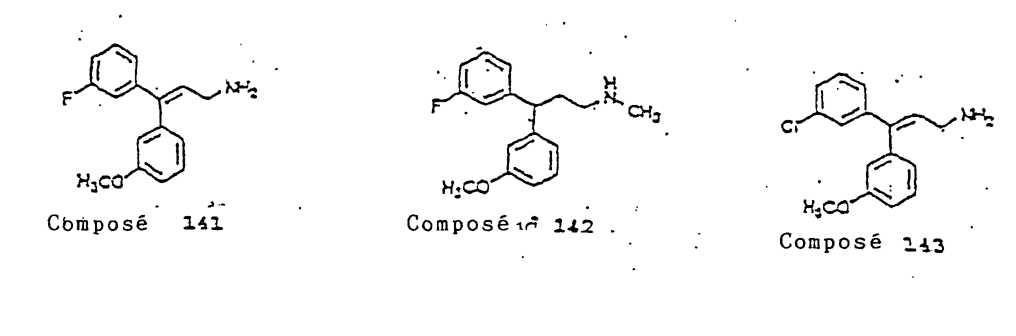 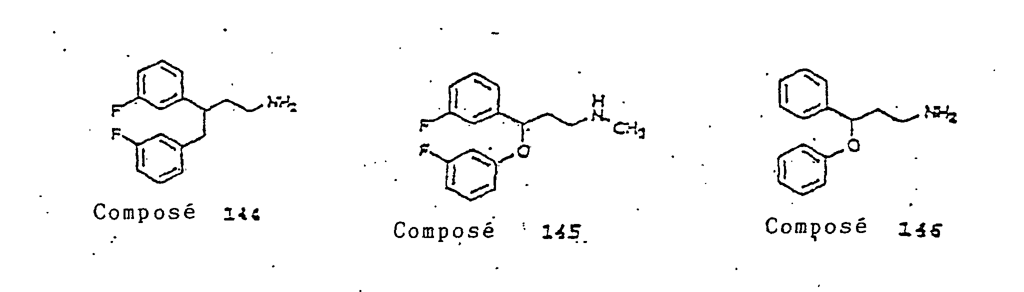 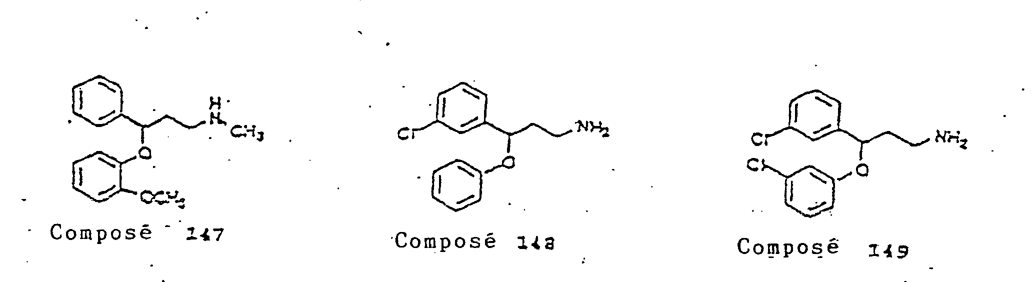 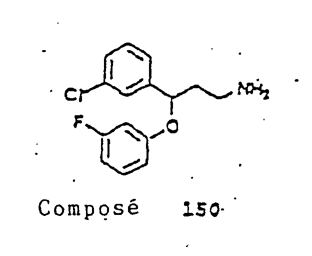 et leurs sels et complexes pharmaceutiquement acceptables pour la préparation d'une composition pharmaceutique pour le traitement d'une maladie ou d'un désordre neurologique.

2. Utilisation selon la revendication 1, dans laquelle le composé est choisi dans le groupe consistant en les composés 54-66, 68-71, 75, 76, 78, 79, 81-90, 92-98, 100, 101, 103, 105, 106, 108, 109, 111, 114-122, 124-136, 138, 139, 141-144, 148-150, et leurs sels et complexes pharmaceutiquement acceptables.

3. Utilisation selon la revendication 1, dans laquelle le composé est choisi dans le groupe consistant en les composés 54-66, 69, 70, 75, 76, 81-83, 85-97, 100-103, 105, 106, 108, 109, 111, 115, 118-122, 125-133, 135-139, 142, 144-150, et leurs sels et complexes pharmaceutiquement acceptables.

4. Utilisation selon la revendication 1, dans laquelle le composé est choisi dans le groupe consistant en les composés 54-66, 69, 70, 75, 76, 81-83, 85-90, 92-97, 100, 101, 103, 105, 106, 108, 109, 111, 115, 118-122, 125-133, 135, 136, 138, 139, 142, 144, 148-150, et leurs sels et complexes pharmaceutiquement acceptables.

5. Utilisation selon la revendication 1, dans laquelle le composé est choisi dans le groupe consistant en les composés 54-66, 69, 82, 83, 89-97, 103, 111, 118-120, 122, 126, 135-138, 142, 144, 145, 147-150, et leurs sels et complexes pharmaceutiquement acceptables.

6. Utilisation selon la revendication 1, dans laquelle le composé est choisi dans le groupe consistant en les composés 54-66, 69, 82, 83, 89-90, 92-97, 103, 111, 118-120, 122, 126, 135, 136, 138, 142, 144, 148-150, et leurs sels et complexes pharmaceutiquement acceptables.

7. Utilisation selon la revendication 1, dans laquelle le composé est choisi dans le groupe consistant en les composés 60, 66, 69, 103, 111, 118-120, 122, 136, 138, 142, 144, 148-150, et leurs sels et complexes pharmaceutiquement acceptables.

8. Utilisation selon la revendication 1, dans laquelle le composé est choisi dans le groupe consistant en les composés 118-122, 137, 145, 148-150, et leurs sels et complexes pharmaceutiquement acceptables.

9. Utilisation selon la revendication 1, dans laquelle le composé est choisi dans le groupe consistant en les composés 118-122, 148-150, et leurs sels et complexes pharmaceutiquement acceptables.

10. Utilisation selon la revendication 1, dans laquelle le composé est choisi dans le groupe consistant en les composés 63 et 64 et leurs sels et complexes pharmaceutiquement acceptables.

11. Utilisation selon la revendication 1, dans laquelle le composé est choisi parmi le composé 119 et ses sels et complexes pharmaceutiquement acceptables.

12. Utilisation selon la revendication 1, dans laquelle le composé est choisi parmi le composé 144 et ses sels et complexes pharmaceutiquement acceptables.

13. Utilisation du composé 60 et de ses sels et complexes pharmaceutiquement acceptables pour la préparation d'une composition pharmaceutique pour le traitement d'une maladie ou d'un désordre neurologique.

14. Utilisation d'un composé de formule : 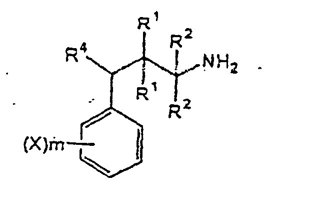 dans laquelle :
X est choisi indépendamment dans le groupe consistant en -Br, -Cl, -F, -I, -CF₃, alkyle, -OH, -OCF₃, -O-alkyle et -O-acyle ;
R₁ est choisi indépendamment dans le groupe consistant en -H, C₁-C₄ alkyle, et -Oacyle ;
R₂ est choisi indépendamment dans le groupe consistant en -H, alkyle et hydroxyalkyle, ou les deux R₂ ensemble sont imino ;
R₄ est phénoxy éventuellement substitué par -F, -Cl, -Br, -I, -CF₃, alkyle, -OH,-O-CF₃, -O-alkyle et -O-acyle ; et
m est indépendamment un nombre entier de 0 à 5 ; et ses sels et complexes pharmaceutiquement acceptables sous réserve que le dit composé n'est pas :
3-(p-isopropoxyphénoxy)-3-phénylpropylamine
3-(2'-methyl-4',5'-dichlorophénoxy)-3-phénylpropylamine
3-(p-tert-butylphénoxy)-3-phénylpropylamine
3-(2',4'-dichlorophénoxy)-3-phényl-2-méthylpropylamine
3-(o-éthylphénoxy)-3-phénylpropylamine
3-(o-méthoxyphénoxy)-3-phénylpropylamine
3-phénoxy-3-phénylpropylamine
pour la préparation d'une composition pharmaceutique pour le traitement d'une maladie ou d'un désordre neurologique.

15. Utilisation d'un composé de formule : 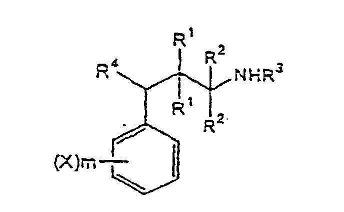 dans laquelle :
X est choisi indépendamment dans le groupe consistant en -F, -Cl, -Br, -I, -CF₃, alkyle, -OH, -OCF₃, -O-alkyle et -O-acyle ;
R₁ est choisi indépendamment dans le groupe consistant en -H, C₁-C₄ alkyle, et-O-acyle ;
R₂ est choisi indépendamment dans le groupe consistant en -H, C₁-C₄ alkyle et hydroxyalkyle, ou les deux R₂ ensemble sont imino ;
R₃ est choisi dans le groupe consistant en méthyle et éthyle ;
R₄ est phénoxy éventuellement substitué par -F, -Cl, -Br, -I, -CF₃, alkyle, -OH,-OCF₃, -O-alkyle et -O-acyle ; et
m est indépendamment un nombre entier de 0 à 5 ; et leurs sels et complexes pharmaceutiquement acceptables sous réserve que le dit composé n'est pas :
N-méthyl 3-(o-chloro-p-tolyloxy)-3-phényl-1-méthylpropylamine
N-méthyl 3-(p-tolyloxy)-3-phénylpropylamine
N-méthyl 3-(o-chloro-p-isopropylphénoxy)-3-phényl-2-méthylpropylamine
N-méthyl 3-(p-iodophénoxy)-3-phényl-propylamine
N-méthyl 3-(3-n propylphénoxy)-3-phénylpropylamine
N-méthyl 3-(p-trifluorométhylphénoxy)-3-phénylpropylamine
N-méthyl 3-(m-chlorophénoxy)-3-phénylpropylamine
N-méthyl 3-(p-fluorophénoxy)-3-phénylpropylamine
N-méthyl 3-(p-méthoxyphénoxy)-3-phénylpropylamine
N-méthyl 3-(o-méthoxyphénoxy)-3-phénylpropylamine
N-méthyl 3-(o-fluorophénoxy)-3-phénylpropylamine
N-méthyl 3-(o-tolyloxy)-3-phénylpropylamine
N-méthyl 3-(p-chlorophénoxy)-3-phénylpropylamine
N-méthyl 3-(m-fluorophénoxy)-3-phénylpropylamine
N-méthyl 3-phénoxy-3 -phényl-2-méthylpropylamine
N-méthyl 3-phénoxy-3-phényl-1-méthylpropylamine
N-méthyl 3-phénoxy-3-phénylpropylamine
N-méthyl 3-(o-trifluorométhylphénoxy)-3-phénylpropylamine
N-méthyl 3-(m-méthoxyphénoxy)-3-phénylpropylamine
N-méthyl 3-(o,p-difluorophénoxy)-3-phénylpropylamine
N-éthyl 3-(o-iodophénoxy)-3-phénylpropylamine
N-méthyl 3-(o-chlorophénoxy)-3-phénylpropylamine
N-méthyl 3-(o-bromophénoxy)-3-phénylpropylamine
pour la préparation d'une composition pharmaceutique pour le traitement d'une maladie ou d'un désordre neurologique.

16. Utilisation d'un composé de formule : 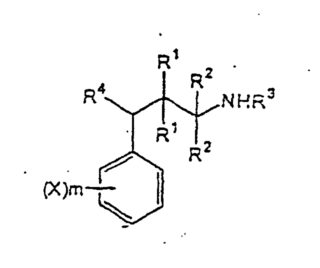 dans laquelle :
(X)m est choisi dans le groupe consistant en méta-fluoro, méta-chloro, ortho-O-C₁-C₄ alkyle, ortho-méthyle, ortho-fluoro, ortho-chloro, méta-O-C₁-C₄ alkyle, métaméthyle, ortho-OH et méta-OH ;
R₁ est H;
R₂ est H;
R₃ est choisi dans le groupe consistant en méthyle et éthyle ;
R₄ est phénoxy éventuellement substitué par -F, -Cl, -Br, -I, -CF₃, alkyle, -OH,-OCF₃, -O-alkyle et -O-acyle ;
et ses sels et complexes pharmaceutiquement acceptables pour la préparation d'une composition pharmaceutique pour le traitement d'une maladie ou d'un désordre neurologique.

17. Utilisation selon l'une quelconque des revendications 1 à 6, dans laquelle la maladie ou le désordre comprend un accident vasculaire cérébral, un traumatisme crânien, une lésion de la moelle épinière, une ischémie de la moelle épinière, un dommage neuronal induit par une ischémie ou une hypoxie, l'épilepsie, la douleur, l'anxiété, des déficits neuropsychiatriques ou cognitifs dus à une ischémie ou une hypoxie tels que ceux survenant fréquemment en tant que conséquence d'une opération chirurgicale cardiaque sous dérivation cardio-pulmonaire, la maladie d'Alzheimer, la maladie de Huntington, la maladie de Parkinson, ou la sclérose amyotrophique latérale.

18. Utilisation selon la revendication 17, dans laquelle l'accident vasculaire cérébral est de nature ischémique global.

19. Utilisation selon la revendication 17, dans laquelle l'accident vasculaire cérébral est de nature ischémique focal.

20. Utilisation selon la revendication 17, dans laquelle l'accident vasculaire cérébral est de nature hémmoragique.

21. Utilisation selon la revendication 17, dans laquelle la maladie ou le désordre neurologique comprend la maladie de Parkinson.

22. Composé choisi dans le groupe consistant en : 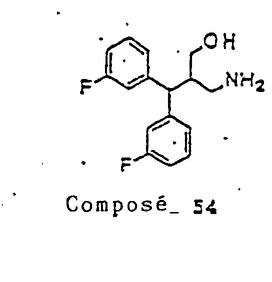 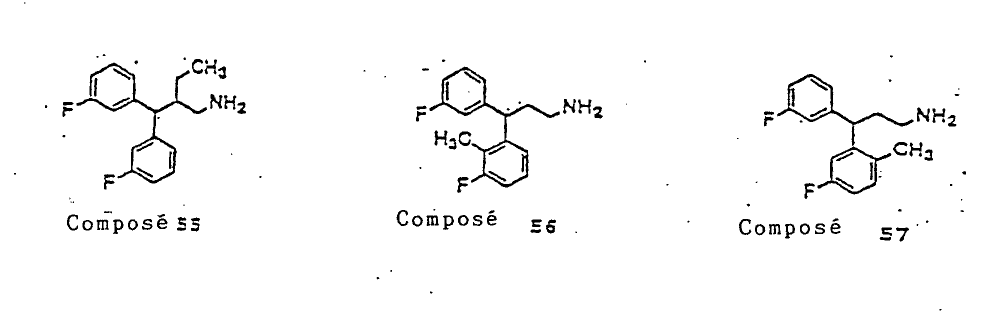 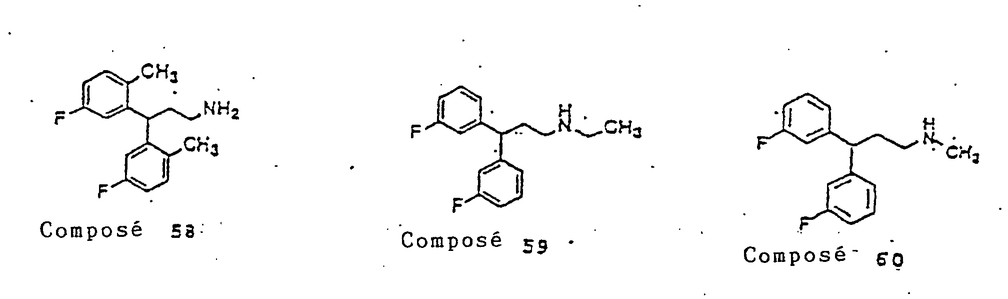 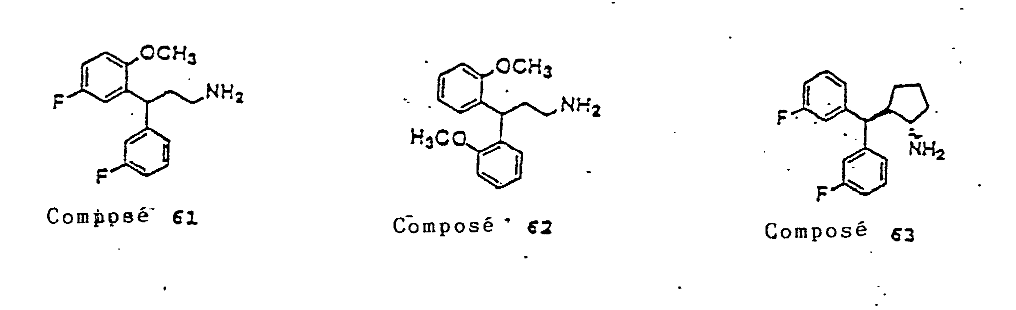 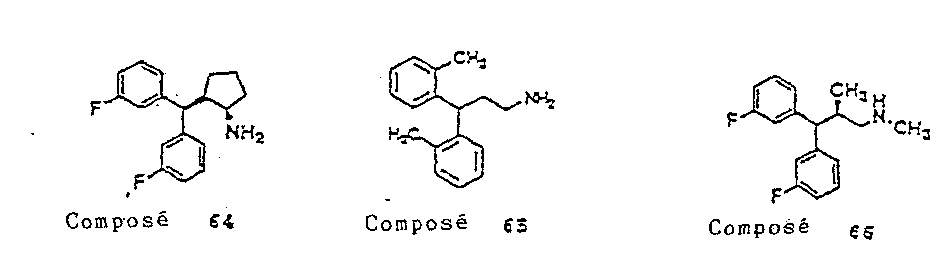 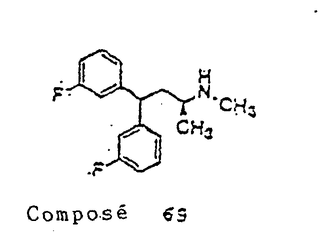 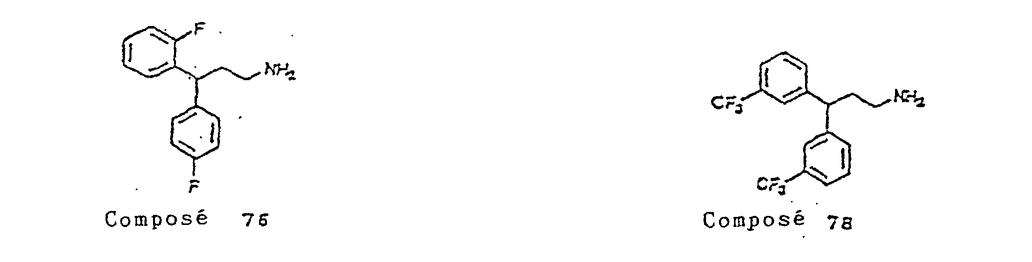 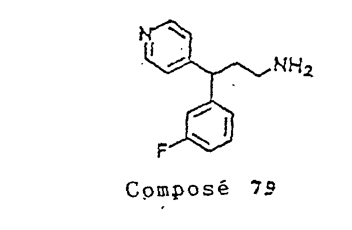 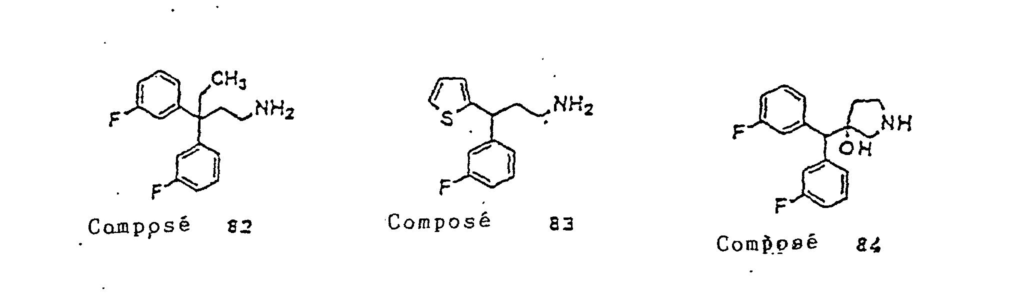 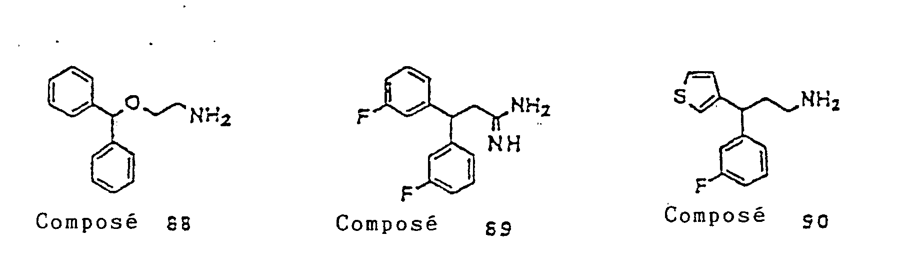 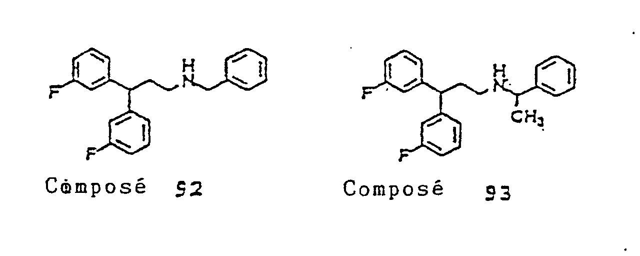 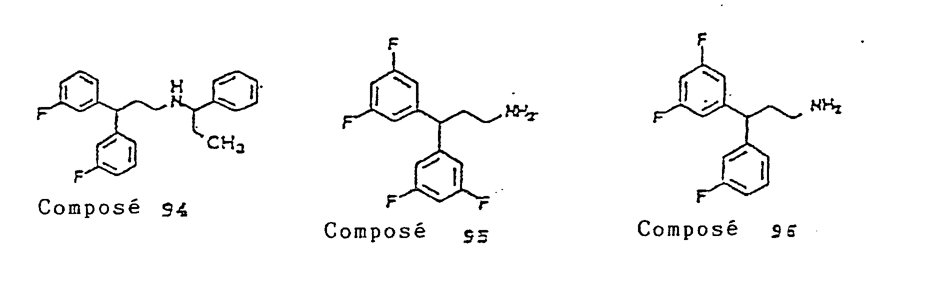 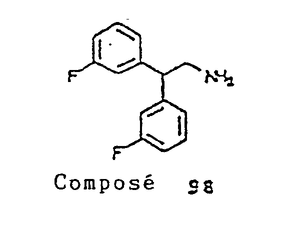 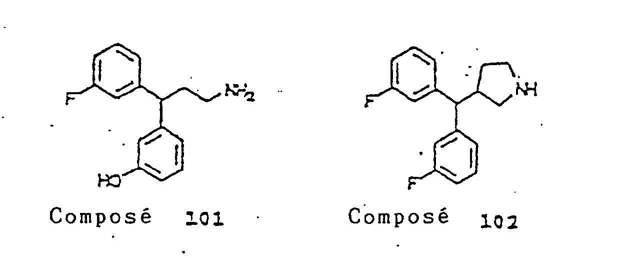 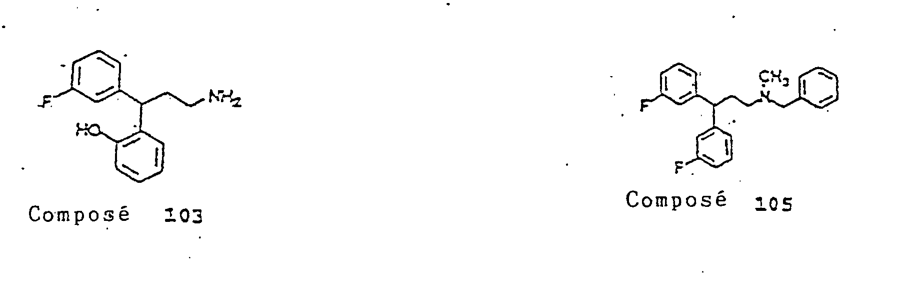 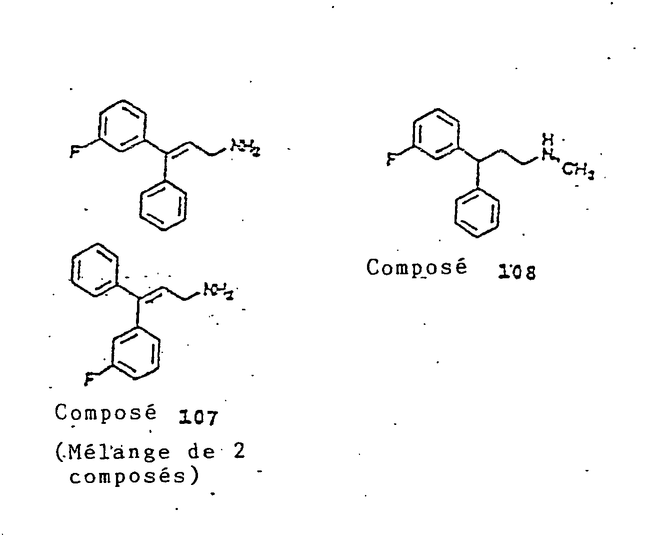 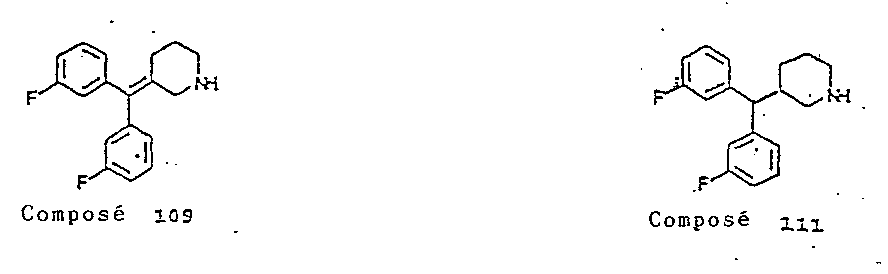 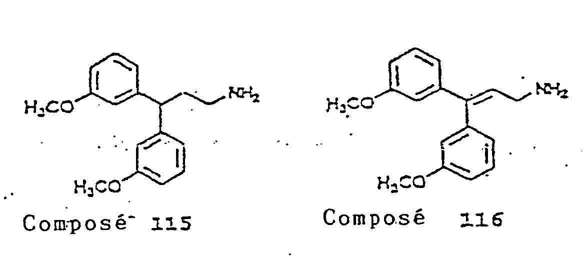 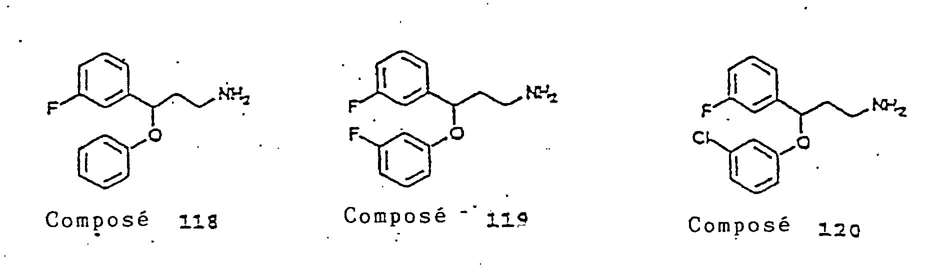 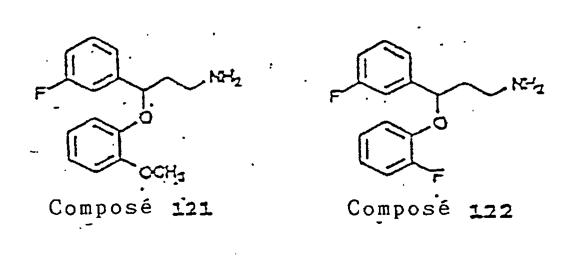 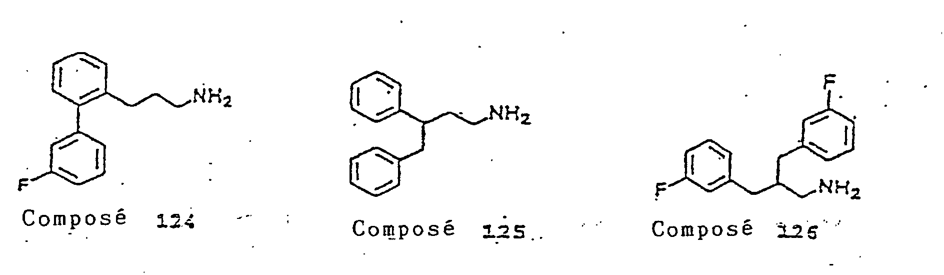 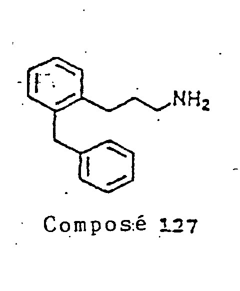 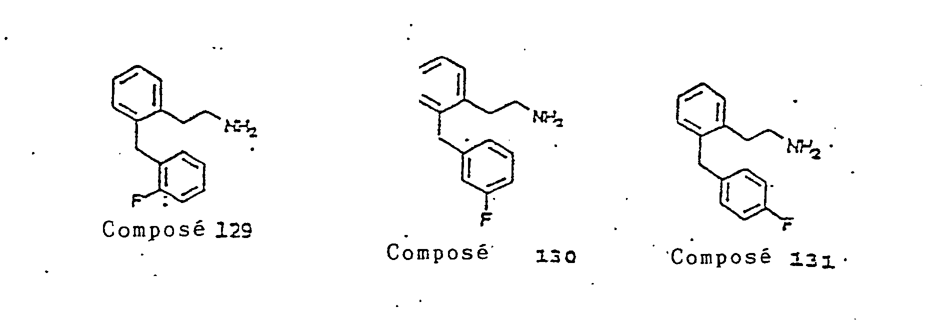 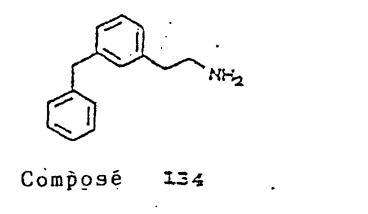 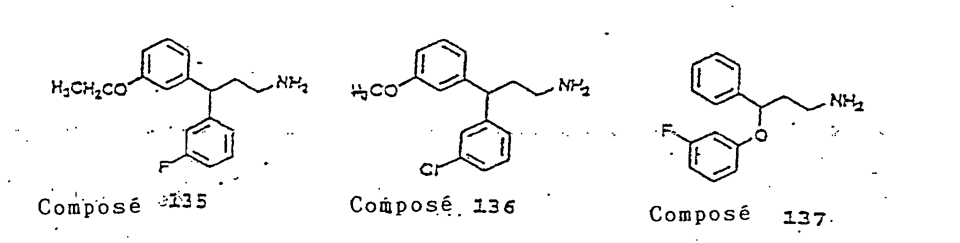 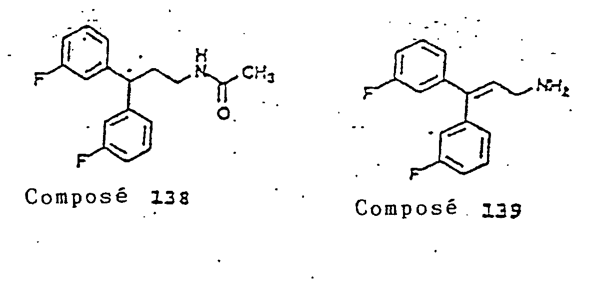 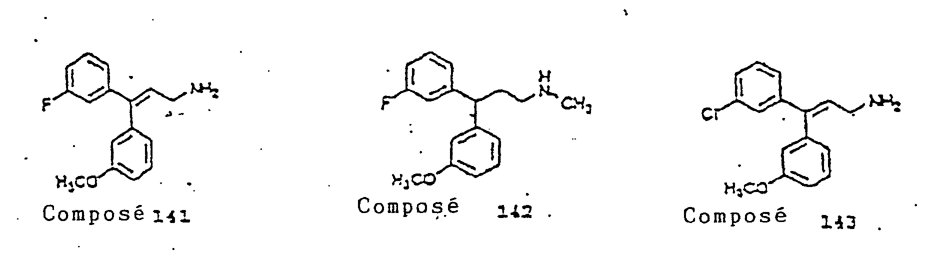 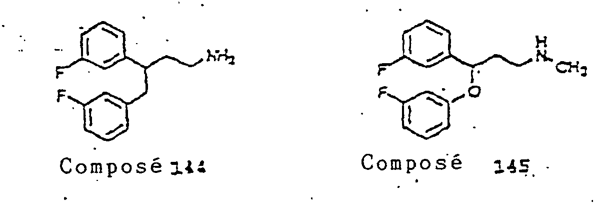 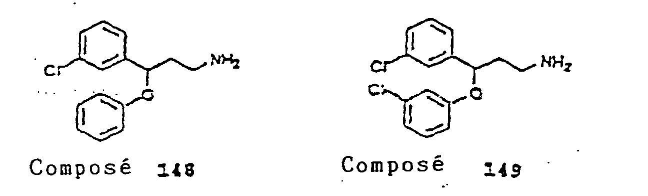 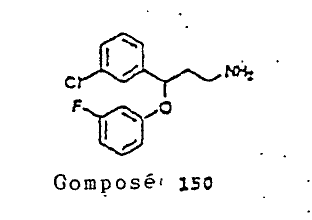 et leurs sels pharmaceutiquement acceptables.

23. Composé selon la revendication 22 choisi dans le groupe consistant en les composés 54-66, 69, 76, 82, 83, 88-90, 92-96, 101, 102, 103, 105, 108, 109, 111, 115, 118-122, 125-127, 129-131, 135-139, 142, 144, 145, 148-150, ou leurs sels pharmaceutiquement acceptables.

24. Composé selon la revendication 22 choisi dans le groupe consistant en les composés 54-66, 69, 82, 83, 89, 90, 93-96, 103, 111, 118-120, 122, 126, 135-138, 142, 144, 145, 148-150, ou ses sels pharmaceutiquement acceptables.

25. Composé selon la revendication 22 choisi dans le groupe consistant en les composés 60-66, 69, 70, 103, 111, 118-120, 122, 136-138, 142, 144, 145, 148-150, ou ses sels pharmaceutiquement acceptables.

26. Composé selon la revendication 22 choisi dans le groupe consistant en les composés, 118-122, 137, 145, 148-150, ou ses sels pharmaceutiquement acceptables.

27. Composé selon la revendication 22 choisi dans le groupe consistant en les composés 118-122, 148-150, ou ses sels pharmaceutiquement acceptables.

28. Composé selon la revendication 22 choisi dans le groupe consistant en les composés 63 et 64 ou ses sels pharmaceutiquement acceptables.

29. Composé selon la revendication 22 choisi parmi le composé 119 ou ses sels pharmaceutiquement acceptables.

30. Composé selon la revendication 22 choisi parmi le composé 144 ou ses sels pharmaceutiquement acceptables.

31. Composé 60 ou ses sels pharmaceutiquement acceptables.

32. Composé de formule : 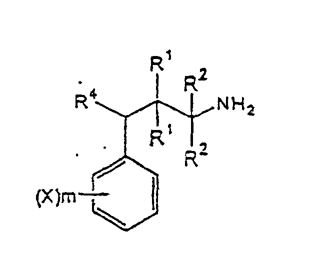 dans laquelle :
X est choisi indépendamment dans le groupe consistant en -Br, -Cl, -F, -I, -CF₃, alkyle, -OH, -OCF₃, -O-alkyle et -O-acyle ;
R₁ est choisi indépendamment dans le groupe consistant en -H, C₁-C₄ alkyle, et-O-acyle ;
R₂ est choisi indépendamment dans le groupe consistant en -H, alkyle et hydroxyalkyle, ou les deux R₂ ensemble sont imino ;
R₄ est phénoxy éventuellement substitué par -F, -Cl, -Br, -I, -CF₃, alkyle, -OH,-OCF₃, -O-alkyle et -O-acyle ; et
m est indépendamment un nombre entier de 1 à 5 ; et ses sels et complexes pharmaceutiquement acceptables

33. Composé de formule : 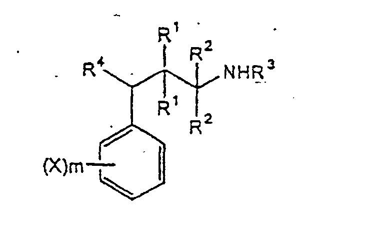 dans laquelle :
X est choisi indépendamment dans le groupe consistant en -F, -Cl, -Br,-I, -CF₃, alkyle, -OH, -OCF₃, -O-alkyle et -O-acyle ;
R₁ est choisi indépendamment dans le groupe consistant en -H, C₁-C₄ alkyle, et-O-acyle ;
R₂ est choisi indépendamment dans le groupe consistant en -H, C₁-C₄ alkyle et hydroxyalkyle, ou les deux R₂ ensemble sont imino ;
R₃ est choisi dans le groupe consistant en méthyle et éthyle ;
R₄ est phénoxy éventuellement substitué par -F, -Cl, -Br, -I, -CF₃, alkyle, -OH,-OCF₃, -O-alkyle ou-O-acyle ; et
m est indépendamment un nombre entier de 1 à 5 ; et ses sels et complexes pharmaceutiquement acceptables sous réserve que le dit composé n'est pas N-méthyl 3-(m-trifluorométhylphénoxy)-3-(4-fluorophényl)propylamine.

34. Composé de formule : 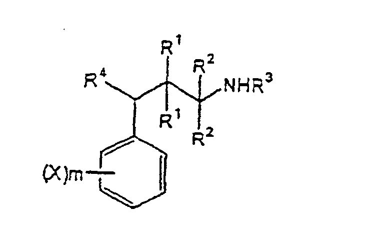 dans laquelle :
(X)m est choisi dans le groupe consistant en méta-fluoro, méta-chloro, ortho-O-C₁-C₄ alkyle, ortho-méthyle, ortho-fluoro, ortho-chloro, méta-O- C₁-C₄ alkyle, métaméthyle, ortho-OH et méta-OH ;
R₁ est H;
R₂ est H;
R₃ est choisi dans le groupe consistant en méthyle et éthyle ;
R₄ est phénoxy éventuellement substitué par -F, -Cl, -Br, -I, -CF₃, alkyle, -OH,-OCF₃, -O-alkyle ou-O-acyle ;
et ses sels et complexes pharmaceutiquement acceptables.

35. Composition pharmaceutique comprenant un composé choisi dans le groupe consistant en 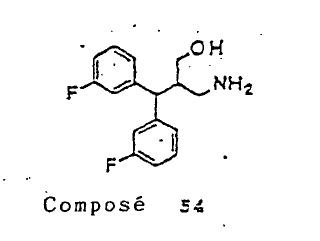 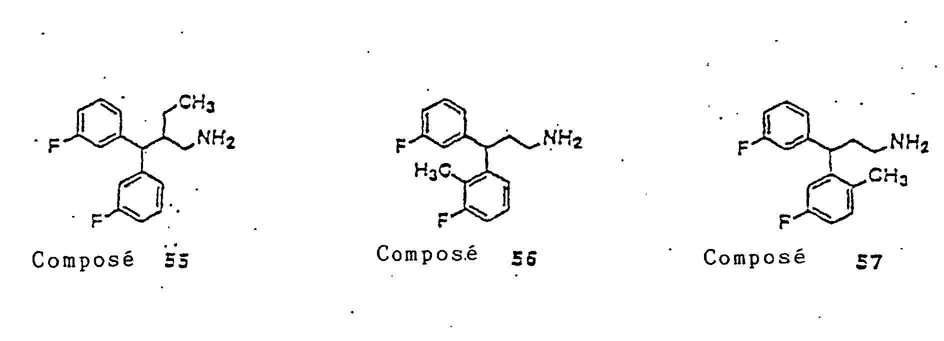 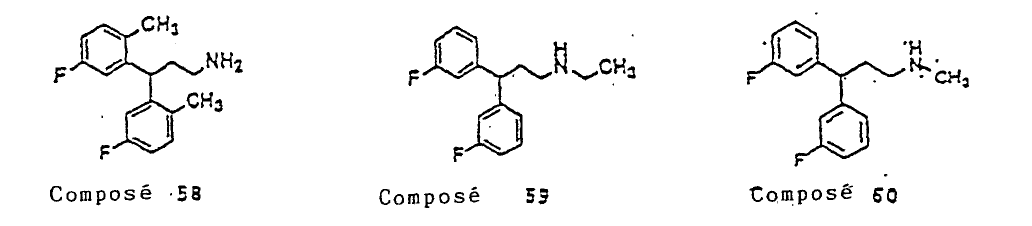 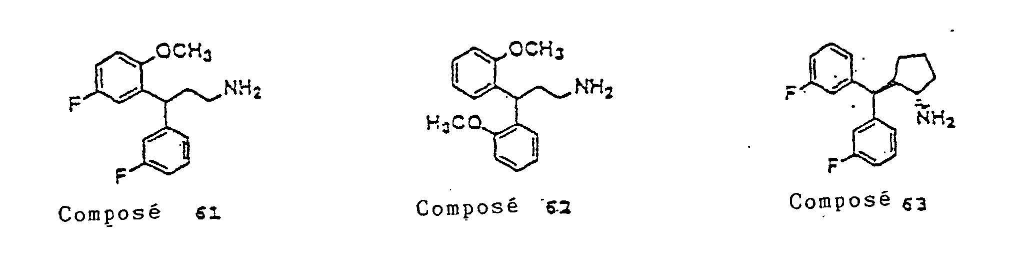 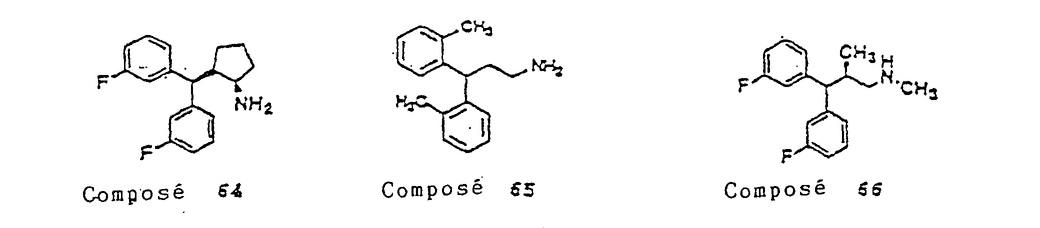 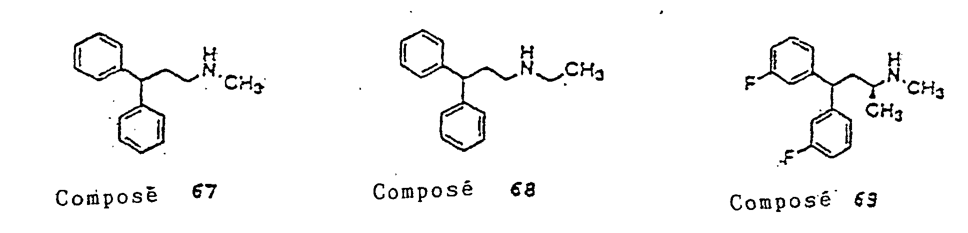 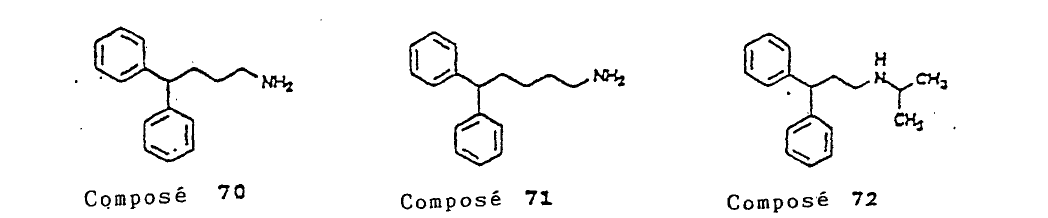 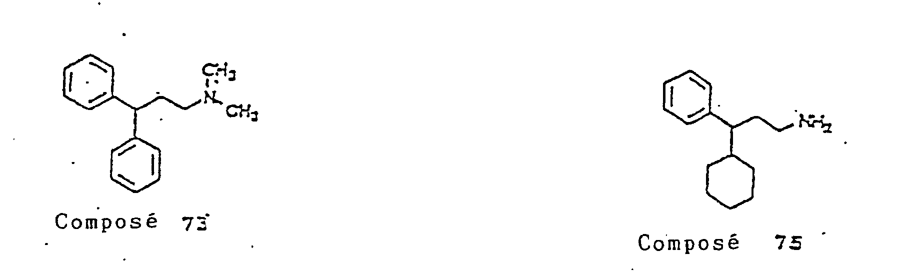 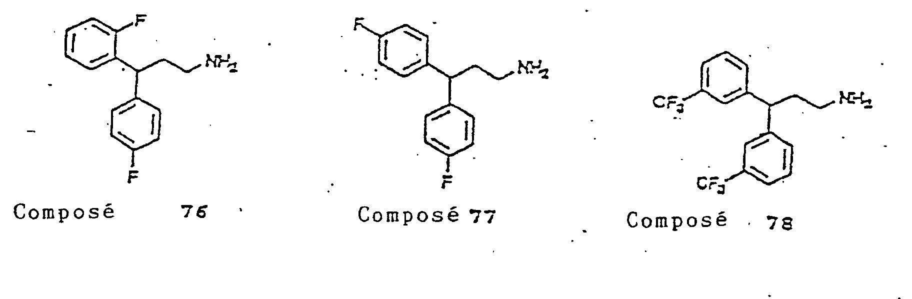 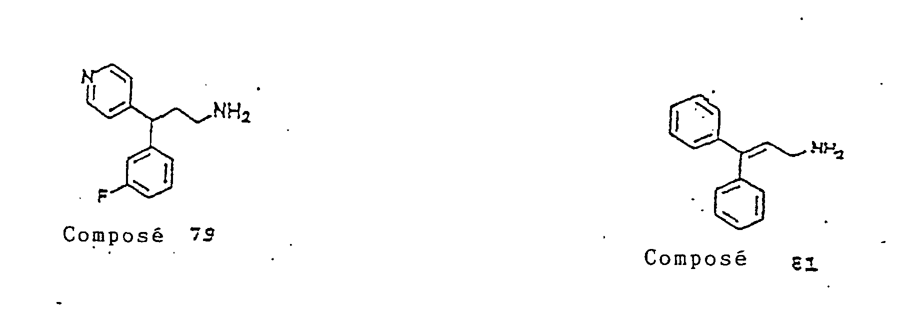 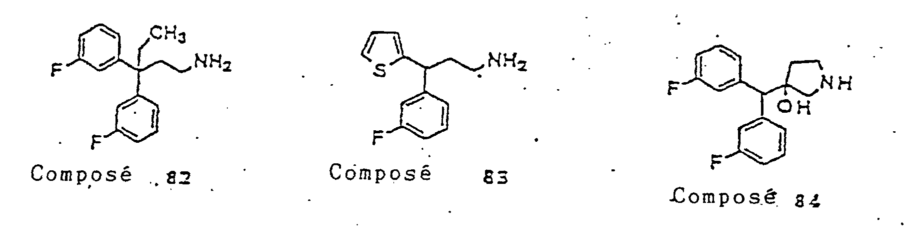 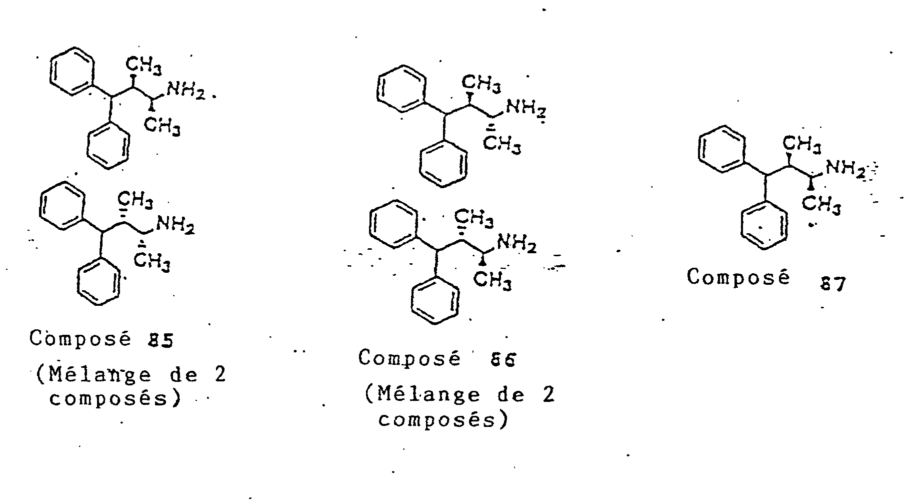 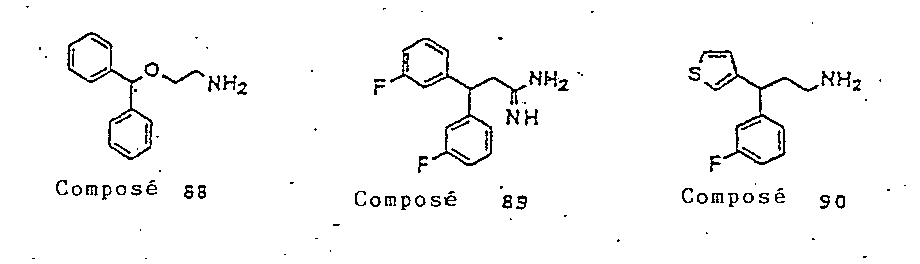 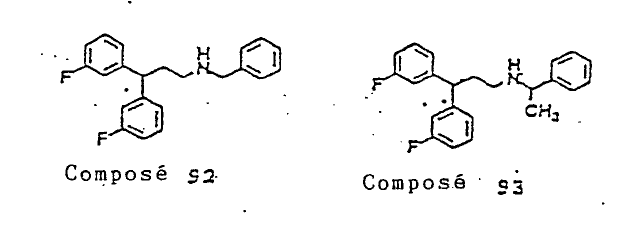 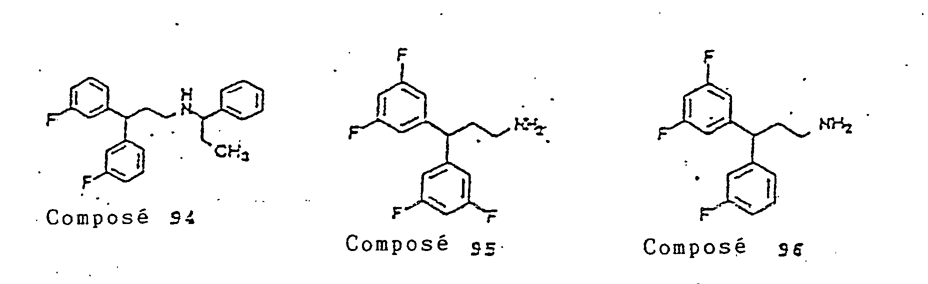 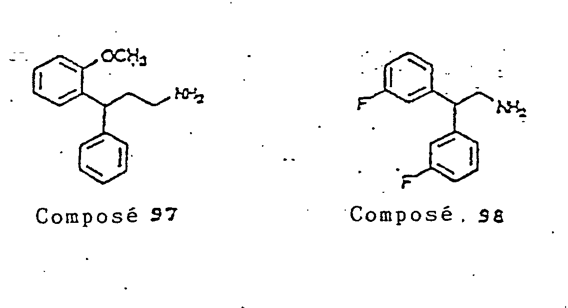 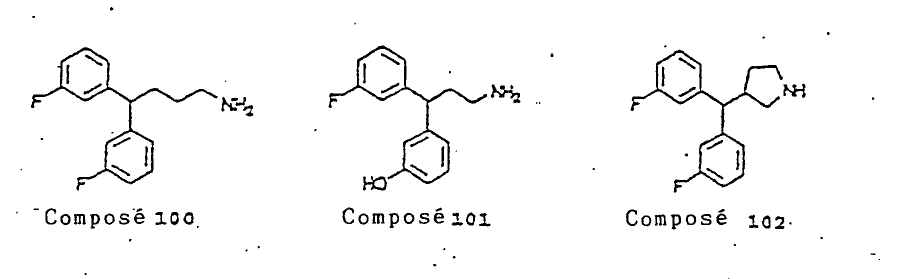 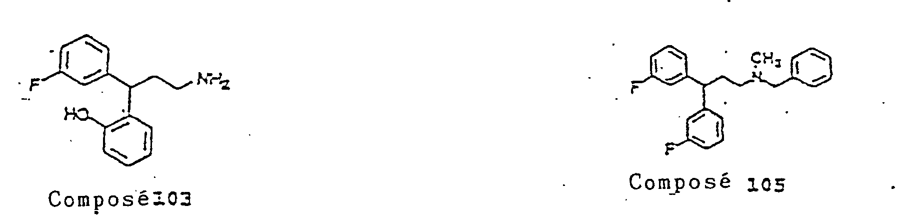 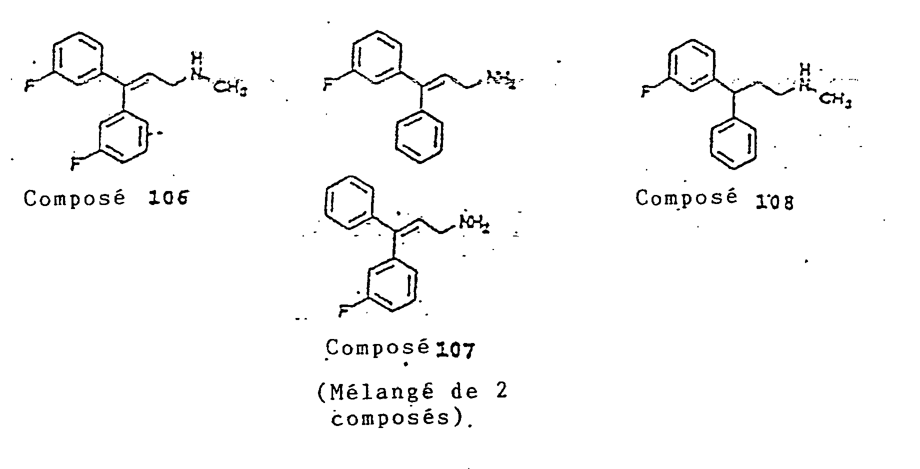 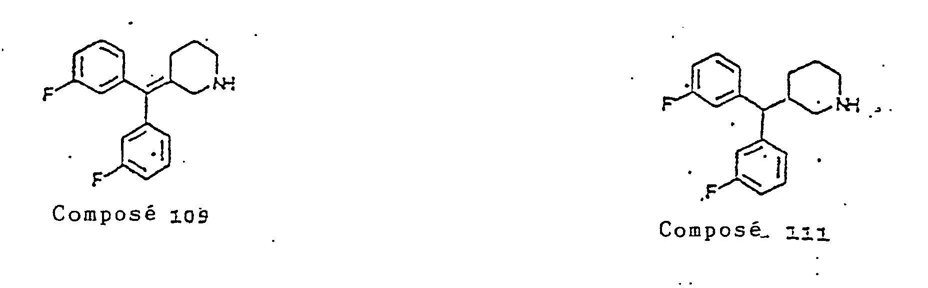 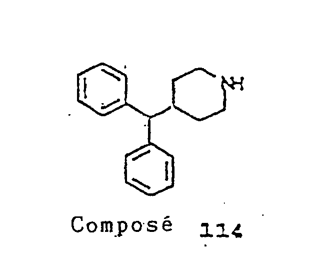 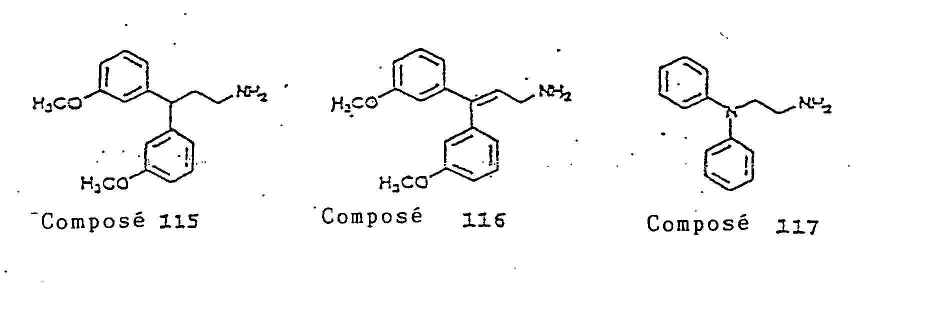 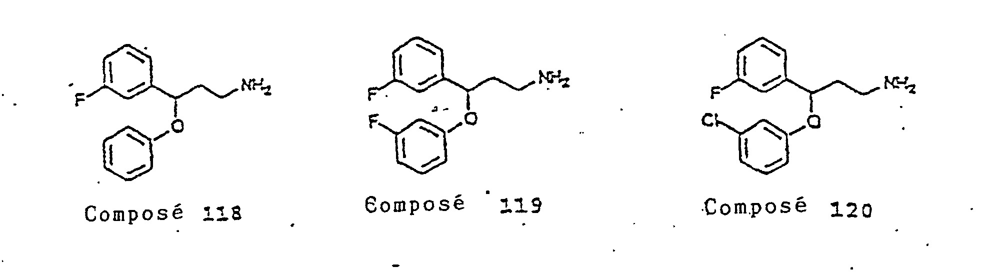 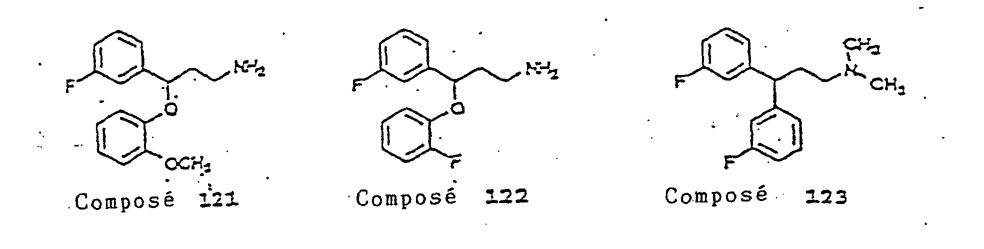 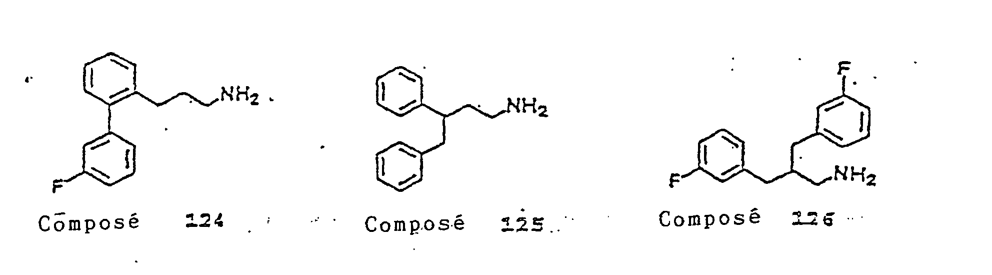 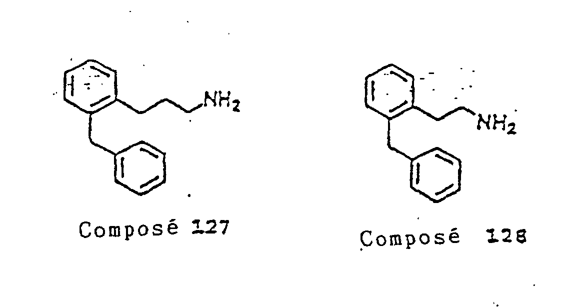 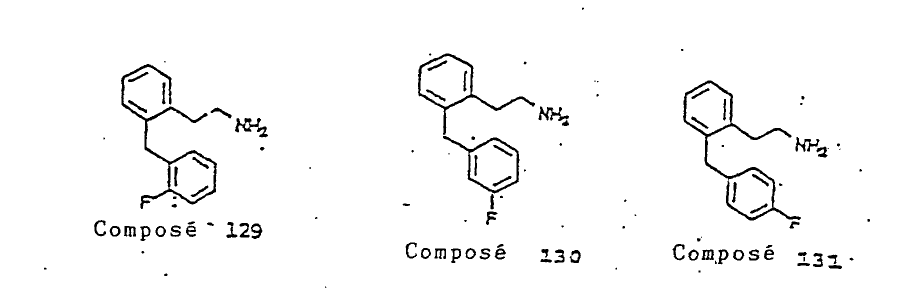 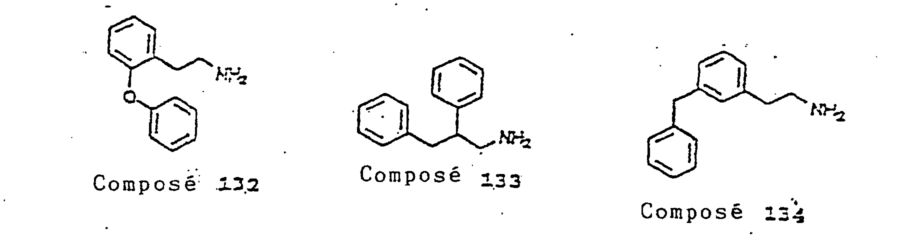 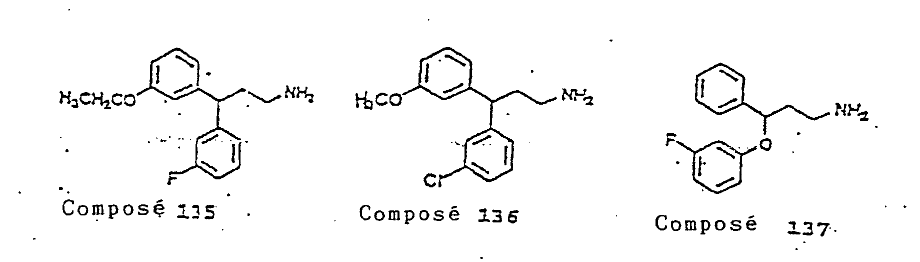 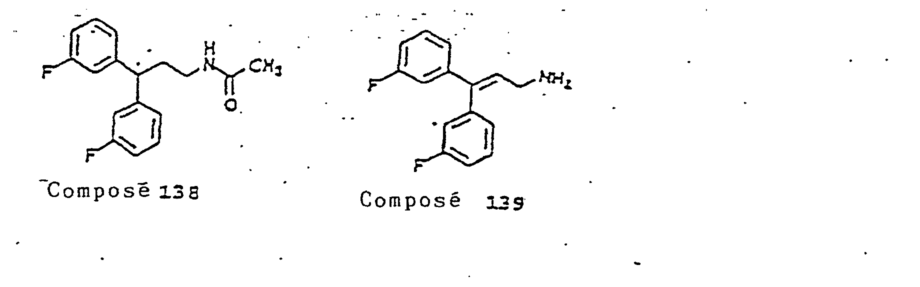 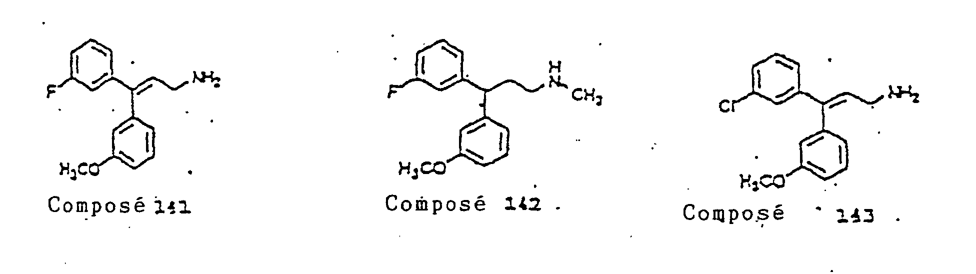 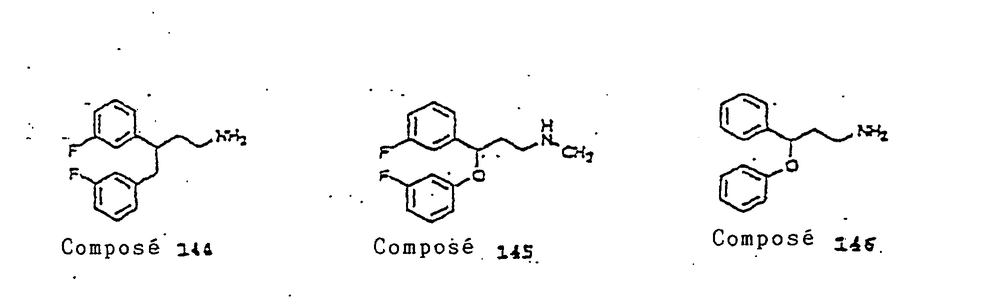 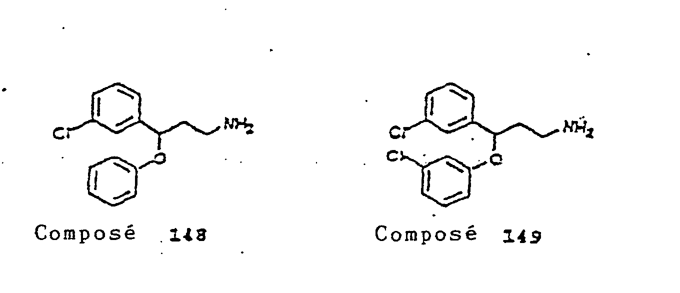 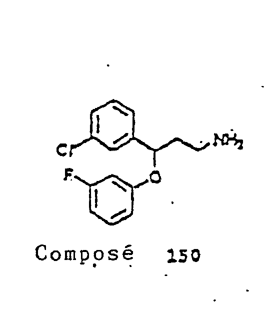 et leurs sels pharmaceutiquement acceptables dans un véhicule pharmaceutiquement acceptable.

36. Composition pharmaceutique selon la revendication 35 comprenant un composé choisi dans le groupe consistant en les composés 54-71, 73, 76-79, 81-84, 88-90, 92-98, 101-103, 105, 107-109, 111, 115, 117-123, 125-127, 129-136, 138, 139, 142, 144-146, 148-150, et leurs sels pharmaceutiquement acceptables, dans un véhicule pharmaceutiquement acceptable.

37. Composition pharmaceutique selon la revendication 35 comprenant un composé choisi dans le groupe consistant en les composés 54-66, 69, 70, 75, 76, 81-83, 85-90, 92-97, 100-103, 105, 106, 108, 109, 111, 115, 118-122, 125-133, 135-139, 142, 144-146, 148-150, et leurs sels pharmaceutiquement acceptables, dans un véhicule pharmaceutiquement acceptable.

38. Composition pharmaceutique selon la revendication 35 comprenant un composé choisi dans le groupe consistant en les composés 54-66, 69, 70, 76, 81-83, 88-90, 92-97, 101-103, 105, 106, 108, 109, 111, 115, 118-122, 125-127, 129-133, 135, 136, 138, 139, 142, 144-146, 148-150, et leurs sels pharmaceutiquement acceptables, dans un véhicule pharmaceutiquement acceptable.

39. Composition pharmaceutique selon la revendication 35 comprenant un composé choisi dans le groupe consistant en les composés 54-66, 69, 82, 83, 89, 90, 93-97; 103, 111, 118-120, 122, 126, 135-138, 142, 144, 145, 148-150, et leurs sels pharmaceutiquement acceptables, dans un véhicule pharmaceutiquement acceptable.

40. Composition pharmaceutique selon la revendication -35 comprenant un composé choisi dans le groupe consistant en les composés 54-66, 69, 82, 83, 89, 90, 93-97, 103, 111, 118-120, 122, 126, 135, 136, 138, 142, 144, 145, 148-150, et leurs sels pharmaceutiquement acceptables, dans un véhicule pharmaceutiquement acceptable.

41. Composition pharmaceutique selon la revendication 35 comprenant un composé choisi dans le groupe consistant en les composés 60, 66, 69, 103, 111, 118-120, 122, 136-138, 142, 144, 145, 148-150, et leurs sels pharmaceutiquement acceptables, dans un véhicule pharmaceutiquement acceptable.

42. Composition pharmaceutique selon la revendication 35 comprenant un composé choisi dans le groupe consistant en les composés 118-122, 137, 145, 148-150, et leurs sels pharmaceutiquement acceptables, dans un véhicule pharmaceutiquement acceptable.

43. Composition pharmaceutique selon la revendication 35 comprenant un composé choisi dans le groupe consistant en les composés 118-122, 148-150, et ses sels pharmaceutiquement acceptables, dans un véhicule pharmaceutiquement acceptable.

44. Composition pharmaceutique selon la revendication 35 comprenant un composé choisi dans le groupe consistant en les composés 63 et 64 et ses sels pharmaceutiquement acceptables, dans un véhicule pharmaceutiquement acceptable.

45. Composition pharmaceutique selon la revendication 35 comprenant un composé choisi parmi le composé 119 et ses sels pharmaceutiquement acceptables, dans un véhicule pharmaceutiquement acceptable.

46. Composition pharmaceutique selon la revendication 35 comprenant un composé choisi parmi le composé 144 et ses sels pharmaceutiquement acceptables, dans un véhicule pharmaceutiquement acceptable.

47. Composition pharmaceutique selon la revendication 35 comprenant un composé choisi parmi le composé 60 et ses sels pharmaceutiquement acceptables, dans un véhicule pharmaceutiquement acceptable.

48. Composition pharmaceutique comprenant un composé selon la revendication 32, dans un véhicule pharmaceutiquement acceptable.

49. Composition pharmaceutique comprenant un composé selon la revendication 33, dans un véhicule pharmaceutiquement acceptable.

50. Composition pharmaceutique comprenant un composé selon la revendication 34, dans un véhicule pharmaceutiquement acceptable.

51. Composition pharmaceutique selon l'une quelconque des revendication 35-50, adaptée au traitement d'une maladie ou d'un désordre neurologique.

52. Composition pharmaceutique selon la revendication 51, dans laquelle la maladie ou le désordre est choisi dans le groupe consistant en un accident vasculaire cérébral, un traumatisme crânien, une lésion de la moelle épinière, l'épilepsie, l'anxiété, la maladie d'Alzheimer, la maladie de Huntington, la maladie de Parkinson, ou la sclérose amyotrophique latérale.

53. Composition pharmaceutique selon la revendication 51, dans laquelle la composition pharmaceutique a une activité neuro-protectrice.

54. Composition pharmaceutique selon la revendication 52, dans laquelle l'accident vasculaire cérébral est de nature ischémique globale.

55. Composition pharmaceutique selon la revendication 52, dans laquelle l'accident vasculaire cérébral est de nature ischémique focale.

56. Composition pharmaceutique selon la revendication 52, dans laquelle l'accident vasculaire cérébral est de nature hémorragique.

57. Composition pharmaceutique selon la revendication 52, dans laquelle.la maladie ou le désordre neurologique est la maladie de Parkinson.
